(19) Europäisches Patentamt
European Patent Office
Office européen des brevets

(11) Publication number: **0 155 973 B1**

# (12) EUROPEAN PATENT SPECIFICATION

(45) Date of publication of patent specification: **14.12.88**

(21) Application number: **84101951.6**

(22) Date of filing: **24.02.84**

(51) Int. Cl.$^4$: **C 07 C 47/198,** C 07 C 47/277, C 07 C 45/50, C 07 C 43/196, C 07 C 43/23, C 07 C 43/178, C 07 C 41/06, A 61 K 7/46

## (54) Hydrocarbyloxy alkanals, organoleptic uses thereof and processors for preparing same.

(43) Date of publication of application:
**02.10.85 Bulletin 85/40**

(45) Publication of the grant of the patent:
**14.12.88 Bulletin 88/50**

(84) Designated Contracting States:
**DE GB**

(58) References cited:
**EP-A-0 018 161**
**US-A-3 354 225**
**US-A-3 847 994**
**US-A-4 029 710**
**US-A-4 391 744**

(73) Proprietor: **INTERNATIONAL FLAVORS & FRAGRANCES INC.**
**521 West 57th Street**
**New York New York 10019 (US)**

(72) Inventor: **Boden, Richard M.**
**1760 Raleigh Court East**
**Ocean New Jersey (US)**
Inventor: **Scheiber, William L.**
**33 Cambridge Drive**
**Jackson New Jersey (US)**
Inventor: **Fujioka, Futoshi**
**1309 Stewart Street**
**Wanamassa New Jersey (US)**
Inventor: **Chant, Patrick**
**1206 Harrison Avenue**
**Leadville Colorado 80461 (US)**
Inventor: **Dekker, Lambert**
**384 West Shore Drive**
**Wyckoff New Jersey (US)**

(74) Representative: **Baillie, Iain Cameron et al**
**c/o Ladas & Parry Isartorplatz 5**
**D-8000 München 2 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 155 973 B1

**Description**

The present invention provides ether carboxaldehydes and ether carbinols.

U.S. Patent 4,029,710 describes production of 4-alkoxy-N-butyraldehydes by hydrogen fluoride catalyzed reaction of allyl alcohol formaldehyde and an alcohol. No particular use of the compounds is suggested.

U.S. Patent 3,847,994 certain 2,2-dimethyl-5-(xylyloxy)-valeraldehydes which are of use for the reduction of serum triglyceride levels. European Patent Publication 18161 describes certain aldehyde ethers that are useful chemical intermediates in the production of, for example, butaine 1,4-diol, butyrolactone and tetrahydrofuran.

U.S. Patent 4,391,774 describes norbornyl oxyacetaldehyde and norbornyl oxyethanols, the aldehyde being taught to have organoleptic uses in the field of perfumery, colognes and perfumed articles. These compounds differ from the compounds of the present invention in that the oxyacetaldehyde or oxyethanol side chain is bound to the 3-position rather than the 2-position of the norbornyl group and in that the side chains of the compounds of the present invention comprise an additional carbon atom. The compounds of the present invention have surprisingly improved perfumery properties as compared to the compounds of U.S. Patent 4,391,744.

U.S. Patent 3,354,225 describes certain camphane derivatives that are similar to the norbornyl compounds of the present invention, but which contain secondary propyloxy or β-hydroxyethoxy side chains. These compounds are taught to have properties rendering them useful as replacements for cedar oil.

The present invention provides ether carboxaldehydes having the generic formula

ZCHO

wherein Q is

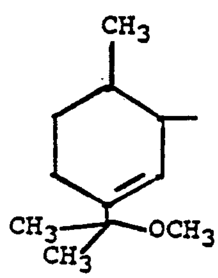

or

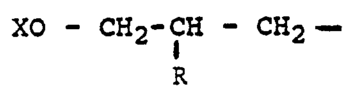

wherein X is selected from

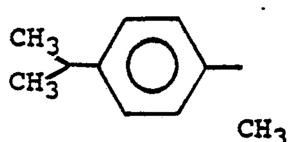

,

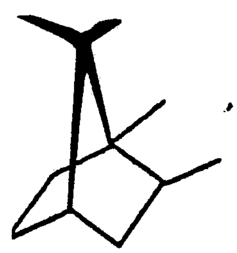

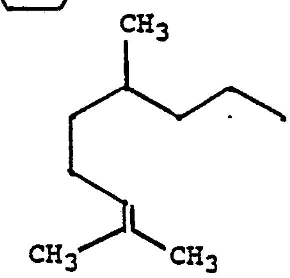

,

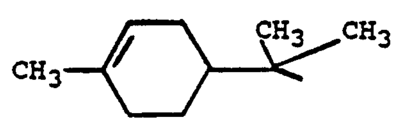

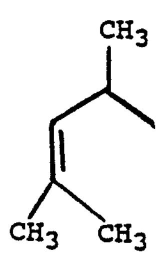

or phenyl

and R is hydrogen or methyl.

The present invention in addition, provides ether carbinols defined according to the generic structure:

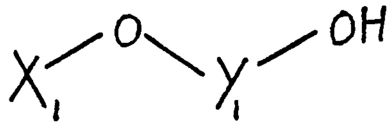

wherein $X_1$ represents a moiety selected from the group consisting of:

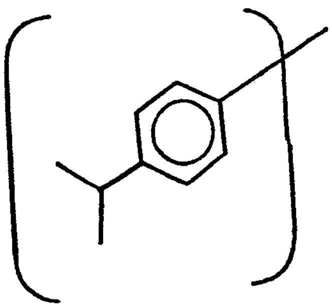
;
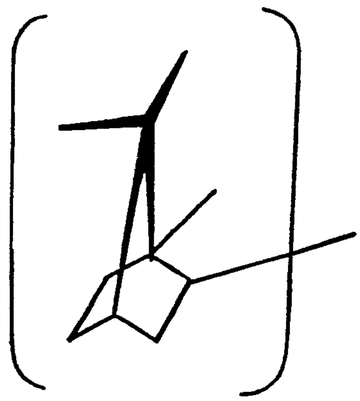

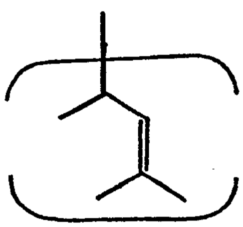
; and
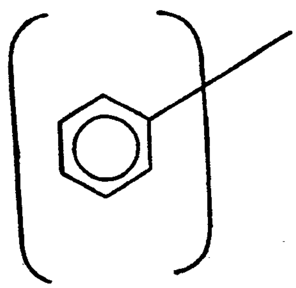

and wherein $Y_1$ represents $C_4$ or $C_5$ alkylene; $C_4$ or $C_5$ alkenylene or $C_4$ or $C_5$ alkynylene.

The present invention also provides a process for preparing ether carboxaldehydes by means of carrying out an oxo reaction on allyl ethers using carbon monoxide and hydrogen, the structure of the allyl ethers being:

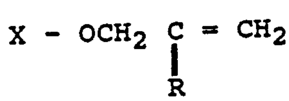
or
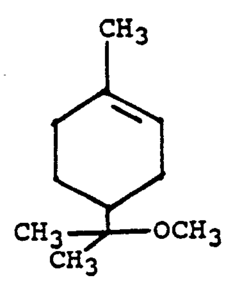

and the reaction being:

alkyl ether

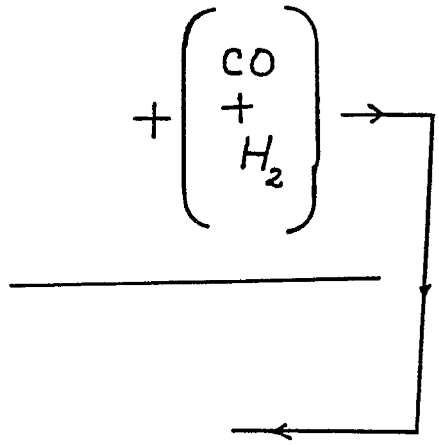

QCHO wherein Q is as defined above.

The present invention also provides products produced according to such process. The resulting compounds, ether carboxaldehydes of our invention produced according to the process of our invention are capable of augmenting or enhancing bitter orange and mango aromas and tastes of foodstuffs, chewing gums, toothpastes, medicinal products and chewing tobaccos.

The present invention also provides processes for preparing ether carbinols by means of carrying out an oxo reaction on an appropriate allyl ether using carbon monoxide and hydrogen and either isolating the resulting carboxaldehyde or further reducing the resulting ether carboxaldehyde to form the appropriate ether carbinol; or reacting camphene with either an alkylene diol, an alkenylene diol or an alkynylene diol in the presence of an appropriate catalyst to form a norbornyloxycarbinol. More specifically, an allyl ether defined according to the formula:

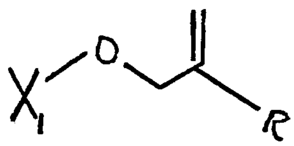

is reacted with a mixture of carbon monoxide and hydrogen using an oxo reaction catalyst whereby a mixture of aldehydes is formed defined according to the structure:

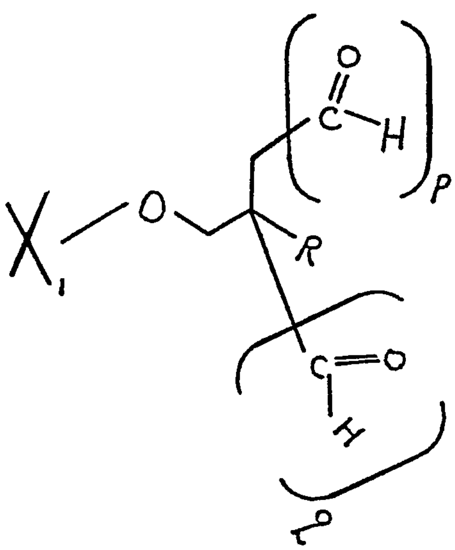

according to the reaction:

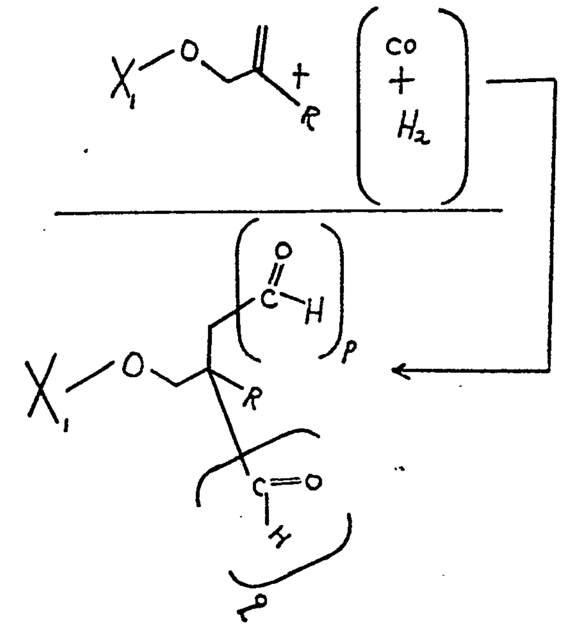

wherein p and q each represent 0 or 1 with the proviso that when p is 0 q is 1 and that when p is 1 q is 0 and wherein R represents hydrogen or methyl. The resulting ether carboxaldehyde mixture defined according to the structure:

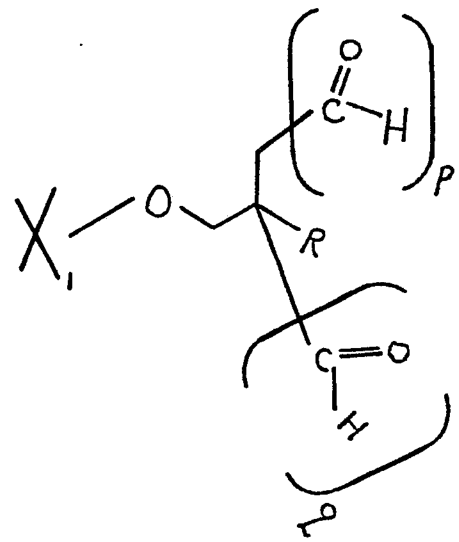

may be used "as-is" for its organoleptic properties or may be further reduced with an appropriate reducing agent to form the ether carbinol defined according to the structure:

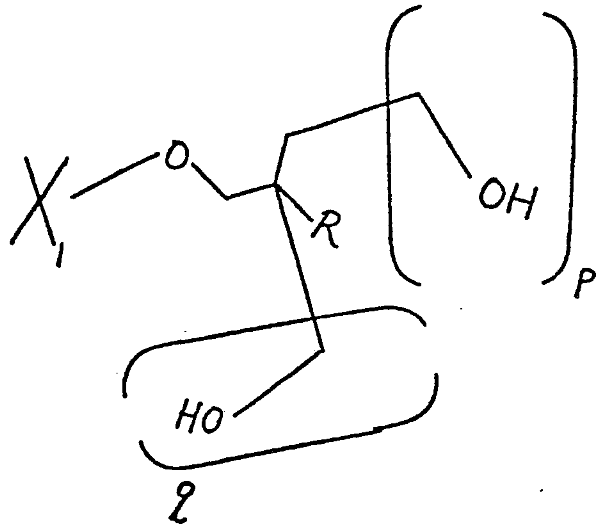

On the other hand, another process of our invention involves the reaction of camphene defined according to the structure:

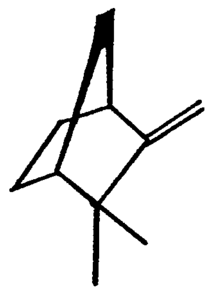

with an alkylene diol, an alkenylene diol or an alkynylene diol defined according to the structure:

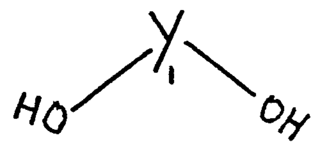

in order to form a norbornyloxycarbinol defined according to the structure:

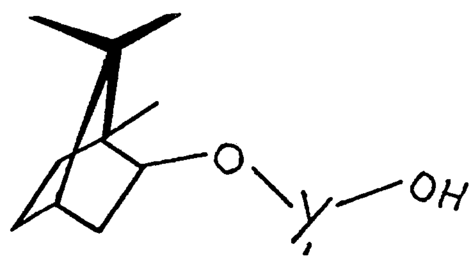

according to the reaction:

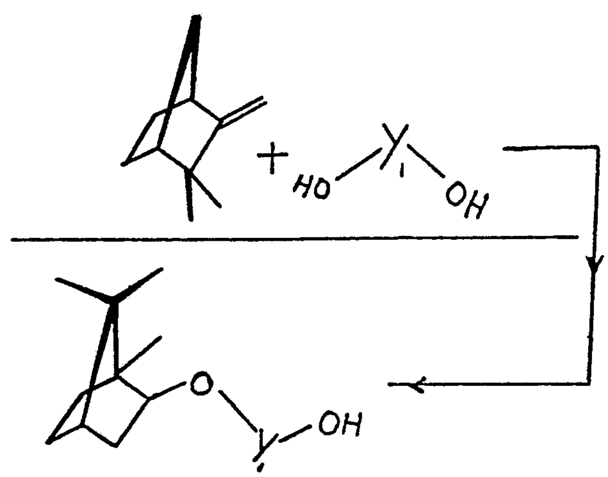

The allyl ethers defined according to the structure:

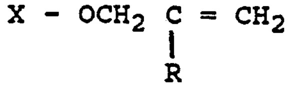

or

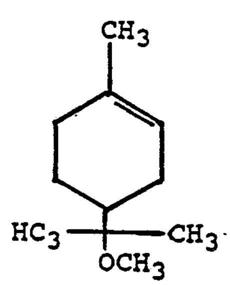

may be prepared by means of any standard ether synthesis, e.g., a "Williamson" synthesis or a synthesis as set forth in United States Letters Patent 4,163,068 issued on July 31, 1979, the specification for which is incorporated by reference herein. Thus, the ethers so useful in our invention may be formed by reacting an alcohol with another alcohol, for example, an allylic alcohol in the presence of an acid catalyst such as para toluene, sulphonic acid at reflux conditions. The reaction mass is refluxed for a period of from two hours up to ten hours, after which a period of time the reaction product is separated from the reaction mass as by distillation.

The ethers so useful in practicing our invention may also be formed by reacting the corresponding alcohol with an appropriate allylic halide or other organic halide as the case may be. This reaction is carried out under the influence of base comprising the step of placing the reactants for the process and the base, respectively, in two immiscible phase; an organic phase and either (i) an aqueous base phase or (ii) a solid base phase with the reactants being located substantially entirely in the first mentioned organic phase and the base being located substantially entirely in the second mentioned phase; and adding to the two phase system a "phase transfer agent" which may be one or more of several organic quaternary ammonium salts.

Specific examples of "phase transfer agents" useful in our invention are as follows:

Tricapryl methyl ammonium chloride;

Cetyl trimethyl ammonium bromide; and

Benzyl trimethyl ammonium hydroxide.

In general, the "phase transfer agents" most preferred have the generic formula:

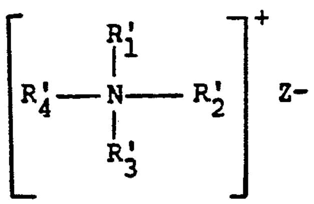

wherein at least one or $R_1'$, $R_2'$, $R_3'$ and $R_4'$ is $C_6$—$C_{14}$ aryl, $C_6$—$C_{10}$ aralkyl, $C_6$—$C_{20}$ alkyl, $C_6$—$C_{14}$ aralkyl and $C_6$—$C_{20}$ alkenyl, and the other of $R_2'$, $R_3'$ and $R_4'$ is alkyl such as methyl, ethyl, n-propyl, i-propyl, 1-butyl, 2-butyl, 1-methyl-2-propyl, 1-pentyl and 1-octyl and Z— is an anion such as chloride, bromide and hydroxide.

This aspect of the process is carried out in an inexpensive solvent which is inert to the reaction system such as toluene, benzene, o-xylene, m-xylene, p-xylene, ethyl benzene, n-hexane, cyclohexane, methylene dichloride and o-dichlorobenzene.

This aspect of the process is carried out at a temperature in the range of from about 10°C up to about 150°C with a temperature range of 30°—120°C being preferred. The reaction time is inversely proportional to the reaction temperature, with lower reaction temperatures giving rise to greater reaction times; and, accordingly, the reaction time ranges from about 30 minutes up to about 10 hours.

In this aspect of the process, the mole ratio of alcohol reactant to organic halide (e.g., allylic halide) is in the range of from 0.5:1.5 up to about 1.5:0.5 with a preferred ratio of alcohol to organic halide (e.g., allylic halide) being from about 1:1 up to about 1:1.2.

The mole ratio of base to alcohol in the reaction mass may be in the range of from about 0.75:1 up to about 1.5:1 with a preferred mole ratio of base:alcohol being from about 1:1 up to about 1.2:1.

The quantity of "phase transfer agent" in the reaction mass based on the amount of alcohol in the reaction mass may vary from 0.5 grams per mole of alcohol up to 25 grams of "phase transfer agent" per mole of alcohol with a preferred concentration of "phase transfer agent" being in the range of from about 2.5 up to about 7.5 grams of "phase transfer agent" per mole of alcohol.

This aspect of the process is preferably carried out at atmospheric pressure since that is the most convenient condition. However, lower or higher pressures can be used without detrimentally affecting the ultimate yield of desired product. The particular base used in the reaction is not critical, but, preferred are sodium hydroxide and potassium hydroxide.

The individual ethers which are reactants for our invention in the oxo process described, infra, can be obtained in pure form or in substantially pure form by conventional purification techniques. Thus, the products can be purified and/or isolated by distillation, extraction, crystallization, preparative chromatographic techniques and the like. It has been found desirable to purify the ethers by fractional distillation in vacuo.

The ethers are then reacted with a mixture of carbon monoxide and hydrogen using a particular range of temperatures and partial pressures of hydrogen and carbon monoxide over one of several alternative "oxo" type reaction catalysts over a period of residence times as previously discussed.

A small number of side products may be formed having the formula:

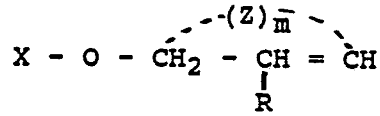

and an even smaller number of alcohols defined according to the structure:

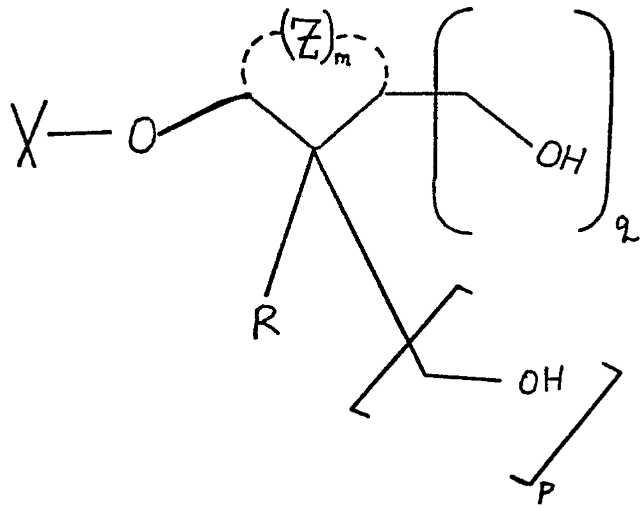

wherein X is as previously defined; wherein Z completes a methyl substituted $C_6$ cycloalkyl ring or represents no moiety; wherein R represents hydrogen or methyl; wherein m represents 0 or 1; wherein n represents 0 or 1; wherein p represents 0 or 1 and wherein q represents 0 or 1 with the provisos that when m is 1, Z completes the alkyl substituted or unsubstituted $C_6$ cycloalkyl ring; that p is 1 when q is 0; and that when p is 0, q is 1. The reaction is carried out at temperatures of between 150°C and 300°C; at pressures of between 20 and 250 atmospheres; with the ratio of partial pressure of carbon monoxide:hydrogen being from 0.1:1 up to 1:0.1. Any oxo type reaction catalyst may be used, but most preferably, the catalyst to yield the best perfume and flavor mixtures are as follows:

Dicobalt octacarbonyl;
Cobalt octanoate;
Palladium chloride;
Rhodium trichloride;
Iron pentacarbonyl;
Nickel tetracarbonyl;
Polymer-bonded rhodium catalyst (e.g., rhodium bonded on a polystyrene substrate);
Tris-triphenyl phosphine rhodium-1-chloride;
Rhodium Aceto acetate dicarbonyl;
Rhodium Aceto acetate/triphenyl phosphine mixture.

The reaction time may vary from about 2 hours up to about 30 hours; and the reaction time is a function of the temperature and pressure of reaction; and the desired ratio of aldehyde:alcohol reaction product. Insofar as the instant invention is concerned, it is most desirable to have as high an aldehyde:alcohol ratio as possible causing the reaction condition to be extreme (high temperature, high pressure and long period of time) will create too high a ratio of alcohol:aldehyde reaction product. Accordingly, it is most desirable to stay within the limits set forth, supra.

At the end of the reaction the reaction product is separated from the catalyst and unreacted materials by standard "work-up" means; e.g., neutralization of catalyst; followed by extraction and fractional distillation; usually an initial fractional distillation by means of distillation through a 3 or 4 plate or stone packed column; followed by a more careful fractionation of the bulked center-cut fractions on, for example, a spinning band column or multiplate (14—50 plate) fractionation column.

Examples of ether reactants which are useful in the practice of our invention and the resulting ether carboxaldehyde and their organoleptic properties are as follows:

TABLE I

| Ether Reactant | Oxo Reaction Product | Perfumery Properties | Food Flavor Properties | Tobacco Flavor Properties |
|---|---|---|---|---|
| 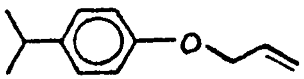 | 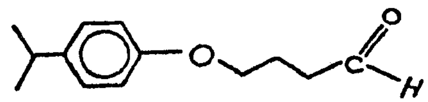 | Diffusive, saw dust-like. | | |
| 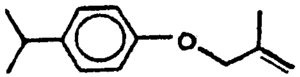 | 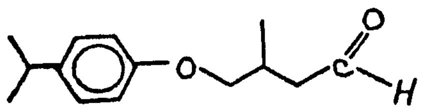 | Green, spicy and calamus-like. | | |
| 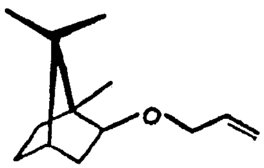 | 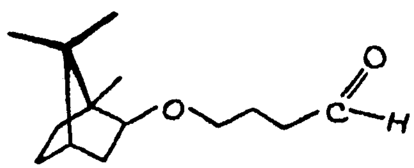 | Natural pine, cypress and fir balsam. | | Adds body, woody, green, herbaceous and slight mouth coating on smoking with woody green and herbaceous aromas prior to smoking. |
| 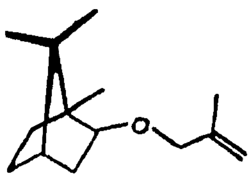 | 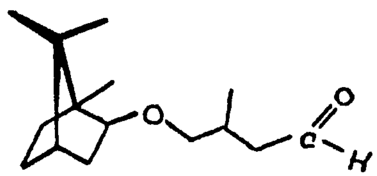 | Patchouli, coriander and woody with a chocolate topnote and dry cedarwood, and patchouli undertones. | | Adds body, woody, herbaceous, spicy and very aromatic on smoking in the main stream and the side stream; with woody, herbaceous and spicy aroma nuances prior to smoking. |
| 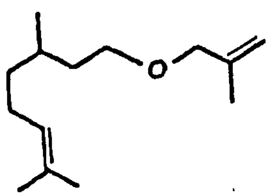 | 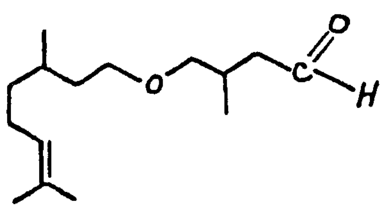 | Floral and ozoney with wood and fruity undertones. | | |

TABLE I (continued)

| Ether Reactant | Oxo Reaction Product | Perfumery Properties | Food Flavor Properties | Tobacco Flavor Properties |
|---|---|---|---|---|
| 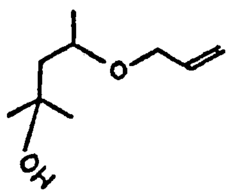 | 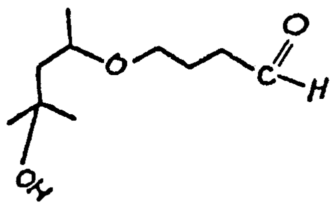 | Spicy (cinnamon), floral (carnation), herbaceous and fruity aroma with caryophyllene—like topnotes. | A bitter orange flavor. | |
| 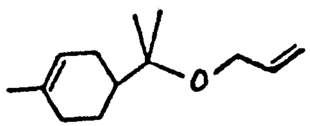 | 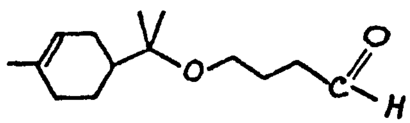 | Citrus (grapefruit)-like aroma. | | |
| 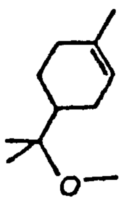 | 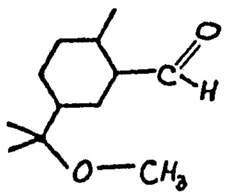 | Green, floral (lilac) aroma. | A mango-like flavor. | |

TABLE I (continued)

| Ether Aldehyde | Perfumery Evaluation | Food Flavor Evaluation | Tobacco Flavor Evaluation |
|---|---|---|---|
| 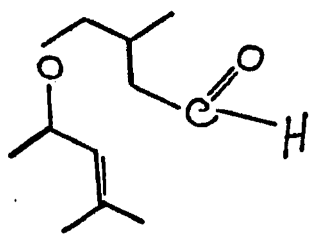 prepared according to Example VIII | A herbaceous, strong green leafy, pepper and spicy aroma profile with herbaceous peppery olibanum and diffusive amber undertones. | A floral flavor profile. | |
| 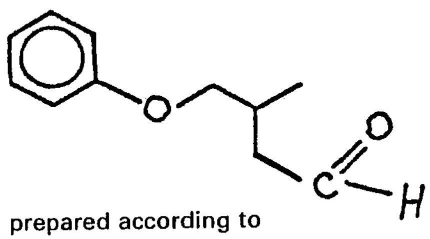 prepared according to Example X | A herbaceous sweet fruity chamomile-like aroma with caramellic undertones. | A fruity flavor. | A fruity aroma and taste profile both prior to and on smoking in the mainstream and the sidestream. |

The ether carbinols of our invention defined according to the structure:

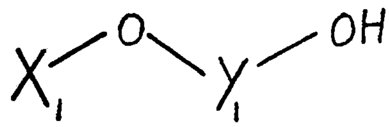

wherein $X_1$ represents a structure selected from the group consisting of:

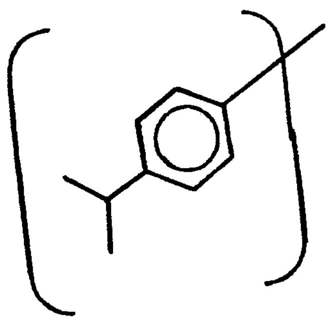

;

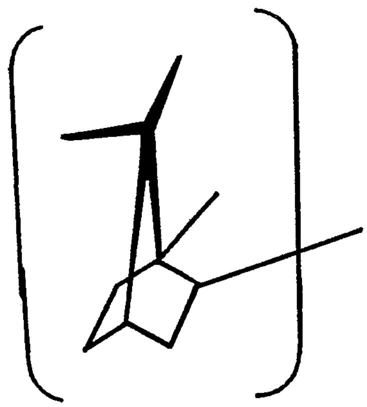

;

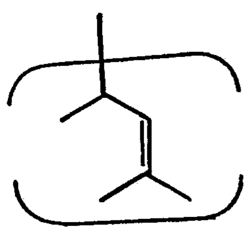

; and

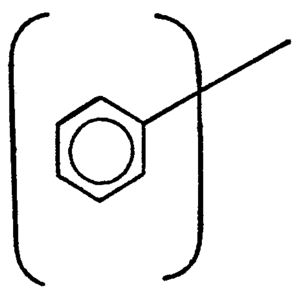

and wherein $Y_1$ represents $C_4$—$C_5$ alkylene, $C_4$—$C_5$ alkenylene and $C_4$—$C_5$ alkynylene may be prepared by means of first reacting allyl ethers defined according to the structure:

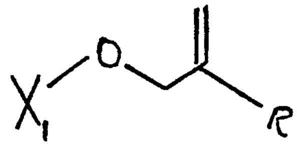

with carbon monoxide and hydrogen thereby carrying out a "oxo" reaction. The allyl ethers defined according to the structure

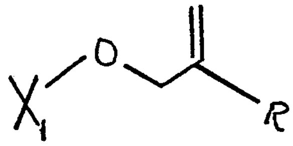

may be prepared by means of any standard ether synthesis, e.g., a "Williamson" synthesis or a synthesis as set forth in United States Letters Patent 4,163,068 issued on July 31, 1979, the specification for which is incorporated by reference herein. Thus, the ethers so useful in our invention may be formed by reacting an alcohol with another alcohol, for example, an allylic alcohol in the presence of an acid catalyst such as para toluene sulphonic acid at reflux conditions. The reaction mass is refluxed for a period of from two hours up to ten hours, after which period of time the reaction product is separated from the reaction mass by distillation.

The ethers so useful in practicing our invention may also be formed by reacting the corresponding alcohol with an appropriate allylic halide or other organic halide as the case may be. This reaction is carried out under the influence of base comprising the step of placing the reactants for the process and the base, respectively, in tow immiscible phases; an organic phase and either (i) an aqueous base phase or (ii) a solid base phase with the reactants being located substantially entirely in the first mentioned organic phase and the base being located substantially intirely in the second mentioned phase; and adding to the two phase system a "phase transfer agent" which may be one or more of several organic quaternary ammonium salts.

Specific examples of "phase transfer agents" useful in our invention are as follows:

Tricapryl methyl ammonium chloride;

Cetyl trimethyl ammonium bromide; and

Benzyl trimethyl ammonium hydroxide.

In general, the "phase transfer agents" most preferred have the generic formula:

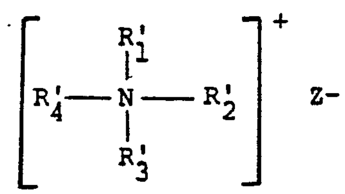

wherein at least one or $R_1'$, $R_2'$, $R_3'$ and $R_4'$ is $C_6$—$C_{14}$ aryl, $C_6$—$C_{10}$ aralkyl, $C_6$—$C_{20}$ alkyl, $C_6$—$C_{14}$ aralkyl and $C_6$—$C_{20}$ alkenyl, and the other of $R_2'$, $R_3'$ and $R_4'$ is alkyl such as methyl, ethyl, n-propyl, i-propyl, 1-butyl, 1-methyl-2-propyl, 1-pentyl and 1-octyl and Z— is an anion such as chloride, bromide and hydroxide.

This aspect of the process is carried out in an inexpensive solvent which is inert to the reaction system such as toluene, benzene, o-xylene, m-xylene, p-xylene, ethyl benzene, n-hexane, cyclohexane, methylene dichloride and o-dichlorobenzene.

This aspect of the process is carried out at a temperature in the range of from about 10°C up to about 150°C with a temperature range of 30°—120°C being preferred. The reaction time is inversely proportional to the reaction temperature, with lower reaction temperatures giving rise to greater reaction times; and, accordingly, the reaction time ranges from about 30 minutes up to about 10 hours.

In this aspect of the process, the mole ratio of alcohol reactant to organic halide (e.g., allylic halide) is in the range of from 0.5:1.5 up to about 1.5:0.5 with a preferred ratio of alcohol to organic halide (e.g., allylic halide) being from about 1:1 up to about 1:1.2.

The mole ratio of base to alcohol in the reaction mass may be in the range of from about 0.75:1 up to about 1.5:1 with a preferred mole ratio of base:alcohol being from about 1:1 up to about 1.2:1.

The quantity of "phase transfer agent" in the reaction mass based on the amount of alcohol in the reaction mass may vary from 0.5 grams per mole of alcohol up to 25 grams of "phase transfer agent" per mole of alcohol with a preferred concentration of "phase transfer agent" being in the range of from about 2.5 up to about 7.5 grams of "phase transfer agent" per mole of alcohol.

This aspect of the process is preferably carried out at atmospheric pressure since that is the most convenient condition. However, lower or higher pressures can be used without detrimentally affecting the ultimate yield of desired product. The particular base used in the reaction is not critical, but, preferred are sodium hydroxide and potassium hydroxide.

The individual ethers which are reactants for our invention in the oxo process described, infra, can be obtained in pure form or in substantially pure form by conventional purification techniques. Thus, the products can be purified and/or isolated by means of distillation, extraction, crystallization, preparative chromatographic techniques and the like. It has been found desirable to purify the ethers by fractional distillation in vacuo.

The thus-formed ethers having the structure:

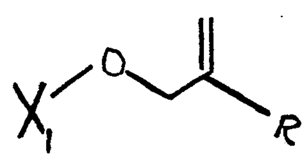

wherein R represents methyl or ethyl and $X_1$ is as defined, supra, are then reacted with a mixture of carbon monoxide and hydrogen using a particular range of temperatures and partial pressures of hydrogen and carbon monoxide over one of several alternative "oxo" type reaction catalysts over a period of various residence times.

Thus, the oxo reaction is carried out thusly:

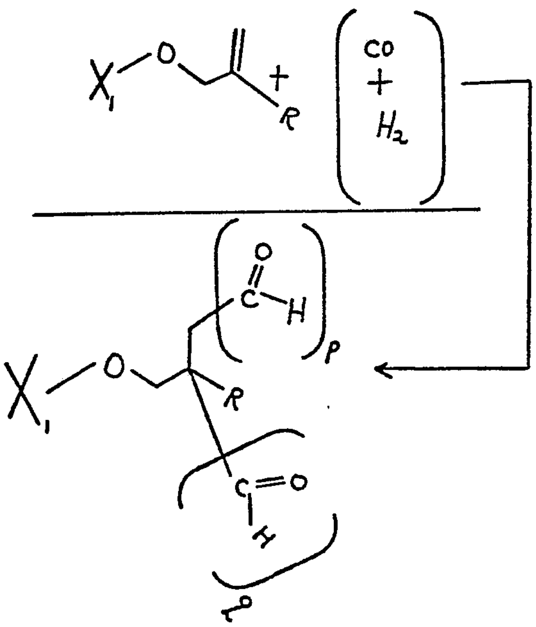

wherein $X_1$, R, p and q are defined, supra, with the production of a small amount of alcohol defined according to the structure:

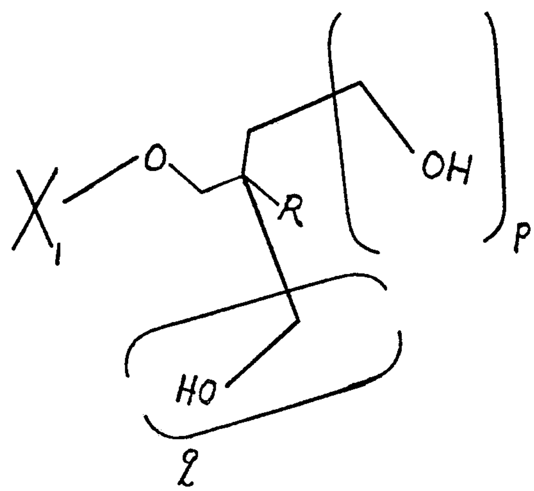

The reaction is carried out at temperatures of between 150°C and 300°C; at pressures of between 20 and 250 atmospheres; with the ratio of partial pressure of carbon monoxide:hydrogen being from 0.1:1 up to 1:0.1. Any oxo type reaction catalyst may be used, but most preferably, the catalyst to yield the best perfume and flavor mixtures are as follows:

Dicobalt octacarbonyl;
Cobalt octanoate;
Palladium chloride;
Rhodium trichloride;
Iron pentacarbonyl;
Nickel tetracarbonyl;
Polymer-bonded rhodium catalyst (e.g., rhodium bonded on a polystyrene substrate);
Tris-triphenyl phosphine rhodium-1-chloride;
Rhodium Aceto acetate dicarbonyl;
Rhodium Aceto acetate/triphenyl phosphine mixture.

The reaction time may vary from about two hours up to about 30 hours; and the reaction time is a function of the temperature and pressure of reaction; and the desired ratio of aldehyde:alcohol reaction product. Insofar as the instant invention is concerned, a high ratio of alcohol:aldehyde reaction product will be created by high temperature high pressure and a long period of time whereas a high ratio of aldehyde:alcohol will be created as a result of low temperature low pressure and short period of time.

At the end of the reaction, the reaction product is separated from the catalyst and unreacted materials by standard "work-up" means if desired; e.g., neutralization of catalyst; followed by extraction and fractional distillation; usually an initial fractional distillation by means of distillation through a 2—4 plate or stone packed column; followed by a more careful fractionation of the bulked center-cut fractions on, for example, a spinning band column or multiplate (14—50 plate) fractionation column.

The resultant aldehyde reaction product may then be reduced by means of using specific reducing agents according to the reaction:

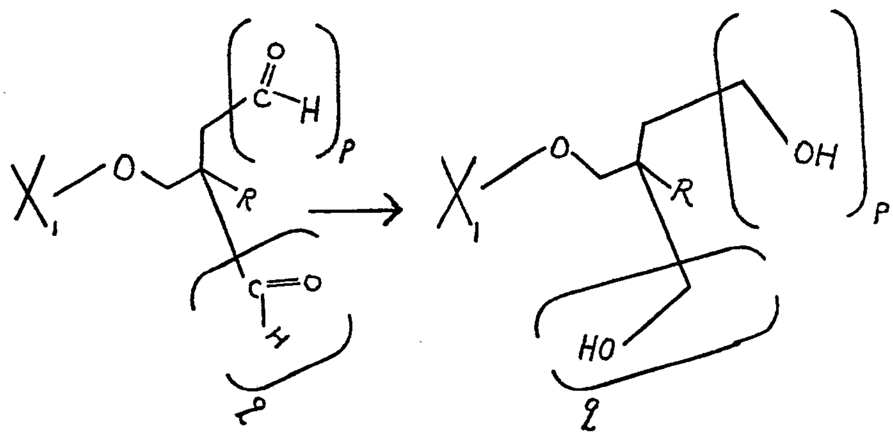

wherein $X_1$, R, p and q are defined, supra. The reduction reaction is carried out at a temperature in the range of from about 60°C up to about 100°C at from about one atmosphere up to about 10 atmospheres but preferably at reflux conditions for a period of time of between about one and 10 hours. The reaction takes place in the presence of a solvent which is inert to the reactants and the reaction product, such as, anhydrous isopropyl alcohol. Such solvent must be capable of being retained during the course of the reaction and not being so volatile as to boil off curing the reaction. The reducing agent may be any standard aldehyde-alcohol reducing agent but preferable reducing agents are alkyl metal borohydrides, for example, sodium borohydride, potassium borohydride and lithium borohydride. Other reducing agents that can be used are lithium aluminum hydride and aluminum isopropoxide and potassium isobutoxide. At the end of the reaction the reaction product which is the ether carbinol of our invention may be isolated from the reaction mass by standard isolation means, e.g., fractional crystallization, fractional distillation and commercial liquid chromatography procedures.

In addition, norbornyloxocarbinols defined according to the generic structure:

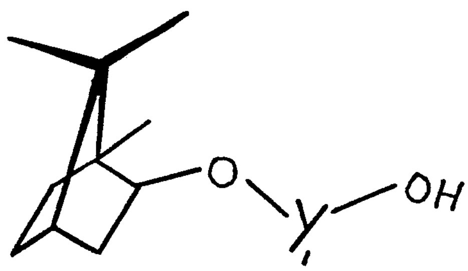

wherein $Y_1$ represents alkylenyl, alkenylenyl and alkynylenyl having from four up to five carbon atoms may be prepared by reacting camphene with a diol defined according to the structure:

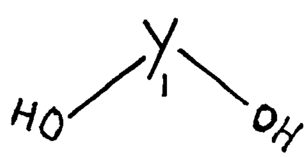

according to the reaction:

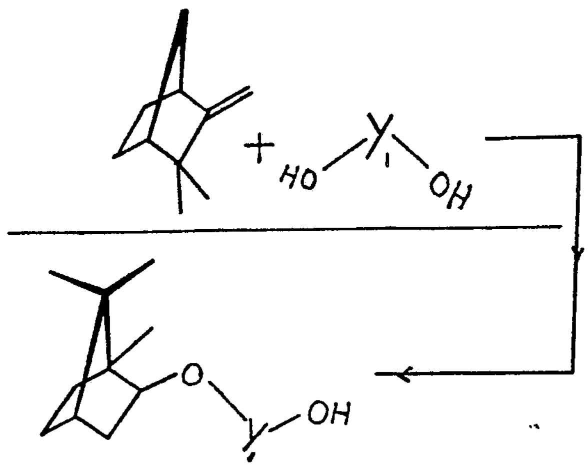

This reaction takes place in the presence of a catalyst which is a Lewis acid, for example, boron trifloride etherate at temperatures in the range of from about 60°C up to about 100°C and at pressures in the range of from about 1 atmosphere up to about 10 atmospheres. Preferably, the reaction takes place at 80°C at atmospheric pressure and at reflux condition. The reaction time may vary from about two hours up to about 20 hours depending upon the temperature of reaction. Higher temperatures of reaction gives rise to lower times of reaction and lower temperatures of reaction require higher times of reaction but a better overall yield. The mole ratio of diol having the structure:

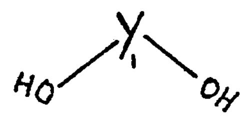

to camphene may vary from about 1:2 up to about 3:1 with a mole ratio of diol:camphene of about 2:1 being preferred. At the end of the reaction the reaction mass is neutralized and the reaction product defined according to the generic structure:

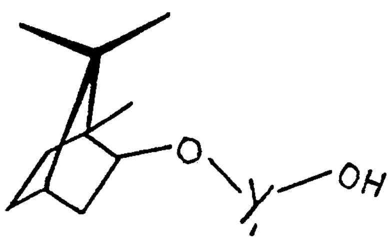

is purified for organoleptic uses as by means of fractional distillation.

Examples of ether carbinols which are useful in the practice of our invention and their organoleptic properties are set forth in the following Table II:

TABLE II

| Ether Carbinol | Perfumery Evaluation | Food Flavor Evaluation | Tobacco Flavor Evaluation |
|---|---|---|---|
| 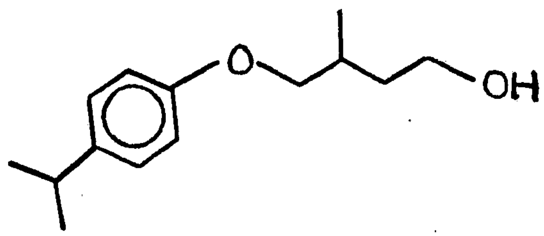 produced according to Example II | Nutty, woody, ozoney, fresh air dried cloth, green-orange aroma profile. | A green aroma and taste with fresh orange nuances. | |
| 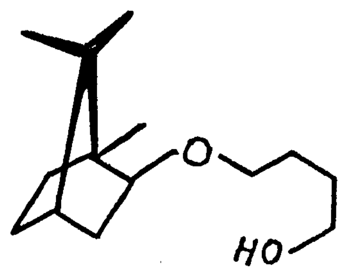 produced according to Examples IV or V. | A minty patchouli aroma with patchouli undertones. | A patchouli, oriental, incense, musky and sandal-wood aroma and taste profile at 5 ppm causing it to be good for oral hygiene and peppermint flavor. | |
| 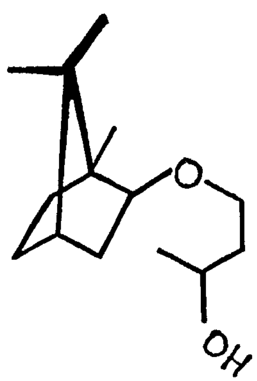 produced according to Example IV | An incense aroma with cedarwood undertones. | A walnut-like flavor. | A woody incense oriental and patchouli aroma and taste profile both prior to and on smoking in the mainstream and the sidestream. |

TABLE II (continued)

| Ether Carbinol | Perfumery Evaluation | Food Flavor Evaluation | Tobacco Flavor Evaluation |
|---|---|---|---|
| 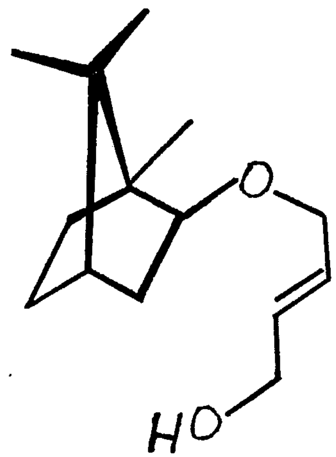 prepared according to Example VII | An onion, garlic and lavender aroma profile with oniony animalic and sweaty undertones. | An onion garlic aroma and taste profile. | |
| 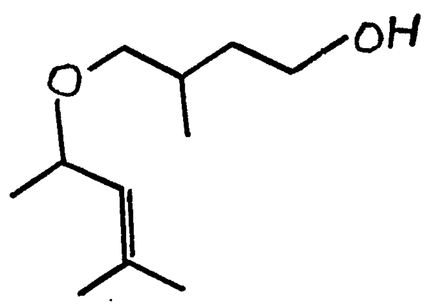 prepared according to Example IX | A camphoraceous, woody lavender and spicy and floral aroma with patchouli and rosy undertones. | A walnut-like flavor. | |

When one or more of the ether carboxaldehydes and/or ether carbinols and reaction products containing same of our invention is used as a food flavor adjuvant, the nature of the co-ingredients included with said one or more ether carboxyaldehydes and/or ether carbinols in formulating the product composition will also serve to alter the organoleptic characteristics of the ultimate foodstuffs treated therewith. As used herein, in regard to flavors, the term "alter" in its various forms means "supplying or imparting flavor character or notes to otherwise bland relatively tasteless substance or augmenting the existing flavor characteristic where a natural flavor is deficient in some regard or supplementing the existing flavor impression to modify its quality, character or taste".

As used herein, the term "foodstuff" includes both solid and liquid ingestible materials which usually do, but need not have nutritional value. Thus, foodstuffs include soups, convenience foods, beverages, dairy products, candies, fruits, cereals, soft drinks, snacks and the like.

Substances suitable for use herein as co-ingredients or flavoring adjuvants are well known in the art for such use being extensively described in the relevant literature. Apart from the requirement that any such material be "ingestibly" acceptable and thus non-toxic or otherwise non-deleterious, nothing particularly critical resides in selected thereof. Accordingly, such materials which may in general be characterized as flavoring adjuvants or vehicles comprise broadly stabilizers, thickeners, surface active agents, conditioners, other flavorants and flavor intensifiers.

Stabilizer compounds include preservatives, e.g., sodium chloride, antioxidants, e.g., calcium and sodium ascorbate, ascorbic acid, butylated hydroxyanisole (mixture of 2 and 3 tertiary butyl-4-hydroxyanisole), butylated hydroxy toluene (2,6-di-tertiary-butyl-4-methyl phenol), propyl gallate and the like, and sequestrants, e.g., citric acid.

Thickener compounds include carriers, binders, protective colloids, suspending agents, emulsifiers and the like, e.g., agaragar; carrageenan; cellulose and cellulose derivatives such as carboxymethyl cellulose and methyl cellulose; natural and synthetic gums such as gum arabic, gum tragacanth; gelatine; proteinaceous materials; lipids; carbohydrates; starches pectins, and emulsifiers, e.g., mono- and diglycerides of fatty acids, skim milk powder, hexoses, pentoses, disaccharides, e.g., sucrose, corn syrup solids and the like.

Surface active agents include emulsifying agents, e.g., fatty acids such as capric acid, caprylic acid, palmitic acid, myristic acid and the like, mono- and diglycerides of fatty acids, lecithin, defoaming and flavor-dispersing agents such as sorbitan monostrearate, potassium stearate hydrogenated tallow alcohol and the like.

Conditioners include compounds such as bleaching and maturing agents, e.g., benzoyl peroxide, calcium peroxide, hydrogen peroxide and the like; starch modifiers such as peracetic acid, sodium chlorite, sodium hypochlorite, propylene oxide, succinic anhydride and the like, buffers and neutralizing agents, e.g., sodium acetate, ammonium bicarbonate, ammonium phosphate, citric acid, lactic acid, vinegar and the like, colorants, e.g., carminic acid, cochineal, tumeric and curcumin and the like; firming agents such as aluminum sodium sulfate, calcium chloride and calcium gluconate; texturizers; anti-caking agents, e.g., aluminum calcium sulfate and tribasic calcium phosphate; enzymes, yeast foods, e.g., calcium lactate and calcium sulfate; nutrient supplements, e.g., iron salts such as ferric phosphate, ferrous gluconate and the like, riboflavin, vitamins, zinc sources such as zinc chloride, zinc sulfate and the like.

Other flavorants and flavor intensifiers include organic acids, e.g., acetic acid, butyric acid, caproic acid, caprylic acid, formic acid, 2-hexenoic acid, 3-hexenoic acid, isobutyric acid, isovaleric acid, propionic acid and valeric acid; ketones and aldehydes, e.g., acetaldehyde, acetone, acetyl methyl carbinol, acrolein, diacetyl, β,β-dimethylacrolein, hexanal, 2-hexenal, cis-3-hexenal, 4(p-hydroxyphenyl)-2-butanone, α-ionone, β-ionone, and 2-pentenal; alcohols, such as 1-butanol, trans-2-buten-1-ol, ethanol, gernaiol, 1-hexanol, cis-3-hexen-1-ol, 3-methyl-3-buten-1-ol, 1-pentalol, 1-penten-3-ol; esters, such as butyl acetate, ethyl acetate, ethyl butyrate, ethyl crotonate, ethyl propionate, 2-hexenyl acetate, 2-hexenyl butyrate, hexyl acetate, hexyl butyrate, isoamyl acetate, isopropyl butyrate, methyl butyrate, methyl caproate, methyl caprylate, propyl acetate, amyl acetate, amyl butyrate, benzyl salicylate, dimethyl anthranilate, ethyl methylphenylglycidate, ethyl succinate, isobutyl cinnamate, and terpenyl acetate; essential oils such as jasmine absolute, rose absolute, orris absolute, lemon essential oil and vanilla; lactones; sulfides, e.g., methyl sulfide and other materials such as maltol and citral as well as natural orange oil, natural peppermint oil, strawberry juice concentrate and the like.

The specific flavoring adjuvants selected for use may be either solid or liquid, depending upon the desired physical form of the ultimate product, i.e., foodstuff, whether simulated or natural and should, in any event, be capable of providing an environment in which the one or more ether carboxaldehydes and/or ether carbinols of our invention can be dispersed or admixed to provide a homogeneous medium. In addition, the selection of one or more adjuvants as well as the quantities thereof, will depend upon the precise organoleptic natural orange character, natural orange juice character, natural peppermint character or natural walnut character desired in the finished product. Thus, in the case of flavoring compositions, ingredients selection will vary in accordance with the foodstuffs to which the flavor and aroma are to be imparted. In contradistinction, in the preparation of solid products, e.g., simulated foodstuffs, ingredients capable of providing normally solid compositions should be selected, such as various cellulose derivatives.

As will be appreciated by those skilled in the art, the amount of one or more ether carboxaldehydes and/or ether carbinols of our invention employed in a particular instance can vary over a relatively wide

range whereby its desired organoleptic effects (having reference to the nature of the product) are achieved. All parts and percentages given herein are by weight unless otherwise specified. Thus, correspondingly greater amounts would be necessary in those instances wherein the ultimate food composition to be flavored is relatively bland to the taste, whereas relatively minor quantities may suffice for the purposes of enhancing the composition merely deficient in natural flavor or aroma. Thus, the primary requirement is that amount which is effective, i.e., sufficient to alter the organoleptic characteristics of the parent composition, whether foodstuff, per se, or flavoring composition. Thus, the use of insufficient quantities of one or more ether carboxaldehydes and/or ether carbinols of our invention will, of course, substantially vitiate any possibility of obtaining the desired results while excess quantities prove needlessly costly and in extreme cases, may disrupt the flavor/aroma balance, thus proving self-defeating. Accordingly, the terminology "effect amount" and "sufficient amount" is to be accorded a significance in the context of the present invention consistent with the obtention of desired flavoring effects.

Thus, and with respect to ultimate food compositions, it has been found that quantities of one or more ether carboxaldehydes and/or ether carbinols of our invention ranging from a small but effective amount, e.g., 0.02 parts per million up to about 100 parts per million by weight based on total composition are suitable. Concentrations in excess of the maximum quantity stated are not normally recommended since they fail to provide commensurate enhancement of organoleptic properties. In those cases wherein the one or more ether carboxaldehydes and/or ether carbinols of our invention is added to the foodstuff as an integral component of the flavoring composition, it is, of course, essential that the total quantity of flavoring composition employed be sufficient to yield an effective ether carboxaldehyde and/or ether carbinol concentration in the foodstuff product.

The compositions described herein can be prepared according to conventional techniques well known as typified by cakes, batters and fruit juices and can be formulated by merely admixing the involved ingredients within the proportions stated in a suitable blender to obtain the desired consistency, homogeneity of dispersion, etc. Alternatively, flavoring compositions in the form of particulate solids can be conveniently prepared by admixing one or more ether carboxaldehydes and/or ether carbinols of our invention with, for example, gum arabic, gum tragacanth, guar gum and the like and thereafter spray-drying the resultant mixture whereby to obtain the particulate solid product. Prepared flavor mixes in powder form, e.g., an orange-flavored powder or a peppermint-flavored powder are obtained by mixing dry solid components, e.g., starch, sugar and the like and one or more ether carboxaldehydes and/or ether carbinols in a dry blender until the requisite degree of uniformity is achieved.

It is presently preferred to combine one or more of the ether carboxaldehydes and/or ether carbinols of our invention with the following adjuvants:

Parahydroxybenzyl acetone;
Vanillin;
Maltol;
β-Ionone;
β-Ionone;
Isobutyl acetate;
Ethyl butyrate;
Dimethyl sulfide;
Acetic acid;
Acetaldehyde;
4-(2,6,6-trimethyl-1,3-cyclohexadien-1-yl)-2-butanone;
4-(6,6-dimethyl-2-methylene-3-cyclohexen-1-yl)-2-butanone;
2-(4-hydroxy-4-methylpentyl)norbornadiene produced according to Example I of U.S. Pat. No. 3,911,028;
β-Damascone (1-crotonyl-2,6,6-trimethylcyclohex-1-ene);
β-Damascenone (1-crotonyl-2,6,6-trimethylcyclohexa-1,3-diene);
Beta-cyclohomocitral (2,6,6-trimethylcyclohex-1-ene carboxaldehyde);
Isoamyl butyrate;
Cis-3-hexenol-1;
Elemecine (4-allyl-1,2,6-trimethoxybenzene);
Isoelemecine (4-propyenyl-1,2,6-trimethoxybenzene);
Cis-2-3-methyl pentenoic acid;
Ethyl-2-methyl-3-pentenoate;
Isobutyl-cis-2-methyl-3-pentenoate;
2-Ethylidene-3-pentenal;
Orange oil;
Lemon oil;
Peppermint oil;
Strawberry juice extract;
Raspberry juice extract;
Cranberry juice extract;
Mango extract;

Pickled mango extract; and
Pulverized walnuts.

One or more carboxaldehyde derivatives and/or ether carbonol derivatives prepared in accordance with the processes of our invention and one or more auxiliary perfume ingredients, including, for example, alcohols other than those of our invention; aldehydes other than those of our invention; ketones; topenic hydrocarbons; nitriles; esters; lactones; natural essential oils; and synthetic essential oils may be admixed so that the combined odors of the individual components produce a pleasant and desired fragrance. Such perfume compositions usually contain (a) the main note or the "bouquet" or foundation stone of the composition; (b) modifiers which round off and accompany the main note; (c) fixatives which include odorous substances which lend a particular note to the perfume throughout all stages of evaporation and substances which retard evaporation; and (d) topnotes which are usually low boiling, fresh smelling materials.

In perfume compositions, it is the individual components which contribute to their particular olfactory characteristics, however, the overall sensory effect of the perfume composition will be at least the sum total of the effects of each of the ingredients. Thus, one or more of the ether carboxaldehyde derivatives and/or ether carbinol derivatives prepared in accordance with the processes of our invention can be used to alter, modify or enhance the aroma characteristics of a perfume composition, for example, by utilizing or moderating the olfactory reaction contributed by another ingredient in the composition.

The amount of one or more ether carboxaldehyde derivatives and/or ether carbinol derivatives and/or ether carbinol derivatives prepared in accordance with the processes of our invention which will be effective in perfume compositions as well as in perfumed articles (e.g., anionic, nonionic, cationic and zwitterionic solid or liquid detergents, soaps, fabric softener compositions, drier-added fabric softener articles, optical brightener compositions, perfumed polymers and textile sizing agents) and colognes depends on many factors, including the other ingredients, their amounts and the effects which are desired. It has been found that perfume compositions containing as little as 0.01% of one or more of the ether carboxaldehyde derivatives and/or ether carbinol derivatives of our invention prepared in accordance with the processes of our invention and less than 50% of one or more of the ether carboxaldehyde derivatives and/or ether carbinol derivatives prepared in accordance with the processes of our invention or even less (e.g., 0.005%) can be used to impart, augment or enhance a large number of aroma properties in or to soaps, cosmetics, solid or liquid anionic, nonionic, cationic and zwitterionic detergents, fabric softener compositions, fabric softener articles, optical brightener compositions, textile sizing compositions, perfumed polymers or other products. The amount employed can range up to 70% of the fragrance components and will depend on considerations, of course, nature of the end product, the effect desired on the finished product and the particular fragrance sought.

One or more ether carboxaldehyde derivatives and/or ether carbinol derivatives prepared in accordance with the processes of our invention are useful (taken alone or together with other ingredients in perfume compositions) as (an) olfactory component (s) in detergents and soaps, space odorants and deodorants, perfumes, colognes, toilet water, bath preparations, such as creams, deodorants, hand lotions and sun screens; powders, such as, talc, dusting powders, face powders, and perfumed polymers and articles of manufacture produced from said perfumed polymers, e.g., garbage bags, children's toys and the like. When used as (an) olfactory component (s) as little as 0.2% of one or more of the ether carboxyaldehyde derivatives and/or ether carbinol derivatives prepared in accordance with the processes of our invention will suffice to impart, augment or enhance various intense aromas in or to floral, piney, lavender, spicy and patchouli formulations. Generally, no more than 6% of one or more of the ether carboxaldehyde derivatives and/or ether carbinol derivatives of our invention based on the ultimate end product as required in the perfumed article composition. Accordingly, the range of ether carboxaldehydes and/or ether carbinols in the perfumed article is from about 0.2% by weight of the ether carboxaldehyde and/or ether carbinol up to about 6% by weight of the ether carboxaldehyde and/or ether carbinol based on the total weight of the perfumed article.

In addition, the perfumed composition or fragrance composition of our invention can contain a vehicle or carrier for one or more of the ether carboxaldehyde derivatives and/or ether carbinol derivatives prepared in accordance with the processes of our invention. The vehicle can be a liquid, such as, a non-toxic alcohol, e.g., ethyl alcohol, a non-toxic glycol, e.g., propylene glycol or the like. The carrier can also be an absorbant solid, such as, gum (e.g., gum arabic or gum orzentane) or components for incapsulating the composition (such as, gelatin as by coacervation) or such as, urea formaldehyde polymer forming a capsule shell around a liquid perfumed center.

Our invention also relates to the utilization of controlled released technology for the controlled release of perfumes into gaseous environments from polymers such as mixtures of epsilon polycaprolactone

polymers and polyethylene which polyepsilon caprolactone polymers are defined according to at least one of the structures:

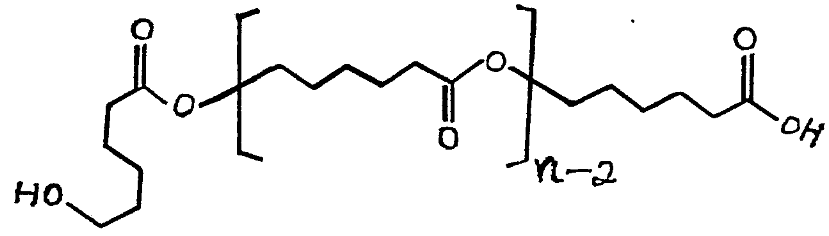

and/or

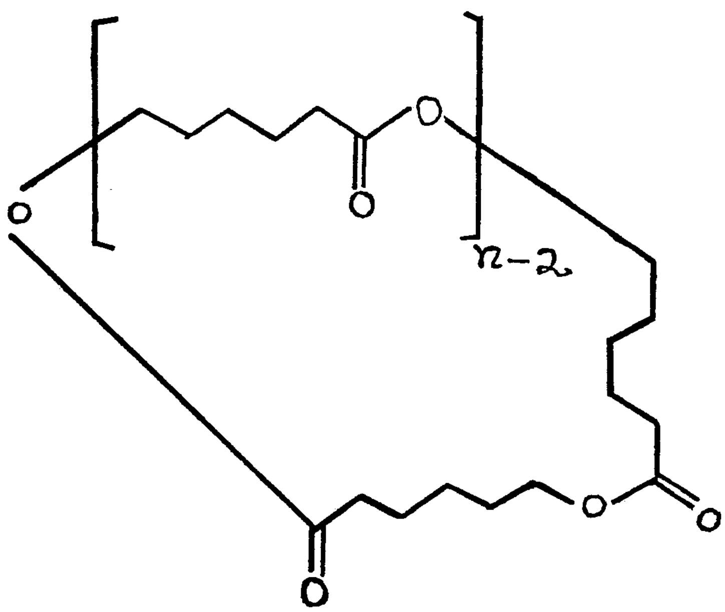

wherein "n" is from about 50 up to about 1,200 with the proviso that the average "n" in the system varies from about 150 up to about 700 according to the mathematical statement:

$$[700 \geqslant \bar{n} \geqslant 150]$$

with the term $\bar{n}$ being the average number of repeating monomeric units for the epsilon polycaprolactone polymer. The perfumed material's release rate from such polymer mixture is close to "zero order". As a general rule, the release rate in a polymeric matrix is proportional to $t^{-1/2}$ until about 60% of the functional fluid is released from the polymeric matrix. The release rate thereafter is related exponentially to time as a general rule according to the equation:

$$\frac{dM_t}{dt} = k_1 e^{-k_2 t}$$

wherein $k_1$ and $k_2$ are constants. According to Kydonieus, "Controlled Release Technologies: Methods, Theory, and Applications" (cited, supra) the amount of perfume composition released is proportional to time as long as the concentration of perfume material present, e.g., the ether carboxaldehydes and/or ether carbinols of our invention is higher than the solubility of the agent in the matrix. Thus, such dispersed systems are similar to the dissolved systems except that instead of a decreased release rate after 60% of the perfume material has been emitted, the relationship holds almost over the complete release curve. Kydonieus further states, that if one assumes that the release of functional fluid by diffusion is negligible in monolithic erodible systems, the speed of erosion will control the release rate and release by erosion by a surface-area-dependent phenomenon, the release being constant (zero order) as long as the surface area does not change during the erosion process. This is the case with the polymers containing the ether carboxaldehydes and/or ether carbinols of our invention.

The polyepsilon caprolactone polymers useful in practicing our invention are more specifically described in the brochure of the Union Carbide Corporation, 270 Park Avenue, New York, New York 10017, entitled "NEW POLYCAPROLACTONE THERMOPLASTIC POLYMERS PCL-300 AND PCL-700". These polyepsilon caprolactone polymers are composed of a repeating sequence of non-polar methylene groups

and relatively polar ester groups. The average number of repeating monomeric units varies between 150 and 700 depending on the particular "PCL" number. Thus, regarding PCL-300 the average number of repeating monomeric units is about 300. Regarding PCL-700, the average number of repeating monomeric units is 700.

The polyepsilon caprolactone homopolymers which are ultimately taken in admixture with such materials as polyethylene useful in the practice of our invention may also be stabilized using stabilizers as defined in United States Letters Patent 4,360,682 issued on November 23, 1982, the specification for which is incorporated herein by reference. The stabilizing materials which stabilize the polyepsilon caprolactone useful in conjunction with our invention against discoloration are dihydroxybenzenes such hydroquinone or compounds having the formula:

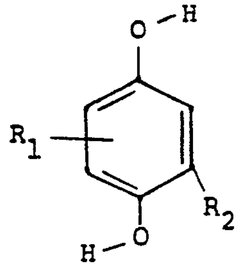

or

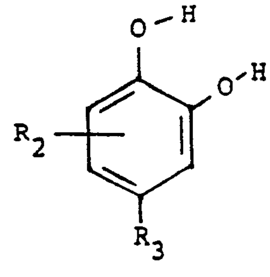

in which $R_1$ is alkyl of from 1 to 8 carbon atoms, and $R_2$ is hydrogen or alkyl of 1 to 8 carbon atoms. It is preferable to have such stabilizer in the polyepsilon caprolactone homopolymer in an amount of from about 100 to 500 ppm. Such stabilizers do not interfere with the functional fluids dissolved and/or absorbed into the polymeric matrix.

The method of incorporating the ether carboxaldehydes and/or ether carbinols of our invention or perfume compositions containing same into the polymers may be according to the techniques of United States Letters Patent 3,505,432 issued on April 7, 1970 (the specification for which is incorporated by reference herein) or United States Letters Patent 4,247,498 issued on January 27, 1981, the disclosure of which is incorporated by reference herein.

Thus, for example, a first amount of liquid polyethylene-polyepsilon caprolactone polymer mixture (50:50) is mixed with one of the ether carboxaldehydes and/or ether carbinols of our invention. Drops are formed from the mixture and the drops are solidified. The solidified drops are then melted, if desired, with a second amount of unscented low density polyethylene, for example, or polypropylene, for example. Usually, but not necessarily, the second amount of polymer is larger than the first amount. The resulting mixture thus obtained, is solidified subsequent to or prior to ultimate casting into a utilitarian shape.

Thus, in accordance with one aspect of our invention, the imparting of scent is effected in two stages. In a first stage, a 50:50 (weight:weight) polyepsilon caprolactone, e.g., PCL-700:polyethylene in molten form is admixed with a high percentage of one of the ether carboxaldehydes and/or ether carbinols of our invention and the mixture is solidified in the form of pellets or beads. These pellets or beads thus contain a high percentage of ether carboxaldehydes and/or ether carbinols (e.g., up to 45% by weight of the entire mixture) and may be used as "master pellets" which thereafter, in a second stage, if desired, may be admixed and liquefied with additional polymers such as additional polyethylene or mixtures of polyethylene and polyepsilon caprolactone in an unscented state, or unscented polypropylene. In addition, additional polymers or copolymers may be used, for example, copolymers specified and described in United Kingdom Patent Specification 1,589,201 published on May 7, 1981, the specification for which is incorporated by reference herein.

In accordance with the present invention at least one of the ether carboxaldehydes and/or ether carbinols of our invention is added to the polymer in a large closed container or drum which is maintained under controlled temperature conditions while the polymer in a melted condition is mixed with at least one of the ether carboxaldehydes and/or ether carbinols under agitation.

In order that the perfume be added uniformly to the polymer, the temperature of the melt is constantly controlled during the process. The polymer-perfume mixture is then directed through an elongated conduit or pipe element having a plurality of orifices adjacent to the lower most portion thereof. The polymer enriched by at least one of the ether carboxaldehydes and/or ether carbinols of our invention is permitted to drip through the orifices onto a continuously moving, cooled conveyor upon which the polymer containing at least one of the ether carboxaldehydes and/or ether carbinols of our invention solidifies into small size pellets with the perfume imprisoned therein. The apparatus useful in conjunction with this process, advantageously includes a conveyor of a material which will not adhere to the polymer which contains at least one of the ether carboxaldehydes and/or ether carbinols of our invention.

In order that the droplets form into uniform pellets or beads, the conveyor is continuously washed with a liquid, such as water to maintain the surface relatively cool. The pellets are delivered by the conveyor into a container and packaged for shipment.

Furthermore, one or more of the ether carboxaldehydes and/or ether carbinols of our invention prepared in accordance with the processes of our invention are capable of supplying and/or potentiating

certain flavor and aroma notes usually lacking in many smoking tobacco flavors and substitute tobacco flavors heretofore provided.

As used herein in regard to smoking tobacco flavors, the terms "alter" and "modify", in their various forms, mean "supplying or imparting flavor character or note to otherwise bland smoking tobacco, smoking tobacco substitutes, or smoking tobacco flavor formulations or augmenting the existing flavor characteristic where a natural flavor is deficient in some regard or supplementing the existing flavor impression to modify its quality, character or taste".

As used herein, the term "enhance" is intended to mean the intensification (without change in kind of quality of aroma or taste) of one or more taste and/or aroma nuances present in the organoleptic impression of smoking tobacco or a smoking tobacco substitute or a smoking tobacco flavor.

Our invention thus provides an organoleptically improved smoking tobacco product and additives therefor, as well as methods of making the same which overcome specific problems heretofore encountered in which specific desired aroma and taste nuances prior to and on smoking in both the main stream and in the side stream are created or enhanced and may be readily controlled and maintained at the desired uniform level regardless of variations in the components of the blend.

This invention further provides various improved smoking tobacco additives and methods, whereby, various aroma and taste nuances are imparted (on smoking in the main stream and in the side stream) to smoking tobacco products and may be readily varied and controlled to produce the desired uniformed flavor characteristics, particularly, insofar as "oriental" like tobacco characteristics are concerned.

In carrying out this aspect of our invention, we add to smoking tobacco materials or a suitable substitute therefor (e.g., dried lettuce leaves) an aroma and flavor additive containing as an active ingredient at least one or more of the ether carboxaldehydes and/or ether carbinols prepared in accordance with the processes of our invention.

In addition to one or more of the ether carboxaldehydes and/or ether carbinols prepared in accordance with the processes of our invention, other flavoring and aroma additives may be added to the smoking tobacco materials or substitute therefor either separately or in admixture with one or more of the ether carboxaldehydes and/or ether carbinols of our invention as follows:

(i) Synthetic Materials
Beta-ethyl-cinnamaldehyde;
Beta-cyclohomocitral;
Eugenol;
Dipentene;
β-Damascenone;
β-Damascone;
Maltol;
Ethyl maltol;
Delta-undecalactone;
Benzaldehyde;
Amyl acetate;
Ethyl butyrate;
Ethyl valerate;
Ethyl acetate;
2-Hexenol-1;
2-Methyl-5-isopropyl-1,3-nonadiene-8-one;
2,6-Dimethyl-2,6-undecadiene-10-one;
2-Methyl-5-isopropyl acetophenone;
2-Hydroxy-2,5,5-8a-tetramethyl-1-(2-hydroxyethyl)-decahydronaphthalene;
Dodecahydro-3a,6,6,9a-tetramethylnaphtho[2,1b]furan;
4-Hydroxy hexanoic acid, gamma lactone; and
Polyisoprenoid hydrocarbons defined in Example V of U.S. Patent No. 3,589,372 issued on June 29, 1971;

(ii) Natural oils
Celery seed oil;
Coffee extract;
Bergamot oil;
Cocoa extract;
Nutmeg oil; and
Origanum oil.

An aroma and flavoring concentrate containing one or more of the ether carboxaldehydes and/or ether carbinols prepared in accordance with the process of our invention, and, if desired, one or more of the above-identified additional flavoring additives may be added to the smoking tobacco material, to the filter or to the leaf or paper wrapper. The smoking tobacco material may be shredded, cured, cased and blended tobacco material or reconstituted tobacco material or tobacco substitutes (e.g., lettuce leaves) or mixture thereof. The proportions of flavoring additives may be varied in accordance with taste but insofar as

enhancement or the imparting of natural and/or spicy notes, we have found that satisfactory results are obtained if the proportion by weight of the sum total of one or more of the ether carboxaldehydes and/or ether carbinols is between 250 ppm and 1,500 ppm (0.025%—0.15%) of the active ingredients to the smoking tobacco material. We have further found that satisfactory results are obtained if the proportion by weight of the sum total of one or more of the ether carboxaldehydes and/or ether carbinols of our invention is between 2,500 and 15,000 ppm (0.25%—1.50%).

Any convenient method for incorporation of one or more of the ether carboxaldehydes and/or ether carbinols prepared in accordance with the processes of our invention in the tobacco product may be employed. Thus, one or more of the ether carboxaldehydes and/or ether carbinols of our invention taken alone or along with other flavoring additives may be dissolved in a suitable solvent, such as ethanol, pentane, diethyl ether and/or other volatile organic solvents and the resulting solution may either be sprayed on the cured, cased and blended tobacco material or the tobacco material may be dipped into such solution.

Under certain circumstances, a solution containing one or more of the ether carboxaldehydes and/or ether carbinols of our invention taken alone or taken further together with other flavoring additives as set forth above, may be applied by means of a suitable applicator such as a brush or roller on the paper or leaf wrapper for the smoking product, or it may be applied to the filter by either spraying, or dipping, or coating.

Furthermore, it will be apparent that only a portion of the smoking tobacco or substitute therefor need be treated and the thus treated tobacco may be blended with other tobaccos before the ultimate tobacco product is formed. In such cases, the tobacco treated may have one or more of the ether carboxaldehydes and/or ether carbinols of our invention in excess of the amounts or concentrations above-indicated so that when blended with other tobaccos, the final product will have the percentage within the indicated range.

In accordance with one specific example of our invention, an aged, cured and shredded domestic Burley tobacco is sprayed with a 20% ethyl alcohol solution of a 50:35:15 weight:weight:weight mixture of compounds containing materials having the following structures (in the order stated):

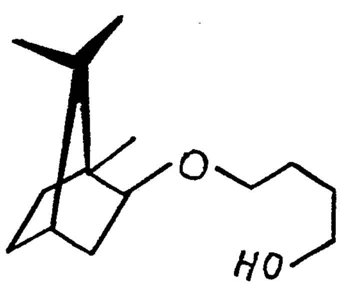
;
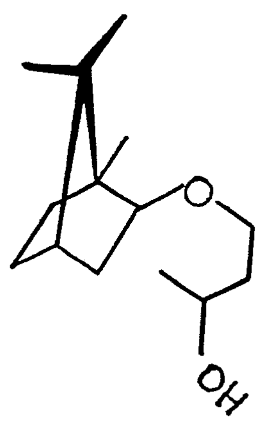
; and
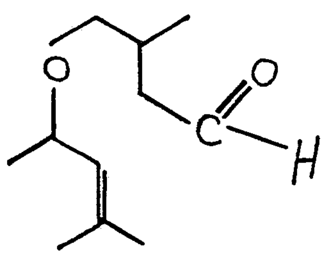

respectively in an amount to provide the tobacco composition containing 800 ppm by weight of the above-mentioned ether carboxaldehydes and/or ether carbinols on a dry basis.

Thereafter, the alcohol is removed by evaporation and the tobacco is manufactured into cigarettes by the usual techniques. The cigarettes, when treated as indicated, have a desired and pleasing aroma prior to smoking which can be described as woody, incense-like, oriental, minty and patchouli-like and, on smoking, in the main stream and in the side stream as spicy, oriental-like, turkish tobacco-like, and woody with a slight mouth coating effect.

While our invention is particularly useful in the manufacture of smoking tobacco, such as cigarette tobacco, cigar tobacco, and pipe tobacco, other smoking tobacco products formed from sheeted tobacco dust or fines may also be used. Likewise, one or more of the ether carboxaldehydes and/or ether carbinols of our invention can be incorporated with materials such as, filter tip materials, seam paste, packaging materials and the like which are used along with tobacco to form a product adapted for smoking. Furthermore, one or more of the ether carboxaldehydes and/or ether carbinols of our invention can be added to certain tobacco substituents of natural or synthetic origin (e.g., dried lettuce leaves) and accordingly, by the term "tobacco" is used throughout this specification is meant any composition

intended for human consumption by smoking or otherwise whether composed of tobacco plant parts or substitute materials or both.

BRIEF DESCRIPTION OF THE DRAWINGS

Figure 1 is the GLC profile for the crude reaction product of Example I containing the compound having the structure:

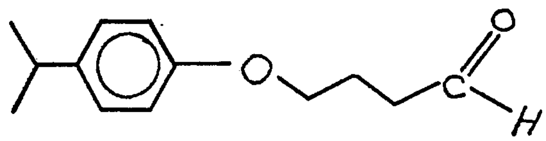

Figure 2 is the NMR spectrum for the compound having the structure:

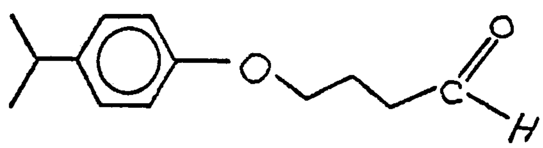

produced according to Example I (conditions: Field strength 100 MHz; solvent: $CFCl_3$).

Figure 3 is the GLC profile for fraction 4 of the distillation product of the reaction product of Example II containing the compound having the structure:

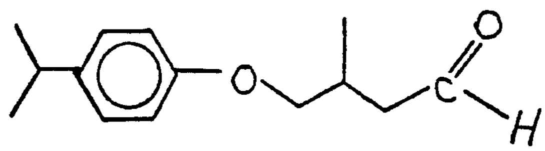

Figure 4 is the NMR spectrum for the peak indicated by reference numeral "30" on Figure 3 which is the GLC profile for fraction 4 of the distillation product of the reaction product of Example II containing the compound having the structure:

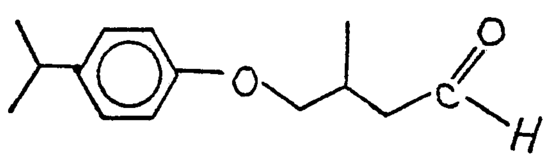

(Conditions: Field strength 100 MHz; solvent: $CFCl_3$).

Figure 5 is the GLC profile for bulked fractions 2—4 of the first distillation product of the reaction product of Example III containing the compound having the structure:

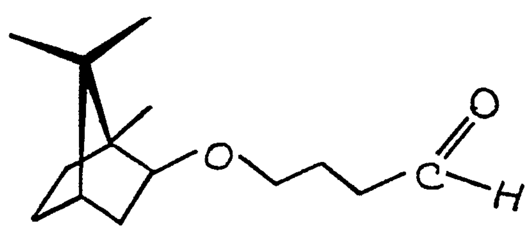

Figure 6 is the GLC profile for fraction 5 of the second distillation product of the reaction product of Example III containing the compound having the structure:

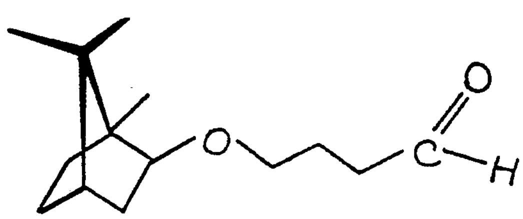

Figure 7 is the NMR spectrum for the compound having the structure:

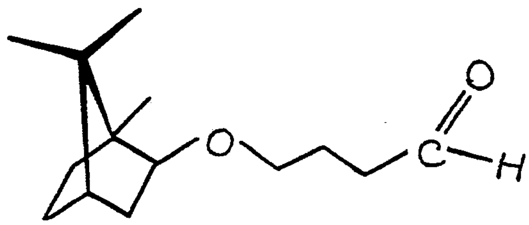

produced according to Example III (conditions: Field strength 100 MHz; solvent: $CFCl_3$).

Figure 8 is the GLC profile for the crude reaction product of Example IV containing the compound having the structure:

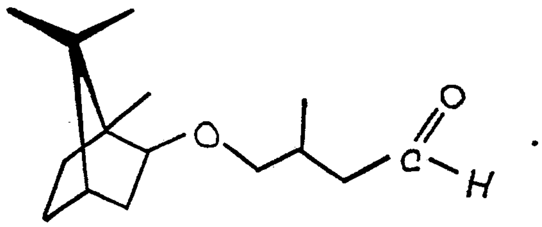

Figure 9 is the GLC profile for fraction 6 of the distillation product of the reaction product of Example IV containing the compound having the structure:

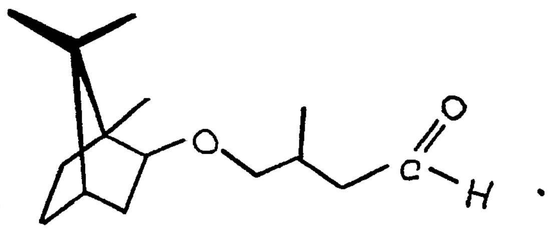

Figure 10 is the NMR spectrum for fraction 6 of the distillation product of the reaction product of Example IV containing the compound having the structure:

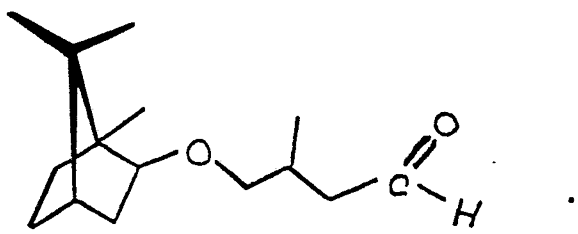

Figure 11 is the GLC profile for fraction 4 of the first distillation product of the reaction product of Example V containing the compound having the structure:

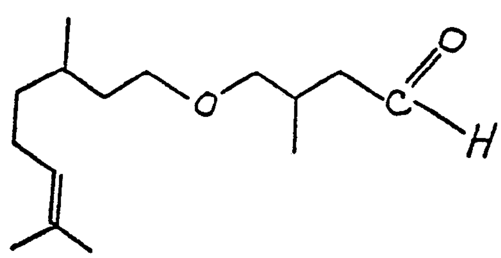

Figure 12 is the GLC profile for fraction 19 of the final distillation product of the reaction product of Example V containing the compound having the structure:

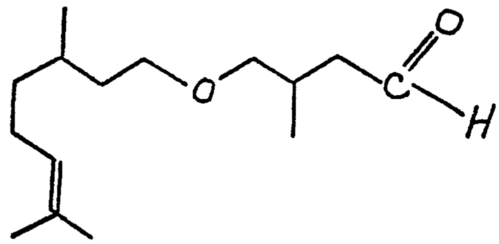

Figure 13 is the NMR spectrum for fraction 19 of the final distillation product of the reaction product of Example V containing the compound having the structure:

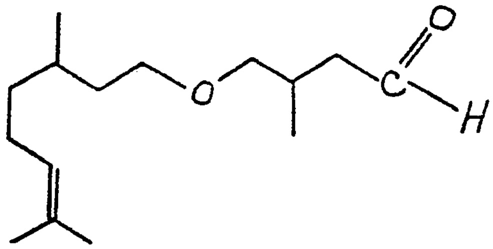

(conditions: Field strength 100 MHz; solvent: $CFCl_3$).

Figure 14 is the GLC profile for the crude reaction product of Example VI containing the compound having the structure:

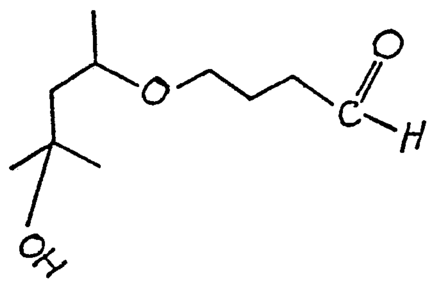

Figure 15 is the GLC profile for fraction 9 of the distillation product of the reaction product of Example VI containing the compound having the structure:

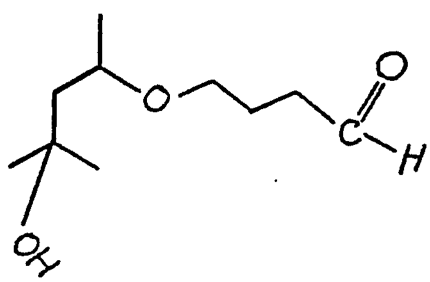

Figure 16 is the NMR spectrum for fraction 9 of the distillation product of the reaction product of Example VI containing the compound having the structure:

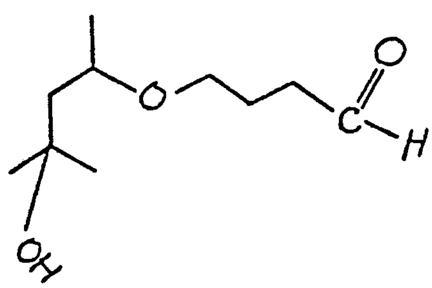

Figure 17 is the GLC profile for the crude reaction product of Example VII containing the compound having the structure:

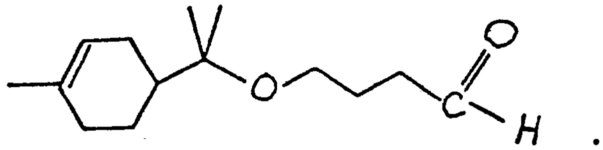

Figure 18 is the GLC profile for fraction 11 of the distillation product of the reaction product of Example VII containing the compound having the structure:

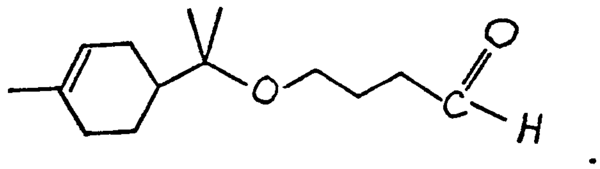

Figure 19 is the NMR spectrum for peak "180" of Figure 18, the GLC profile for fraction 11 of the distillation product of the reaction product of Example VII containing the compound having the structure:

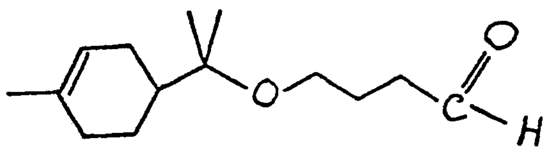

(Conditions: field strength 100 MHz; solvent: $CFCl_3$).

Figure 20 is the GLC profile for the crude reaction product of Example VIII containing the compound having the structure:

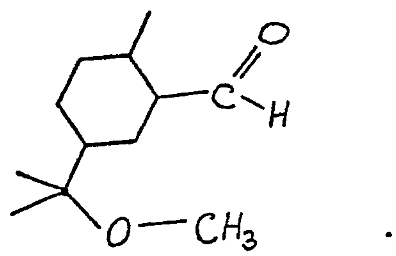

Figure 21 is the GLC profile for fraction 8 of the distillation product of the reaction product of Example VIII containing the compound having the structure:

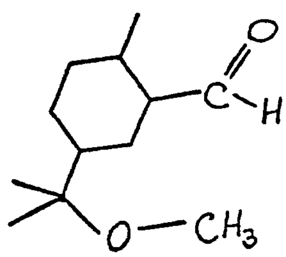

Figure 22 is the NMR spectrum for the compound having the structure:

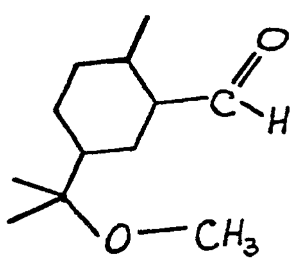

produced according to Example VIII (conditions: Field strength: Field strength: 100 MHz; Solvent: $CFCl_3$).

Figure 23 is the GLC profile for the crude reaction product of Example XXXII containing the compound having the structure:

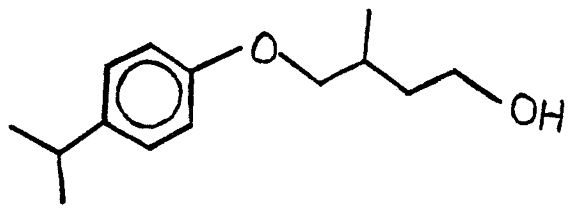

Figure 24 is the GLC profile for fraction 5 of the distillation product of the reaction product of Example XXXII containing the compound having the structure:

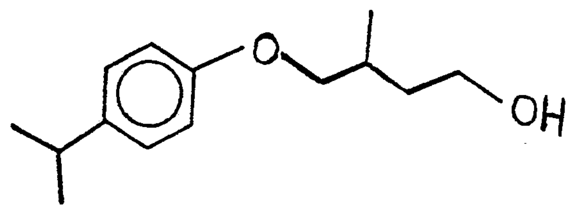

Figure 25 is the NMR spectrum for peak 149 of the GLC profile of Figure 24 for the compound having the structure:

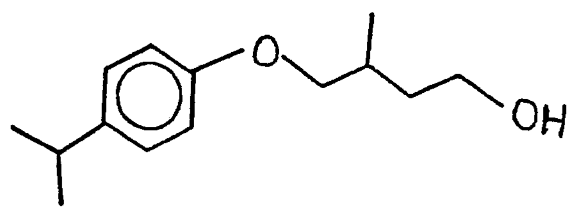

(Conditions: Field strength: 100 MHz; solvent: $CFCl_3$).

Figure 26 is the GLC profile for the crude reaction product of Example XXXIII containing the compounds having the structure:

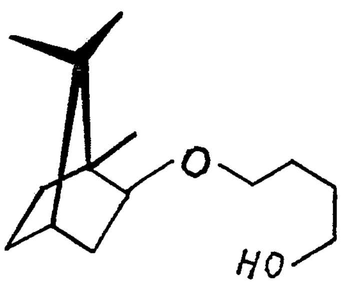

and

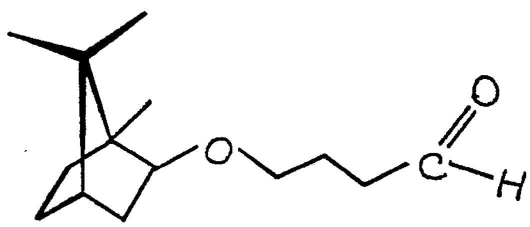

Figure 27 is the GLC profile for fraction 2 of the distillation product of the reaction product of Example XXXIII containing the compound having the structure:

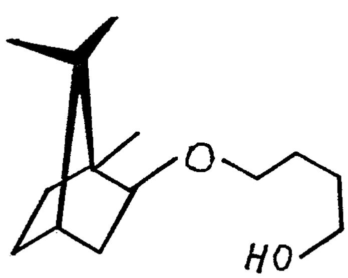

Figure 28 is the NMR spectrum for the compound having the structure:

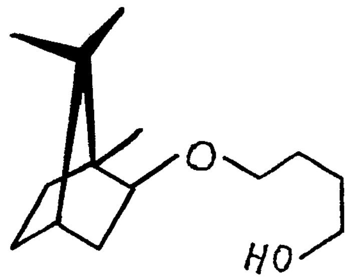

produced according to Example XXXIII (Conditions: Field strength: 100 MHz; solvent: $CFCl_3$).

Figure 29 is the infra-red spectrum for the compound having the structure:

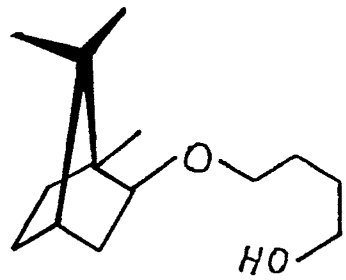

produced according to Example XXXIII.

Figure 30 is the GLC profile of the crude reaction product of Example XXXIV containing the compound having the structure:

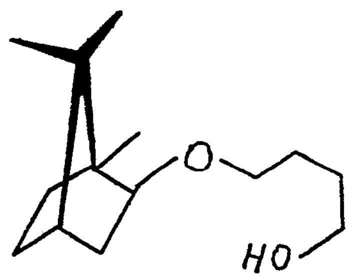

Figure 31 is the NMR spectrum for fraction 4 of the distillation product of the reaction product of Example XXXIV containing the compound having the structure:

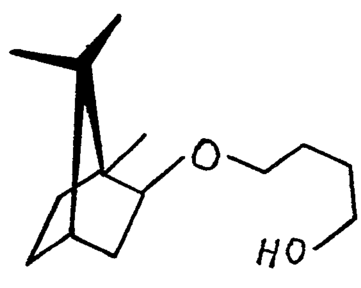

Figure 32 is the GLC profile for the crude reaction profile of Example XXXV containing the compound having the structure:

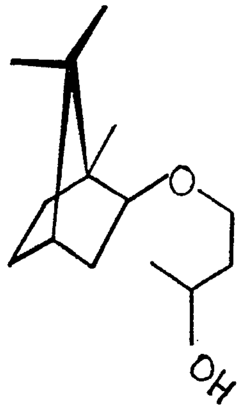

Figure 33 is the NMR spectrum for fraction 2 of the distillation product of the reaction product of Example XXXV containing the compound having the structure:

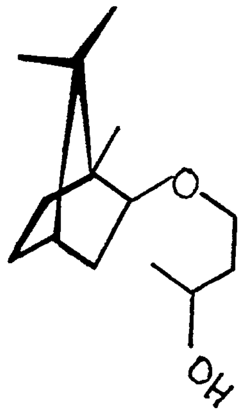

(Conditions: Field strength: 100 MHz; solvent: $CFCl_3$).

Figure 34 is the GLC profile for the crude reaction product of Example XXXVI containing the compound having the structure:

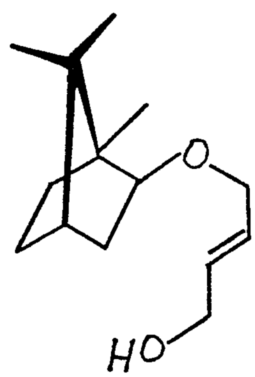

(Conditions: SE-30 column programmed at 100—200°C at 8°C per minute).

Figure 35 is the NMR spectrum for fraction 4 of the distillation product of the reaction product of Example XXXVI containing the compound having the structure:

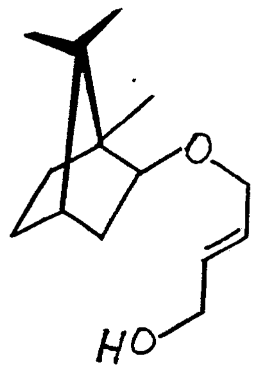

Figure 36 is the GLC profile for fraction 4 of the distillation product of the reaction product of Example XXXVII containing the compound having the structure:

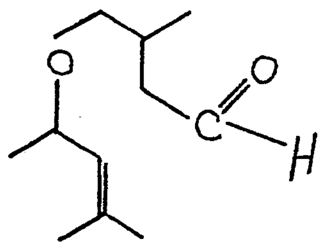

Figure 37 is the NMR spectrum for fraction 8 of the distillation product of the reaction product of Example XXXVII containing the compound having the structure:

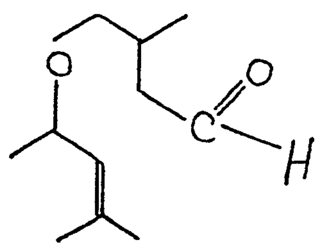

(Conditions: Field strength: 100 MHz; solvent: $CFCl_3$).

Figure 38 is the GLC profile for the crude reaction product of Example XXXVIII containing the compound having the structure:

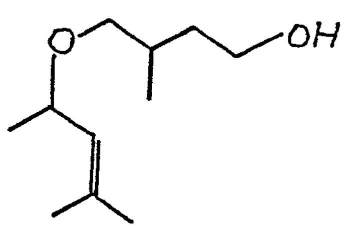

Figure 39 is the NMR spectrum for fraction 4 of the distillation product of the reaction product of Example XXXVIII containing the compound having the structure:

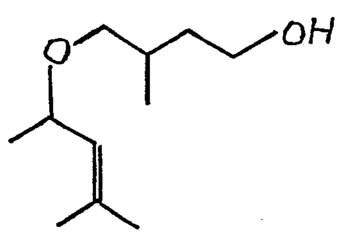

(Conditions: Field strength: 100 MHz; solvent: $CFCl_3$).

Figure 40a is the GLC profile for the crude reaction product of Example XXXIX containing the compound having the structure:

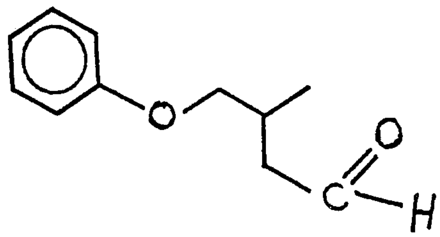

Figure 40B is the NMR spectrum for the compound having the structure:

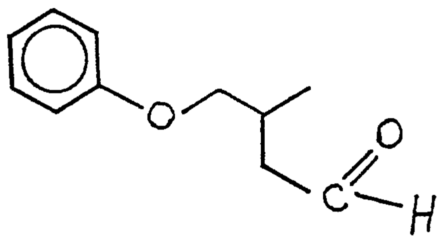

produced according to Example XXXIX (Conditions: Solvent; $CFCl_3$: Field strength: 100 MHz).

Figure 41 represents a cut-away side elevation view of apparatus used in forming perfumed polymers which contain embedded therein at least one of the ether carbinols of our invention.

Figure 42 is a front view of the apparatus of Figure 45 looking in the direction of the arrows.

DETAILED DESCRIPTION OF THE DRAWINGS

Figure 1 is the GLC profile for the crude reaction product produced according to Example I containing the compound having the structure:

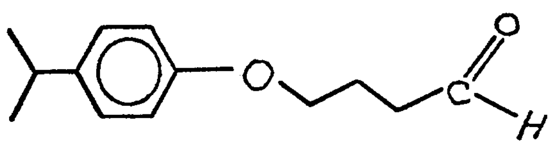

The peak indicated by reference numeral "10" is the peak for the compound having the structure:

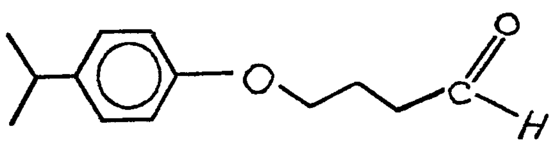

Figure 3 is the GLC profile for fraction 4 of the distillation product of the reaction product of Example II containing the compound having the structure:

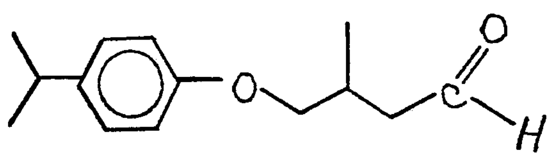

The peak indicated by reference numeral "30" is the peak for the compound having the structure:

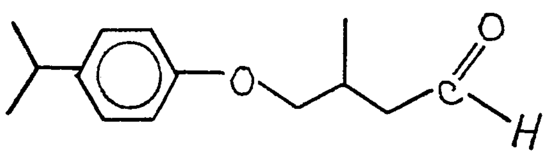

Figure 18 is the GLC profile for fraction 11 of the distillation product of the reaction product of Example VII containing the compound having the structure:

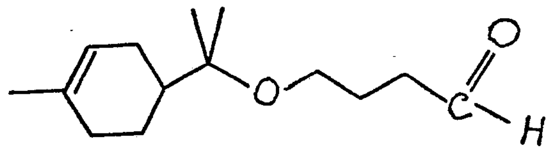

The peak indicated by reference numeral "180" is the peak for the compound having the structure:

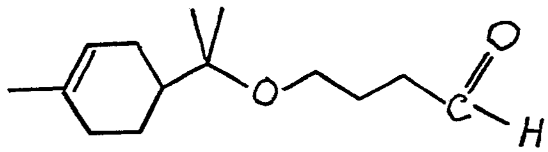

Figure 24 is the GLC profile for the crude reaction product of Example XXXII containing the compounds having the structures:

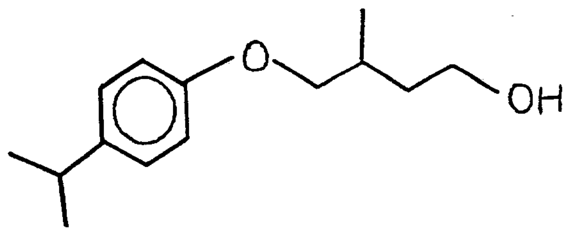

and

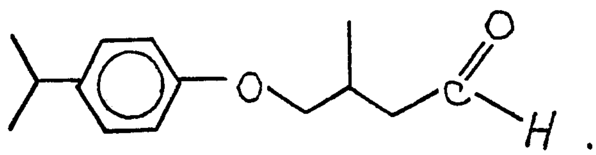

The peak indicated by Reference numeral 130 is the peak for the compound having the structure:

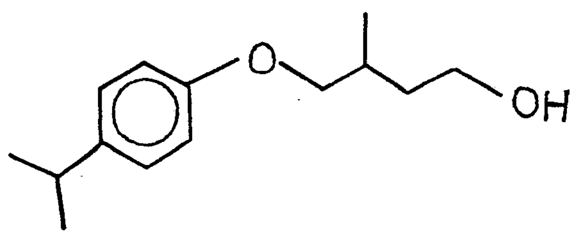

The peak indicated by Reference 131 is the peak for the compound having the structure:

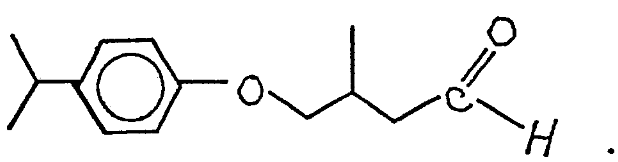

Figure 24 is the GLC profile for fraction 5 of the distillation product of the reaction product of Example XXXII containing the compound having the structure:

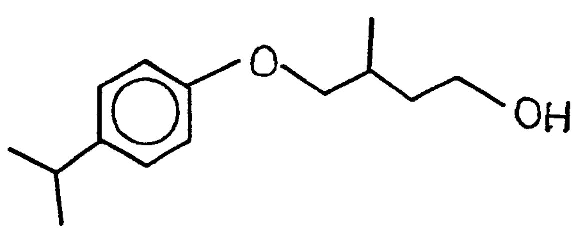

The peak indicated by reference numeral 150 is the peak for the compound having the structure:

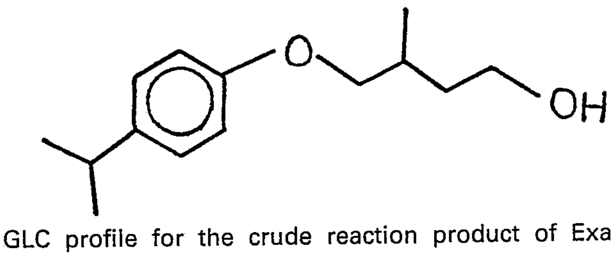

.

Figure 26 is the GLC profile for the crude reaction product of Example XXXIII containing the compounds having the structures:

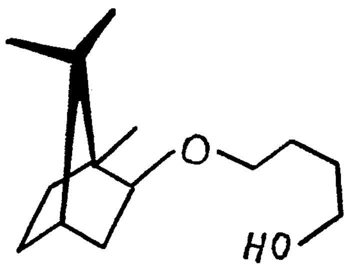

;

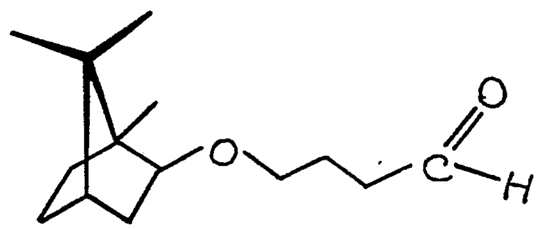

;

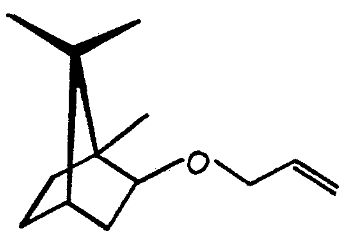

; and

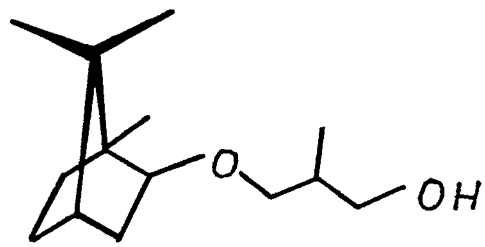

.

The peak indicated by Reference 179 is the peak for the compound having the structure:

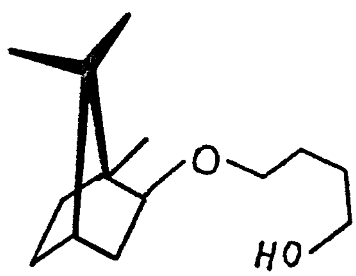

The peak indicated by Reference 181 is the peak for the compound having the structure:

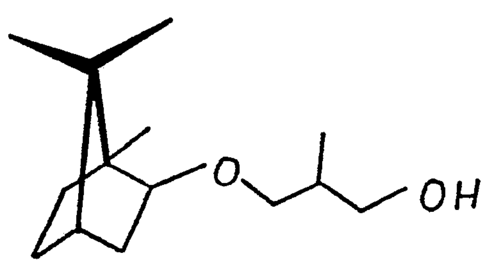

The peak indicated by Reference 182 is the peak for the compound having the structure:

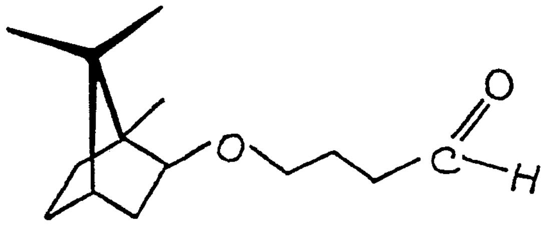

The peak indicated by Reference 183 is the peak for the compound having the structure:

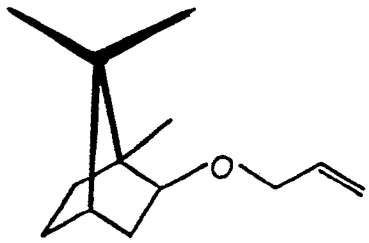

Figure 27 is the GLC profile for fraction 2 of the distillation product of the reaction product of Example XXXIII containing the compound having the structure:

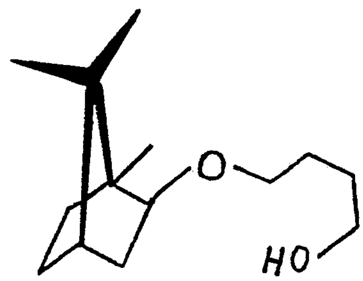

as well as the compound having the structure:

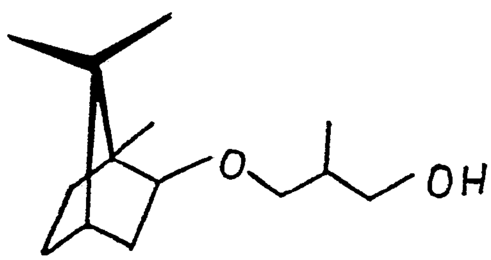

The peak indicated by Reference 191 is the peak for the compound having the structure:

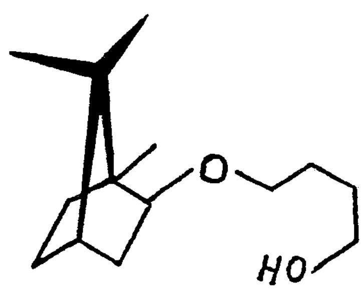

The peak indicated by Reference 192 is the peak for the compound having the structure:

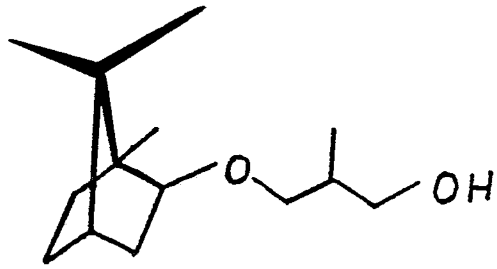

Referring to Figures 42 and 42, there is provided a process for forming scented polymer elements (wherein the polymer may be a thermoplastic polymer such as low density polyethylene or polypropylene or copolymers of ethylene and vinyl acetate or mixtures of polymers and copolymers such as copolymers of ethylene and vinyl acetate and polyethylene) such as pellets useful in the formation of plastic particles useful in fabricating certain articles which may be perfumed. This process comprises heating the polymer or mixture of polymers to the melting point of said polymer or mixture of polymers, e.g., 250°C in the case of low density polyethylene. The lower most portion of the container is maintained at a slightly lower temperature and the material in the container is taken off at such location for delivery through the conduit. Thus, referring to Figures 23 and 24 in particular, the apparatus used in producing such elements comprises a device for forming the polymer containing perfume, e.g., polyethylene or polyethylene-polyvinyl acetate or mixtures of same or polypropylene, which comprises a vat or container *212* into which the polymer taken alone or in admixture with other copolymers and the perfuming substance which is at least one of the ether carbinols or ether carboxaldehydes of our invention or mixtures of ether carbinols and ether carboxaldehydes and other compatible perfumes is placed. The container is closed by means of an air-tight lid *228* and clamped to the container by bolts *265.* A stirrer *273* traverses the lid or cover *228* in an air-tight manner and is rotatable in a suitable manner. A surrounding cylinder *212A* having heated coils which are supplied with electric current through cable *214* from a rheostat or control *216* is operated to maintain the temperature inside the container *212* such that the polymer in the container will be maintained in the molten or liquid state. It has been found advantageous to employ polymers at such a temperature that the viscosity will be in the range of 90—100 Sayboldt seconds. The heater *218* is operated to maintain the upper portion of the container *212* within a temperature range of, for example, 220°—270°C in the case of low density polyethylene. The bottom portion of the container *212* is heated by means of heating coils *212A* regulated through the control *220* connected thereto through a connecting wire *222* to maintain the lower portion of the container *212* within a temperature range of 220°—270°C.

Thus, the polymer or mixture of polymers added to the container *212* is heated from 10—12 hours, whereafter the perfume composition or perfume material which contains one or more of the ether carbinols and/or ether carboxaldehydes of our invention is quickly added to the melt. Generally, about 10—45 percent by weight of the resulting mixture of the perfumery substance is added to the polymer.

After the perfume material is added to the container *212*, the mixture is stirred for a few minutes, for example, 5—15 minutes and maintained within the temperature ranges indicated previously by the heating coil *212A.* The controls *216* and *220* are connected through cables *224* and *226* to a suitable supply of electric current for supplying the power for heating purposes.

Thereafter, the valve "V" is opened permitting the mass to flow outwardly through conduit *232* having a multiplicity of orifices *234* adjacent to the lower side thereof. The outer end of the conduit *232* is closed so that the liquid polymer in intimate admixture with one or more of the ether carbinols and/or ether carboxaldehydes of our invention or mixture of perfume substances and one or more of the ether carbinols and/or ether carboxaldehydes of our invention, will continuously drop through the orifices *234* downwardly from the conduit *232.* During this time, the temperature of the polymer intimately admixed with the perfumery substance in the container *212* is accurately controlled so that a temperature in the range of from about 240°—250°C, for eample, (in the case of low density polyethylene) will exist in the conduit *232.* The regulation of the temperature through the controls *216* and *220* is essential in order to insure temperature balance to provide for the continuous dripping or dropping of molten polymer intimately admixed with the perfume substance which is all of or which contains one or more of the ether carbinols and/or ether carboxaldehydes of our invention, through the orifices *234* at a rate which will insure the formation of droplets *236* which will fall downwardly onto a moving conveyor belt *238* caused to run between conveyor wheels *240* and *242* beneath the conduit *232.*

When the droplets *236* fall onto the conveyor *238*, they form pellets *244* which harden almost instantaneously and fall off the end of the conveyor *238* into a container *250* which is advantageously filled with water or some other suitable cooling liquid to insure the rapid cooling of each of the pellets *244.* The pellets *244* are then collected from the container *250* and utilized for the formation of other functional products, e.g., garbage bags and the like.

Example I

Preparation of 4-(p-cumenyloxy) butyraldehyde

Reaction:

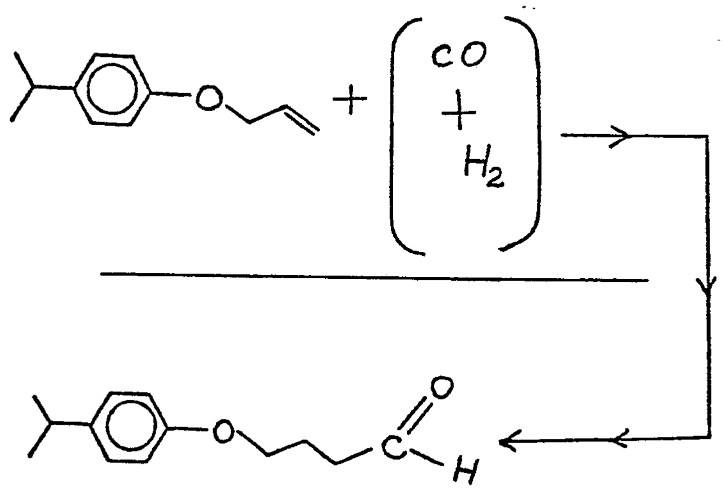

Into a 500 cc autoclave are placed the following materials:
176 grams (1 mole) of the ether having the structure:

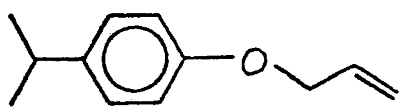

0.2 grams of rhodium carbonyl-di(triphenyl phosphine) chloride
50 ml — Toluene

The autoclave is sealed and heated to 160°C at a pressure of between 500 and 1,000 psig (using a 50:50 mole:mole mixture of carbon monoxide and hydrogen) and maintained at that pressure and temperature for a period of 12 hours.

At the end of the 12 hour period, the autoclave is cooled down and opened and the reaction mass is filtered. The resulting liquid is distilled on a 2" splash column yielding the following fractions:

| Fraction No. | Vapor Temp. (°C) | Liquid Temp. (°C) | Vacuum mm/Hg. Pressure | Weight of Fraction |
|---|---|---|---|---|
| 1 | /35 | /125 | 12:0 | 15.9 |
| 2 | 100 | 125 | 9:0 | 1.1 |
| 3 | 103 | 126 | 9:0 | 13.8 |
| 4 | 101 | 135 | 6:0 | 28.9 |
| 5 | 112 | 145 | 6:0 | 19.6 |
| 6 | 143 | 162 | 5:5 | 20.8 |
| 7 | 159 | 227 | 6:5 | 35.2 |

Fractions 2—6 are then bulked and redistilled on a spinning band column yielding the following fractions:

| Fraction No. | Vapor Temp. (°C) | Liquid Temp. (°C) | Vacuum mm/Hg. Pressure |
|---|---|---|---|
| 1 | /62 | /134 | 1:3 |
| 2 | 64 | 145 | 1:2 |
| 3 | 132 | 152 | 1:2 |
| 4 | 181 | 161 | 1:2 |
| 5 | 185 | 225 | 1:2 |

Figure 1 is the GLC profile for the crude reaction product containing the compound having the structure:

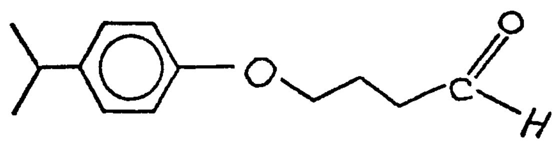

The peak indicated by reference numeral "10" on Figure 1 is the peak for the compound having the structure:

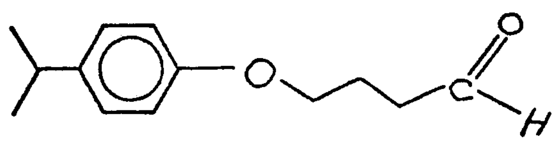

Figure 2 is the NMR spectrum for the compound having the structure:

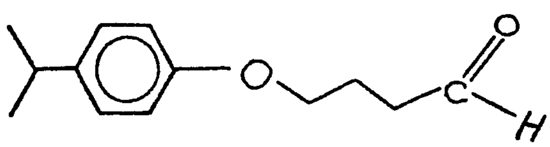

(conditions: Field strength 100 MHz; solvent: $CFCl_3$).

Example II

Preparation of 4-(p-cumenyloxy)-3-methylbutyraldehyde

Reaction:

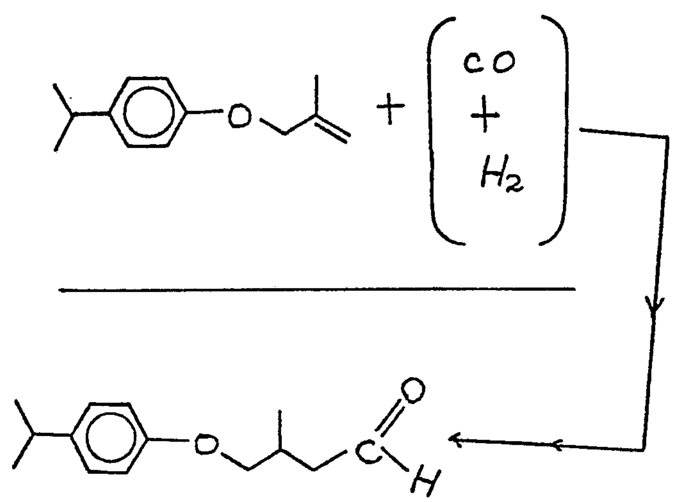

Into a 500 cc autoclave is placed:

200 grams of the compound having the structure:

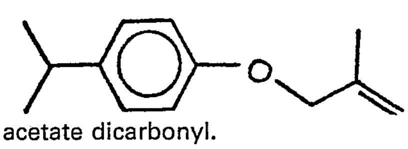

0.1 gram of Rhodium aceto acetate dicarbonyl.

The autoclave is sealed and pressurized to 1,000 psig at 120°C using a 50:50 mole:mole mixture of carbon monoxide and hydrogen and maintained at that pressure and temperature for a period of 14.5 hours. GLC analysis indicates 83% product formed.

The reaction mass is cooled and the autoclave is opened. The contents are filtered and the liquid material is distilled on a 2" splash column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 97/103 | 133/140 | 3:5 | 18.3 |
| 2 | 104 | 140 | 3:0 | 27.8 |
| 3 | 134 | 158 | 2:4 | 47.1 |
| 4 | 140 | 180 | 4:0 | 39.5 |

Fractions 2, 3 and 4 are bulked and redistilled on a micro Vigreux column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 65/ | 142/ | | 2.9 |
| 2 | 75 | 145 | 3:4 | 3.2 |
| 3 | 73 | 148 | 3:4 | 3.0 |
| 4 | 70 | 150 | 3:4 | 2.3 |
| 5 | 98 | 155 | 3:4 | 7.6 |
| 6 | 95 | 157 | 3:4 | 7.6 |
| 7 | 80 | 164 | 3:6 | 11.5 |
| 8 | 94 | 172 | 3:6 | 1.5 |
| 9 | 104 | 193 | 3:6 | 3.1 |
| 10 | 100 | 200 | 3:6 | 5.7 |
| 11 | 80 | 220 | 3:6 | 3.2 |

Figure 3 is the GLC profile for Fraction 4 of the foregoing distillation.

The peak indicated by reference numeral "30" is the peak for the compound having the structure:

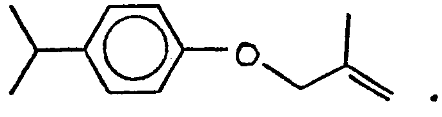

Figure 4 is the NMR spectrum for the peak indicated by reference numeral "30" on Figure 3 for the compound having the structure:

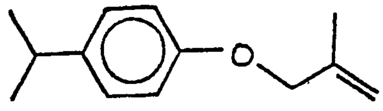

(Conditions: $CFCl_3$ solvent; Field strength: 100 MHz).

Example III

Preparation of 4-(2-bornyloxy)butyraldehyde

Reaction:

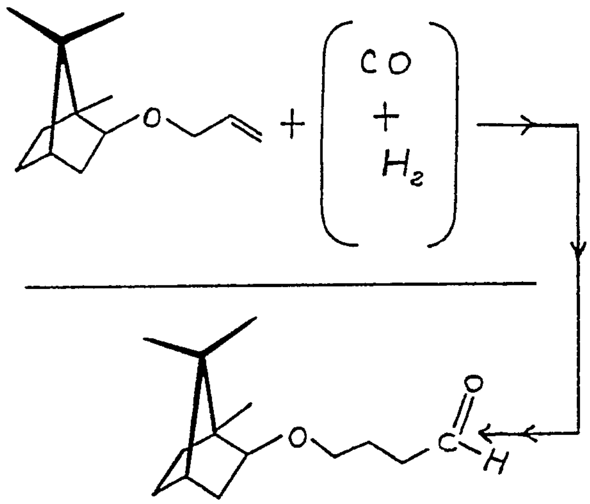

Into a 500 ml autoclave are placed the following ingredients:

Isobornyl allyl ether having the structure:

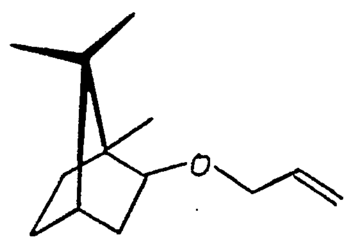

194 grams (1 mole)

Triphenylphosphine 2.0 grams

Rhodium Chloride-mono-carbonyltriphenylphosphine — 0.2 grams

Toluene — 50 ml

The autoclave is sealed and pressurized to 500 psig using a 50:50 mole:mole mixture of carbon monoxide and hydrogen. The autoclave is heated to 200°C and maintained at 500—700 psig for a period of 13 hours. At the end of the 13 hour period, the autoclave is cooled and opened yielding 462.9 grams of product (including toluene).

The reaction product is then distilled through a 12" Goodloe column yielding the following fractions:

| Fraction No. | Vapor Temp. (°C) | Liquid Temp. (°C) | Vacuum mm/Hg. Pressure | Wgt. of Fraction |
|---|---|---|---|---|
| 1 | 119/ | 165/ | 4:0 | 13.9 |
| 2 | 125 | 175 | 3:0 | 16.1 |
| 3 | 156 | 244 | 3:0 | 17.7 |
| 4 | 195 | 284 | 3:0 | 13.0 |

Fractions 2, 3 and 4 are bulked and redistilled through a spinning band column yielding the following fractions:

| Fraction No. | Vapor Temp. (°C) | Liquid Temp. (°C) | Vacuum mm/Hg. Pressure | Wgt. of Fraction |
|---|---|---|---|---|
| 1 | /94 | /134 | 2:8 | 9.7 |
| 2 | 95 | 140 | 2:6 | 8.7 |
| 3 | 62 | 147 | 2:6 | 3.5 |
| 4 | 102 | 163 | 2:4 | 2.8 |
| 5 | 114 | 178 | 2:4 | 3.3 |
| 6 | 70 | 194 | 2:8 | 5.9 |
| 7 | 64 | 240 | 2:9 | 4.4 |

The resulting product contains 70 percent by weight of the compound having the structure:

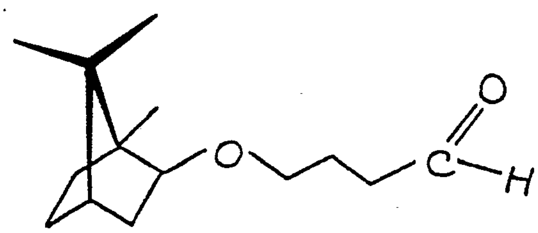

and 30 percent by weight of the compound having the structure:

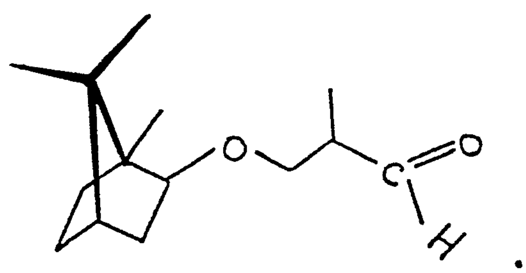

.

Figure 5 is the GLC profile of bulked fractions 2—4 of the first distillation. This material contains a mixture of compounds having the structures:

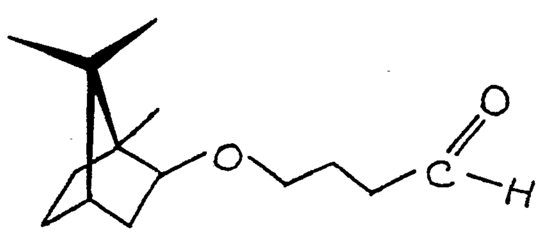

and

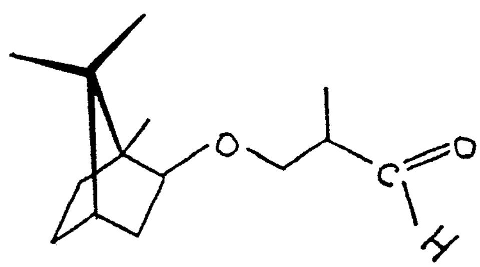

.

Figure 6 is the GLC profile for fraction 5 of the spinning band distillation and contains the compounds having the structures:

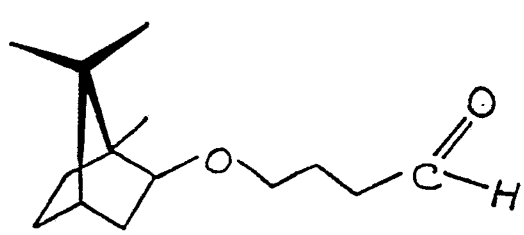

and

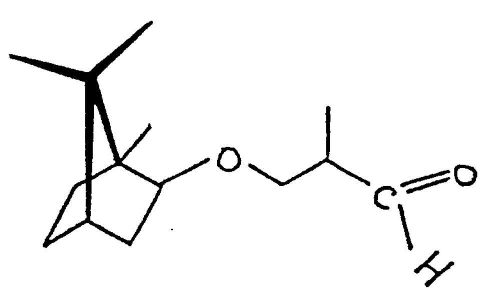

Figure 7 is the NMR spectrum for the mixture of compounds containing 70 percent by weight of the compound having the structure:

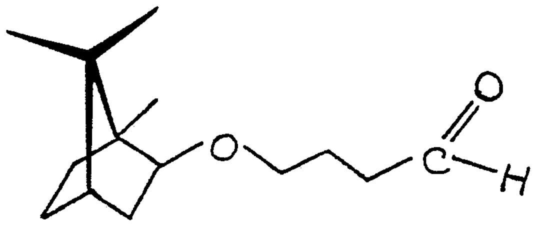

and 30 percent by weight of the compound having the structure:

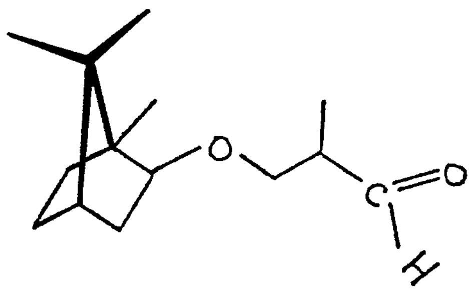

(conditions: $CFCl_3$ solvent; 100 MHz field strength).

Example IV

Preparation of 3-methyl-4-isobornyloxybutyraldehyde

Reaction:

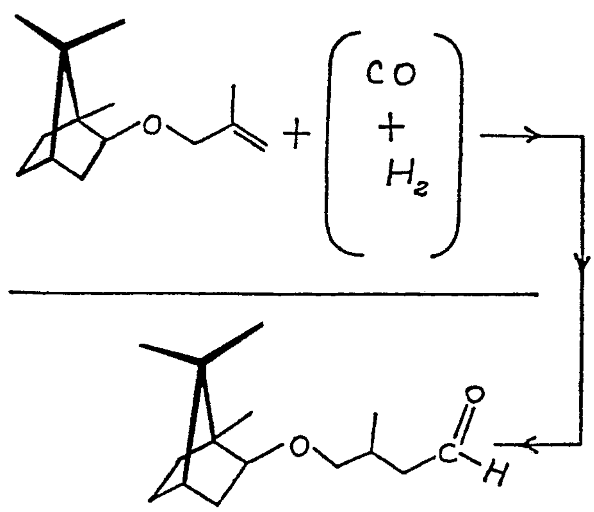

Into a 500 cc autoclave is placed the following materials:

(i) Isobornyl methallyl ether having the structure:

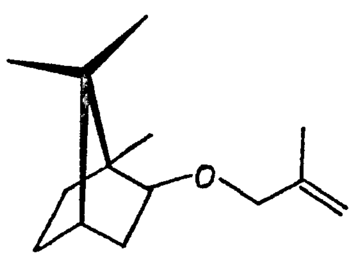

271 grams (77% pure);

(ii) Rhodium dicarbonyl acetoacetate 0.08 grams. The autoclave is sealed, heated to 100—140°C and pressurized to 1,000 psig using a 50:50 (mole:mole) mixture of carbon monoxide and hydrogen and maintained at that pressure and temperature for a period of seven hours.

At the end of the seven hour period, the autoclave contents are cooled and the autoclave is opened and the contents are filtered.

The reaction mass is then distilled on a 2" splash column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 110/ | 135/ | 10:2 | - |
| 2 | 118 | 140 | 14:0 | 12.0 |
| 3 | 123 | 142 | 4:4 | 39.1 |
| 4 | 142 | 206 | 5:0 | 35.0 |
| 5 | 173 | 250 | 5:6 | 26.8 |

Fractions 3, 4 and 5 are bulked and redistilled on a spinning band distillation column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 80/ | 120/ | 1:5 | 12.5 |
| 2 | 68 | 125 | 1:5 | 7.7 |
| 3 | 68 | 139 | 1:4 | 6.0 |
| 4 | 95 | 137 | 1:3 | 8.9 |
| 5 | 95 | 146 | 1:3 | 9.0 |
| 6 | 96 | 141 | 1:3 | 9.0 |
| 7 | 98 | 143 | 1:4 | 8.5 |
| 8 | 99 | 145 | 1:4 | 10.0 |
| 9 | 100 | 152 | 2:0 | 10.5 |
| 10 | 102 | 166 | 1:4 | 10.1 |
| 11 | 100 | 195 | 1:5 | 8.9 |
| 12 | 104 | 225 | 1:5 | 6.9 |

Figure 8 is the GLC profile for the crude reaction product of this example prior to the distillation containing the compound having the structure:

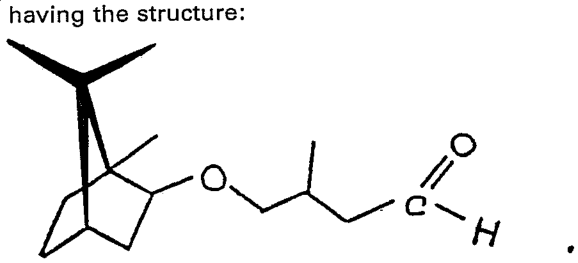

Figure 9 is the GLC profile for fraction 6 of the spinning band distillation containing the compound having the structure:

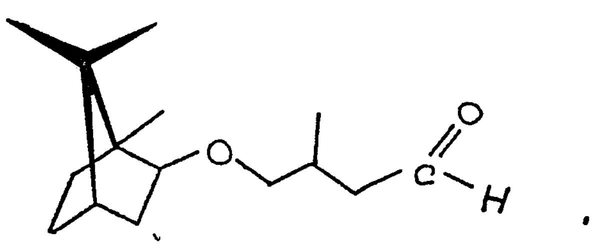

Figure 10 is the NMR spectrum for fraction 6 of the spinning band distillation, for the compound having the structure:

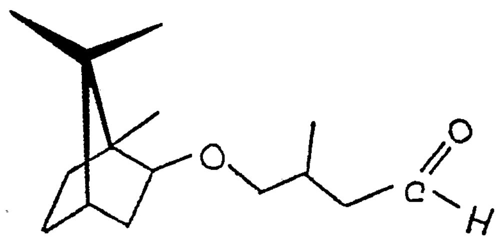

(Conditions: Solvent: $CFCl_3$; Field strength: 100 MHz).

Example V

Preparation of 4-(3,7-dimethyl-6-octenyloxy)-3-methylbutyraldehyde

Reaction:

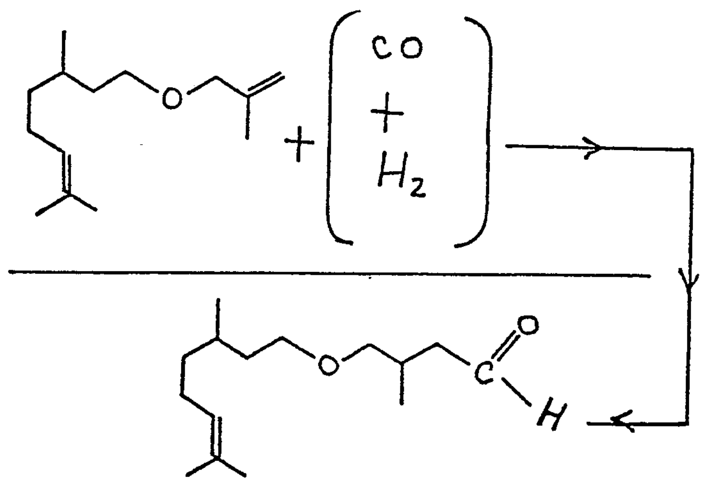

Into a 500 cc autoclave is placed the following materials:

(i) Citronellyl metallyl ether — 305 grams (1.5 moles) (having the structure:

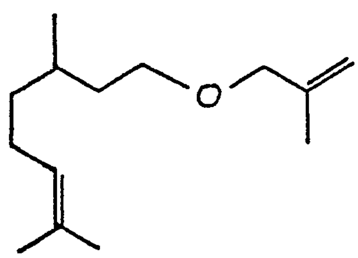

)

(ii) Triphenyl phosphine — 4 grams

(iii) Rhodium mono-carbonyl(triphenyl phosphine) chloride — 0.4 grams

(iv) Toluene — 50 ml

The autoclave is sealed and the contents heated to 140—200°C at a pressure of 1,000 psig using a 50:50 mole:mole ratio of carbon monoxide and hydrogen to pressurize same. The autoclave is maintained at that temperature and pressure for a period of seven hours. At the end of the seven hour period, the autoclave is cooled, opened and the contents filtered. The resulting product is distilled on a 2" splash column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 29/39 | 52/104 | 1:7/1:7 | 28.1 |
| 2 | 104 | 130 | 2:5 | 12.2 |
| 3 | 138 | 153 | 1:4 | 30.2 |
| 4 | 155 | 170 | 2:2 | 39.2 |
| 5 | 150 | 65 | 8:0 | 22.5 |
| 6 | 135 | 176 | 4:8 | 46.8 |
| 7 | 148 | 205 | 4:8 | 29.2 |
| 8 | 140 | 221 | 4.0 | 5.6 |

Fractions 2—9 are then bulked and redistilled on a spinning band column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 46/54 | 124/117 | 1:3/1:3 | 2.3 |
| 2 | 64 | 117 | 1:2 | 5.0 |
| 3 | 68 | 118 | 1:2 | 5.9 |
| 4 | 69 | 130 | 1:2 | 7.9 |
| 5 | 70 | 132 | 1:2 | 9.6 |
| 6 | 72 | 133 | 1:2 | 8.5 |
| 7 | 73 | 138 | 1:2 | 9.0 |
| 8 | 78 | 140 | 1:2 | 10.4 |
| 9 | 88 | 141 | 1:2 | 9.4 |
| 10 | 98 | 136 | 1:1 | 5.7 |
| 11 | 100 | 137 | 1:1 | 6.0 |
| 12 | 100 | 138 | 1:1 | 11.7 |
| 13 | 102 | 138 | 1:1 | 8.1 |
| 14 | 102 | 142 | 1:1 | 10.1 |
| 15 | 102 | 142 | 1:1 | 10.3 |
| 16 | 107 | 144 | 1:1 | 10.8 |
| 17 | 102 | 146 | 1:1 | 9.1 |
| 18 | 66 | 146 | 1:1 | 5.7 |
| 19 | 104 | 171 | 1:0 | 7.1 |
| 20 | 96 | 196 | 1:1 | 7.4 |
| 21 | 60 | 220 | 1:0 | 2.8 |

Figure 11 is the GLC profile for fraction 4 of the first distillation set forth above of the reaction product of this example containing the compound having the structure:

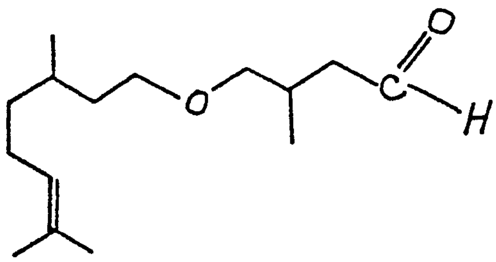

Figure 12 is the GLC profile for fraction 19 of the final distillation and fraction 19 consists of the compound having the structure:

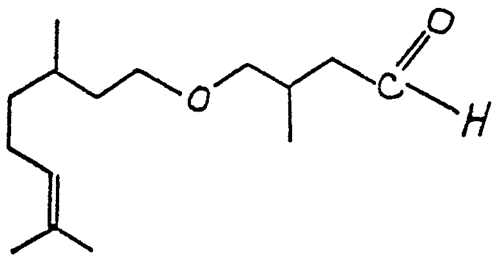

Figure 13 is the NMR spectrum for fraction 19 of the second distillation (spinning band column distillation) and consists of the compound having the structure:

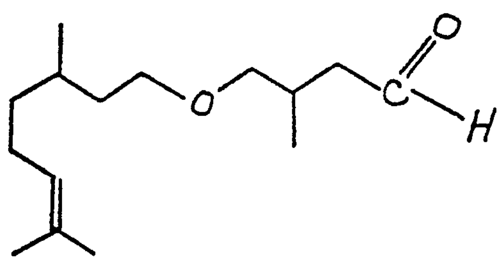

(conditions: Field strength: 100 MHz; solvent: $CFCl_3$).

Example VI

Preparation of 4-hydroxy-4-methyl-2-pentyloxy-butyraldehyde

Reaction:

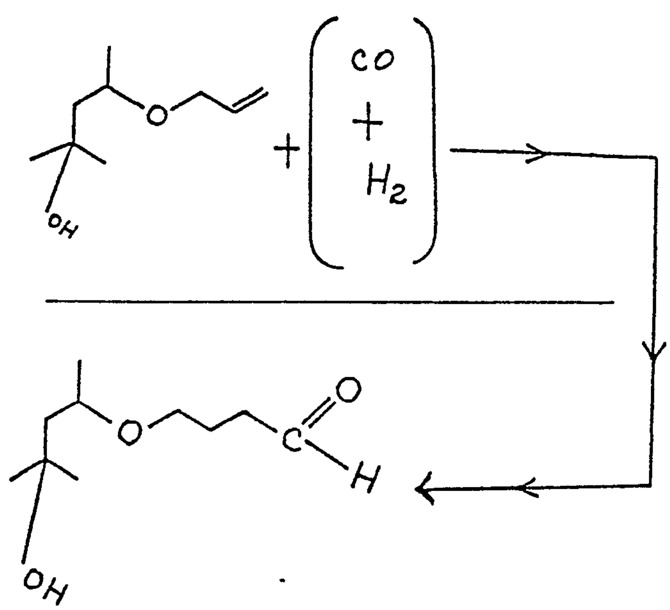

Into a 500 cc autoclave is placed the following materials:

(i) 4-allyloxy-2-methyl-2-pentanol (having the structure:

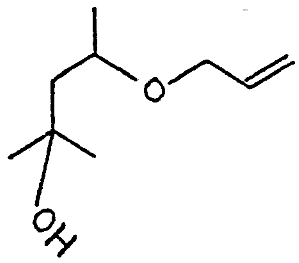

)

185 grams;

(ii) Rhodium-mono-carbonyl-di(triphenylphosphine) chloride — 0.2 grams

(iii) Triphenylphosphine — 2 grams

(iv) Toluene — 50 ml

The autoclave is sealed and pressurized with a 50:50 mole:mole mixture of carbon monoxide and hydrogen to 500 psig and heated to 140°C. The temperature of 140°C and pressure of 500 psig is maintained in the autoclave for a period of fourteen hours. At the end of the fourteen hour period, the autoclave is cooled and opened and the contents are filtered. The resulting product is then distilled on a spinning band column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 53/ | 98/ | 6:0 | 13.5 |
| 2 | 60 | 90 | 5:5 | 7.6 |
| 3 | 58 | 92 | 4:8 | 9.28 |
| 4 | 58 | 96 | 4:8 | 10.0 |
| 5 | 78 | 101 | 4:8 | 15.1 |
| 6 | 74 | 117 | 5:0 | 12.2 |
| 7 | 76 | 131 | 4:8 | 4.0 |
| 8 | 80 | 190 | 1:2 | 5.8 |
| 9 | 80 | 213 | 4:0 | 6.9 |
| 10 | 65 | 218 | 4:5 | 5.5 |

Figure 14 is the GLC profile for the crude reaction product of this example.

Figure 15 is the GLC profile for fraction 9 of the foregoing distillation.

Figure 16 is the NMR spectrum for fraction 9 of the foregoing distillation (conditions: Solvent: $CFCl_3$; Field strength: 100 MHz).

Example VII

Preparation of terpinyloxybutanol

Reaction:

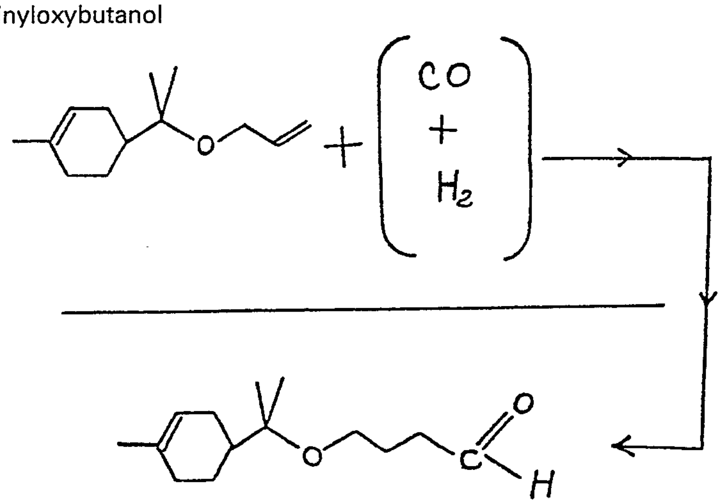

Into a 500 cc autoclave is placed the following ingredients:
(i) Terpinyl allyl ether (having the structure:

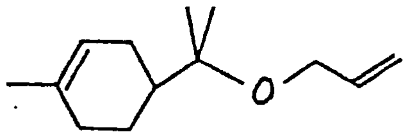

)

135 grams
(ii) Rhodium chloride-mono-carbonyl-di(triphenylphosgene) — 0.1 grams
(iii) Triphenylphosphene — 2 grams
(iv) Toluene — 50 ml

The autoclave is sealed and pressurized to 500 psig using a 50:50 mole:mole mixture of carbon monoxide and hydrogen and the temperature of the autoclave is raised to 140°C. The autoclave is maintained at 500 psig and 140°C for a period of twelve hours using the said carbon monoxide:hydrogen mixture.

At the end of the twelve hour period, the autoclave is cooled and the contents are removed and filtered. The resulting liquid is then distilled on a 1" splash column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 32/ | 130/ | 8:0 | 14.5 |
| 2 | 120 | 150 | 14:0 | 9.0 |
| 3 | 138 | 162 | 13:0 | 18.9 |
| 4 | 110 | 155 | 7:0 | 28.2 |
| 5 | 149 | 172 | 12:0 | 27.9 |
| 6 | 157 | 290 | 12:0 | 13.8 |

Fractions 2—6 of this first distillation are bulked and redistilled on a spinning band column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 40/54 | 100/106 | 3:2 | 1.3 |
| 2 | 74 | 115 | 3:4 | 7.6 |
| 3 | 80 | 117 | 4:4 | 3.6 |
| 4 | 84 | 120 | 4:6 | 4.8 |
| 5 | 90 | 123 | 4:4 | 4.0 |
| 6 | 90 | 125 | 4:8 | 3.4 |
| 7 | 98 | 128 | 5:0 | 4.2 |
| 8 | 84 | 135 | 5:0 | 4.4 |
| 9 | 110 | 142 | 5:0 | 5.6 |
| 10 | 110 | 145 | 5.0 | 6.3 |
| 11 | 110 | 154 | 5:0 | 6.9 |
| 12 | 110 | 180 | 4:8 | 4.7 |

Figure 17 is the GLC profile for the crude reaction product prior to distillation containing the compound having the structure:

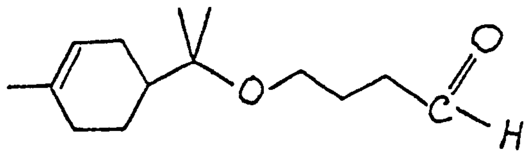

Figure 18 is the GLC profile for fraction 11 of the foregoing distillation containing the compound having the structure:

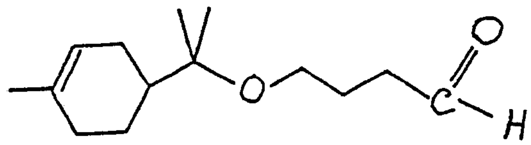

The peak indicated by reference numeral "180" on the GLC profile of Figure 18 is the peak for the compound having the structure:

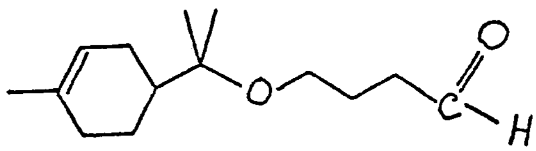

Figure 19 is the NMR spectrum for peak 180 of Figure 18 (Fraction 11 of the foregoing distillation) which is the peak for the compound having the structure:

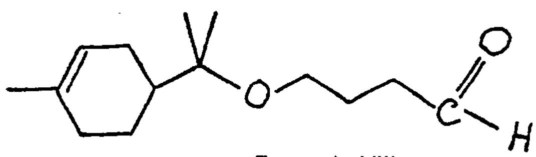

Example VIII

Preparation of 8-methoxy-p-methane-2-carboxaldehyde

Reaction:

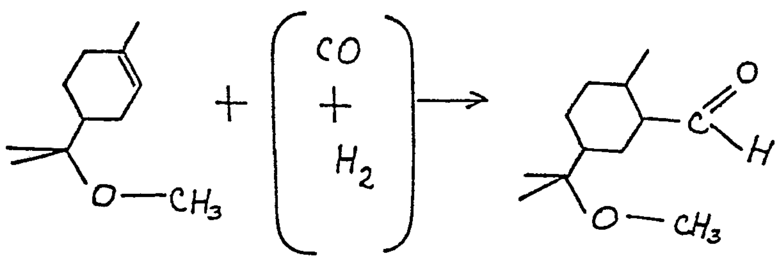

Into a 1000 cc autoclave is placed the following ingredients:

(i) "Orange flower ether: (having the structure:

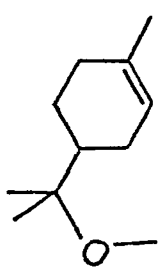

504 grams (3 moles);

(ii) Rhodium acetoacetate-dicarbonyl — 0.1 grams

The autoclave is sealed and pressurized to 1,000 psig using a 50:50 mole:mole mixture of carbon monoxide and hydrogen and heated to a temperature of 160°C. The autoclave contents are maintained at 1,000 psig and 60°C for a period of seven hours.

At the end of the seven hour period, the autoclave contents are cooled, the autoclave is opened and the contents are removed and filtered. The resulting liquid filtrate is then distilled on a 2" splash column yielding the following fractions:

| Fraction No. | Vapor Temp. (°C) | Liquid Temp. (°C) | Vacuum mm/Hg. Pressure |
|---|---|---|---|
| 1 | 82/ | 102/ | 10 |
| 2 | 93 | 113 | 18 |
| 3 | 98 | 118 | 17 |
| 4 | 114 | 128 | 14 |
| 5 | 132 | 142 | 13 |
| 6 | 142 | 151 | 9 |
| 7 | 143 | 160 | 6 |
| 8 | 168 | 220 | 6 |

Fractions 5—7 of this distillation product are bulked and redistilled on a spinning band column yielding the following fractions:

| Fraction No. | Vapor Temp. (°C) | Liquid Temp. (°C) | Vacuum mm/Hg. Pressure | Weight of Fraction |
|---|---|---|---|---|
| 1 | 50/59 | 102/ | 9:3 | 4.3 |
| 2 | 70 | 120 | 8:7 | 13.7 |
| 3 | 87 | 116 | 5:4 | 11.6 |
| 4 | 84 | 117 | 2:5 | 20.0 |
| 5 | 84 | 116 | 2:5 | 17.4 |
| 6 | 84 | 116 | 2:5 | 15.9 |
| 7 | 90 | 103 | 2:6 | 10.7 |
| 8 | 91 | 125 | 2:6 | 17.4 |
| 9 | 94 | 127 | 2:6 | 35.2 |
| 10 | 108 | 134 | 2:8 | 32.0 |
| 11 | 120 | 161 | 2:8 | 31.9 |
| 12 | 43 | 200 | 2:8 | 4.1 |

Figure 20 is the GLC profile for the crude reaction product of this example containing the compound having the structure:

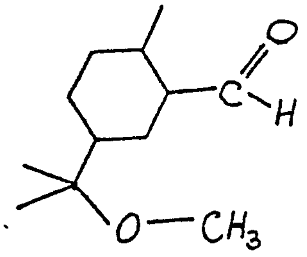

Figure 21 is the GLC profile of fraction 8 of the foregoing spinning band distillation containing the compound having the structure:

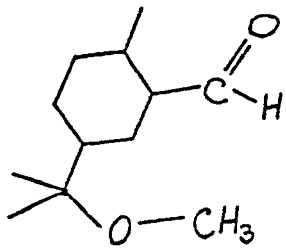

Figure 22 is the NMR spectrum for the compound having the structure:

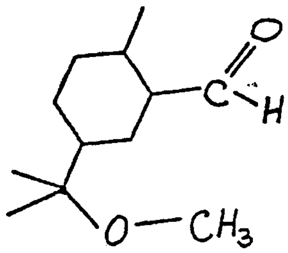

(Conditions: Field strength: 100 MHz; solvent: $CFCl_3$).

Example IX

Pine fragrance

The following pine fragrance formulations are prepared:

| | Parts By Weight | | |
|---|---|---|---|
| Ingredients | IX—A | IX—B | IX—C |
| Isobornyl acetate | 100 | 100 | 100 |
| Camphor | 10 | 10 | 10 |
| Terpineol | 25 | 25 | 25 |
| Fir balsam absolute (50% in diethyl phthalate) | 20 | 20 | 20 |
| Coumarin | 4 | 4 | 4 |
| Linalool | 30 | 30 | 30 |
| Frenchyl alcohol | 10 | 10 | 10 |
| Anethol | 12 | 12 | 12 |
| Lemon terpenes washed | 50 | 50 | 50 |
| Borneol | 5 | 5 | 5 |
| Galbanum oil | 5 | 5 | 5 |
| Turpentine Russian | 150 | 150 | 150 |
| Eucalyptol | 50 | 50 | 50 |
| 2,2,6-trimethyl-1-cyclohexene-1-carboxaldehyde | 12 | 12 | 12 |
| Maltol (1% in diethyl phthalate) | 5 | 5 | 5 |

| | Parts by Weight | | |
|---|---|---|---|
| Ingredients | IX-A | IX-B | IX-C |
| Compound having the structure: 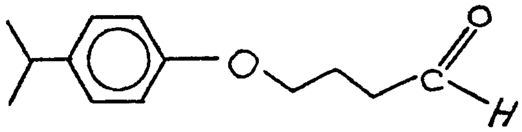 prepared according to Example I | 28 | 0 | 0 |
| Compound having the structure: 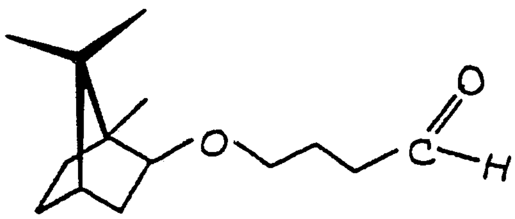 prepared according to Example III | 0 | 28 | 0 |
| Compound having the structure: 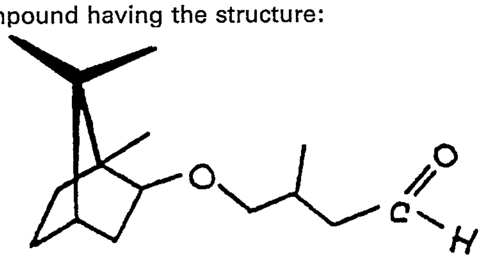 prepared according to Example IV | 0 | 0 | 28 |

The compound having the structure:

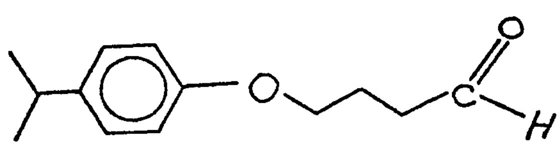

imparts to the pine formulation an intense diffusive saw dust nuance. Accordingly, the pine formulation can be described as "piney with an intense saw dust-like undertone".

The compound having the structure:

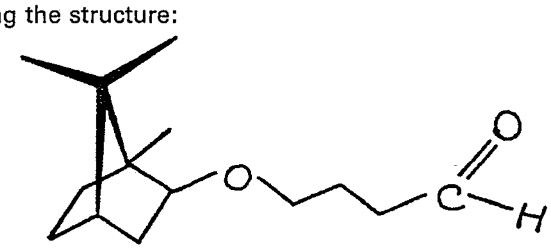

imparts to this piney formulation a very natural-like cypress, fir balsam-like aroma with lavender spiked topnotes. Accordingly, the formulation thus prepared can be described as "natural piney with cypress-like and fir balsam undertones and lavender spiked topnotes".

The compound having the structure:

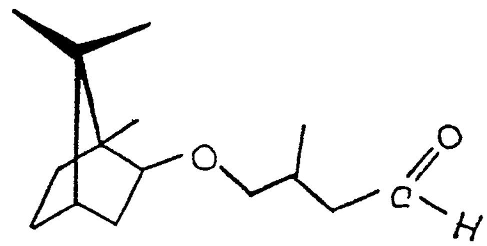

imparts to this piney formulation a patchouli, coriander, mahogany-wood-like character. Accordingly, the formulation can be described from a perfumery standpoint as "piney with patchouli, coriander and mahogany-like and dry cedarwood undertones with faint chocolate topnotes".

Example X

Floral perfume compositions

The compound having the structure:

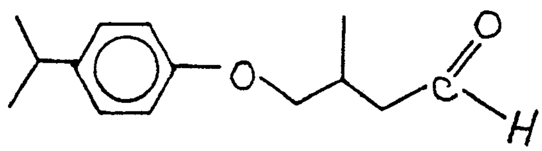

produced according to Example II has a green, spicy, calamus-like aroma. This material has great warmth and richness and blends well many floral concepts. It is a rather unique floral note of great value to perfumery. Its use may be demonstrated by the following floral fragrance whereby the compound having the structure:

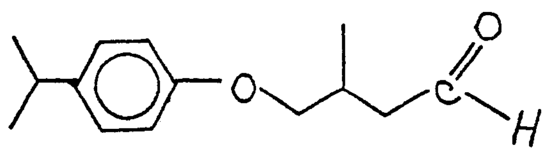

is used to the extent of 5% by weight.

The compound having the structure:

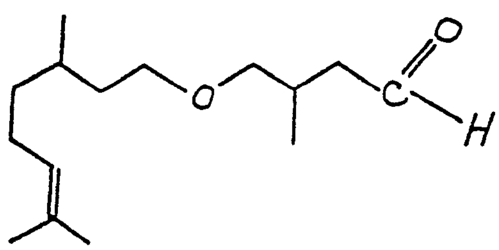

produced according to Example V imparts to this floral fragrance a very "ozoney" nuance causing it to be quite useful in the "fresh air dried-cloves aroma" type fragrance. The addition of 5 percent by weight of the compound having the structure:

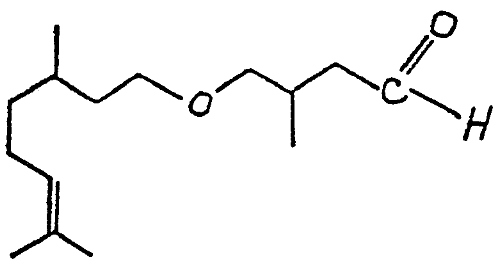

imparts a very desirable fresh air character.

The compound having the structure:

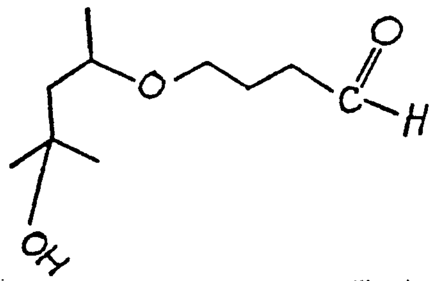

imparts to the floral formulation a spicy (cinnamon), carnation-like, herbaceous and fruity undertone.

All four of these products perform quite well in fragrances and are judged to be very valuable fragrance materials.

Floral Fragrance

| | "A" | "B" | "C" | "D" |
|---|---|---|---|---|
| Citronellol | 12.3 | 12.3 | 12.3 | 5.0 |
| Geraniol | 2.5 | 2.5 | 2.5 | 5.0 |
| Amyl Cinnamic Aldehyde | 24.6 | 24.6 | 24.6 | 5.0 |
| Galaxolide® 50 (Trademark Tricyclic Isochroman of International Flavors & Fragrances Inc.) | 9.8 | 9.8 | 9.8 | 5.0 |
| Vertenex High Cis (Cis-t Butylcyclohexenyl Acetate; Para Isomer) | 7.4 | 7.4 | 7.4 | 5.0 |
| Rose Oxide | 0.7 | 0.7 | 0.7 | 5.0 |
| Cinnamic Alcohol | 19.6 | 19.6 | 19.6 | 5.0 |
| Aldehyde C—11 (n-Undecylenic Aldehyde) | 0.5 | 0.5 | 0.5 | 5.0 |
| Aldehyde C—12 (n-Dodecyl Aldehyde in 10% solution in diethyl phthalate | 0.5 | 0.5 | 0.5 | 5.0 |
| Citronellal (10% solution in diethyl phthalate) | 0.5 | 0.5 | 0.5 | 5.0 |
| Phenyl Ethyl Acetate | 2.5 | 2.5 | 2.5 | 5.0 |
| Ylang Oil | 1.2 | 1.2 | 1.2 | 5.0 |
| Indisan (Hydrogenated derivative of reaction product of Camphene and Resorcinol) | 3.7 | 3.7 | 3.7 | 5.0 |
| Musk Ketone | 5.0 | 5.0 | 5.0 | 5.0 |
| Oakmoss Resin | 0.5 | 0.5 | 0.5 | 5.0 |
| Liatrix Absolute (10% in diethyl phthalate | 2.5 | 2.5 | 2.5 | 5.0 |
| Vetiver Acetate | 1.2 | 1.2 | 1.2 | 5.0 |
| Diethyl Phthalate | 5.0 | 5.0 | 5.0 | 5.0 |

FLORAL FRAGRANCE (Cont'd.)

| | "A" | "B" | "C" | "D" |
|---|---|---|---|---|
| Compound having the structure: 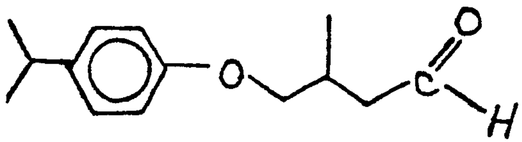 produced according to Example II. | .. 5.0 | 0 | 0 | 0 |
| Compound having the structure: 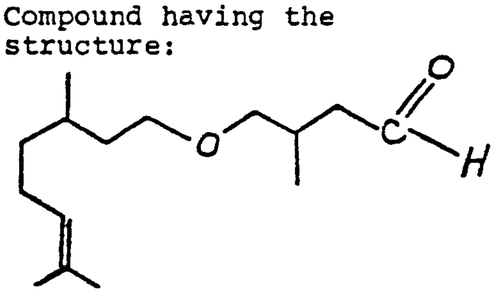 produced according to Example V. | .. 0 | 5.0 | 0 | 0 |
| Compound having the structure: 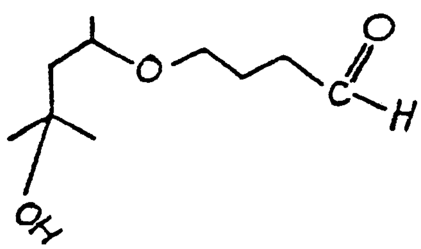 produced according to Example VI. | .. 0 | 0 | 5.0 | 0 |
| Compound having the structure: 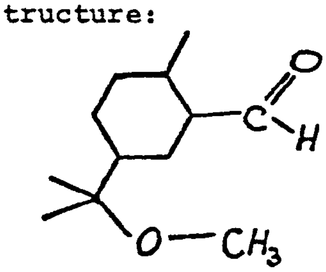 produced according to Example VIII. | .. 0 | 0 | 0 | 5.0 |

The compound having the structure:

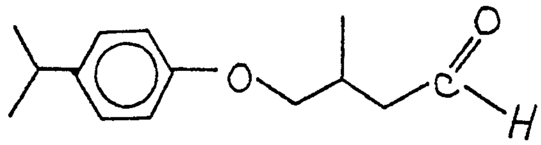

imparts to this floral fragrance green, spicy and calamus-like aroma nuances. Accordingly, the fragrance can be described as "floral with green, spicy and calamus-like undertones".

The compound having the structure:

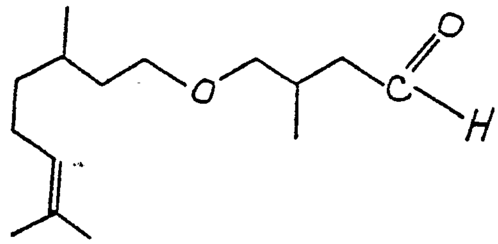

imparts to this floral formulation a very ozoney type nuance. Accordingly, the fragrance thus produced can be described as "floral with ozoney and fresh air dried cloth undertones".

The compound having the structure:

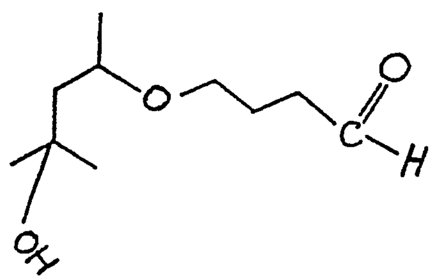

imparts to this floral formulation a cinnamon-like, carnation-like, herbaceous and fruity aroma profile. Accordingly, the fragrance can be described as "floral with spicy (cinnamon), carnation-like, herbaceous and fruity undertones".

The compound having the structure:

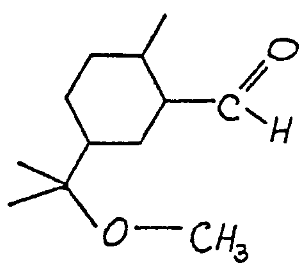

imparts to this floral formulation an intense lilac and green undertone. Accordingly, the fragrance can be described as "floral with lilac and green undertones".

Example XI

Preparation of lilac fragrance

The following mixture is prepared:

| | |
|---|---|
| Hydroxycitronellal | 22% |
| Phenyl Ethyl Alcohol | 12% |
| Heliotropine | 12% |
| Linalool | 8% |
| Cinnamic Alcohol | 4% |
| Indole — 10% in Diethyl Phthalate | 2% |
| Benzyl Acetate | 8% |
| Anisic Alcohol | 8% |
| Coumarin — 10% in Diethyl Phthalate | 4% |
| Compound having the structure: 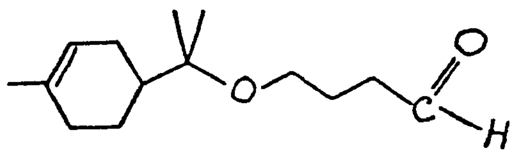 | 4% |

The compound having the structure:

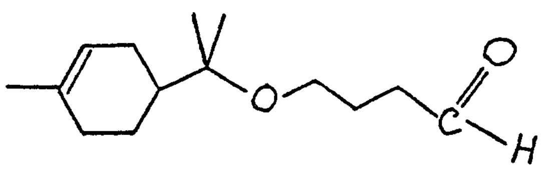

imparts to this lilac fragrance an interesting citrusy (grapefruit-like) undertone. According the fragrance can be described as "lilac with a citrusy, grapefruit-like undertone".

Example XII

Preparation of cosmetic powder compositions

Cosmetic powder compositions are prepared by mixing in a ball mill 100 grams of talcum powder with 0.25 grams of each of the substances set forth in Table III below. Each of the cosmetic powder compositions has an excellent aroma as described in Table III below:

TABLE III

| SUBSTANCE | AROMA DESCRIPTION |
|---|---|
| 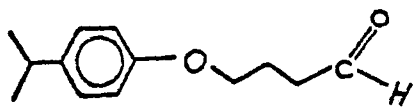 | Diffusive, saw dust-like. |
| 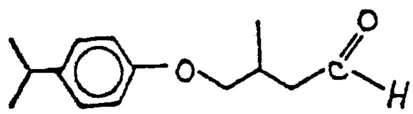 | Green, spicy and calamus-like. |
| 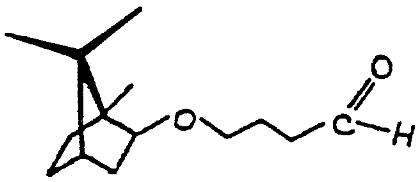 | Natural pine, cypress and fir balsam. |
| 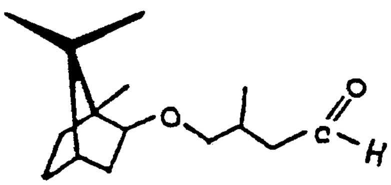 | Patchouli, coriander and woody with a chocolate topnote and dry cedarwood and patchouli undertones. |

TABLE III - (Cont'd.)

| SUBSTANCE | AROMA DESCRIPTION |
|---|---|
| 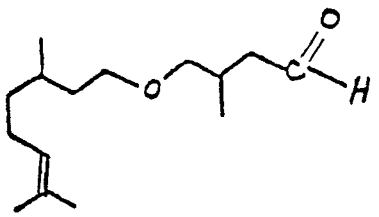 | Floral and ozoney with woody and fruity undertones. |
| 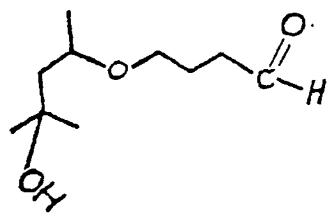 | Spicy (cinnamon), floral (carnation), herbaceous and fruity aroma with caryophyllene-like topnotes. |
| 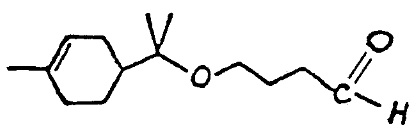 | Citrus (grapefruit)-like aroma. |
| 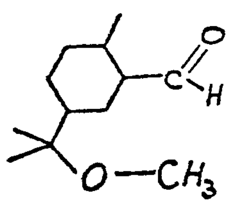 | Green, floral (lilac) aroma. |

TABLE III (Cont'd)

| | |
|---|---|
| Fragrance prepared according to Example IX(A). | Piney with an intense saw dust-like undertone. |
| Fragrance prepared according to Example IX(B). | Natural piney with cypress-like and fir balsam undertones and lavender spike topnotes. |
| Fragrance prepared according to Example IX(C). | Piney with patchouli, coriander and mahogany-like and dry cedarwood undertones with faint chocolate topnotes. |
| Fragrance prepared according to Example X(A). | Floral with green, spicy and calamus-like undertones. |
| Fragrance prepared according to Example X(B). | Floral with ozoney and fresh air dried cloth undertones. |
| Fragrance prepared according to Example X(C). | Floral with spicy (cinnamon), carnation-like, herbaceous and fruity undertones. |
| Fragrance prepared according to Example X(D). | Floral with lilac and green undertones. |
| Fragrance prepared according to Example XI. | Lilac with a citrusy, grapefruit-like undertone. |

Example XIII

Perfumed Liquid Detergents

Concentrated liquid detergents (lysine salt of n-dodecylbenzene sulfonic acid as more specifically described in U.S. Patent No. 3,948,818 issued on April 6, 1976 incorporated by reference herein) with aroma nuances as set forth in Table III of Example XII are prepared containing 0.10%, 0.15%, 0.20%, 0.25%, 0.30% and 0.35% of the substance set forth in Table III of Example XII. They are prepared by adding and homogeneously mixing the appropriate quantity of substance set forth in Table III of Example XII in the liquid detergent. The detergents all possess excellent aromas as set forth in Table III of Example XII, the intensity increasing with greater concentrations of substance as set forth in Table III of Example XII.

Example XIV

Preparations of Colognes and Handkerchief Perfumes

Compositions as set forth in Table III of Example XII are incorporated into colognes at concentrations of 2.0%, 2.5%, 3.0%, 3.5%, 4.0%, 4.5% and 5.0% in 80%, 85%, 90% and 95% aqueous food grade ethanol solutions; and into handkerchief perfumes at concentrations of 15%, 20%, 25% and 30% (in 80%, 85%, 90% and 95% aqueous food grade ethanol solutions). Distinctive and definitive fragrances as set forth in Table III of Example XII are imparted to the colognes and to the handkerchief perfumes at all levels indicated.

Example XV

Preparations of Soap Compositions

One hundred grams of soap chips (per sample (IVORY®, produced by the Procter & Gamble Company of Sincinnati, Ohio), are each mixed with one gram samples of substances as set forth in Table III of Example XII until homogeneous compositions are obtained. In each of the cases, the homogeneous compositions are heated under 8 atmospheres pressure at 180°C for a period of three hours and the resulting liquids are placed into soap molds.

The resulting soap cakes, on cooling, manifest aromas as set forth in Table III of Example XII.

Example XVI

Preparation of Solid Detergent Compositions

Detergents are prepared using the following ingredients according to Example I of Canadian Patent No. 1,007,948 (incorporated herein by reference):

| Ingredient | Percent by Weight |
|---|---|
| Neodol® 45—11 (a $C_{14}$—$C_{15}$ alcohol ethoxylated with 11 moles of ethylene oxide) | 12 |
| Sodium carbonate | 55 |
| Sodium citrate | 20 |
| Sodium sulfate, water brighteners | q.s. |

The detergent is a phosphate-free detergent. Samples of 100 grams each of this detergent are admixed with 0.10, 0.15, 0.20 and 0.25 grams of each of the substances as set forth in Table III of Example XII. Each of the detergent samples has an excellent aroma as indicated in Table III of Example XII.

Example XVII

Utilizing the procedure of Example I at column 15 of U.S. Patent No. 3,632,396 (the disclosure of which is incorporated herein by reference), nonwoven cloth substrates useful as drier-added fabric softening articles of manufacture are prepared wherein the substrate, the substrate coating, the outer coating and the perfuming material are as follows:

1. A water "dissolvable" paper ("Dissolvo Paper")
2. Adogen 448 (m.p. about 140°F) as the substrate coating; and
3. An outer coating having the following formulation (m.p. about 150°F):
   - 57% $C_{20-22}$ HAPS
   - 22% isopropyl alcohol
   - 20% antistatic agent
   - 1% of one of the substances as set forth in Table III of Example XII.

Fabric softening compositions prepared according to Example I at column 15 of U.S. Patent No. 3,632,396 having aroma characteristics as set forth in Table III of Example XII, supra, consist of a substrate coating having a weight of about 3 grams per 100 square inches of substrate; a first coating located directly on the substrate coating consisting of about 1.85 grams per 100 square inches of substrate; and an outer coating coated on the first coating consisting of about 1.4 grams per 100 square inches of substrate. One of the substances of Table III of Example XII is admixed in each case with the outer coating mixture, thereby providing a total aromatized outer coating weight ratio to substrate of about 0.5:1 by weight of the substrate. The aroma characteristics are imparted in a pleasant manner to the head space in a dryer on operation thereof in each case using said drier-added fabric softener non-woven fabrics and these aroma characteristics are described in Table III of Example XII, supra.

Example XVIII

Hair Spray Formulations

The following hair spray formulation is prepared by first dissolving PVP/VA E—735 copolymer manufactured by the GAF Corporation of 140 West 51st Street, New York, New York, in 91.62 grams of 95% food grade ethanol. 8.0 Grams of the polymer is dissolved in the alcohol.

The following ingredients are added to the PVP/VA alcoholic solution:

| | Weight Percent |
|---|---|
| Dioctyl sebacate | 0.05 |
| Benzyl alcohol | 0.10 |
| Dow Corning 473 fluid (prepared by the Dow Corning Corporation) | 0.10 |
| Tween 20 surfactant (prepared by ICI America Corporation) | 0.03 |
| One of the perfumery substances as set forth in Table III of Example XII, supra | 0.10 |

The perfume substances as set forth in Table III of Example XII add aroma characteristics as set forth in Table III of Example XII which are rather intense and aesthetically pleasing to the users of the soft-feel, good-hold pump hair sprays.

Example XIX

Conditioning Shampoos

Monamid CMA (prepared by the Mona Industries Company) (3.0 weight percent) is melted with 2.0 weight percent coconut fatty acid (prepared by Procter & Gamble Company of Cincinnati, Ohio); 1.0 weight percent ethylene glycol distearate (prepared by the Armak Corporation) and triethanolamine (a product of Union Carbide Corporation) (1.4 weight percent). The resulting melt is admixed with Stepanol WAT produced by the Stepan Chemical Company (35.0 weight percent). The resulting mixture is heated to 60°C and mixed until a clear solution is obtained (at 60°C). This material is "COMPOSITION A".

Gafquat® 755 N polymer (manufactured by GAF Corporation of 140 West 51st Street, New York, New York) (5.0 weight percent) is admixed with 0.1 weight percent sodium sulfite and 1.4 weight percent polyethylene glycol 6000 distearate produced by Armak Corporation. This material is "COMPOSITION B".

The resulting COMPOSITION A & COMPOSITION B are then mixed in a 50:50 wt ratio of A:B and cooled to 45°C and 0.3 wt percent of perfuming substance as set forth in Table III of Example XII is added to the mixture. The resulting mixture is cooled to 40°C and blending is carried out for an additional one hour in each case. At the end of this blending period, the resulting material has a pleasant fragrance as indicated in Table III of Example XII.

Example XX

Tobacco Formulation

A tobacco mixture is prepared by admixing the following ingredients:

| Ingredients | Parts by Weight |
|---|---|
| Bright | 40.1 |
| Burley | 24.9 |
| Maryland | 1.1 |
| Turkish | 11.6 |
| Stem (flue-cured) | 14.2 |
| Glycerine | 2.8 |
| Water | 5.3 |

Cigarettes are prepared from this tobacco.
The following flavor formulation is prepared:

| Ingredients | Parts by Weight |
|---|---|
| Ethyl butyrate | .05 |
| Ethyl valerate | .05 |
| Maltol | 2.00 |
| Cocoa extract | 26.00 |
| Coffee extract | 10.00 |
| Ethyl alcohol | 20.00 |
| Water | 41.90 |

The above-stated tobacco flavor formulation is applied at the rate of 1.0% to all of the cigarettes produced using the above tobacco formulation. Half of the cigarettes are then treated with 500 or 1000 ppm of the compound having the structure:

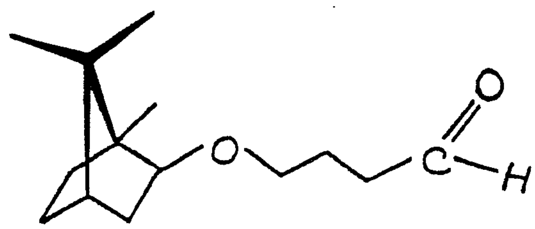

produced according to Example III. The control cigarettes not containing the compound having the structure:

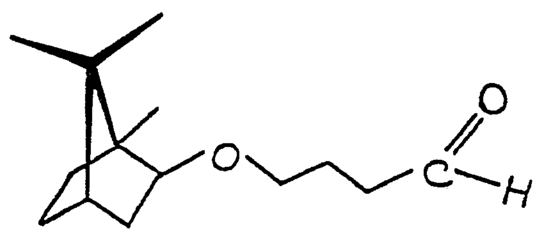

and the experimental cigarettes which contain the compound having the structure:

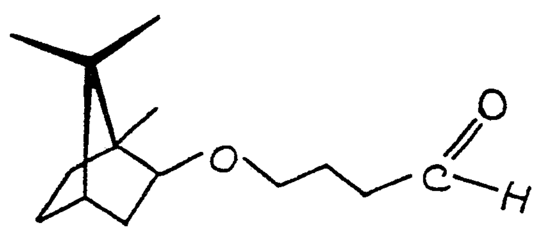

are evaluated by paired comparison and the results are as follows:

The experimental cigarettes are found to have a woody, green and herbaceous aroma prior to smoking and are found to have body and a slight mouth coating with woody, green and herbaceous nuances on smoking in both the main stream and the side stream. The experimental cigarettes are found to be sweeter and more aromatic on smoking in the main stream. All cigarettes are evaluated for smoke flavor with a 20 mm cellulose acetate filter.

When used in the filter rather than on the tobacco the cigarettes having in the filter the compound having the structure:

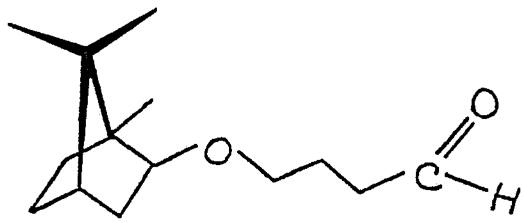

have a sweet, woody, green, herbaceous flavor and taste prior to and on smoking.

Example XXI

Tobacco Formulation

A tobacco mixture is prepared by admixing the following ingredients:

| Ingredients | Parts by Weight |
|---|---|
| Bright | 40.1 |
| Burley | 24.9 |
| Maryland | 1.1 |
| Turkish | 11.6 |
| Stem (flue-cured) | 14.2 |
| Glycerine | 2.8 |
| Water | 5.3 |

Cigarettes are prepared from this tobacco.

The following flavor formulation is prepared:

| Ingredients | Parts by Weight |
|---|---|
| Ethyl butyrate | .05 |
| Ethyl valerate | .05 |
| Maltol | 2.00 |
| Cocoa extract | 26.00 |
| Coffee extract | 10.00 |
| Ethyl alcohol | 20.00 |
| Water | 41.90 |

The above-stated tobacco flavor formulation is applied at the rate of 1.0% to all of the cigarettes produced using the above tobacco formulation. Half of the cigarettes are then treated with 500 or 1000 ppm of the compound having the structure:

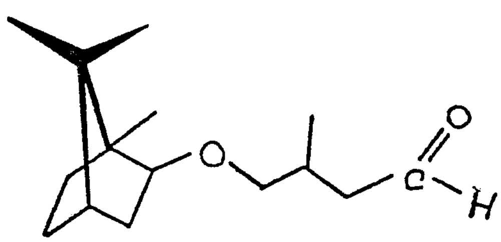

produced according to Example IV. The control cigarettes not containing the compound having the structure:

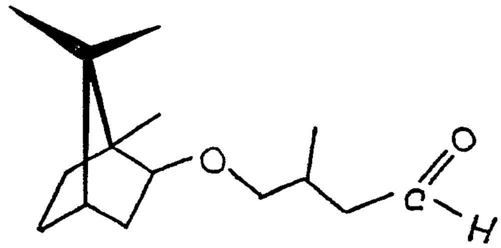

and the experimental cigarettes which contain the compound having the structure:

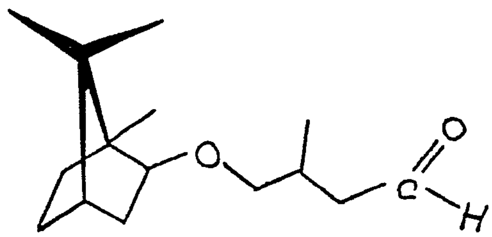

are evaluated by paired comparison and the results are as follows:

The experimental cigarettes are found to have woody, herbaceous and spicy aroma nuances prior to smoking and have body and added aromatically with woody, herbaceous and oriental-like, spicy nuances on smoking in both the main stream and the side stream. All cigarettes are evaluated for smoke flavor with a 20 mm cellulose acetate filter.

When used in the filter rather than on the tobacco the cigarettes having in the filter the compound having the structure:

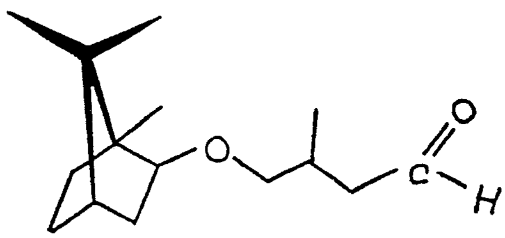

have an aromatic, woody, herbaceous, spicy aroma and taste both prior to and on smoking.

Example XXII

Each of the fragrance materials of Table III of Example XII, Supra, are added to a 50:50 weight:weight mixture of low density polyethylene:polyepsilon caprolactone PCL—700 forming pellets with scents as set forth in Table III of Example XII, supra.

75 Pounds of a 50:50 mixture of PCL—700 polyepsilon caprolactone (manufactured by the Union Carbide Corporation of New York, New York having a melting point of about 180—190°F): Low density polyethylene, are heated to about 250°F in a container of the kind illustrated in Figures 41 and 42; 25 Pounds of each of the fragrance materials as set forth in Table III of Example XII, is then quickly added to the liquified polymer mixture, the lid *228* is put in place and the agitating means *273* are actuated. The temperature is then raised to about 260°F and the mixing is continued for 5—15 minutes. The valve "V" is then opened to allow flow of the molten polymer enriched with perfume ingredient to exit through the orifices *234*. The liquid falling through the orifices *234* solidifies almost instantaneously upon impact with the moving cooled conveyor *238*. Polymer beads or pellets *244* having pronounced scents as described in Table III of Example XII, supra, are thus formed. Analysis demonstrates that the pellets contain about 25% of the perfume material so that almost no losses in the scenting substance did occur. These pellets may be called "master pellets".

50 Pounds of each batch of the scent containing "master pellets" are then added to one thousand pounds of unscented polypropylene and the mass is heated to the liquid state. The liquid is molded into thin sheets of films. The thin sheets of films have pronounced aromas as set forth in Table III of Example XII, supra. The sheets of films are cut into strips of 0.25" in width × 3" in length and placed into room air fresheners.

On operation of the room air freshener, after four minutes, the room in each case has an aesthetically pleasing aroma with no foul odor being present, the aroma being described in Table III of Example XII, supra.

Example XIII

Flavor Formulation

The following natural orange formulation is prepared:

| Ingredients | Parts by Weight |
|---|---|
| Compound defined according to the structure: 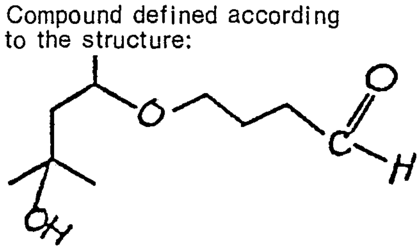 prepared according to Example VI. | 26.0 |
| Natural Lemon Oil Terpeneless | 10.0 |
| Acetaldehyde | 0.6 |
| Alpha-terpineol | 2.1 |
| Citral | 1.8 |
| Carvone | 0.24 |
| Terpinolene | 1.2 |
| alpha-terpinene | 0.25 |
| Diphenyl | 0.25 |
| Alpha Fenchyl Alcohol | 0.25 |
| Limonene | 0.35 |
| Linalool | 0.25 |
| Geranyl Acetate | 0.25 |
| Nootkatone | 0.25 |
| Neryl Acetate | 0.25 |

The second flavor formulation is prepared which is identical to the above formulation, except without the compound having the structure:

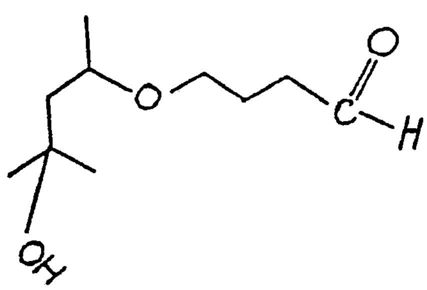

The flavor formulation with the compound having the structure:

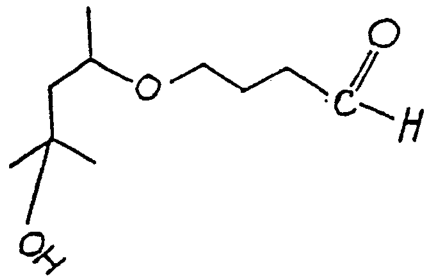

has a definite "natural orange" aroma due to the addition of bitter orange principals to this citrus flavor.

The citrus flavor with the compound having the structure:

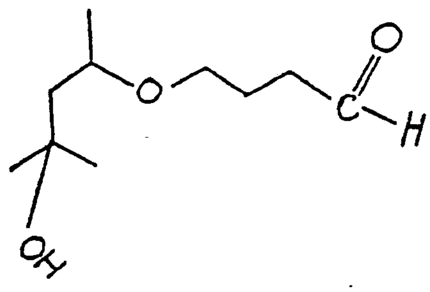

is used in the following examples.

Example XXIV

A. Powder Flavor Composition

20 Grams of the flavor composition of Example XXIII containing the compound having the structure:

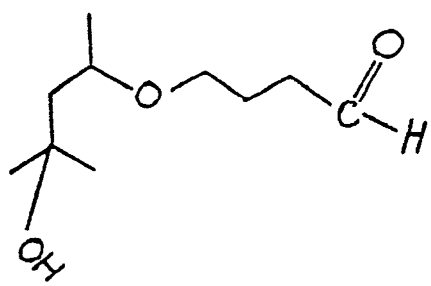

is emulsified in a solution containing 300 grams gum acacia and 700 grams water. The emulsion is spray-dried with a Bowen Lab Model Drier utilizing 260 c.f.m. of air with an inlet temperature of 500°F, and outlet temperature of 200°F, and a wheel speed of 50,000 rpm.

B. Sustained Release Flavor

The following mixture is prepared:

| Ingredients | Parts by Weight |
|---|---|
| Liquid Citrus Flavor Composition of Example XXIII | 20.0 |
| Propylene glycol | 9.0 |
| Cab-O-Sil® M—5 (Brand of Silica produced by the Cabot Corporation of 125 High Street, Boston, Massachusetts 02110: Physical Properties: Surface area: 200 $m^2$/gm Nominal particle size: 0.012 microns Density: 2.3 lbs/cu.ft. | 5.00 |

The Cabo-O-Sil is dispersed in the liquid citrus flavor compositions of Example XXIII with vigorous stirring, thereby resulting in a viscous liquid. 71 Parts by weight of the powder flavor compositions of Part "A", supra, is then blended into the said viscous liquid, with stirring, at 25°C for a period of 30 minutes resulting in a dry, free flowing sustained release flavor powder.

Example XXV

10 Parts by weight of 50 Bloom pigskin gelatin is added to 90 parts by weight of water at a temperature of 150°F. The mixture is agitated until the gelatin is completely dissolved and the solution is cooled to 120°F. 20 Parts by weight of the liquid flavor composition of Example XXIII is added to the solution which is then homogenized to form an emulsion having particle size typically in the range of 5—40 microns. This material is kept at 120°F under which conditions the gelatin will not jell.

Coacervation is induced by adding, slowly and uniformly 40 parts by weight of a 20% aqueous solution of sodium sulphate. During coacervation the gelatin molecules are deposited uniformly about each oil droplet as a nucleus.

Gelatin is effected by pouring the heated coacervate mixture into 1,000 parts by weight of 7% aqueous solution of sodium sulphate at 65°F. The resulting jelled coacervate may be filtered and washed with water at temperatures below the melting point of gelatin, to remove the salt.

Hardening of the filtered cake, in this example, is effected by washing with 200 parts by weight of 37% solution of formaldehyde in water. The cake is then washed to remove residual formaldehyde.

Example XXVI

Chewing gum

100 Parts by weight of chicle are mixed with 4 parts by weight of the flavor prepared in accordance with Example XXIV(B). 300 Parts of sucrose and 100 parts of corn syrup are added. Mixing is effected in a ribbon blender with jacketed side walls of the type manufactured by the Baker Perkins Company.

The resultant chewing gum blend is then manufactured into strips 1 inch in width and 0.1 inches in thickness. The strips are cut into lengths of 3 inches each. On chewing, the chewing gum has a pleasant, long lasting citrus flavor.

Example XXVII

Chewing Gum

100 Parts by weight of chicle are mixed with 18 parts by weight of the flavor prepared in accordance with Example XXV. 300 Parts of sucrose and 100 parts of corn syrup are then added. Mixing is effected in a ribbon blender with jacketed side walls of the type manufactured by the Baker Perkins Company.

The resultant chewing gum blend is then manufactured into strips 1 inch in width and 0.1 inches in thickness. The strips are cut into lengths of 3 inches each. On chewing, the chewing gum has a pleasant, long lasting citrus flavor.

Example XXVIII

Toothpaste Formulation

The following separate groups of ingredients are prepared:

| Parts by Weight | Ingredients |
|---|---|
| Group "A" | |
| 30.200 | Glycerine |
| 15.325 | Distilled Water |
| .100 | Sodium Benzoate |
| .125 | Saccharin Sodium |
| .400 | Stannous Fluoroide |
| Group "B" | |
| 12.500 | Calcium Carbonate |
| 37.200 | Dicalcium Phosphate (Dihydrate) |
| Group "C" | |
| 2.000 | Sodium N-Lauroyl Sarcosinate (foaming agent) |
| Group "D" | |
| 1.200 | Flavor Materials of Example XXIV(B) |

Procedure:

1. The ingredients in Group "A" are stirred and heated in a steam jacketed kettle to 160°F;
2. Stirring is continued for an additional three to five minutes to form a homogeneous gel;
3. The powders of Group "B" are added to the gel, while mixing, until a homogeneous paste is formed;
4. With stirring, the flavor of "D" is added and lastly the sodium n-lauroyl sarcosinate;
5. The resultant slurry is then blended for one hour. The completed paste is then transferred to a three roller mill and then homogenized, and finally tubed.

The resulting toothpaste when used in a normal toothbrushing procedure yields a pleasant citrus flavor, of constant strong intensity throughout said procedure (1—1.5 minutes).

Example XXIX

Chewable Vitamin Tablets

The flavor material produced according to the process of Example XXIV(B) is added to a Chewable Vitamin Tablet Formulation at a rate of 10 gm/Kg which chewable vitamin tablet formulation is prepared as follows:

In a Hobart Mixer the following materials are blended to homogeneity:

| | Gms/1000 tablets |
|---|---|
| Vitamin C (ascorbic acid) as ascorbic acid-sodium ascorbate mixture 1:1 | 70.00 |
| Vitamin $B_1$ (thiamine mononitrate) as Rocoat® thiamine mononitrate 33⅓% (Hoffman La Roche) | 4.0 |
| Vitamin $B_2$ (riboflavin) as Rocoat® riboflavin 33⅓% | 5.0 |
| Vitamin $B_6$ (pyridoxine hydrochloride) as Rocoat® pyridoxine hydrochloride 33⅓% | 4.0 |
| Niacinamide as Rocoat® niacinamide 33⅓% | 33.0 |
| Calcium pantothenate | 11.5 |
| Vitamin $B_{12}$ (cyanocobalamin) as Merck 0.1% in gelatin | 3.5 |
| Vitamin E (dl-alpha tocopheryl acetate) as dry Vitamin E acetate 33⅓% Roche | 6.6 |
| d-Biotin | 0.044 |
| Flavor of Example XXIV(B) | (as indicated above) |
| Certified lake color | 5.0 |
| Sweetener — sodium saccharin | 1.0 |
| Magnesium stearate lubricant | 10.0 |
| Mannitol q.s. to make | 100.0 |

Preliminary tablets are prepared by slugging with flatfaced punches and grinding the slugs to 14 mesh. 13.5 G dry Vitamin A Acetate and 0.6 g Vitamin D are then added as beadlets. The entire blend is then compressed using concave punches at 0.5 g each.

Chewing of the resultant tablets yields a pleasant, long-lasting, consistently strong citrus flavor for a period of 12 minutes.

Example XXX

Chewing Tobacco

Onto 100 pounds of tobacco for chewing (85% Wisconsin leaf and 15% Pennsylvania leaf) the following casing is sprayed at a rate of 30%:

| Ingredients | Parts by Weight |
|---|---|
| Corn Syrup | 60.0 |
| Licorice | 10.0 |
| Glycerine | 20.0 |
| Fig Juice | 4.6 |
| Prune Juice | 5.0 |
| Flavor Material of Example XXIV(B) | 0.4 |

The resultant product is redried to a moisture content of 20%. On chewing, this tobacco has an excellent substantially consistent, long-lasting citrus and licorice aroma and taste profile in conjunction with the tobacco note.

Example XXXI

To 100 parts by weight of GOYA® mango nectar (produced by the Goya Corporation of New York, New York) is added 10 ppm of the compound having the structure:

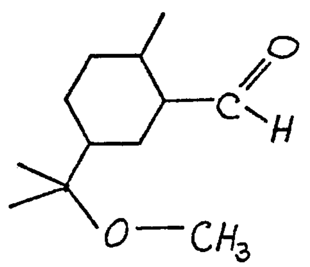

prepared according to Example VIII. The compound having the structure:

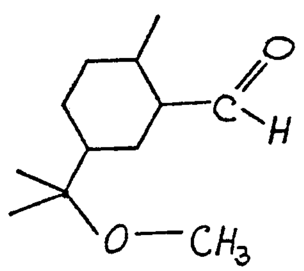

adds to the mango nectar a very natural nuance which although present in natural mango is lost in the canning process when mango nectar is prepared and canned in the usual manner.

The ether caboxaldehydes of our invention are indicated, for example, as having structures such as:

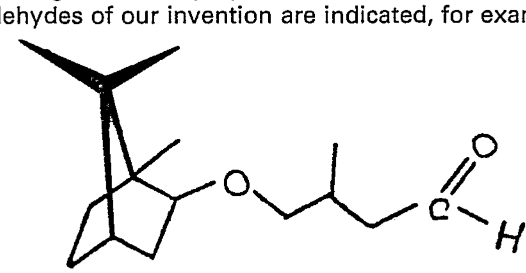

it is to be understood that the instant invention also covers the use of the individual "cis" and "trans" and stereoisomers of such compounds, for example, the compound having the structure:

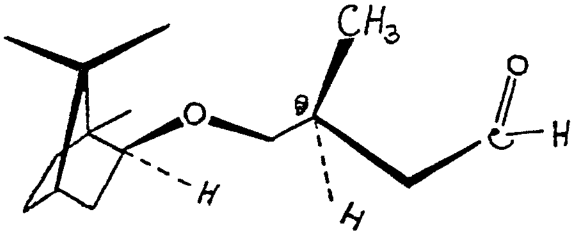

Example XXXII

Preparation of 4(p-cumenyloxy)-3-methyl-1-butanol

Reaction:

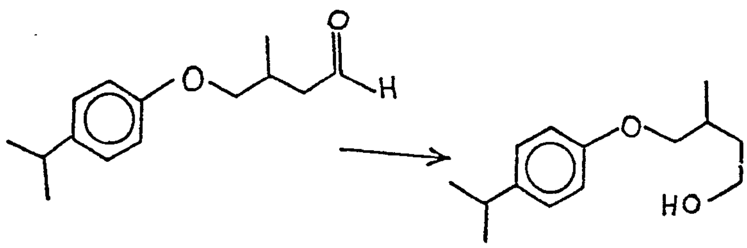

Into a one liter reaction flask is placed 56.7 grams (1.5 moles) of sodium borohydride and 200 ml of anhydrous isopropyl alcohol. Over a period of 45 minutes, 225 grams of the aldehyde having the structure:

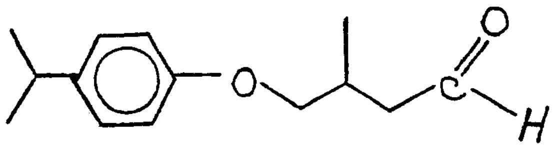

prepared according to Example II is added to the reaction mass. The reaction mass is maintained at 20°C for a period of three hours with stirring. At the end of the three hour period, the reaction mass is filtered and the filtrate is washed with water until neutral. The crude reaction mass is then distilled through a 2" splash column followed by a fractional distillation through a microvigreux column. The distillation through the microvigreux column yields the following fractions:

| Fraction No. | Vapor Temp. (°C) | Liquid Temp. (°C) | Vacuum mm/Hg. Pressure | Weight of Fraction (gms) |
|---|---|---|---|---|
| 1 | 65 | 142 | 3.4 | 2.9 |
| 2 | 75 | 145 | 3.4 | 3.2 |
| 3 | 73 | 148 | 3.4 | 3.0 |
| 4 | 70 | 150 | 3.4 | 2.3 |
| 5 | 98 | 155 | 3.4 | 7.6 |
| 6 | 95 | 157 | 3.4 | 7.6 |
| 7 | 80 | 164 | 3.6 | 11.5 |
| 8 | 94 | 172 | 3.6 | 1.5 |
| 9 | 104 | 193 | 3.6 | 3.1 |
| 10 | 100 | 200 | 3.6 | 5.7 |
| 11 | 80 | 220 | 3.6 | 3.2 |

Figure 24A is the GLC profile for the crude reaction product containing the compound having the structure:

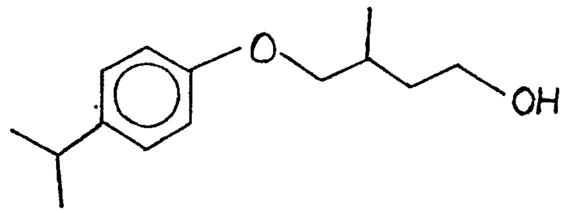

Figure 24B is the GLC profile for fraction 5 of the foregoing distillation containing the compound having the structure:

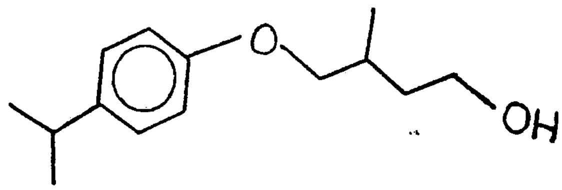

Figure 25 is the NMR spectrum for the peak indicated by reference numeral 149 of the GLC profile of Figure 3B for the compound having the structure:

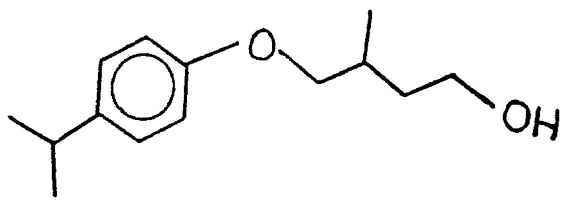

(Conditions: Field strength: 100 MHz; solvent: $CFCl_3$).

Example XXXIII

Preparation of 4-(2-Bornyloxy)-1-butanol)

Reaction:

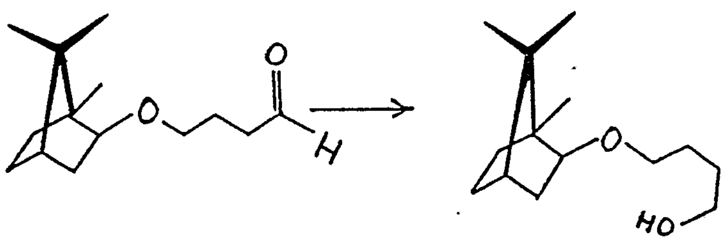

Into a one liter reaction flask equipped with stirrer, thermometer, reflux condenser and heating mantle is placed 200 ml of anhydrous isopropyl alcohol and 56.7 grams (1.5 moles) of sodium borohydride. The resulting mixture is heated to reflux and during refluxing, over a 45 minute period, the aldehyde having the structure:

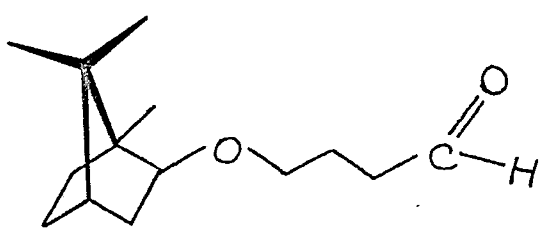

produced according to Example III is added to the reaction mass. The reaction mass is then continued to be stirred at reflux for a period of three hours. At the end of the three hour period, the reaction mass is cooled to room temperature and the undissolved sodium borohydride is filtered. The reaction mass is then washed with water until neutral. The crude reaction mass, weighing 309.3 grams, is then rushed over through a 2" splash column yielding the following fractions:

| Fraction No. | Vapor Temp. (°C) | Liquid Temp. ("C) | Vacuum mm/Hg. Pressure | of Fraction (gms) |
|---|---|---|---|---|
| 1 | 95/ | 119/ | 1.3 | 2.2 |
| 2 | 100 | 120 | 1.4 | 9.4 |
| 3 | 104 | 130 | 1.4 | 30.8 |
| 4 | 105 | 135 | 1.5 | 34.4 |
| 5 | 107 | 145 | 1.6 | 37.0 |
| 6 | 106 | 173 | 1.7 | 16.3 |
| 7 | 106 | 179 | 1.7 | 1.0 |

Figure 26 is GLC profile for the crude reaction product containing the compounds having the structure:

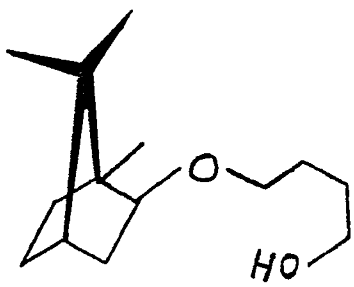

and

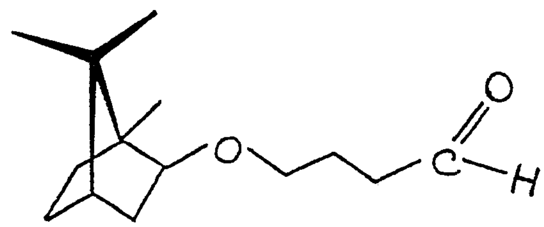

Figure 27 is the GLC profile for fraction 2 of the foregoing distillation product containing the compound having the structure:

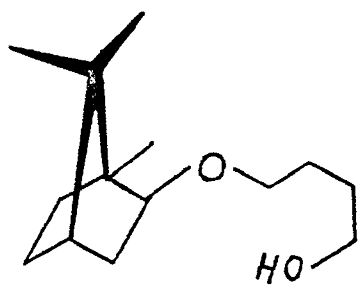

Figure 28 is the NMR spectrum for the compound having the structure:

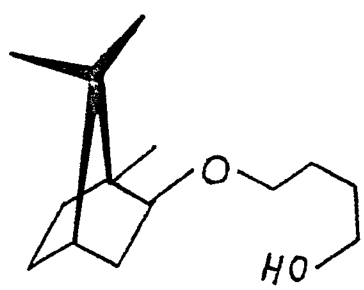

produced according to this example (Conditions: Field strength: 100 MHz; solvent: $CFCl_3$).

Figure 29 is the infrared spectrum for the compound having the structure:

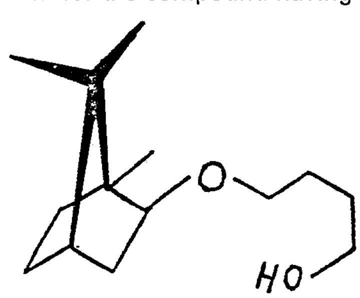

produced according to this Example.

Example XXXIV

Production of 4-(2-Bornyloxy)-1-butanol
Reaction:

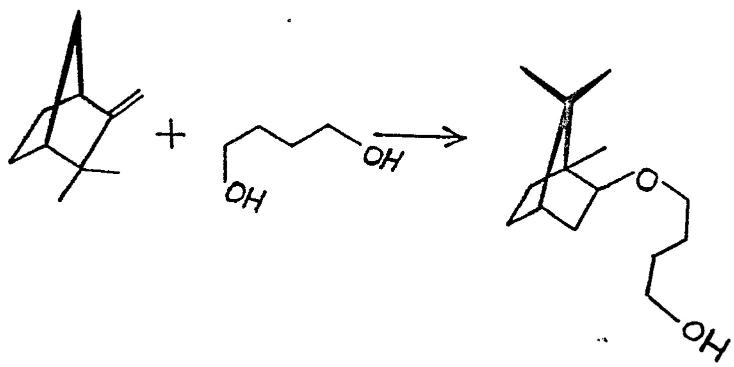

Into a one liter reaction flask equipped with stirrer, thermometer and reflux condenser is placed 500 grams of 1,4-butanediol and 15 grams of born trifluoride. The reaction mass is heated to 80°C. Over a period of one hour, a mixture of 500 grams of camphene and 500 grams of 1,4-butanediol is added to the reaction mass. During the addition period, the reaction mass is maintained at 79—80°C with stirring. The reaction mass is continued to be stirred at 80°C for a period of seven hours. At the end of the seven hour period, the reaction mass is washed with water and extracted with toluene. The organic phase is then separated and is washed with saturated sodium carbonate solution. The thus washed material is then dried over anhydrous sodium sulfate and distilled on a 12" Goodloe column yielding the following fractions.

| Fraction No. | Vapor Temp. (°C) | Liquid Temp. (°C) | Vacuum mm/Hg. Pressure | Weight of Fraction (gms) |
|---|---|---|---|---|
| 1 | 118/127 | 157/143 | 1.2/3 | 39 |
| 2 | 128 | 143 | 2.7 | 38 |
| 3 | 128 | 143 | 2.8 | 42 |
| 4 | 128 | 143 | 2.7 | 46 |
| 5 | 128 | 143 | 2.8 | 35 |
| 6 | 128 | 145 | 2.8 | 55 |
| 7 | 129 | 150 | 2.8 | 39 |
| 8 | 133 | 207 | 3.0 | 27 |

Figure 30 is the GLC profile for crude reaction product for this Example containing the compound having the structure:

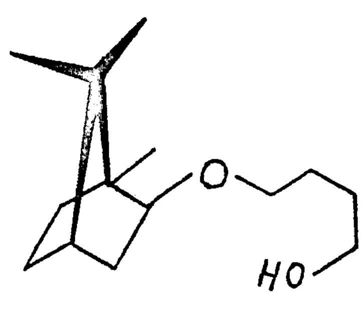

Figure 31 is the NMR spectrum for fraction 4 of the foregoing distillation product containing the compound having the structure:

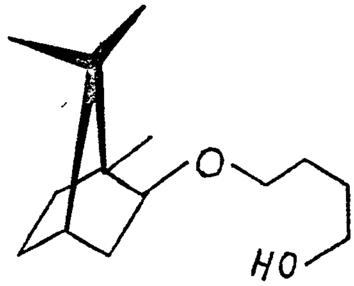

Example XXXV

Preparation of 4-(2-bornyloxy)2-butanol

Reaction:

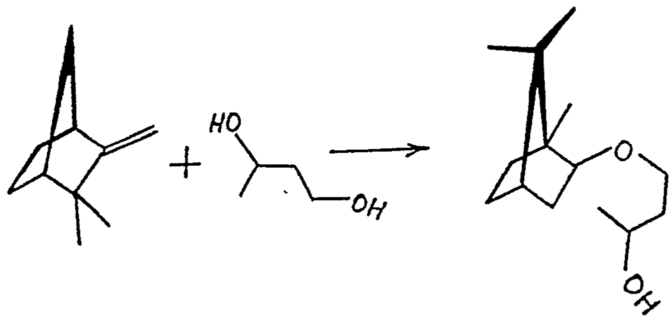

Into a one liter reaction vessel equipped with a stirrer, thermometer, reflux condenser and heating mantel is placed 340 grams of t,3-butanidiol and 10 grams of boron trifluoride. The reaction mass is heated to 80°C and while maintaining the reaction mass at 80°C, 340 grams of camphene is added over a two hour period. At the end of the camphene feeding period, the reaction mass is stirred at a temperature of 80°C for a period of 18 hours.

After the 18 hour period, the reaction mass is quenched with water and the reaction mass is washed with saturated sodium carbonate solution until neutral. The aqueous phase is separated from the organic phase. The aqueous phase is extracted with toluene and the toluene extract are added to the organic phase. The resulting organic material is then charged to an evaporator and the toluene solvent is recovered.

The resulting product is distilled on a column packed with splash saddles yielding the following fractions:

| Fraction No. | Vapor Temp. (°C) | Liquid Temp. (°C) | Vacuum mm/Hg. Pressure | Weight of Fraction (gms) |
|---|---|---|---|---|
| 1 | 93/ | 127/ | 5.0 | 7.0 |
| 2 | 115 | 127 | 5.0 | 15.0 |
| 3 | 123 | 136 | 4.8 | 211.0 |
| 4 | 175 | 220 | 3.8 | 190.0 |

Figure 32 is the GLC profile for the crude reaction product containing the compound having the structure:

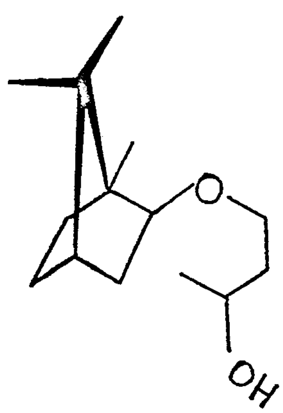

Figure 33 is the NMR spectrum for fraction 2 of the foregoing distillation product containing the compound having the structure:

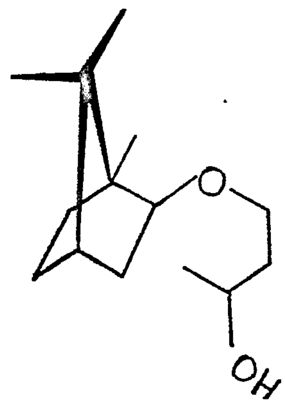

(Conditions: Field strength: 100 MHz; solvent: $CFCl_3$).

Example XXXVI

Preparation of 4-(2-bornyloxy)-2-buten-1-ol

Reaction:

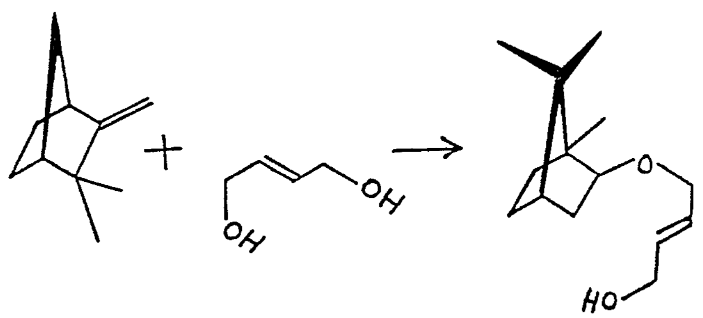

Into a two liter reaction vessel equipped with stirrer, thermometer, heating mantel and reflux condenser is placed 1000 grams (7.35 moles) of 2-pentene-1,4-diol and 15 grams of boron trifluoride etherate. The reaction mass is then heated to 80°C. Over a period of two hours while maintaining the reaction mass at 80°C, 500 grams (3.7 moles) of camphene is added. The reaction is continued to be stirred at 80°C for a period of 12.5 hours after the feeding of the camphene.

At the end of the 12.5 hour period, the reaction mass is cooled to room temperature, quenched with water and neutralized with saturated sodium carbonate. The organic phase is then distilled on a 2″ splash column packed with saddles yielding the following fractions:

| Fraction No. | Vapor Temp. (°C) | Liquid Temp. (°C) | Vacuum mm/Hg. Pressure | Weight of Fraction (gms) |
|---|---|---|---|---|
| 1 | 80/102 | 118/124 | 1.2 | 14.0 |
| 2 | 110 | 126 | 1.2 | 19.0 |
| 3 | 120 | 131 | 1.2 | 64.0 |
| 4 | 130 | 143 | 1.2 | 205.0 |
| 5 | 155 | 180 | 1.2 | 142.0 |
| 6 | 170 | 220 | 1.2 | 60.0 |

Fractions 2—5 are bulked and redistilled on a 12″ Goodloe column yielding the following fractions:

| Fraction No. | Vapor Temp. (°C) | Liquid Temp. (°C) | Vacuum mm/Hg. Pressure | Weight of Fraction (gms) |
|---|---|---|---|---|
| 1 | 128/130 | 153/151 | 2.6 | 16.0 |
| 2 | 130 | 146 | 2.6 | 18.0 |
| 3 | 131 | 147 | 2.6 | 20.0 |
| 4 | 131 | 147 | 2.6 | 19.0 |
| 5 | 131 | 147 | 2.6 | 27.0 |
| 6 | 131 | 147 | 2.6 | 22.0 |
| 7 | 131 | 147 | 2.6 | 18.0 |
| 8 | 131 | 147 | 2.6 | 25.0 |
| 9 | 131 | 147 | 2.6 | 26.0 |
| 10 | 131 | 148 | 2.6 | 23.0 |
| 11 | 131 | 149 | 2.6 | 27.0 |
| 12 | 132 | 150 | 2.6 | 20.0 |
| 13 | 131/132 | 150/152 | 2.5 | 21.0 |
| 14 | 132 | 155 | 2.5 | 26.0 |
| 15 | 132 | 162 | 2.5 | 25.0 |
| 16 | 132 | 187 | 2.5 | 20.0 |
| 17 | 139 | 207 | 2.5 | 13.0 |
| 18 | 155 | 210 | 2.5 | 8.0 |

Figure 34 is the GLC profile for the crude reaction product containing the compound having the structure:

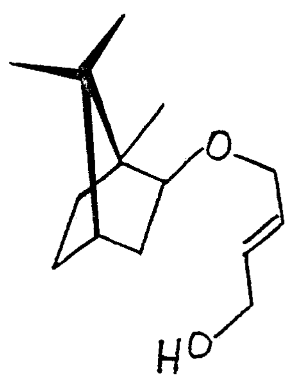

(Conditions: SE—30 column programmed at 100—200°C at 8°C per minute).

Figure 35 is the NMR spectrum for fraction 4 of the foregoing distillation product containing the compound having the structure:

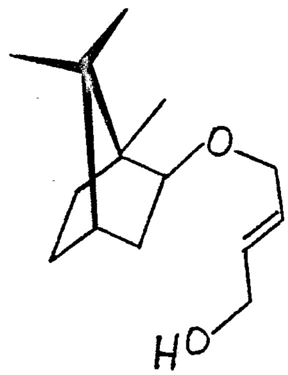

Example XXXVII

Preparation of 4-[1,3-dimethyl-2-butenyl)oxy]-3-methylbutyraldehyde

Reaction:

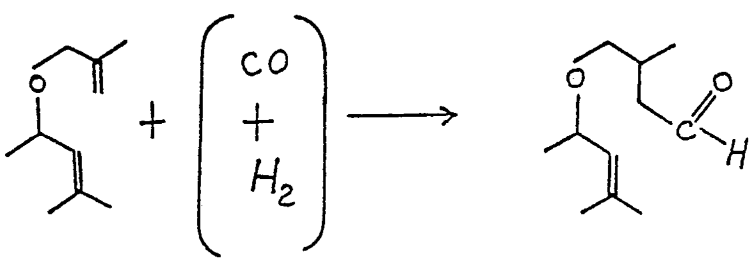

Into a high-pressure oxo-reactor is placed 98 grams of the methallyl ether having the structure:

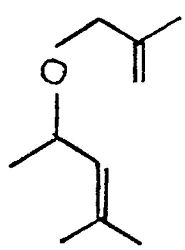

0.3 grams of rhodium-chlorocarbonyl (triphenyl phosphine), 3 grams of triphenyl phosphine and 300 ml of anhydrous toluene. The oxo-reactor is sealed, heated to 130°C and pressurized to 1000 psig with a 50:50 mole:mole mixture of carbon monoxide and hydrogen. The oxo-reactor is stirred for a period of one hour at 1000 psig and 130°C and then the temperature is raised for a period of four hours at 140°C. The reaction mass is then cooled to room temperature, and the reactor is opened. The reaction product is then filtered. The filtrate weighs 355.5 grams. The filtrate is then distilled on a 2" splash column yielding the following fractions:

| Fraction No. | Vapor Temp. (°C) | Liquid Temp. (°C) | Vacuum mm/Hg. Pressure | Weight of Fraction (gms) |
|---|---|---|---|---|
| 1 | /53 | /63 | 10.4 | 58.2 |
| 2 | 52 | 67 | 10.0 | 67.5 |
| 3 | 50 | 127 | 9.5 | 70.1 |
| 4 | 61 | 97 | 7.0 | 8.9 |
| 5 | 89 | 95 | 7.0 | 12.2 |
| 6 | 82 | 98 | 7.9 | 10.4 |
| 7 | 85 | 97 | 4.9 | 17.6 |
| 8 | 80 | 105 | 3.7 | 12.5 |
| 9 | 78 | 123 | 3.6 | 10.9 |
| 10 | 80 | 137 | 3.6 | 7.5 |
| 11 | 86 | 210 | 3.8 | 6.9 |

Figure 36 is the GLC profile for fraction 4 of the foregoing distillation product containing the compound having the structure:

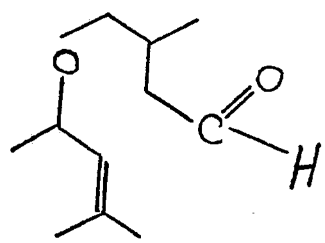

Figure 37 is the NMR spectrum for fraction 8 of the foregoing distillation containing the compound having the structure:

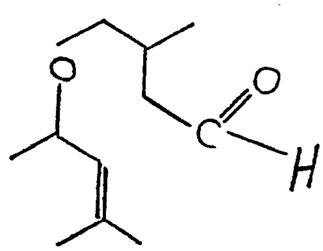

(Conditions: Field strength: 100 MHz; Solvent: $CFCl_3$).

Example XXXVIII

Preparation of 4-[(1,3-dimethyl-2-butenyl)oxy]-3-methyl-1-butanol

Reaction:

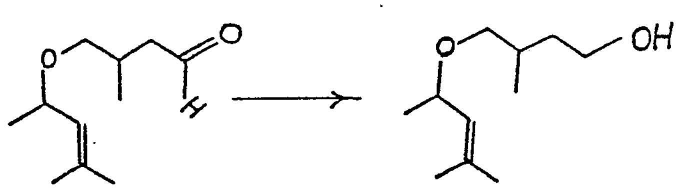

Into a 250 cc reaction vessel equipped with stirrer, thermometer, reflux condenser and heating mantel is placed 10 grams of sodium borohydride and 100 ml anhydrous isopropyl alcohol.

While maintaining the reaction mass at room temperature, 66.5 grams of the aldehyde produced according to Example VIII, supra, containing the compound having the structure:

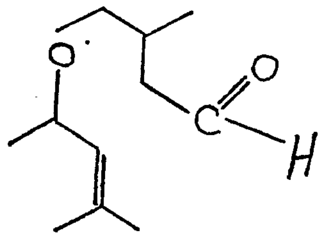

is added to the reaction mass over a period of two hours. During the addition of this material, the reaction mass temperature rises to 40°C as a result of the exothermic reaction. The reaction mass is cooled and maintained at 40°C with stirring for a period of four hours. At the end of the four hour period, the reaction mass is cooled to room temperature. A 5% aqueous solution of hydrochloric acid is added to the reaction mass. The reaction mass is then washed with water followed by a saturated sodium carbonate solution until neutral. The resulting reaction mass is then distilled on a 2" splash column yielding the following fractions:

| Fraction No. | Vapor Temp. (°C) | Liquid Temp. (°C) | Vacuum mm/Hg. Pressure | Weight of Fraction (gms) |
|---|---|---|---|---|
| 1 | 83/ | 94/ | 1.6 | 3.0 |
| 2 | 81 | 86 | 1.3 | 4.0 |
| 3 | 81 | 86 | 1.3 | 5.1 |
| 4 | 90 | 96 | 2.2 | 2.5 |
| 5 | 90 | 110 | 2.0 | 10.0 |
| 6 | 94 | 210 | 2.0 | 5.9 |

Figure 38 is the GLC profile for the crude reaction product containing the compound having the structure:

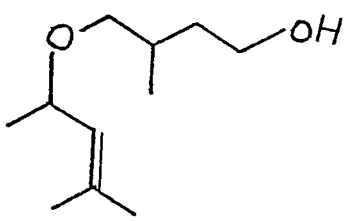

Figure 39 is the NMR spectrum for fraction 4 of the foregoing distillation product containing the compound having the structure:

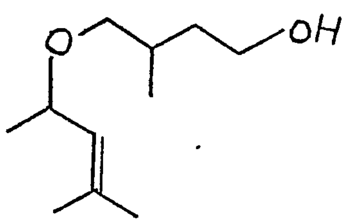

(Conditions: Field strength: 100 MHz; solvent: $CFCl_3$).

Example XXXIX

Preparation of 3-methyl-4-phenoxy-butanal

Reaction:

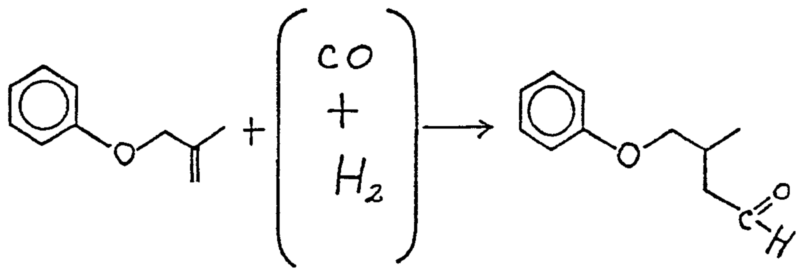

Into a high-pressure oxo-reactor are placed the following materials:

Allyl ether having the structure:

| | |
|---|---|
| 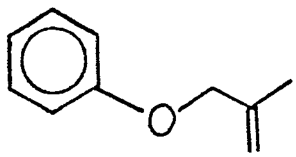 | 148 grams (1 mole) |
| Triphenyl phosphine | 6 grams |
| Rhodium chloride carbonyl (triphenyl phosphine) | 0.6 grams |

The oxo-reactor is sealed, heated to 140°C and pressurized with a 50:50 mole:mole mixture of hydrogen and carbon monoxide. The reaction mass is stirred while maintaining the temperature of 140°C and maintaining the pressure at 1000 psig for a period of ten hours.

At the end of the ten hour period, the oxo-reactor is cooled, opened and contents removed and filtered. The resulting filtrate is then distilled on a 2" splash column yielding the following fractions:

| Fraction No. | Vapor Temp. (°C) | Liquid Temp. (°C) | Vacuum mm/Hg. Pressure | Weight of Fraction (gms) |
|---|---|---|---|---|
| 1 | 35/ | 45/ | 1.6 | 21.3 |
| 2 | 72 | 100 | 1.6 | 9.2 |
| 3 | 100 | 108 | 1.6 | 35.1 |
| 4 | 99 | 108 | 1.5 | 51.8 |
| 5 | 97 | 108 | 1.3 | 50.1 |
| 6 | 95 | 135 | 1.3 | 32.2 |

Fractions 2—6 are bulked and redistilled on a 12" Goodloe column yielding the following fractions:

| Fraction No. | Vapor Temp. (°C) | Liquid Temp. (°C) | Vacuum mm/Hg. Pressure | Weight of Fraction (gms) |
|---|---|---|---|---|
| 1 | 50/58 | 112/117 | 2.8/3.0 | 6.6 |
| 2 | 62 | 112 | 1.6 | 10.0 |
| 3 | 94 | 127 | 1.6 | 12.7 |
| 4 | 94 | 128 | 1.5 | 14.2 |
| 5 | 88 | 128 | 1.2 | 12.1 |
| 6 | 88 | 128 | 1.2 | 13.0 |
| 7 | 100 | 128 | 1.2 | 8.4 |
| 8 | 104 | 127 | 2.3 | 10.4 |
| 9 | 100 | 128 | 2.0 | 17.9 |
| 10 | 104 | 131 | 2.3 | 18.0 |
| 11 | 102 | 147 | 2.3 | 14.8 |
| 12 | 80 | 100 | 2.3 | 8.9 |

Figure 40A is the GLC profile for the crude reaction product containing the compound having the structure:

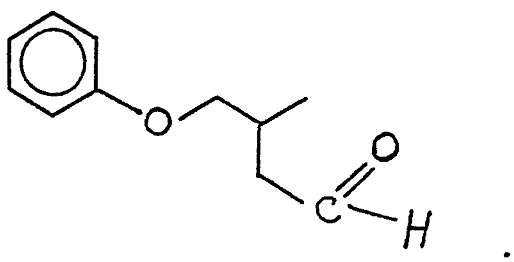

Figure 40B is the NMR spectrum for the compound having the structure:

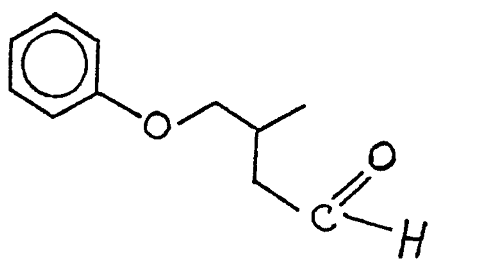

(Conditions: Solvent: $CFCl_3$; Field Strength: 100 MHz).

Example XL

Pine Fragrance

The following pine fragrance formulations are prepared:

| Ingredients | Parts by Weight XL—A | XL—B | XL—C |
|---|---|---|---|
| Isobornyl acetate | 100 | 100 | 100 |
| Camphor | 10 | 10 | 10 |
| Terpineol | 25 | 25 | 25 |
| Fir balsam absolute (50% in diethyl phthalate) | 20 | 20 | 20 |
| Coumarin | 4 | 4 | 4 |
| Linalool | 30 | 30 | 30 |
| Frenchyl alcohol | 10 | 10 | 10 |
| Anethol | 12 | 12 | 12 |
| Lemon terpenes washed | 50 | 50 | 50 |
| Borneol | 5 | 5 | 5 |
| Galbanum oil | 5 | 5 | 5 |
| Turpentine Russian | 150 | 150 | 150 |
| Eucalyptol | 50 | 50 | 50 |
| 2,2,6-trimethyl-1-cyclohexene-1-carboxyaldehyde | 12 | 12 | 12 |
| Maltol (1% in diethyl phthalate) | 5 | 5 | 5 |
| Compound having the structure: 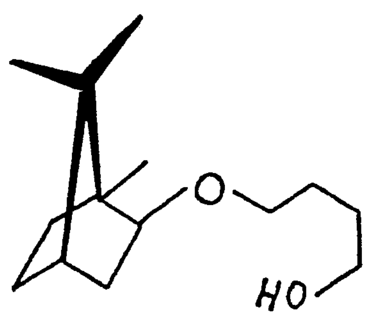 prepared according to Examples XXXIII or XXXIV | 28 | 0 | 0 |

| Ingredients | Parts by weight XL–A | XL–B | XL–C |
|---|---|---|---|
| Compound having the structure: 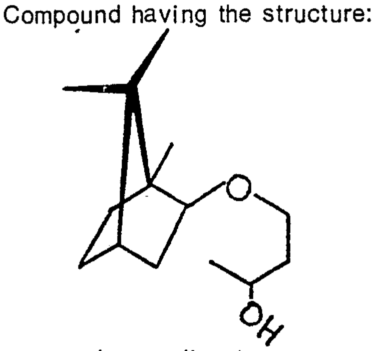 prepared according to Example XXXV | 0 | 28 | 0 |
| Compound having the structure: 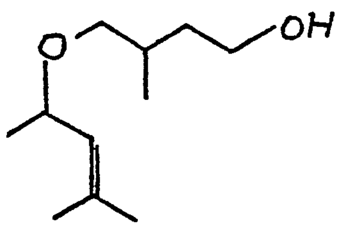 prepared according to Example XXXVIII | 0 | 0 | 28 |

The compound having the structure:

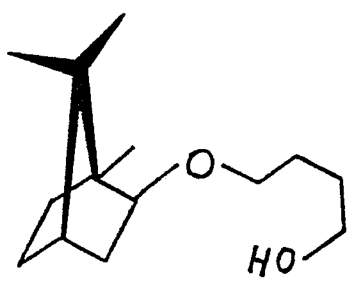

imparts to the pine formulation an intense minty patchouli aroma and causes it to have intense patchouli topnotes. Accordingly, the pine formulation can be described as "piney with an intense patchouli and minty aroma having patchouli topnotes".

The compound having the structure:

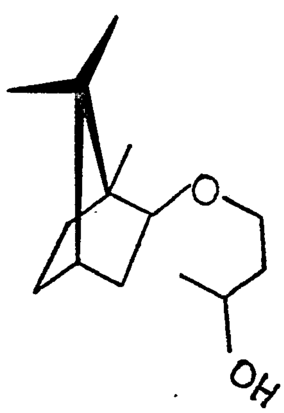

imparts to this piney formulation a very natural-like incense and cedarwood aroma. Accordingly, the formulation thus prepared can be described as "natural piney with cedarwood-like and incense topnotes".

The compound having the structure:

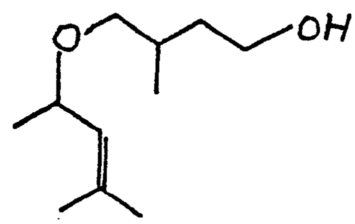

imparts to this piney formulation a camphoraceous, woody, lavender, spicy and floral aroma with patchouli and rosey undertones. Accordingly, the formulation can be described from a perfumery standpoint as "piney with camphoraceous woody, lavender, spicy, floral, patchouli and rosey undertones."

Example XLI

Floral Perfume Compositions

The compound having the structure:

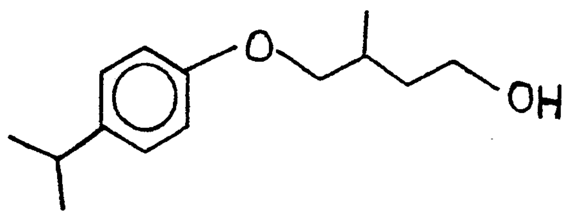

produced according to Example XXXII has an ozoney fresh air dried cloth-like, green, orange, nutty and woody aroma. This material has great warmth and richness and blends well with many floral concepts. It is a rather unique floral note of great value to perfumery. Its use may be demonstrated by the following floral fragrance whereby the compound having the structure:

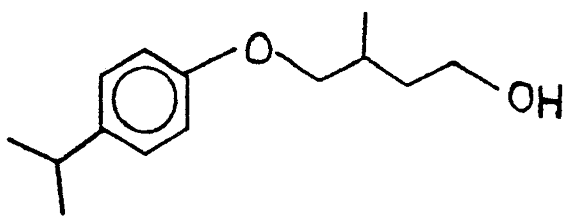

is used to the extent of 5% by weight.

The compound having the structure:

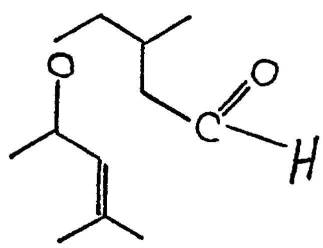

produced according to Example XXXVII imparts to this floral fragrance a herbaceous strong green, leafy, peppery and spicy aroma with herbaceous, peppery, olibanum and diffusive amber-like undertones. The addition of 5% by weight of the compound having the structure:

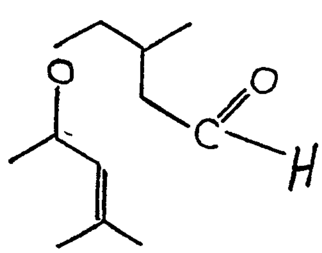

imparts a very desirable spicy and green character.

The compound having the structure:

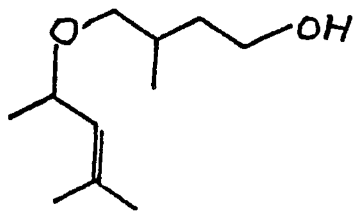

imparts to the floral formulation a camphoraceous, woody, lavender and spicy aroma.

The compound having the structure:

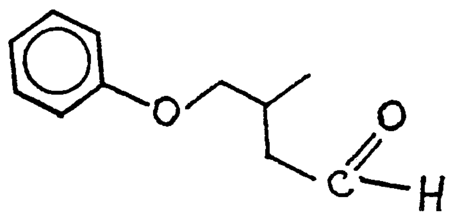

imparts to the floral formulation a herbaceous, sweet fruity, chamomile-like undertone.

All four of these products perform quite well in fragrances and are judged to be very valuable fragrance materials:

FLORAL FRAGRANCE

| | "A" | "B" | "C" | "D" |
|---|---|---|---|---|
| Citronellol | 12.3 | 12.3 | 12.3 | 5.0 |
| Geraniol | 2.5 | 2.5 | 2.5 | 5.0 |
| Amyl Cinnamic Aldehyde | 24.6 | 24.6 | 24.6 | 5.0 |
| Galaxolide® 50 (Trademark Tricyclic Isochroman of International Flavours and Fragrances Inc.) | 9.8 | 9.8 | 9.8 | 5.0 |
| Vertenex High Cis (Cis-t Butylcyclohexenyl Acetate; Para Isomer; | 7.4 | 7.4 | 7.4 | 5.0 |
| Rose Oxide | 0.7 | 0.7 | 0.7 | 5.0 |
| Cinnamic Alcohol | 19.6 | 19.6 | 19.6 | 5.0 |
| Aldehyde C—11 (n-Undecylenic Aldehyde) | 0.5 | 0.5 | 0.5 | 5.0 |
| Aldehyde C—12 (n-Dodecyl Aldehyde in 10% solution in diethyl phthalate) | 0.5 | 0.5 | 0.5 | 5.0 |
| Citronellal (10% solution in diethyl phthalate) | 0.5 | 0.5 | 0.5 | 5.0 |
| Phenyl Ethyl Acetate | 2.5 | 2.5 | 2.5 | 5.0 |
| Ylang Oil | 1.2 | 1.2 | 1.2 | 5.0 |

FLORAL FRAGRANCE (continued)

| | "A" | "B" | "C" | "D" |
|---|---|---|---|---|
| Indisan (Hydrogenated derivative of reaction product of Camphene and Resorcinol) | 3.7 | 3.7 | 3.7 | 5.0 |
| Musk Ketone | 5.0 | 5.0 | 5.0 | 5.0 |
| Oakmoss Resin | 0.5 | 0.5 | 0.5 | 5.0 |
| Liatrix Absolute (10% in diethyl phthalate) | 2.5 | 2.5 | 2.5 | 5.0 |
| Vetiver Acetate | 1.2 | 1.2 | 1.2 | 5.0 |
| Diethyl Phthalate | 5.0 | 5.0 | 5.0 | 5.0 |
| Compound having the structure: 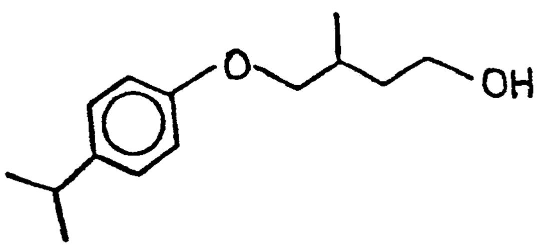 produced according to Example XXXII | 5.0 | 0 | 0 | 0 |
| Compound having the structure: 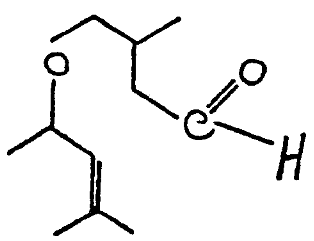 | 0 | 5.0 | 0 | 0 |
| Compound having the structure: 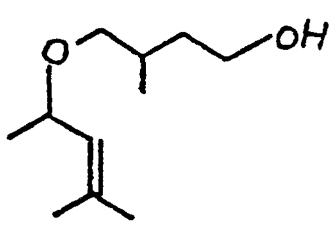 | 0 | 0 | 5.0 | 0 |

FLORAL FRAGRANCE (Cont'd)

| | "A" | "B" | "C" | "D" |
|---|---|---|---|---|
| Compound having the structure: 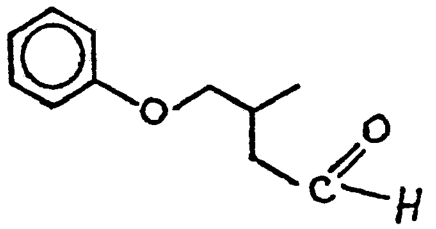 | 0 | 0 | 0 | 5.0 |

Thus, as a result of adding the compound having the structure:

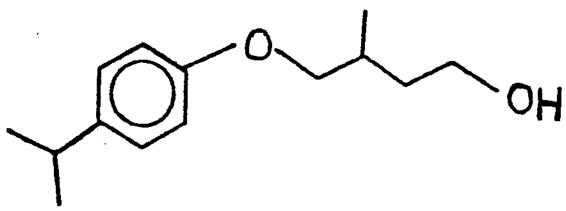

the fragrance can be described as "floral with ozoney, fresh air dried cloth-like, green and fresh orange undertones".

The fragrance produced by having the compound having the structure:

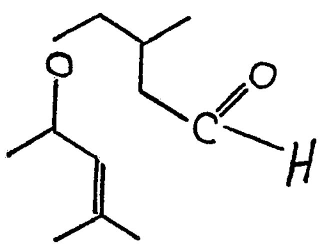

added thereto, can be described as "floral with herbaceous strong green, leafy, pepper, spicy, olibanum and diffusive amber undertones".

The fragrance produced by having added thereto the compound having the structure:

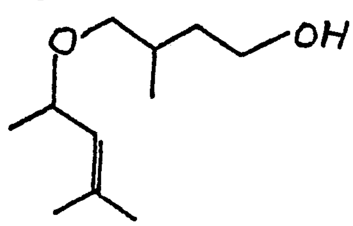

can be described as "floral with camphoraceous, woody, lavender, spicy, patchouli and rosey undertones".

The fragrance having added thereto the compound having the structure:

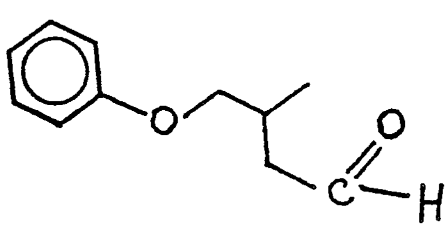

can be described as "floral with herbaceous, sweet fruity, chamomile-like and caramellic undertones".

Example XLII

Preparation of Lilac Fragrance

The following mixture is prepared:

| | |
|---|---|
| Hydroxcitronellal | 22% |
| Phenyl Ethyl Alcohol | 12% |
| Heliotropine | 12% |
| Linalool | 8% |
| Cinnamic Alcohol | 4% |
| Indole — 10% in Diethyl Phthalate | 2% |
| Benzyl Acetate | 8% |
| Anisic Alcohol | 8% |
| Coumarin — 10% in Diethyl Phthalate | 4% |
| Compound having the structure: 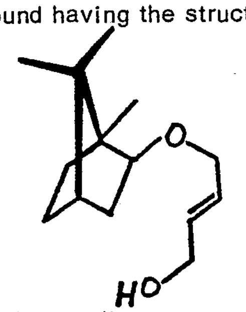 prepared according to Example XXXVI | 4% |

The compound having the structure:

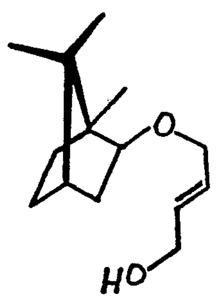

imparts to this lilac fragrance an interesting animalic sweaty lavender-like undertone. Accordingly, the fragrance can be described as "lilac with an animalic sweaty lavender-like undertone".

Example XLIII

Preparation of Cosmetic Powder Compositions

Cosmetic power compositions are prepared by mixing in a ball mill 100 grams of talcum powder with 0.25 grams of each of the substances set forth in Table IV below. Each of the cosmetic powder compositions has an excellent aroma as described in Table IV below:

TABLE IV

| SUBSTANCE | AROMA DESCRIPTION |
| --- | --- |
| Compound having the structure 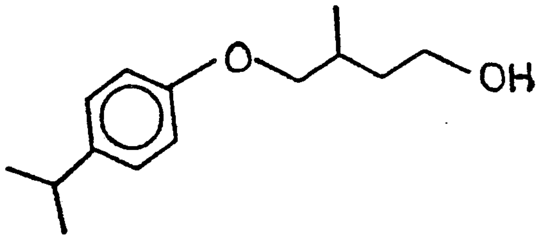 produced according to Example XXXII | A nutty, woody, ozoney, fresh air dried cloth, green, orange aroma profile. |
| Compound having the structure: 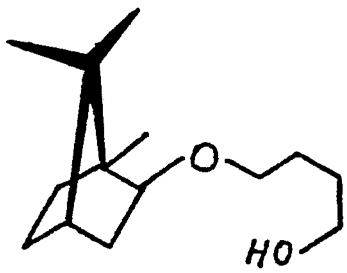 produced according to Example XXXIII or XXXIV | A minty patchouli aroma with patchouli undertones. |
| Compound having the structure: 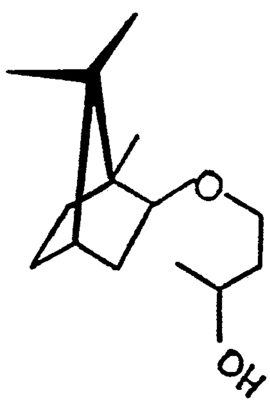 prepared according to Example XXXV | An incense aroma with cedarwood undertones. |

| SUBSTANCE | AROMA DESCRIPTION |
| --- | --- |
| Compound having the structure: 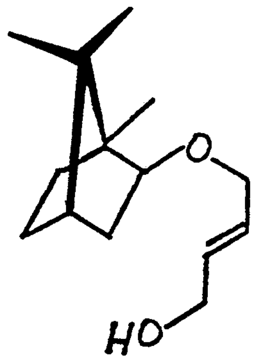 | An onion, garlic and lavender aroma profile with oniony, animalic sweaty undertones. |
| Compound having the structure: 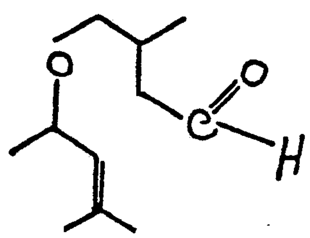 prepared according to Example XXXVII | A herbaceous, strong green leafy, pepper and spicy aroma with herbaceous peppery olibanum and diffusive amber undertones. |
| Compound having the structure: 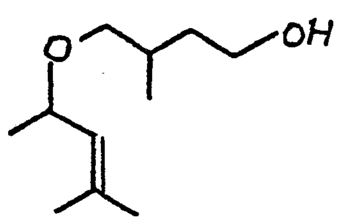 produced according to Example XXXVIII | A camphoraceous, woody, lavender, spicy and floral aroma with patchouli and rosey undertones. |

| SUBSTANCE | AROMA DESCRIPTION |
|---|---|
| Compound having the structure: 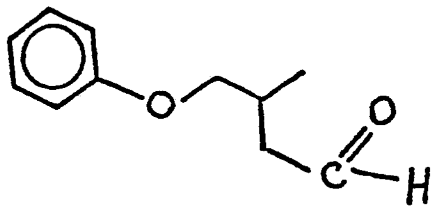 prepared according to Example XXXIX | A herbaceous, sweet fruity, and chamomile-like aroma with caramellic undertones. |
| Fragrance of Example XL(A). | A piney aroma with an intense patchouli and minty aroma having patchouli topnotes. |
| Fragrance of Example XL(B). | A natural piney aroma with cedarwood-like and incense topnotes. |
| Fragrance of Example XL (C). | A piney aroma with camphoraceous, woody, lavender, spicy floral patchouli and rosey undertones. |
| Perfume composition of Example XLI (A). | Floral with ozoney, fresh air dried cloth-like, green and orange undertones. |
| Perfume composition of Example XLI(B) | Floral with herbaceous, strong green, leafy, peppery, spicy, olibanum and diffusive amber undertones. |
| Perfume composition of Example XLI(C) | Floral with camphoraceous, woody, lavender, spicy, patchouli and rosey undertones. |

| SUBSTANCE | AROMA DESCRIPTION |
|---|---|
| Perfume composition of Example XLI (D) | Floral with herbaceous, sweety fruity, chamomile-like and caramellic undertones. |
| Perfume composition of Example XLII | Lilac with an animalic, sweaty, lavender-like undertones. |

Example XLIV

Perfumed Liquid Detergent

Concentrated liquid detergents with aromas as set forth in Table IV of Example XLIII (which detergents are prepared from Lysine salt of n-dodecyl benzene sulfonic acid as more specifically described in United States Letters Patent, Serial No. 3,948,818 issued on April 6, 1976 the specification for which is incorporated by reference herein) are prepared containing each of the substances set forth in Table IV of Example XLIII supra. They are prepared by adding and homogeneously mixing the appropriate quantity of perfumery substance as set forth in Table IV of Example XLIII in the liquid detergent. The detergents all possess aromas as set forth in Table IV of Example XLIII, the intensity increasing with greater concentrations of perfumery substance of Table IV of Example XLIII, supra.

Example XLV

Preparation of a Cologne and Handkerchief Perfume

The perfume substances of Table IV of Example XLIII supra, are incorporated into colognes at concentrations of 1.5%, 2.0%, 2.5%, 3.0% and 4.0% in 80%, 85% and 90% aqueous ethanols; and into a handkerchief perfume composition at concentrations of 10%, 15%, 20%, 25% and 30% (in 85%, 90% and 95% aqueous ethanols). Distinct and definitive aromas as set forth in Table IV of Example XLIII are imparted to the cologne and to the handkerchief perfume compositions.

Example XLVI

Preparation of a Detergent Composition

A total of 100 grams of a detergent powder (a non-ionic detergent powder containing a proteolytic enzyme prepared according to Example I of Canadian Letters Patent 985,190 issued on March 9, 1976 the disclosure of which is incorporated by reference herein) is mixed with 0.15 grams of each of the substances set forth in Table IV of Example XLIII, supra, until substantially homogeneous compositions are obtained. These compositions have excellent aromas as set forth in Table IV of Example XLIII.

Example XLVII

Preparation of Soap

Each of the perfumery substances of Table IV of Example XLIII are incorporated into soap (LVU—1) at 0.1% by weight of each substances. After two weeks in the oven at 90°F. each of the soaps showed no visual effect from the heat. Each of the soaps manifested an excellent aroma as set forth in Table IV of Example XLIII, supra.

Example XLVIII

Preparation of Soap Composition

One hundred grams of soap chips (IVORY®, registered trademark of the Procter & Gamble Co. of Cincinnati, Ohio) are mixed individually with one gram each of the perfumery substances of Table IV of Example XLIII, supra, until a homogeneous composition is obtained. The homogeneous composition is then treated under three atmospheres pressure at 180°C. for a period of three hours and the resulting liquid is placed into a soap mold. The resulting soap cakes, on cooling, manifest excellent aromas as set forth in Table IV of Example XLIII, supra.

Example XLIX

Preparation of a Solid Detergent Composition

A detergent is prepared from the following ingredients according to Example I of Canadian Letters Patent No. 1,007,948 the specification for which is incorporated by reference herein):

| Ingredients | Parts by Weight |
|---|---|
| "Neodol 45—II" (a $C_{14}$—$C_{15}$ alcohol ethoxylated with 11 moles of ethylene oxide) | 12 |
| Sodium carbonate | 55 |
| Sodim citrate | 20 |
| Sodium sulfate, water brighteners | q.s. |

This detergent is a "phosphate-free" detergent. A total of 100 grams of this detergent is admixed separately with 0.15 grams of each of the perfume substances of Table IV of Example XLIII, supra. The detergent samples each have excellent aromas as set forth in Table IV of Example XLIII, supra.

Example L

Utilizing the procedure of Example I at column 15 of U.S. Letters Patent 3,632,396 (the specification for which is incorporated by reference herein), a non-woven cloth substrate useful as a dryer-added fabric softening article of manufacture is prepared, wherein the substrate, the substrate coating and the outer coating and the perfuming material are as follows:

1. a water "dissolvable" paper ("Dissolvo Paper")
2. Adogen 448 (m.p. about 140°F.) as the substrate coating; and
3. an outer coating having the following formulation (m.p. about 150°F.);

57 percent $C_{20-22}$ HAPS
22 percent isopropyl alcohol
20 percent antistatic agent
1 percent of one of the perfume substances of Table IV of Example XLIII, supra.

A fabric softening composition prepared as set forth above having the above aroma characteristics as set forth in Table IV of Example XLIII, supra, essentially consists of a substrate having a weight of about 3 grams per 100 square inches, a substrate coating of about 1.85 grams per 100 square inches of substrate and an outer coating of about 1.4 grams per 100 square inches of substrate, thereby providing a total aromatized substrate and outer coating weight ratio of about 1:1 by weight of substrate. The aroma set forth in Table IV of Example XLIII is imparted in a pleasant manner to the headspace in the dryer on operation thereof, using said dryer-added fabric softening non-woven fabric.

Example LI

Flavor Composition

The following basic walnut flavor formulation is prepared:

| Ingredients | Parts by Weight |
|---|---|
| Ethyl-2-Methyl Butyrate | 10 |
| Vanillin | 40 |
| Butyl Valerate | 40 |
| 2.3-Diethyl Pyrazine | 5 |
| Methyl Cyclopentenolone | 80 |
| Benzaldehyde | 60 |
| Valerian Oil Indian (1% in 95% aqueous ethanol alcohol) | 0.5 |
| Propylene Glycol | 764.5 |

The compound having the structure:

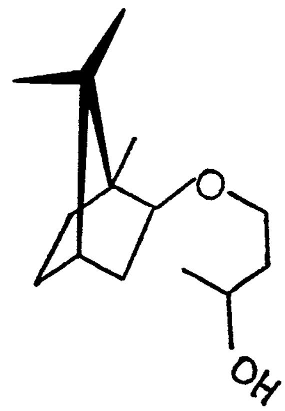

prepared according to the process of Example XXXV is added to the above formulation at the rate of 1.5%. The formulation is compared to a formulation which does not have the compound having the structure:

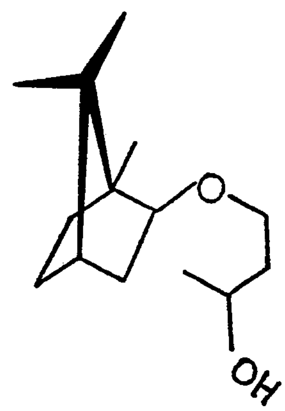

The formulation containing the compound having the structure:

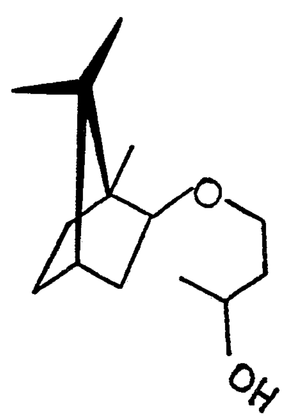

has a woody-balsamic, fresh walnut kernel and walnut skin-like taste and, in addition, has a fuller mouthfeel and longer lasting taste. The flavor that has added to it, the compound having the structure:

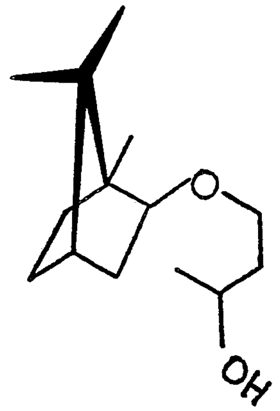

is preferred by a group of flavor panelists not associated with the assignee of the instant application or the inventorship entity and they consider it to be a substantially improved walnut flavor.

Example LII

Orange Flavor Formulation

An orange formulation is prepared by admixing:

| Ingredients | Parts by Weight |
|---|---|
| Natural orange oil | 13.00 |
| Acetaldehyde | 1.50 |
| Ethyl acetate | 0.10 |
| Ethyl butyrate | 0.50 |
| Propanol | 0.10 |
| trans-2-Hexenal | 0.10 |
| Ethyl alcohol (95%) | 60.00 |
| Fusel oil | 0.05 |
| Propylene glycol | 24.65 |

This is denominated Flavor A. A second formulation, Flavor B is prepared by adding the compound having the structure:

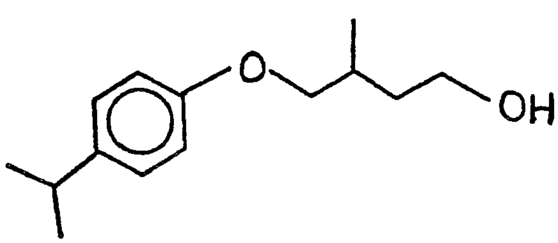

prepared according to Example XXXII to a portion of Flavor A and the ratio of two parts to 100 parts of Flavor A.

Each of Flavors A and B is added in the amount of 2 ounces per gallon of 32° Baume sugar syrup to produce a syrup for combination with water to form a drink. The beverage prepared using Flavor A is a passable orange beverage of good character, flavor and intensity.

The beverage prepared using Flavor B has a much improved flavor. The improvement contributed by the compound having the structure:

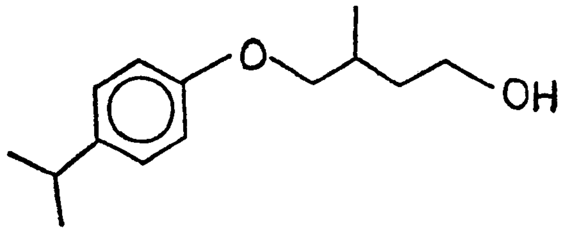

is due to:

i. a greater degree of natural character of freshly squeezed orange juice,
ii. an increase in the pulplike notes and
iii. greater orange juice flavor depth.

Example LIII

0.5 Grams of the mixture:

| Ingredients | Parts by Weight |
|---|---|
| Dimethyl sulfide | 4 |
| Methyl propyl disulfide | 25 |
| Methyl propenyl disulfide | 2 |
| Dipropyl disulfide | 12 |
| Propyl propenyl disulfide | 8 |
| Diallyl disulfide | 1 |
| Compound having the structure 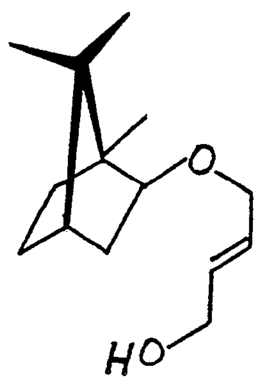 prepared according to Example XXXVI | 12 |

is emulsified in a solution containing the following materials:

100 grams gum arabic
300 grams water
0.5 grams 20 percent solution in ethanol of butylated hydroxy anisole.

The resultant emulsion is spray-dried in a Bowen Lab. Model spray-drier, inlet temperature 500°F., outlet temperature 200°F. 12 Grams of this spray-dried material is mixed with 29.2 grams of the following soup base:

| Ingredients | Parts by Weight |
|---|---|
| Fine ground sodium chloride | 35.62 |
| Hydrolized vegetable protein [4 BE:Nestle's] | 27.40 |
| Mono sodium glutamate | 17.81 |
| Mono calcium glutamate | 17.81 |
| Sucrose | 10.96 |
| Beef Fat | 5.48 |
| Sethness caramel color [powder B and C] | 2.73 |

The resultant mixture is then added to 12 ounces of boiling water and an excellent onion-flavored soup is obtained which is comparative to that created using natural onions or natural onion oil.

Example LIV

Toothpaste

The compound having the structure:

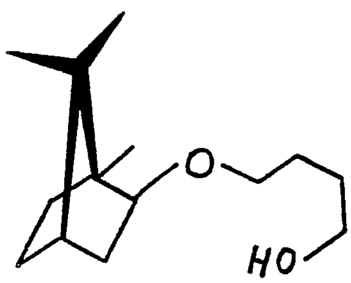

prepared according to Example XXXIII or XXXIV is incorporated at the rate of 0.1% in toothpaste (Colgate MFP Fluoride Gel "Winterfresh" manufactured by the Colgate Palmolive Corporation of New York, N.Y.) and evaluated by a panel of nine people for its aroma characteristics against a control without the addition of the compound having the structure:

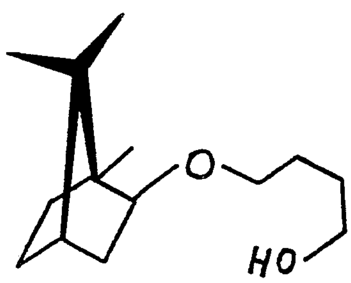

The entire panel unanimously preferred the toothpaste with the compound having the structure:

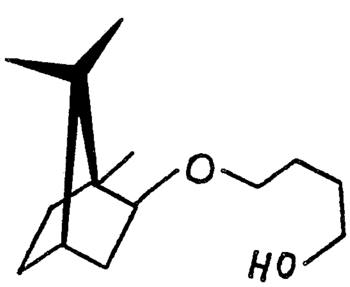

as having a fresher, more cooling aroma with patchouli overtones and oriental, incense, musky and sandalwood nuances than the control.

A small group of panelists (three in number) brushed their teeth with the same toothpaste using 0.1%, 0.2% and 0.5% of the compound having the structure:

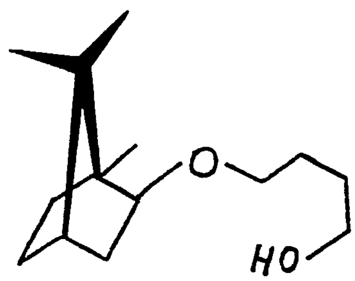

produced according to Example XXXIII or XXXIV brushing their teeth at three hour intervals. All members of the panel are of the opinion that the clean, fresh sensation lasted longer with the toothpaste containing the compound having the structure:

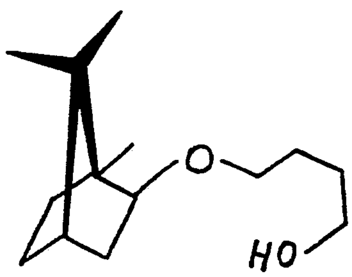

than without it. In addition, all members of the panel state than the bitter after-taste related to the saccharin in the toothpaste is depressed and a more pleasant after-taste is created. No off-note is created by the compound having the structure:

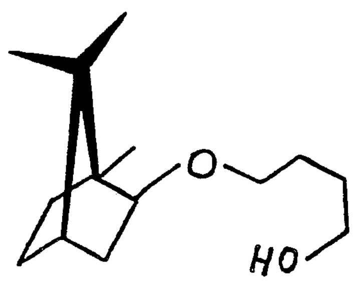

In addition, when the compound having the structure:

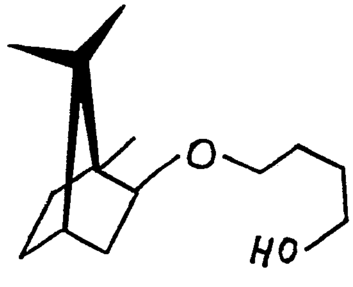

is used at a level 0.5% the same results are obtained with no off-note created by the compound having the structure:

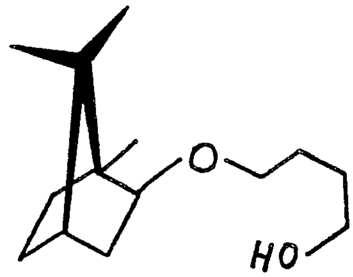

Therefore, it is concluded that the compound having the structure:

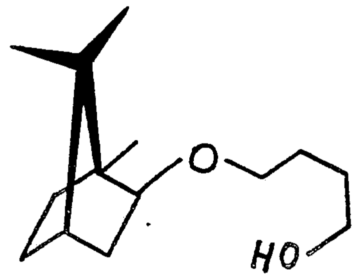

is effective in improving oral hygiene products including toothpaste, mouthwash, mouth sprays and sugar-based tablets and in addition, chewing gum.

The compound having the structure:

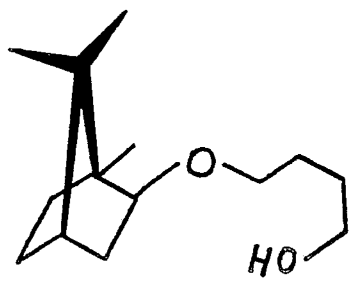

improves the organoleptic properties of toothpaste, chewing gum, mouthwash, mouth sprays and sugar-based mouth freshener tablets:

(i) by adding fresher topnotes

(ii) by enhancing the menthol-like cooling notes (without using menthol)

(iii) by extending the fresh after-taste without having an effect similar to that of chloroform (without the use of chloroform), and

(iv) by imparting oriental incense, musky and sandalwood and patchouli nuances to the toothpaste.

The foregoing effects are unexpected, unobvious and advantageous.

Example LV(A)

Flavor Preparation

The following flavor formulation is prepared by admixing the ingredients in the proportion set forth below:

| Ingredients | Parts by Weight |
|---|---|
| Clove oil | 1.0 |
| Cardamom oil | 0.1 |
| Spearmint oil | 5.0 |
| Anethol | 2.0 |
| Compound having the structure: 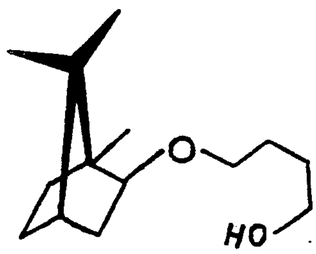 produced according to Examples XXXIII or XXXIV | 4.0 |
| Peppermint oil, redistilled | 83.9 |
| Prenyl methyl carbonate | 2.0 |

Example LV(B)

Ten parts by weight of 50 Bloom pigskin gelatin is added to ninety parts by weight of water at a temperature of 150°F. The mixture is agitated until the gelatin completely dissolves and the solution is cooled to 120°F. Twenty parts by weight of the flavor of Part (A) supra is added to the solution which is then homogenized to form an emulsion, having a particle size typically in the range of 2—5 microns. The material is kept at 120°F. under which conditions the gelatin will not gel.

Coacervation is induced by adding, slowly and uniformly 40 parts by weight of a 20% aqueous solution of sodium sulfate. During coacervation, the gelatin molecules are deposited uniformly about each oil droplet as a nucleus.

Gelatin is effected by pouring the heated coacervate mixture into 1,000 parts by weight of a 7% aqueous solution of sodium sulfate at 65°F. The resulting jelled coacervate may be filtered and washed with water at temperatures below the melting point of gelatin to remove the salt.

Hardening of the filter cake, in this example, is effected by washing with 200 parts by weight of 37% solution of formaldehyde in water. The cake is then washed to remove residual formaldehyde.

Example LV(C)

The following mixture is prepared:

| Ingredients | Parts by Weight |
|---|---|
| Liquid flavor composition of Part (A) supra | 48.4 |
| Cab-O-Sil M—5 (brand of silica produced by the Cabot Corporation of 125 High Street, Boston, Mass. 02110 Physical properties: Surface Area: $200 m^2/gm$. Nominal particle size: 0.012 microns Density: 2.3 lbs/cu. ft. | 3.2 |

The Cab-O-Sil is dispersed in the liquid flavor composition of Part (A) with vigorous stirring thereby resulting in a viscous liquid. 48.4 Parts by weight of the powder flavor composition produced according to Part (B) supra, is then blended into said viscous liquid with stirring at 25°C. for a period of 30 minutes resulting in a thixotropic sustained release patchouli-like and incense/ethereal/"cooling effect" flavored composition.

Example LVI

Chewing Gum

One hundred parts by weight of chicle are mixed with four parts by weight of the flavor prepared in accordance with Example LV(B). Three hundred parts of sucrose and one hundred parts of corn syrup are added. Mixing is effected in a ribbon blender with jacketed side walls of the type manufactured by the Baker Perkins Co.

The resultant chewing gum blend is then manufactured into strips 1 inch in width and 0.1 inches in thickness. The strips are cut into lengths of 3 inches each. On chewing, the chewing gum has a pleasant long-lasting, patchouli-like and incense/cooling/freshening flavor.

Example LVII

Toothpaste Formulation

The following separate groups of ingredients are prepared:

| Ingredients | Parts by Weight |
|---|---|
| Group "A" | |
| Glycerine | 30.200 |
| Distilled water | 15.325 |
| Sodium benzoate | 0.100 |
| Saccharin sodium | 0.125 |
| Stannous fluoride | 0.400 |
| Group "B" | |
| Calcium carbonate | 12.500 |
| Dicalcium phosphate (dihydrate) | 37.200 |
| Group "C" | |
| Sodium N-lauroyl sarcosinate (foaming agent) | 2.000 |
| Group "D" | |
| Flavor materials of Example LV(B) | 1.200 |

Procedure:

1. The ingredients in Group "A" are stirred and heated in a steam jacketed kettle to 160°F.
2. Stirring is continued for an additional three to five minutes to form a homogeneous gel.
3. The powders of Group "B" are added to the gel while mixing until a homogeneous paste is formed.
4. With stirring, the flavor of "D" is added and lastly the sodium n-lauroyl sarcosinate.
5. The resultant slurry is then blended for one hour. The completed paste is then transferred to a three roller mill and then homogenized and finally tubed.

The resulting toothpaste when used in a normal toothbrushing procedure yields a pleasant patchouli-like and incense/cooling/freshening flavour of constant strong intensity throughout said procedure (1—1.5 minutes).

Example LVIII

Tobacco Flavor Formulation

Cigarettes are produced using the following tobacco formulation:

| Ingredients | Parts by Weight |
|---|---|
| Bright | 40.1 |
| Burley | 24.9 |
| Maryland | 1.1 |
| Turkish | 11.6 |
| Stem (flue-cured) | 14.2 |
| Glycerine | 2.8 |
| $H_2O$ | 5.3 |

At the rate of 0.2%, the following tobacco flavor formulation is applied to all of the cigarettes produced with the above tobacco formulation.

| Ingredients | Parts by Weight |
|---|---|
| Ethyl Butyrate | .05 |
| Ethyl Valerate | .05 |
| Maltol | 2.00 |
| Cocoa Extract | 26.00 |
| Coffee Extract | 10.00 |
| Ethyl Alcohol (95%) | 20.00 |
| $H_2O$ | 41.90 |

To 50% of the cigarettes, 10 and 20 ppm of the compound having the structure:

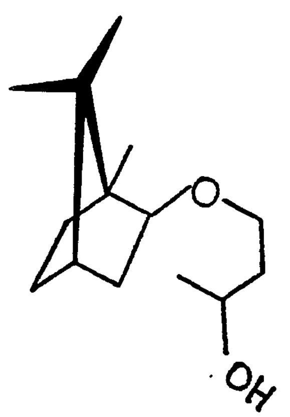

are added. These cigarettes are hereinafter called "experimental" cigarettes and the cigarettes without the compound having the structure:

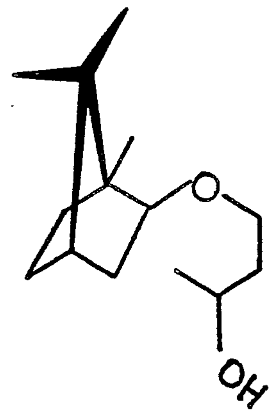

produced according to Example XXXV are hereinafter called "control" cigarettes. The control and experimental cigarettes are then evaluated by paired comparison and the results are as follows:

a. In aroma, the experimental cigarettes are found to be more aromatic with a woody, incense, oriental and patchouli aroma and taste.

b. In smoke flavor, the experimental cigarettes are found aromatic, more sweet, more bitter, richer and slightly less harsh in the mouth and more cigarette tobacco-like than the control cigarettes with woody, incense, oriental and patchouli-like aroma and taste nuances.

In summary, the experimental cigarettes containing 20 ppm is the compound having the structure:

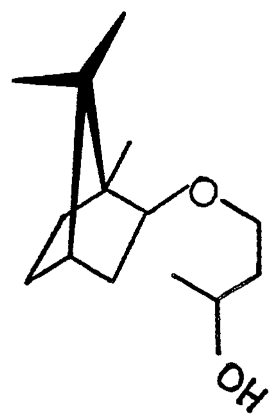

produced according to Example VI are found to be woody, incense, oriental and patchouli-like and turkish tobacco-like in the main stream and in the side stream.

All cigarettes both control and experimental are evaluated for smoke flavor with 20 mm cellulose acetate filters.

A similar effect occurs when using the compound having the structure:

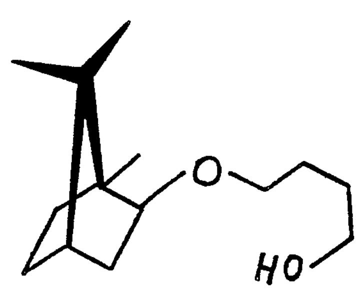

produced according to Examples XXXIII or XXXIV.

## Claims

1. A compound of the formula Q—CHO wherein Q is

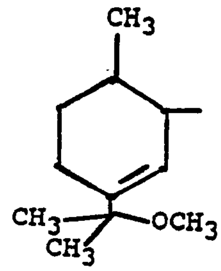

or 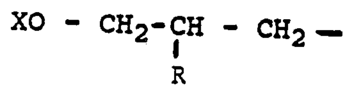

wherein X is selected from

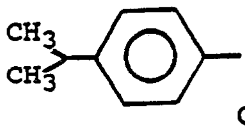

,

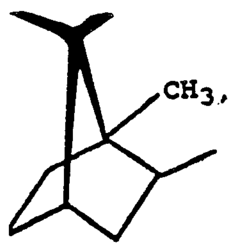

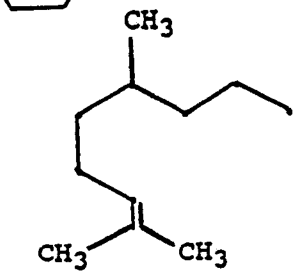

,

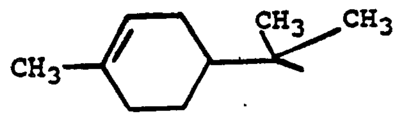

,

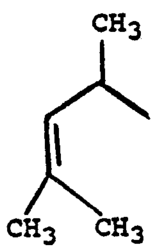

or phenyl

and R is hydrogen or methyl.

2. The compound of claim 1, having the formula:

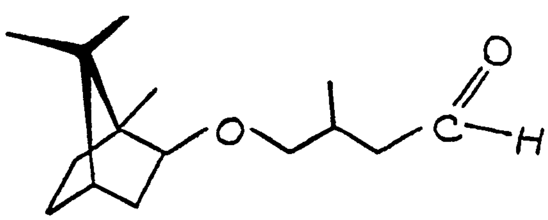

.

3. The compound of claim 1, having the formula:

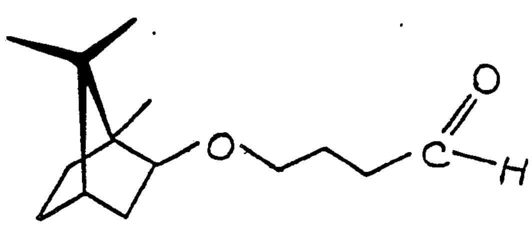

.

4. The compound of claim 1, having the formula:

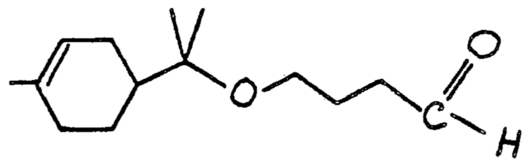

5. The compound of claim 1, having the formula:

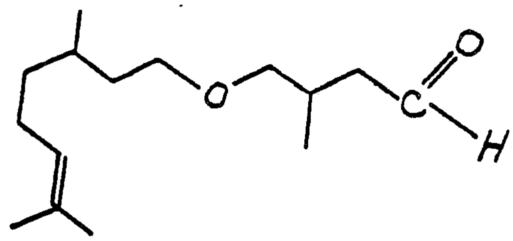

6. The compound of claim 1, having the formula:

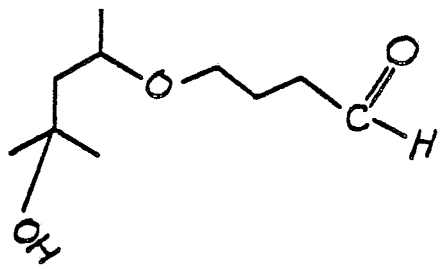

7. The compound of claim 1, having the formula:

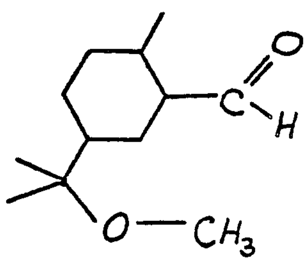

8. The compound of claim 1, having the formula:

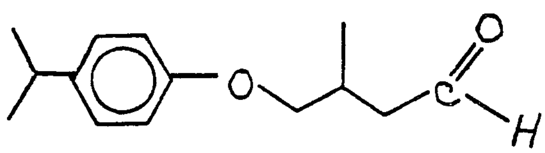

9. The compound of claim 1, having the formula:

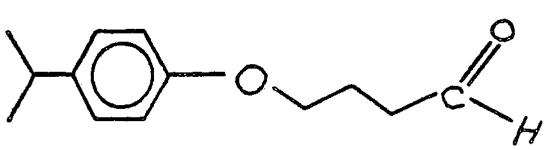

10. The ether aldehyde of claim 1, having the structure:

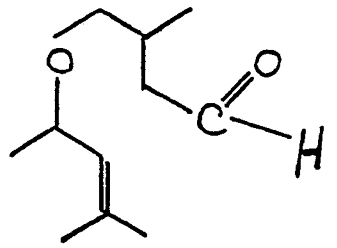

11. The ether aldehyde of claim 1, having the structure:

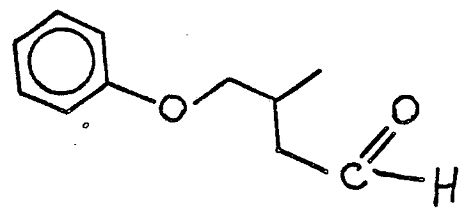

12. A process for the production of a compound as described in claim 1, which comprises reacting a mixture of carbon monoxide and hydrogen with an ether having the formula:

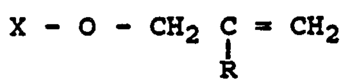

or

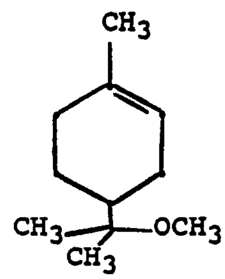

in the presence of an "oxo" reaction catalyst whereby a product is produced containing a major proportion of compounds as defined in claim 1.

13. A process for augmenting or enhancing the aroma or taste of a consumable material selected from the group consisting of perfume compositions, perfumed articles, colognes, foodstuffs, chewing gums, toothpastes, medicinal products, chewing tobaccos, smoking tobaccos and smoking tobacco articles comprising the step of adding to said consumable material an aroma or taste augmenting or enhacing quantity of a produced defined according to any one of claims 1—11.

14. An ether carbinol defined according to the structure:

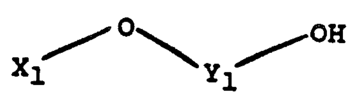

wherein $X_1$ is a moiety selected from the group consisting of:

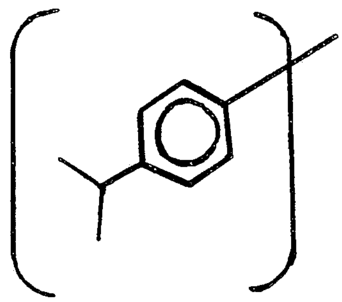 ; 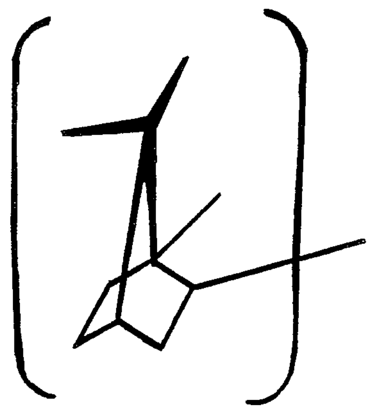 ;

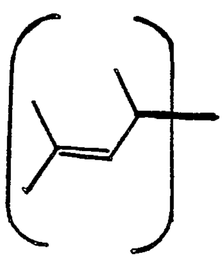 and 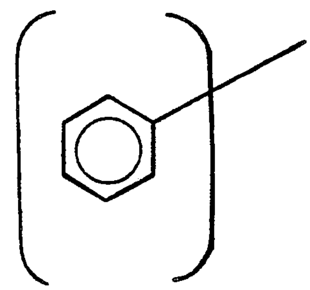

and wherein $Y_1$ is a moiety selected from the group consisting of:

$C_4$—$C_5$ alkylene;

$C_4$—$C_5$ alkenylene; and

$C_4$—$C_5$ alkynylene.

15. The ether carbinol of claim 14, having the structure:

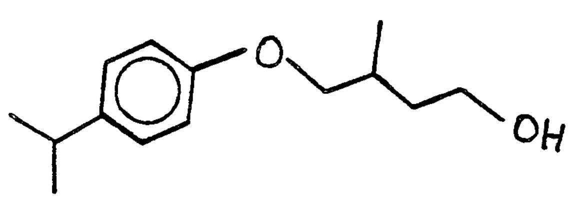

16. The ether carbinol of claim 14, having the structure:

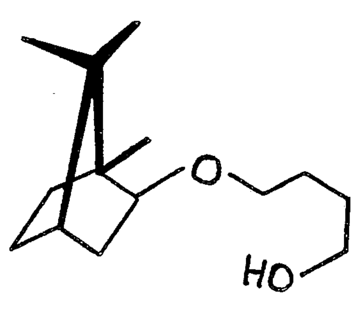

17. The ether carbinol of claim 14, having the structure:

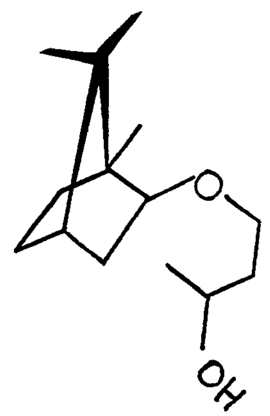

18. The ether carbinol of claim 14, having the structure:

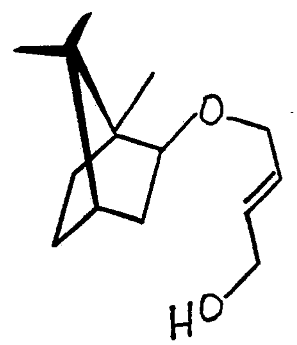

19. The ether carbinol of claim 14, having the structure:

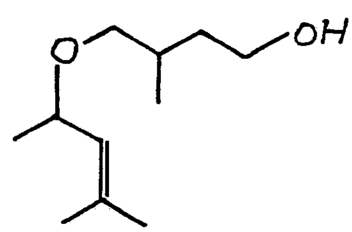

20. The ether carbinol of claim 14, having the structure:

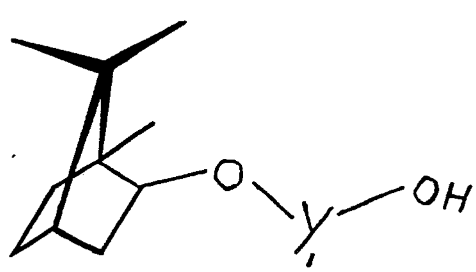

wherein $Y_1$ is selected from the group consisting of:

$C_4$—$C_5$ alkylene;

$C_4$—$C_5$ alkenylene; and

$C_4$—$C_5$ alkynylene.

21. A process for augmenting or enhancing the aroma or taste a consumable material selected from the group consisting of perfumed compositions, perfumed articles, colognes, foodstuffs, chewing gum, chewing tobaccos, medicinal products, toothpastes, smoking tobaccos and smoking tobacco articles

comprising the step of adding to said consumable material an aroma or taste augmenting or enhancing quantity of at least one ether carbinol defined according to any one of claims 14—20.

22. A process for producing a compound defined according to the formula:

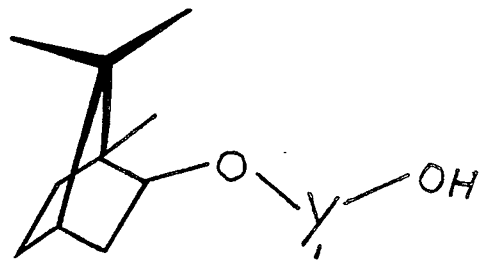

wherein $Y_1$ is a moiety selected from the group consisting of:

$C_4$—$C_5$ alkylene;
$C_4$—$C_5$ alkenylene; and
$C_4$—$C_5$ alkynylene.

comprising the step of adding to camphene defined according to the formula:

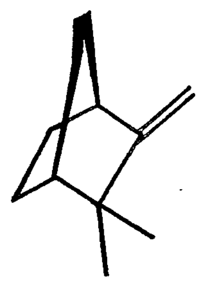

a diol defined according to the structure:

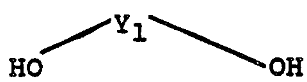

thereby effecting the reaction:

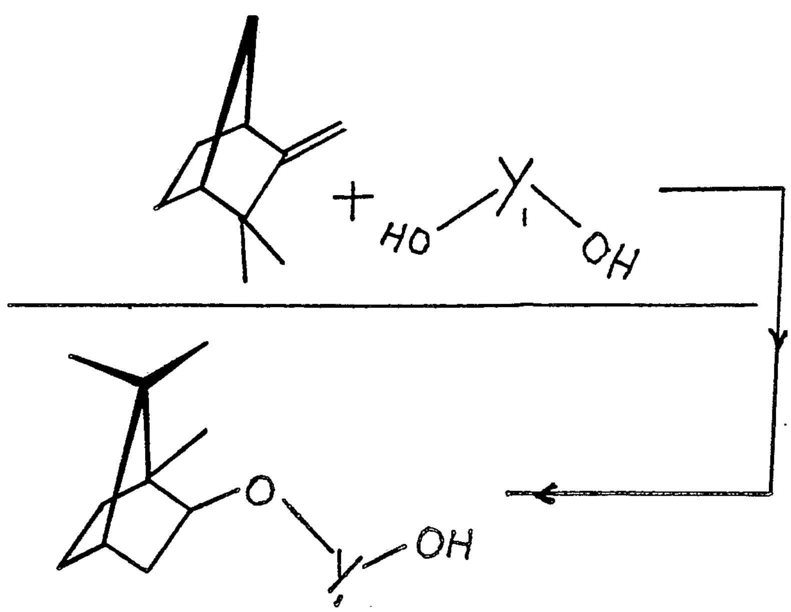

23. A process for preparing a mixture of compounds containing a major proportion of compounds defined according to the formula:

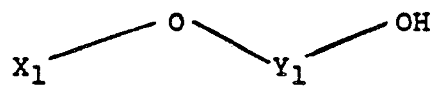

wherein $X_1$ represents a moiety selected from:

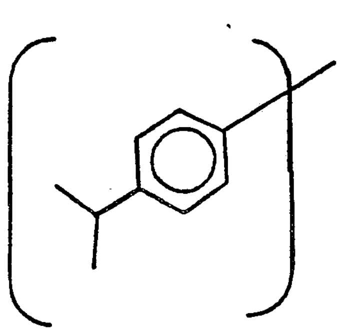

;

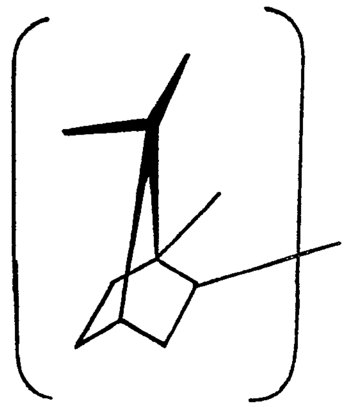

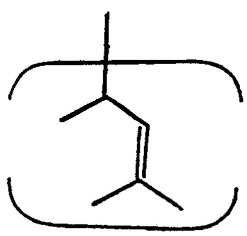

and

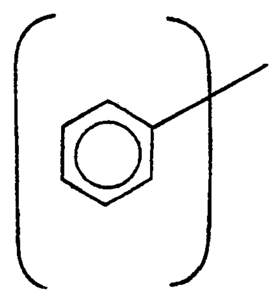

and wherein $Y_1$ is $C_4$—$C_5$ alkylene comprising the step of reacting an allyl ether of the formula:

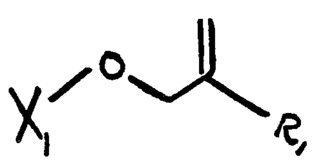

with a mixture of carbon monoxide and hydrogen in the presence of an oxo reaction catalyst thereby forming a mixture of aldehydes of the formula:

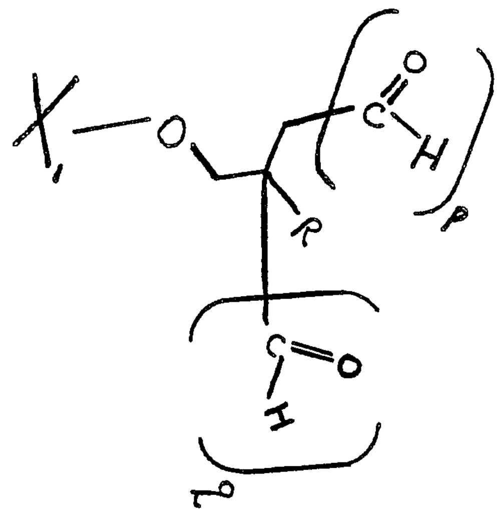

wherein R represents hydrogen or methyl and wherein p is 0 or 1 and q is 0 or 1 and wherein the sum of p + q = 1 and then subjecting the mixture of aldehydes of the formula:

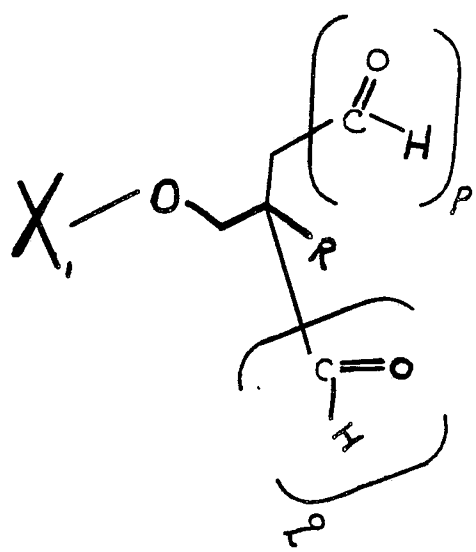

to hydrogenation using hydrogen and a hydrogenation catalyst thereby forming a mixture of compounds defined according to the structure:

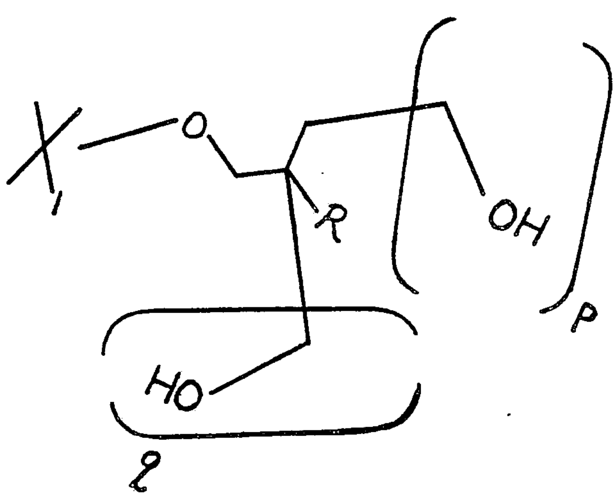

24. The process for augmenting or enhancing the aroma or taste of a consumable material selected from the group consisting of perfume compositions, perfumed articles, colognes, foodstuffs, chewing gums, toothpastes, medicinal products, chewing tobaccos, smoking tobaccos and smoking tobacco articles comprising the step of adding to said consumable material a mixture of ether carbinols defined according to claim 23.

**Patentansprüche**

1. Verbindung der Formel Q—CHO in der Q

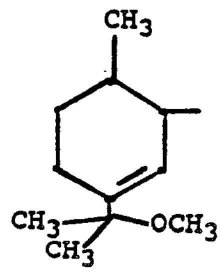

oder oder 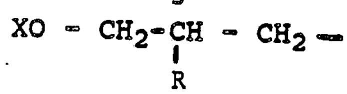

ist, wobei X ausgewählt ist aus

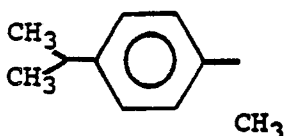

,

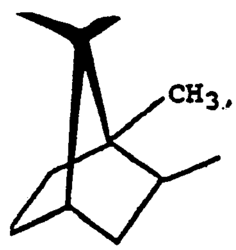

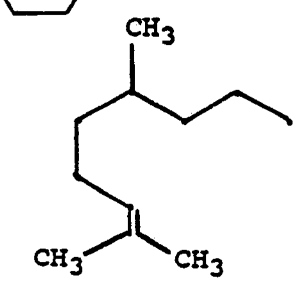

,

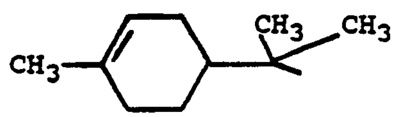

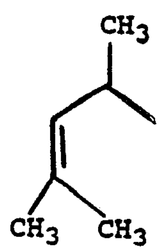

oder Phenyl und R Wasserstoff oder Methyl ist.

2. Verbindung nach Anspruch 1 mit der Formel

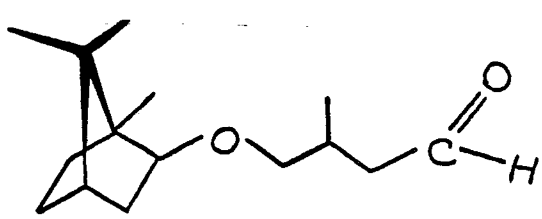

3. Verbindung nach Anspruch 1 mit der Formel

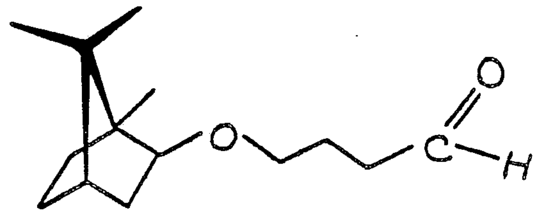

4. Verbindung nach Anspruch 1 mit der Formel

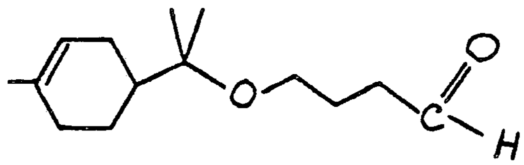

5. Verbindung nach Anspruch 1 mit der Formel

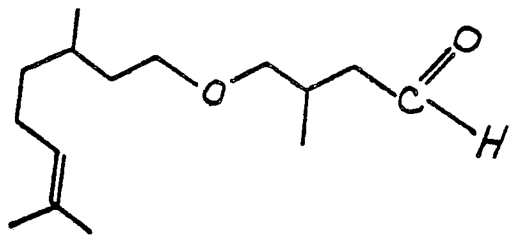

6. Verbindung nach Anspruch 1 mit der Formel

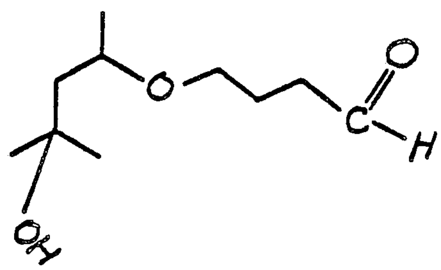

7. Verbindung nach Anspruch 1 mit der Formel

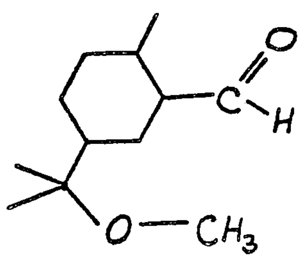

8. Verbindung nach Anspruch 1 mit der Formel

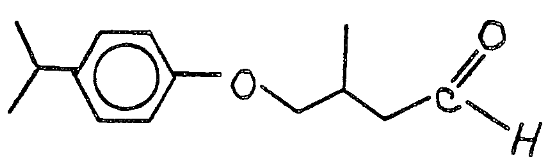

9. Verbindung nach Anspruch 1 mit der Formel

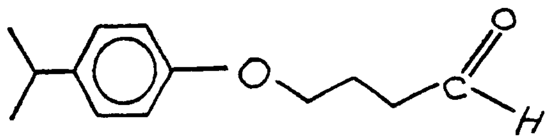

.

10. Etheraldehyd nach Anspruch 1 mit der Struktur

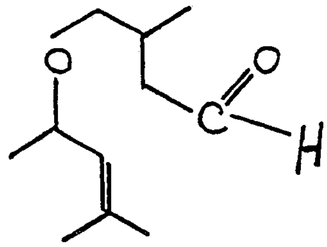

11. Etheraldehyd nach Anspruch 1 mit der Struktur

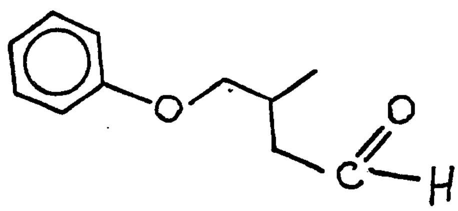

.

12. Verfahren zum Erzeugen einer Verbindung nach Anspruch 1, in dem ein Gemisch von Kohlenmonoxid und Wasserstoff mit einem Ether der Formel

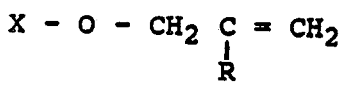 oder 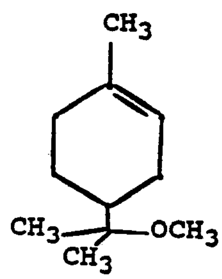

in Gegenwart eines Oxoreaktionskatalysators zu einem Produkt umgesetzt wird, das zum größten Teil aus Verbindungen nach Anspruch 1 besteht.

13. Verfahren zum Verstärken oder Verbessern des Aromas oder Geschmacks eines Verbrauchsgutes, das aus der Gruppe ausgewählt ist, die aus den Parfümzusammensetzungen, parfümierten Artikeln, Kölnischwässern, Nahrungs- und Genußmitteln, Kaugummis, Zahnpasten, medizinischen Produkten, Kautabaken, Rauchtabaken und Rauchtabakartikeln besteht, in dem dem Verbrauchsgut eine des Aroma oder den Geschmack verstärkende oder verbessernde Menge eines Produkts nach einem der Ansprüche 1 bis 11 zugesetzt wird.

14. Ethercarbinol mit der Struktur

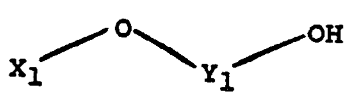

in der $X_1$ ein Anteil ist, der aus der Gruppe ausgewählt ist, die aus

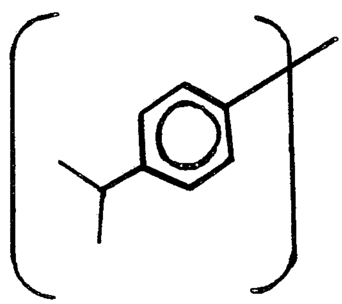

;

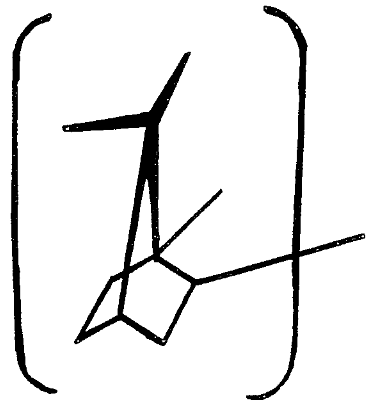

;

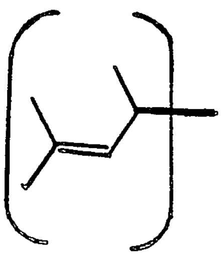

und

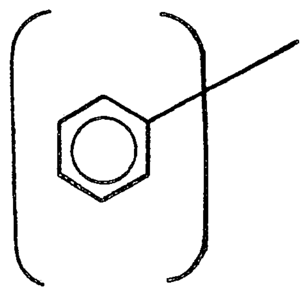

besteht
und $Y_1$ ein Anteil ist, der aus der Gruppe ausgewählt ist, die aus
$C_4$—$C_5$-Alkylen;
$C_4$—$C_5$-Alkenylen und
$C_4$—$C_5$-Alkynylen
besteht.

15. Ethercarbinol nach Anspruch 14 mit der Struktur

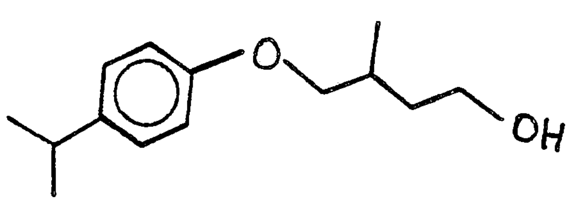

16. Ethercarbinol nach Anspruch 14 mit der Struktur

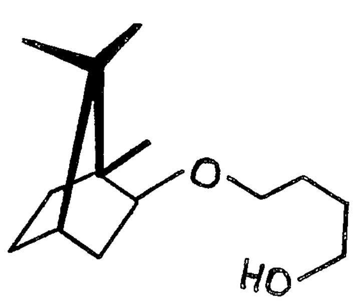

17. Ethercarbinol nach Anspruch 14 mit der Struktur

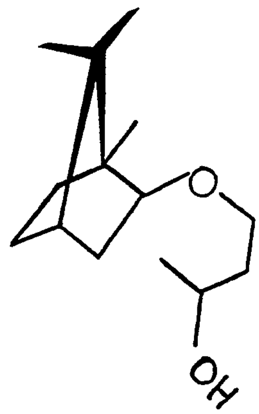

18. Ethercarbinol nach Anspruch 14 mit der Struktur

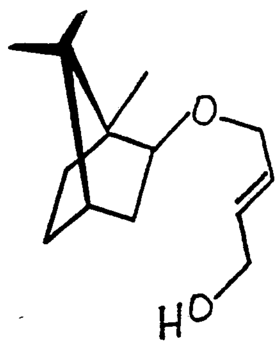

19. Ethercarbinol nach Anspruch 14 mit der Struktur

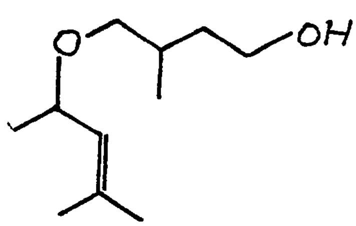

20. Ethercarbinol nach Anspruch 14 mit der Struktur

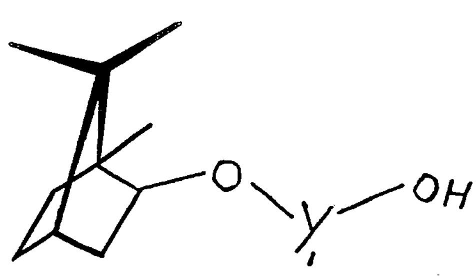

wobei $Y_1$ aus der Gruppe ausgewählt ist, die aus

$C_4$—$C_5$-Alkylen;

$C_4$—$C_5$-Alkenylen und

$C_4$—$C_5$-Alkynylen

besteht.

21. Verfahren zum Verstärken oder Verbessern des Aromas oder Geschmacks eines Verbrauchsgutes, das aus der Gruppe ausgewählt ist, die aus den Parfümzusammensetzungen, parfümierten Artikeln, Kölnischwässern, Nahrungs- und Genußmitteln, Kaugummis, Zahnpasten, medizinischen Produkten,

Kautbaken, Rauchtabaken und Rauchtabakartikeln besteht, in dem dem Verbrauchsgut eine des Aroma oder den Geschmack verstärkende oder verbessernde Menge mindestens Ethercarbinols nach einem der Ansprüche 14 bis 20 zugesetzt wird.

22. Verfahren zum Erzeugen einer Verbindung der Formel

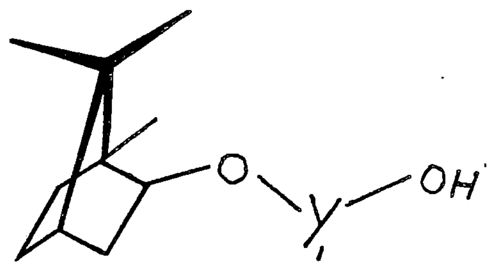

in der $Y_1$ ein Anteil ist, der aus der Gruppe ausgewählt ist, die aus

$C_4$—$C_5$-Alkylen;
$C_4$—$C_5$-Alkenylen und
$C_4$—$C_5$-Alkynylen

besteht

in dem zu einem Camphen der Formel

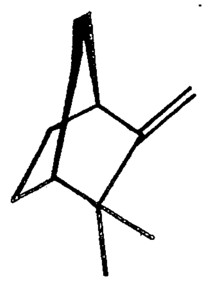

ein Diol mit der Struktur

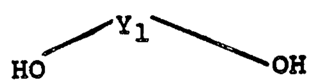

zugesetzt und dadurch die Reaktion

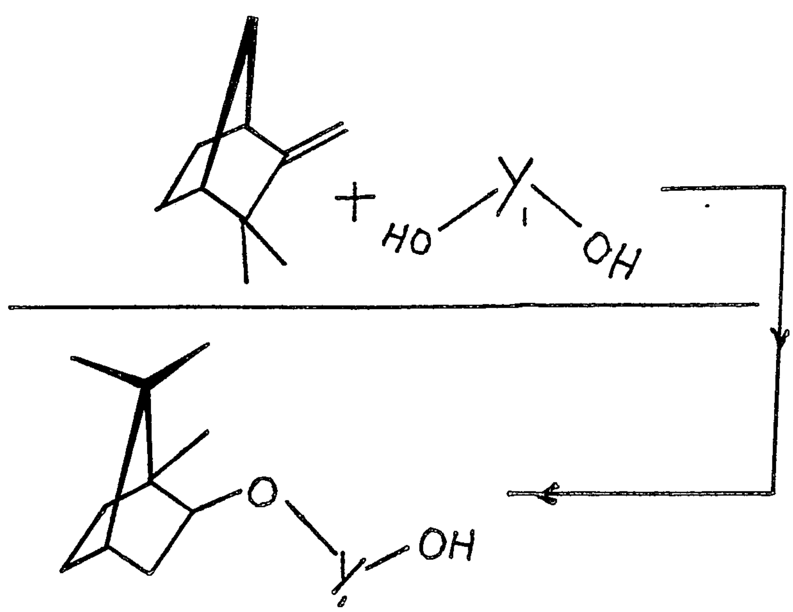

durchgeführt wird.

23. Verfahren zum Herstellen einem Gemisches won Verbindungen, das zum größten Teil aus Verbindungen der Formel

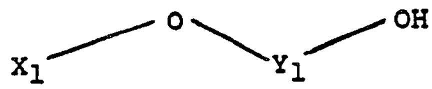

besteht, in der $X_1$ einen Anteil darstellt, der ausgewählt ist aus

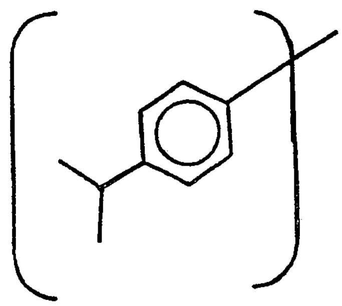

;

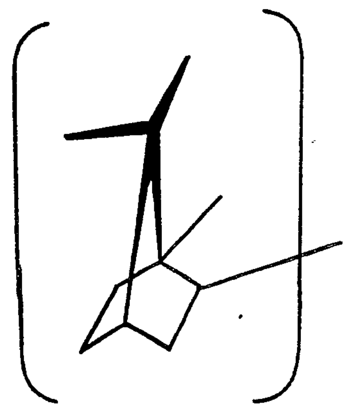

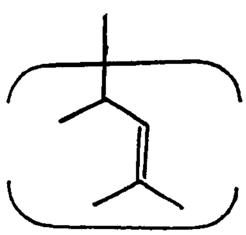

und

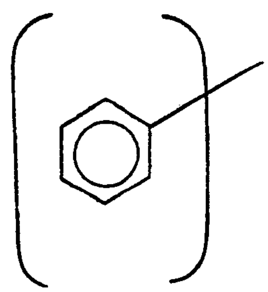

und $Y_1$ ein $C_4$—$C_5$-Alkylen ist,
wobei ein Allylether der Formel

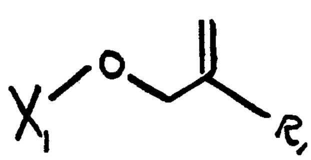

mit einem Gemisch von Kohlenmonoxid und Wasserstoff in Gegenwart eines Oxoreaktionskatalysators umgesetzt und dadurch ein Gemisch von Aldehyden der Formel

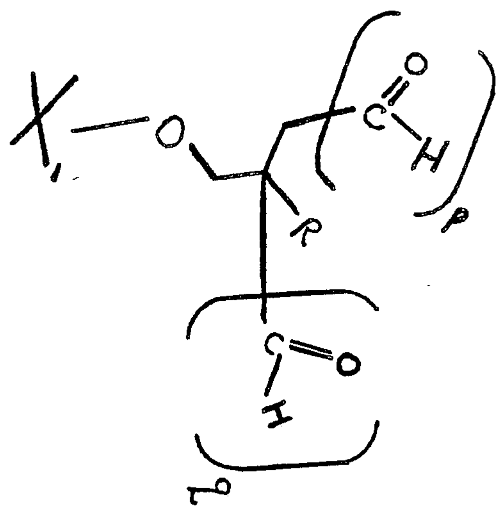

erzeugt wird, in der R Wasserstoff oder Methyl, p gleich 0 oder 1, q gleich 0 oder 1 und die Summe von p + q = 1 ist, und danach das Gemisch von Aldehyden der Formel

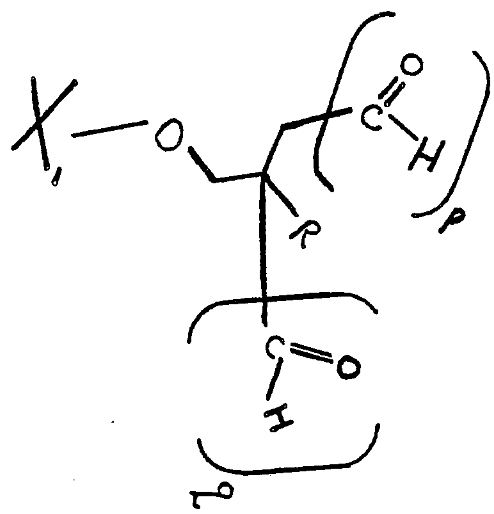

unter Verwendung von Wasserstoff und eines Hydrierkatalysators hydriert und dadurch ein Gemisch von Verbindungen der Struktur

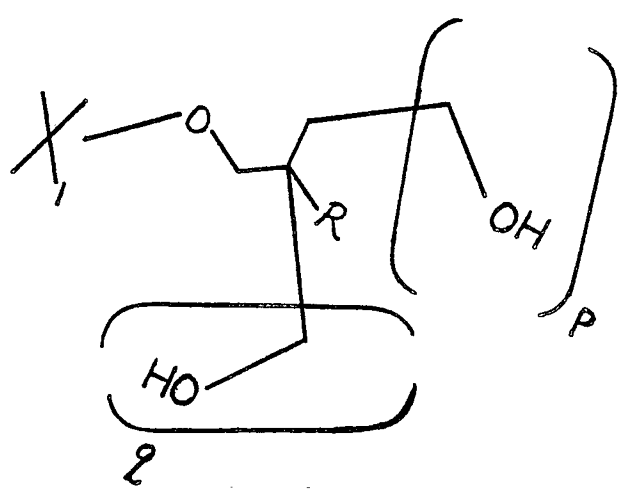

gebildet wird.

24. Verfahren zum Verstärken oder Verbessern des Aromas oder Geschmacks eines Verbrauchsgutes, das aus der Gruppe ausgewählt ist, die aus den Parfümzusammensetzungen, parfümierten Artikeln, Kölnischwässern, Nahrungs- und Genußmitteln, Kaugummis, Zahnpasten, medizinischen Produkten, Kautabaken, Rauchtabaken und Rauchtabakartikeln besteht, in dem dem Verbrauchsgut eine des Aroma oder den Geschmack verstärkende oder verbessernde Menge ein Gemisch von Ethercarbinolen nach Anspruch 23 zugestzt wird.

**Revendications**

1. Composé de formule Q—CHO où Q représente

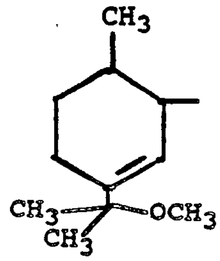

ou

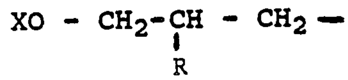

où X est choisi entre

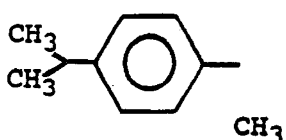

,

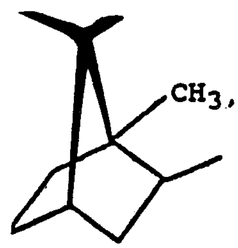

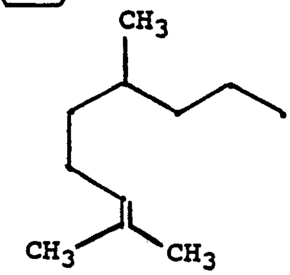

,

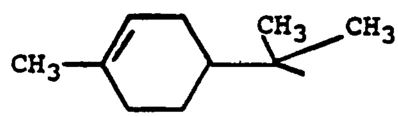

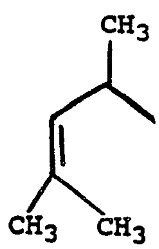

ou phényle

et R est un hydrogène ou un méthyle.

2. Composé de la revendication 1, de formule

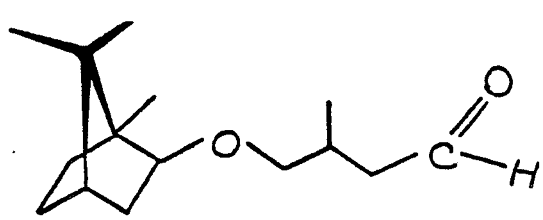

.

3. Composé de la revendication 1, de formule

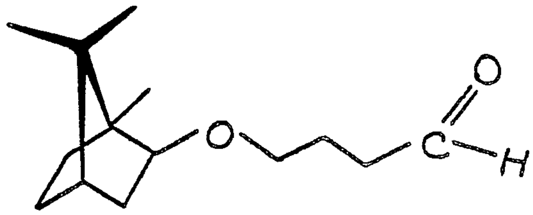

4. Composé de la revendication 1, de formule

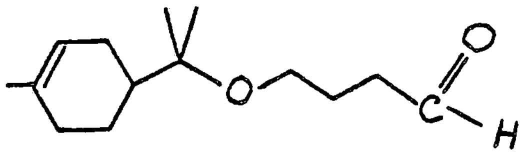

5. Composé de la revendication 1, de formule

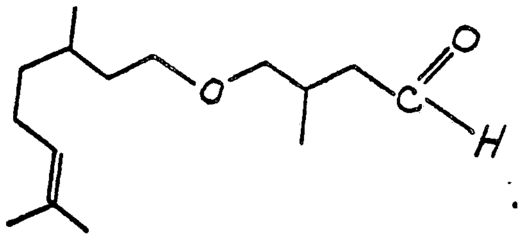

6. Composé de la revendication 1, de formule

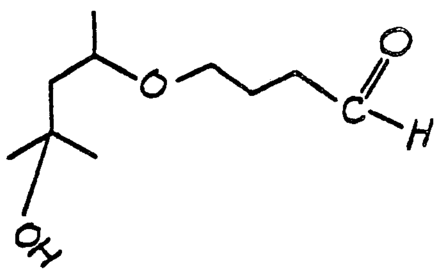

7. Composé de la revendication 1, de formule

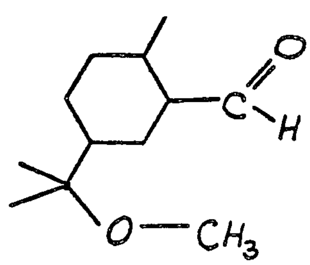

8. Composé de la revendication 1, de formule

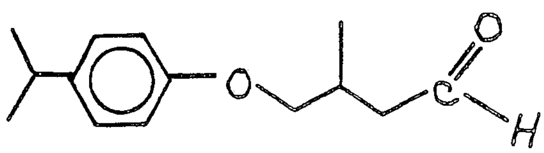

9. Composé de la revendication 1, de formule

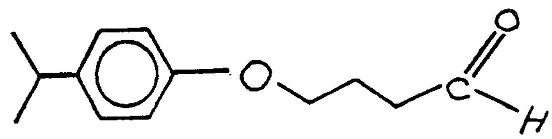

10. Aldéhyde éther de la revendication 1, de structure

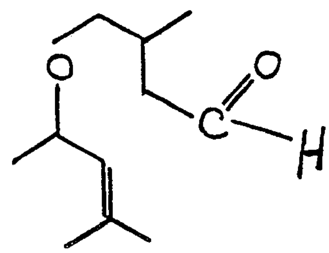

11. Aldéhyde éther de la revendication 1, de structure

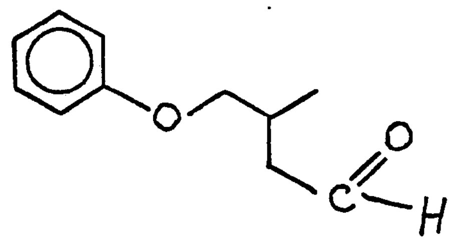

12. Procédé de préparation d'un composé selon la revendication 1, dans lequel on fait réagir un mélange du monoxyde de carbone et d'hydrogène avec un éther de formule

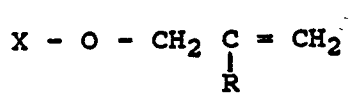

ou

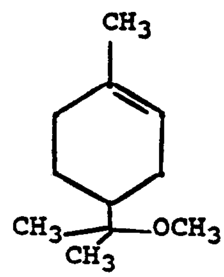

en présence d'un catalyseur de réaction "oxo" par lequel on obtient un produit contenant une proportion majeure de composés selon la revendication 1.

13. Procédé pour augmenter ou accroître l'arôme ou le goût d'un produit consommable choisi dans le groupe constitué par les compositions de parfum, les articles parfumés, les eaux de Cologne, les aliments, les gommes à mâcher, les dentifrices, les produits médicinaux, les tabacs à mâcher, les tabacs à fumer et les articles de tabac à fumer comprenant l'étape d'addition au dit produit consommable d'une quantité augmentant ou accroissant l'arôme ou le goût d'un produit défini selon l'une quelconque des revendications 1—11.

14. Carbinol éther défini selon la structure

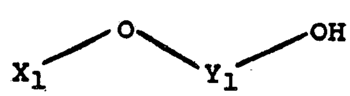

où X1 est une fraction choisie dans le groupe constitué par

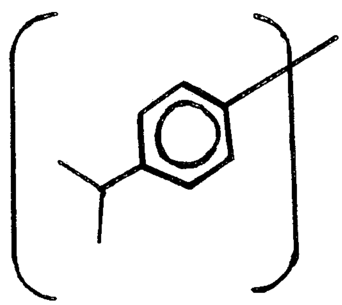

;

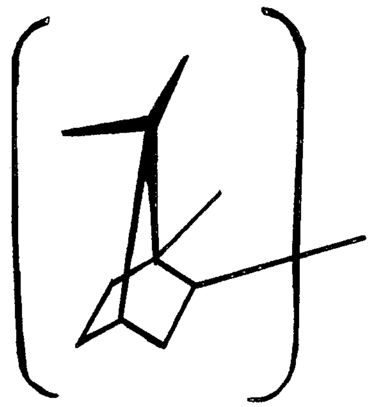

;

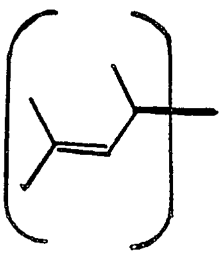

et

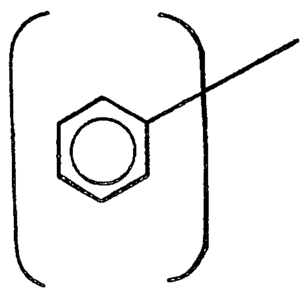

et où Y1 est une fraction choisie dans le groupe constitué par:

alcoylène en C1 à C5;

alcénylène en C4 à C5; et

alcynylène en C4 à C5.

15. Carbinol éther de la revendication 14, de structure

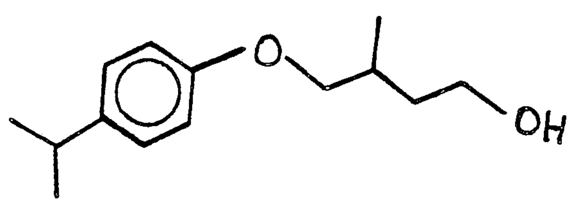

16. Carbinol éther de la revendication 14, de structure

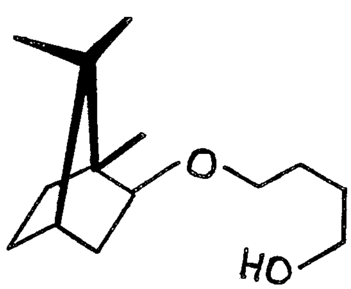

17. Carbinol éther de la revendication 14, de structure

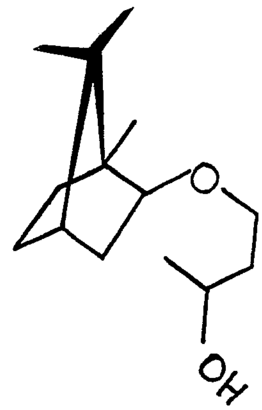

18. Carbinol éther de la revendication 14, de structure

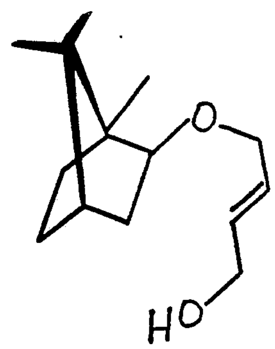

19. Carbinol éther de la revendication 14, de structure

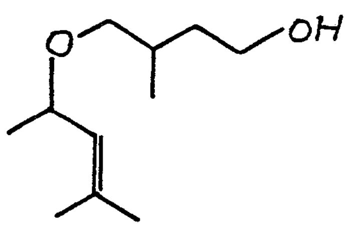

20. Carbinol éther de la revendication 14, de structure

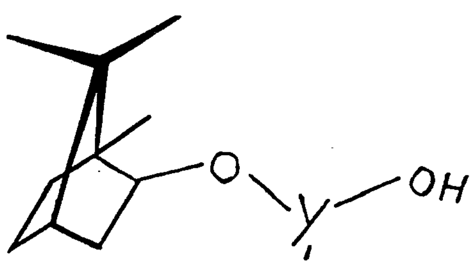

où Y1 est choisi dans le groupe constitué par:

alcoylène en C4 à C5;

alcénylène en C4 à C5; et

alcynylène en C4 à C5.

21. Procédé pour augmenter ou accroître l'arôme ou le goût d'un produit consommable choisi dans le groupe constitué par les compositions de parfumées, les articles parfumés, les eaux de Cologne, les aliments, la gomme à mâcher, les tabacs à mâcher, les produits médicinaux, les dentifrices, les tabacs à fumer et les articles de tabac à fumer comprenant l'étape d'addition au dit produit consommable d'une

quantité augmentant ou accroissant l'arôme ou le goût d'au moins un carbinol éther défini selon l'une quelconque des revendications 14—20.

22. Procédé de préparation d'un composé défini selon la formule

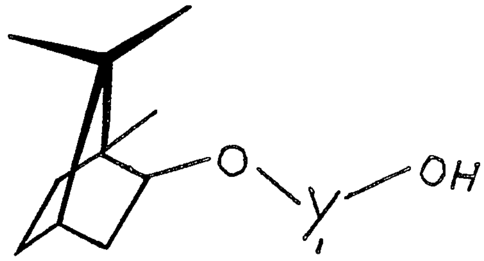

où Y1 est une fraction choisie dans le groupe constitué par:

alcoylène en C4 à C5;
alcénylène en C4 à C5; et
alcynylène en C4 à C5

comprenant l'étape d'addition à du camphène défini selon la formule

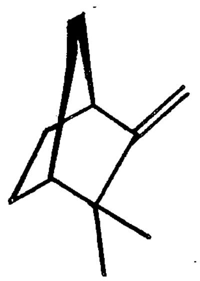

d'un diol défini selon la structure

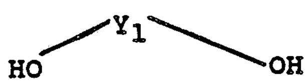

effectant ainsi la réaction:

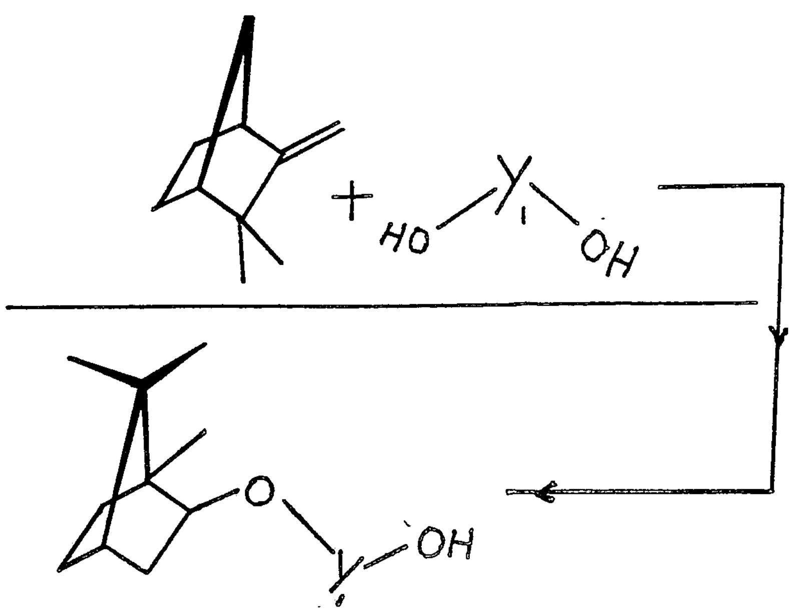

23. Procédé de préparation d'un mélange de composés contenant une proportion majeure de composés définis selon la formule

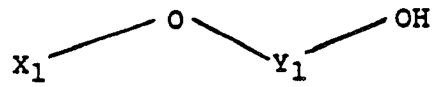

où X1 représente une fraction choisie entre

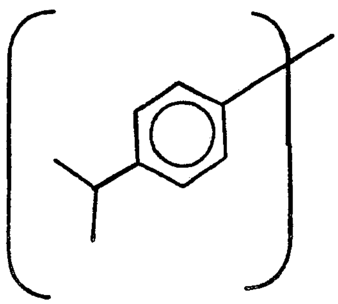

;

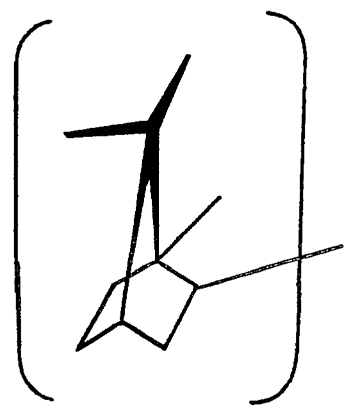

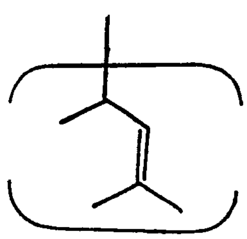

et

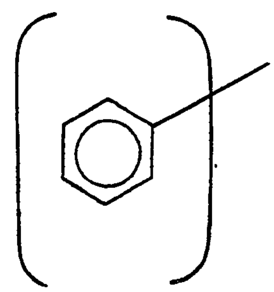

et où X1 est un alcoylène en C4 à C5 comprenant l'étape de réaction d'un allyl éther de formule

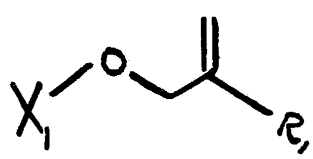

avec un mélange de monoxyde de carbone et d'hydrogène en présence d'un catalyseur de réaction oxo, formant ainsi un mélange d'aldéhydes de formule

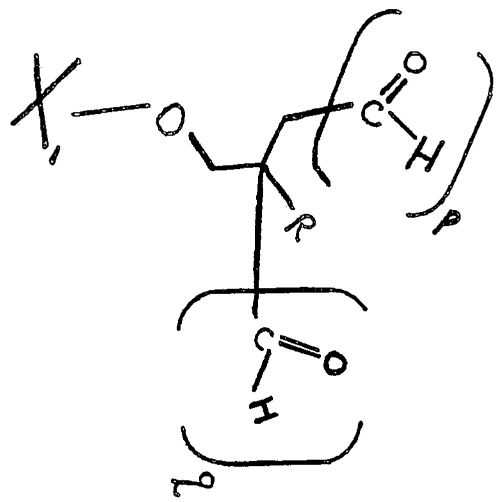

où R représente un hydrogène ou un méthyle et où p vaut 0 ou 1 et q vaut 0 ou 1 et où la somme de p + q = 1 puis de soumission du mélange d'aldéhydes de formule:

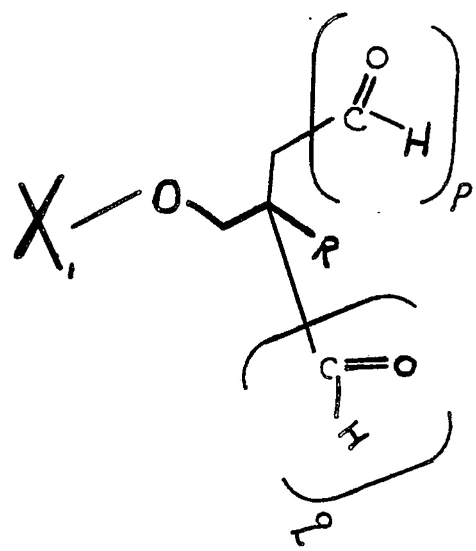

à une hydrogénation en utilisant de l'hydrogène et un catalyseur d'hydrogénation, formant ainsi un mélange de composés définis selon la structure

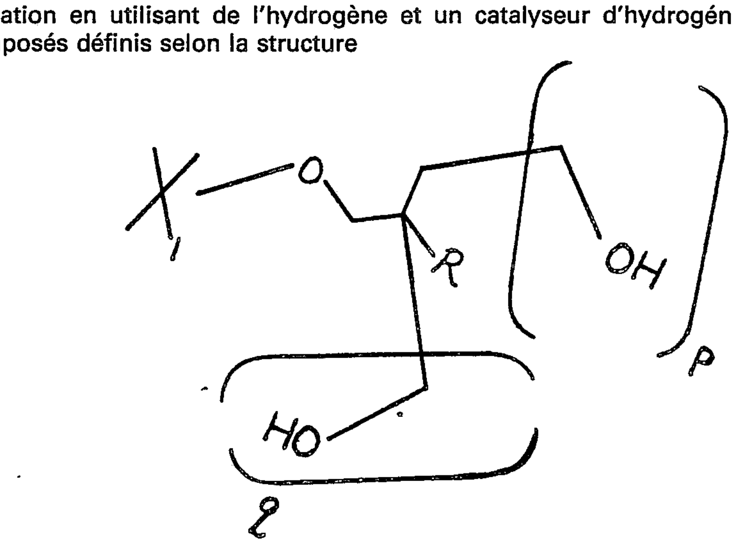

24. Procédé pour augmenter ou accroître l'arôme ou le goût d'un produit consommable choisi dans le groupe constitué par les compositions de parfum, les articles parfumés, les eaux de Cologne, les aliments, les gommes à mâcher, les dentifrices, les produits médicinaux, les tabacs à mâcher, les tabacs à fumer et les articles de tabac à fumer comprenant l'étape d'addition au dit produit consommable d'un mélange de carbinol éthers défini selon la revendication 23.

FIG.1

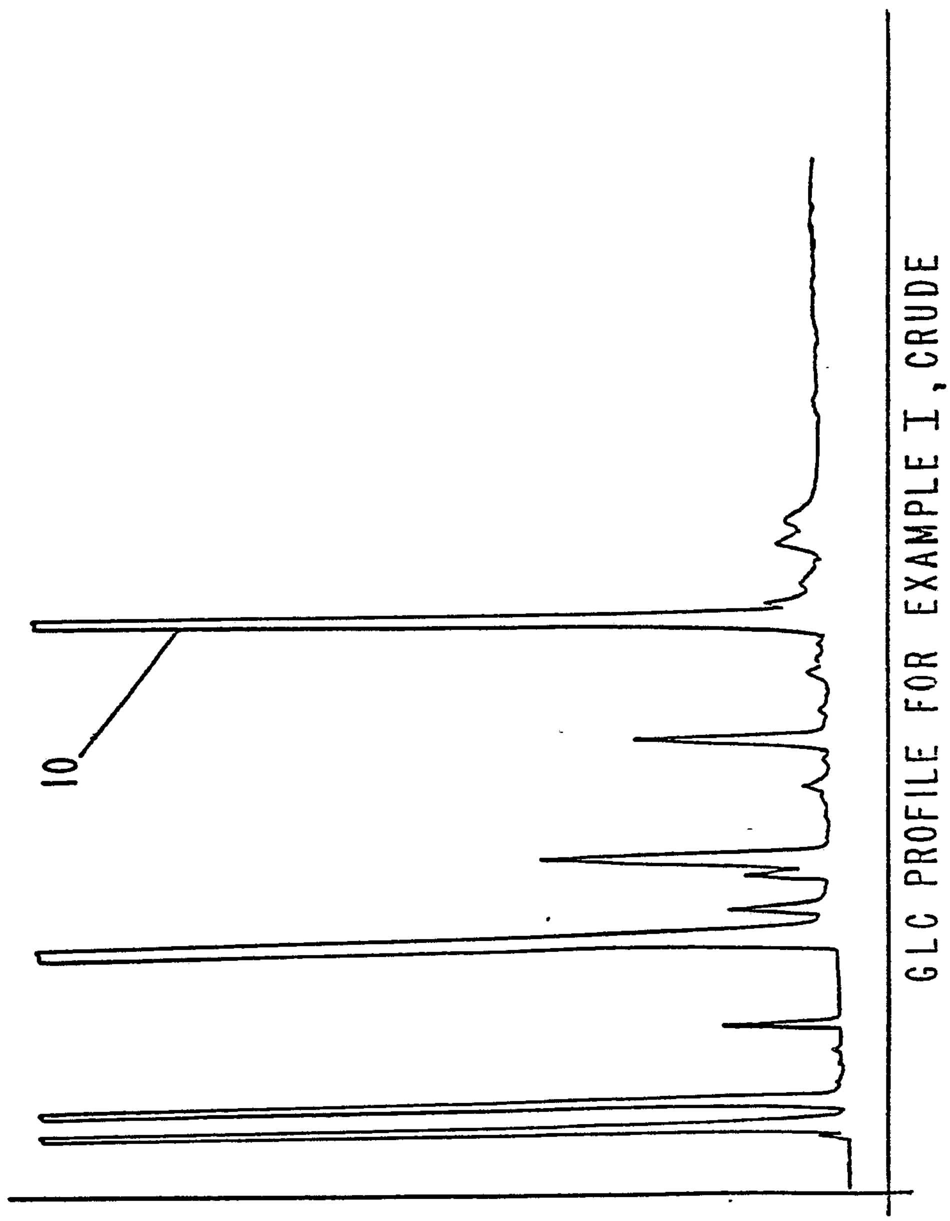


GLC PROFILE FOR EXAMPLE I, CRUDE

FIG.2

N M SPECTRUM FOR EXAMPLE I.

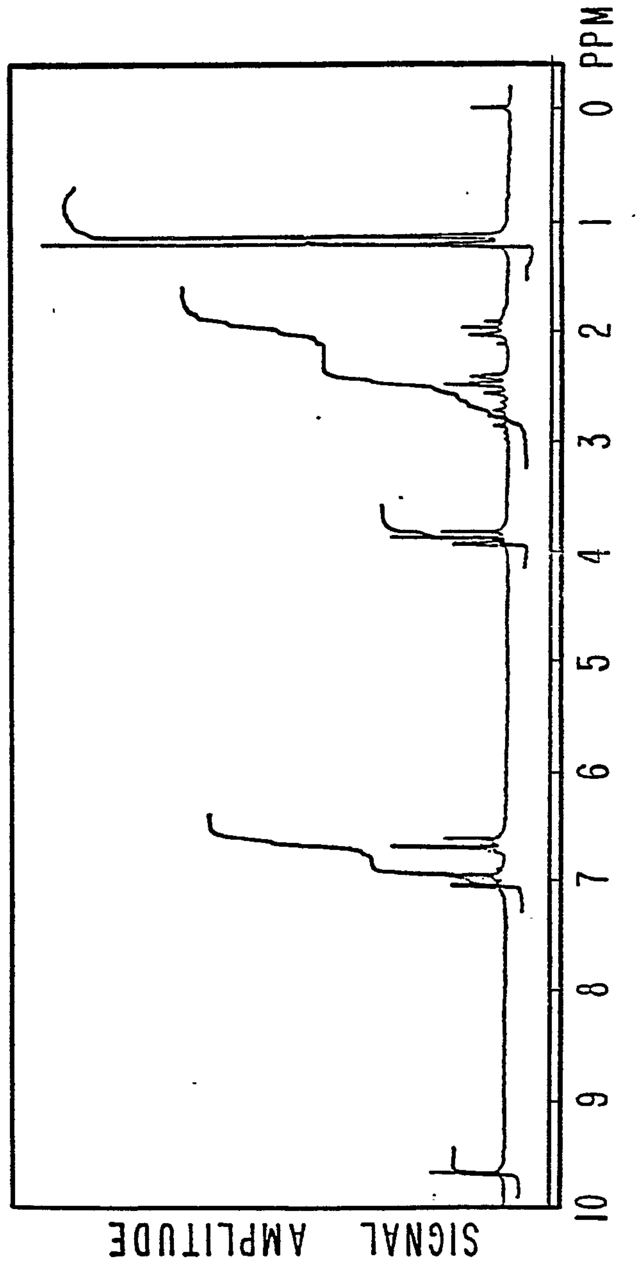

FIG.5

GLC PROFILE FOR BULKED FRACTIONS 2-4 . 1st DISTILLATION.

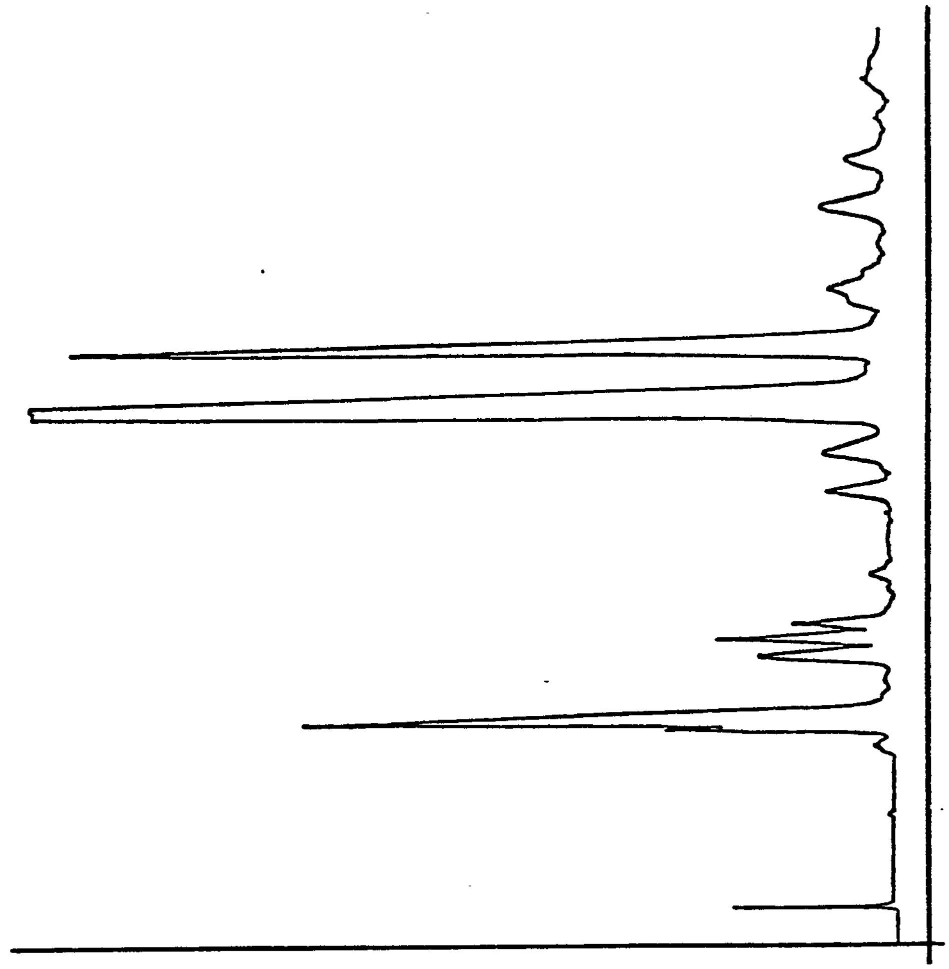

FIG.3

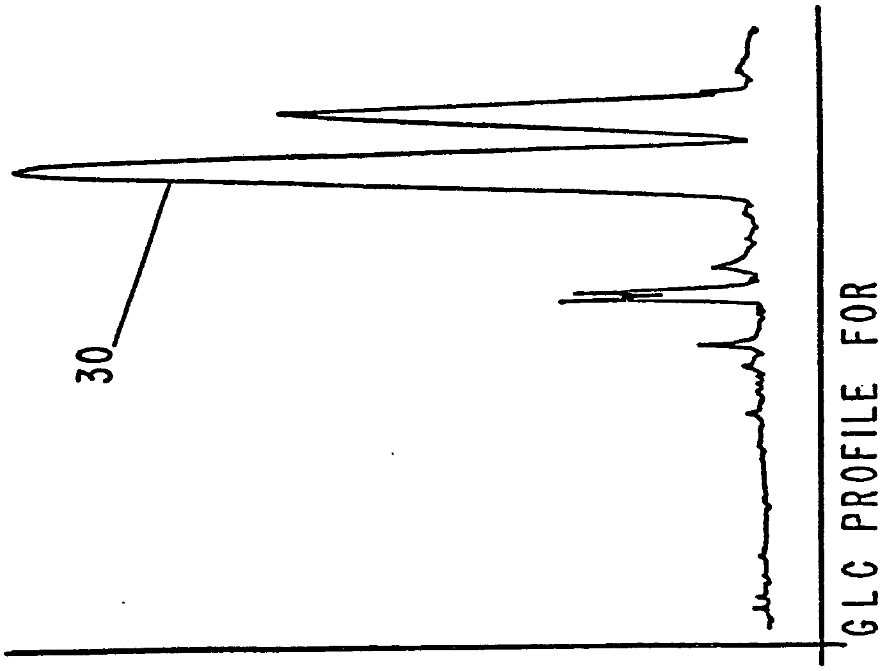


GLC PROFILE FOR FRACTION 4 OF EXAMPLE II.

FIG.4

NMR SPECTRUM FOR FRACTION 4 OF EXAMPLE II, PEAK "30" OF FIG. 3.

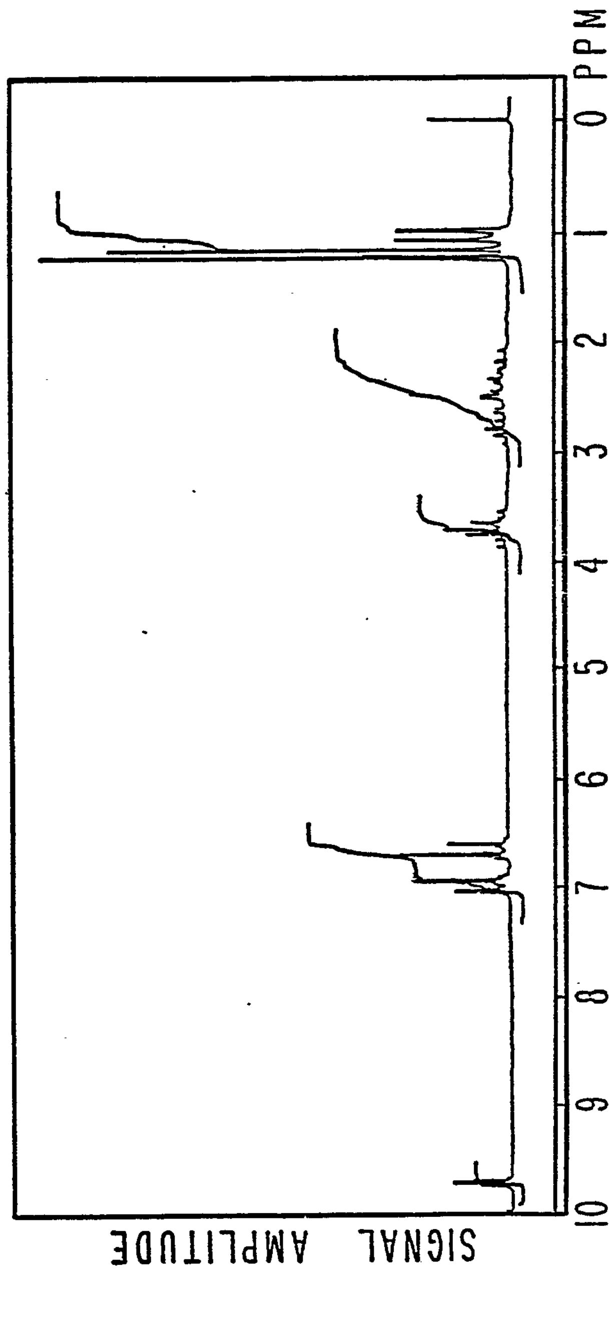

FIG.6

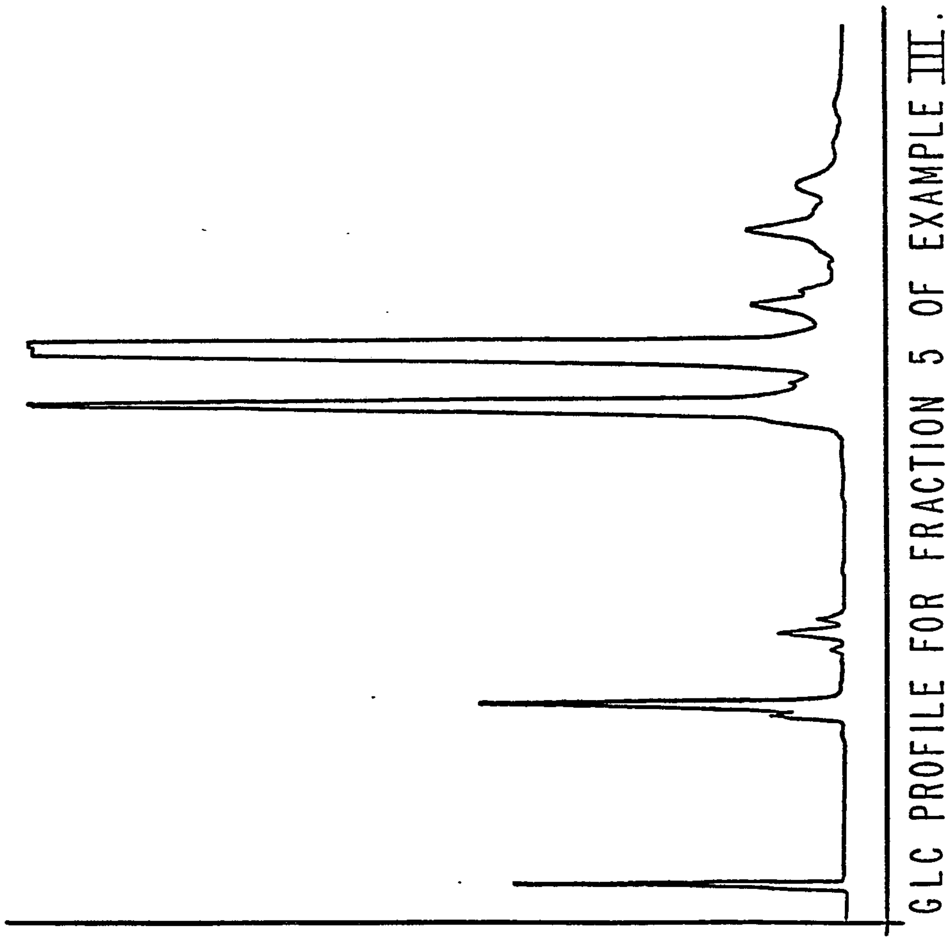

GLC PROFILE FOR FRACTION 5 OF EXAMPLE III.

FIG.8

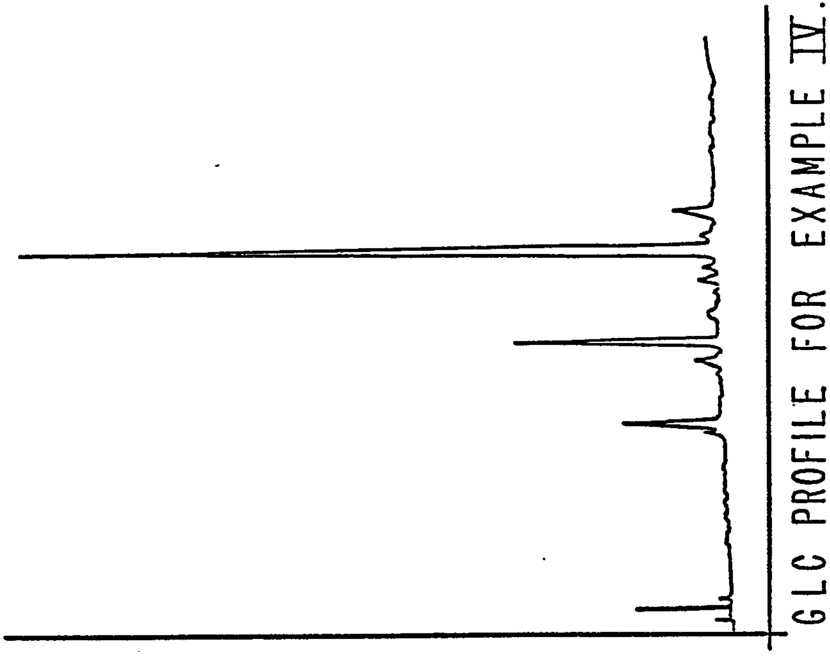

GLC PROFILE FOR EXAMPLE IV.
CRUDE

FIG.7

NMR SPECTRUM FOR EXAMPLE III.

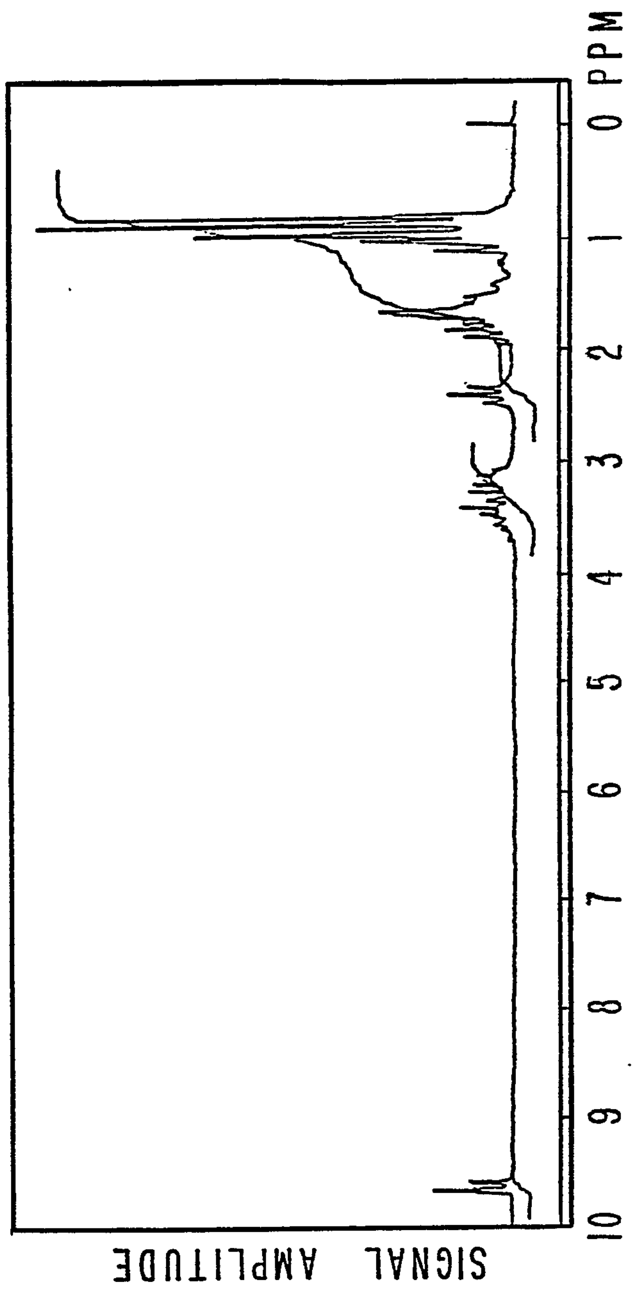

FIG.9

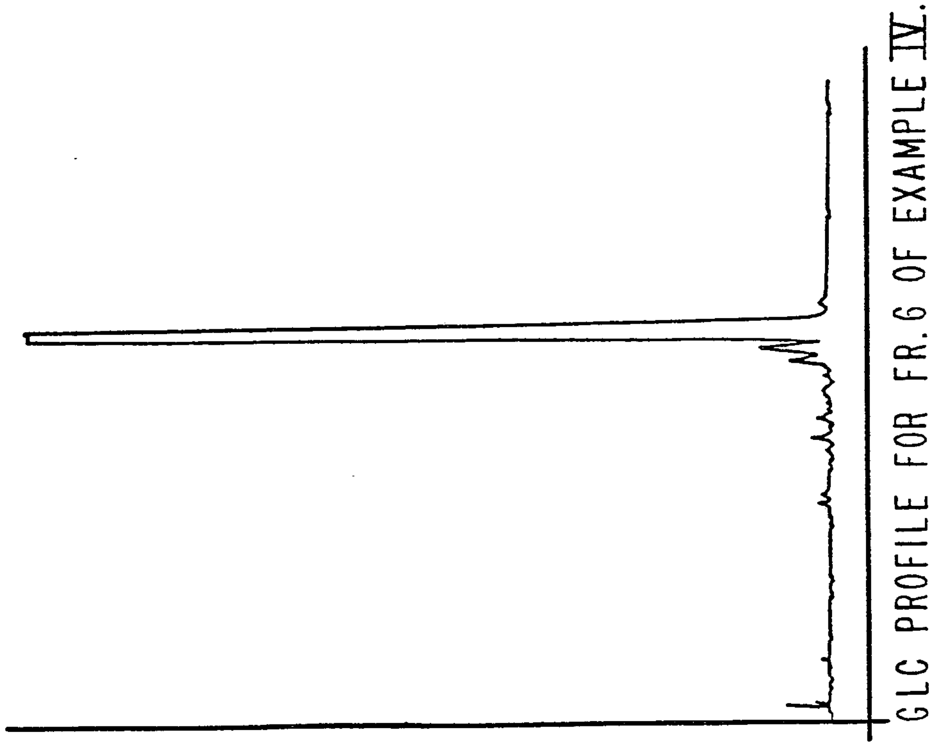

GLC PROFILE FOR FR. 6 OF EXAMPLE IV.

FIG.11

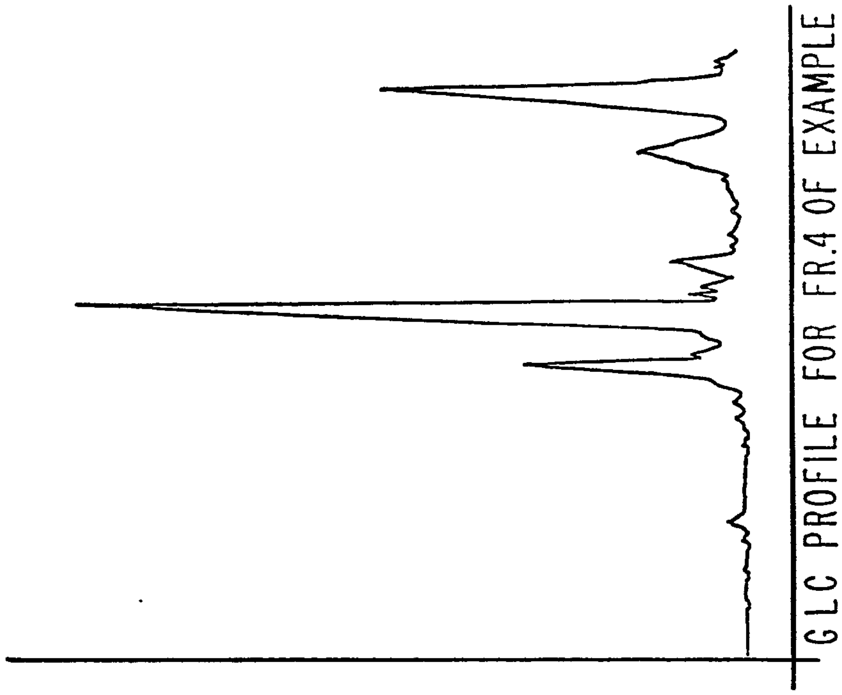

GLC PROFILE FOR FR.4 OF EXAMPLE V.
FIRST DISTILLATION

FIG.10

N M R SPECTRUM FOR FRACTION 6 OF EXAMPLE IV.

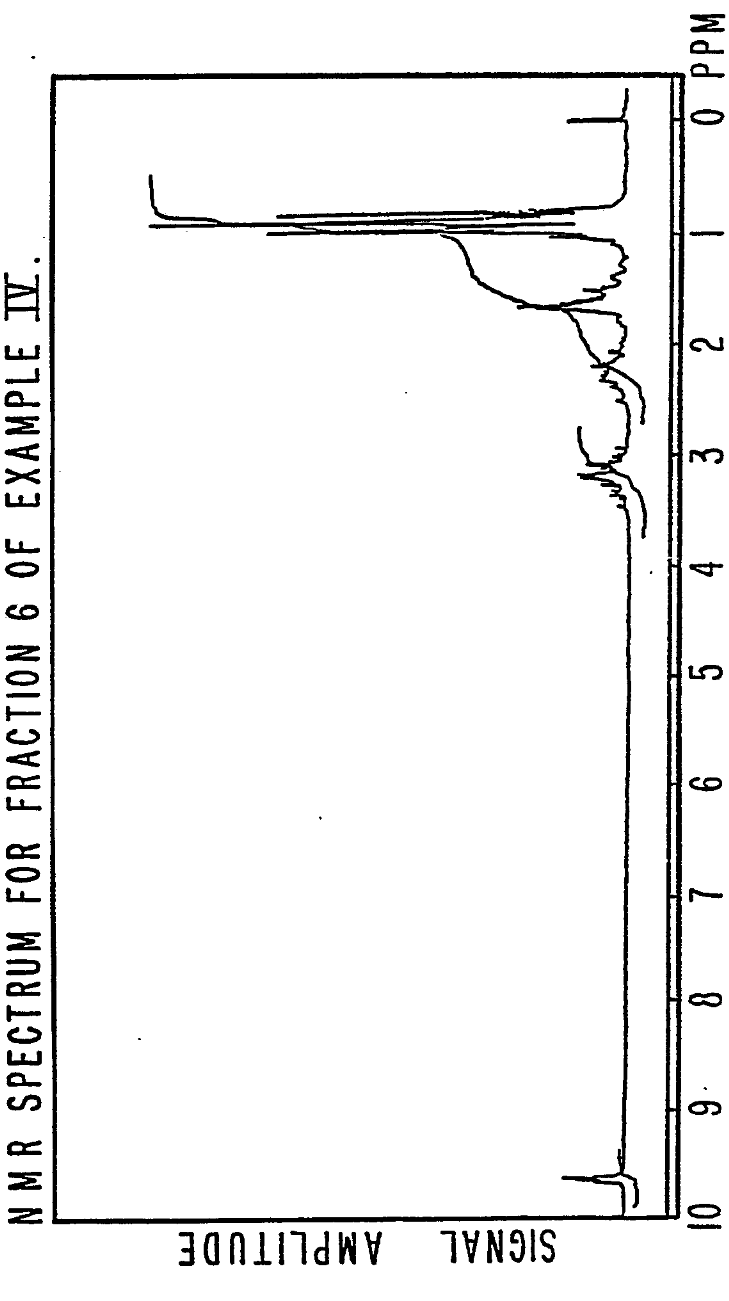

FIG.14

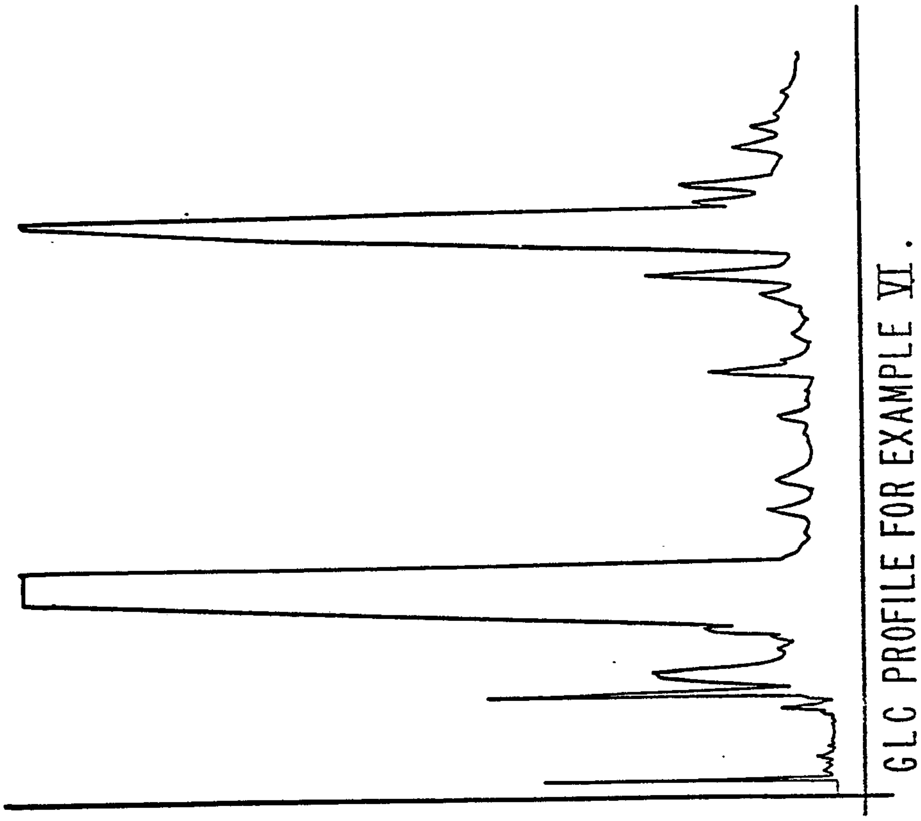

GLC PROFILE FOR EXAMPLE VI.
CRUDE

FIG.12

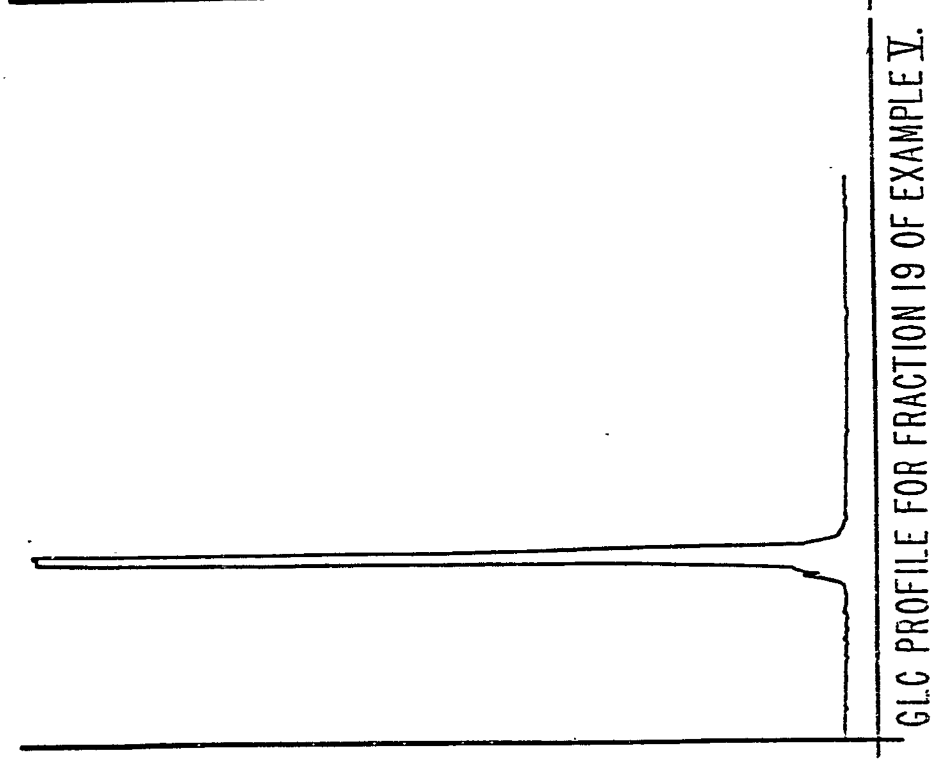

GLC PROFILE FOR FRACTION 19 OF EXAMPLE V.
FINAL DISTILLATION

FIG.13

NMR SPECTRUM FOR FRACTION 19 OF EXAMPLE V.

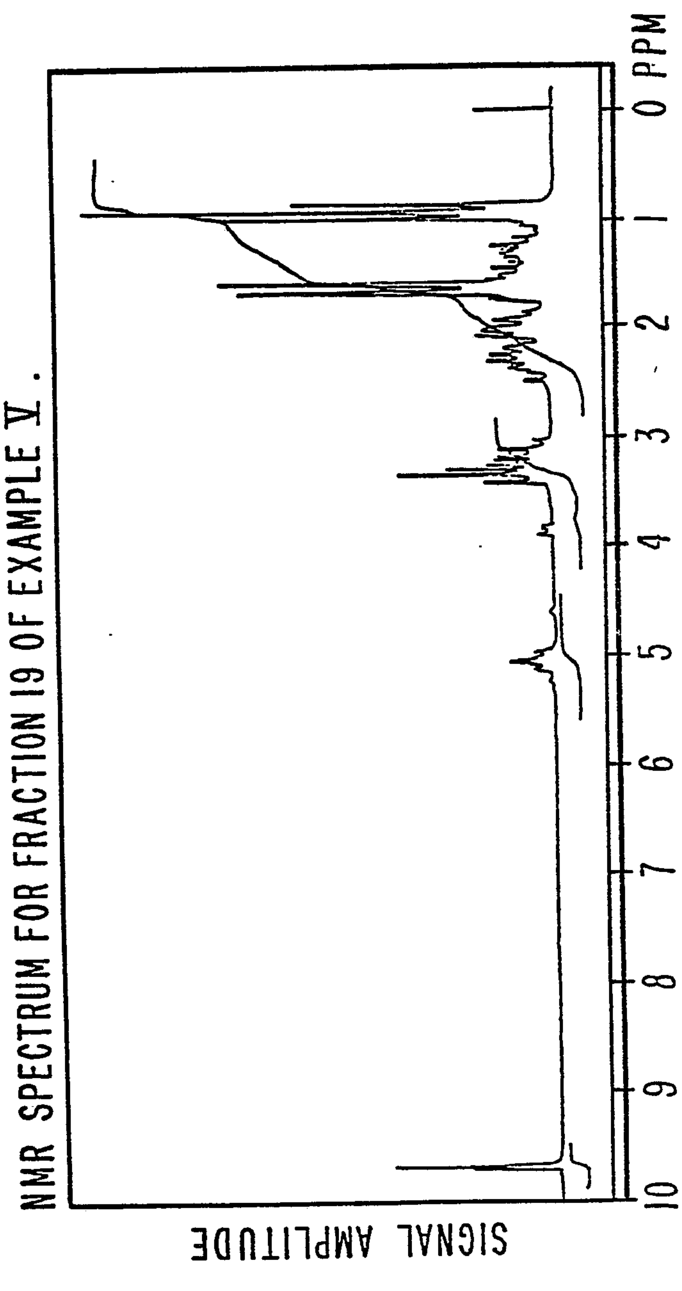

FIG.15

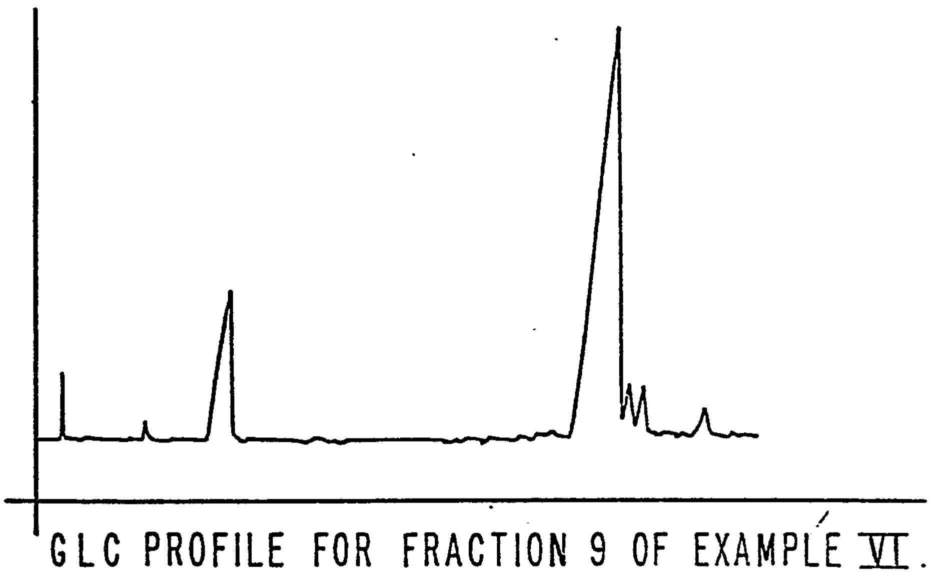

GLC PROFILE FOR FRACTION 9 OF EXAMPLE VI.

FIG.18

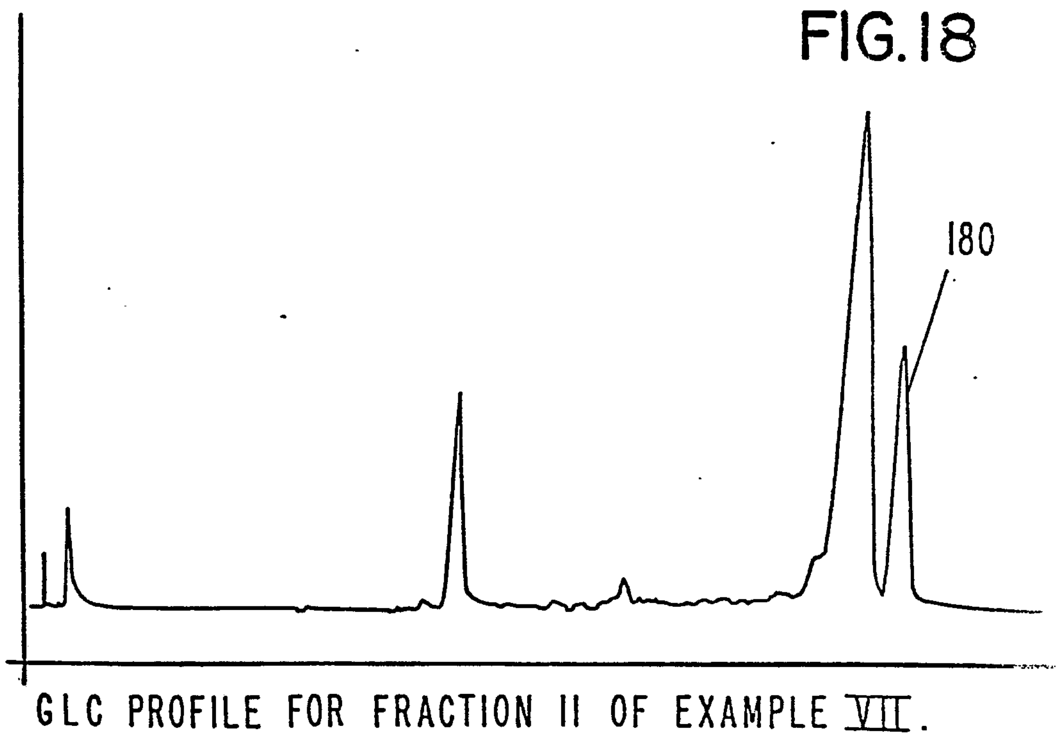


GLC PROFILE FOR FRACTION II OF EXAMPLE VII.

FIG.16

NMR SPECTRUM FOR FRACTION 9 OF EXAMPLE VI.

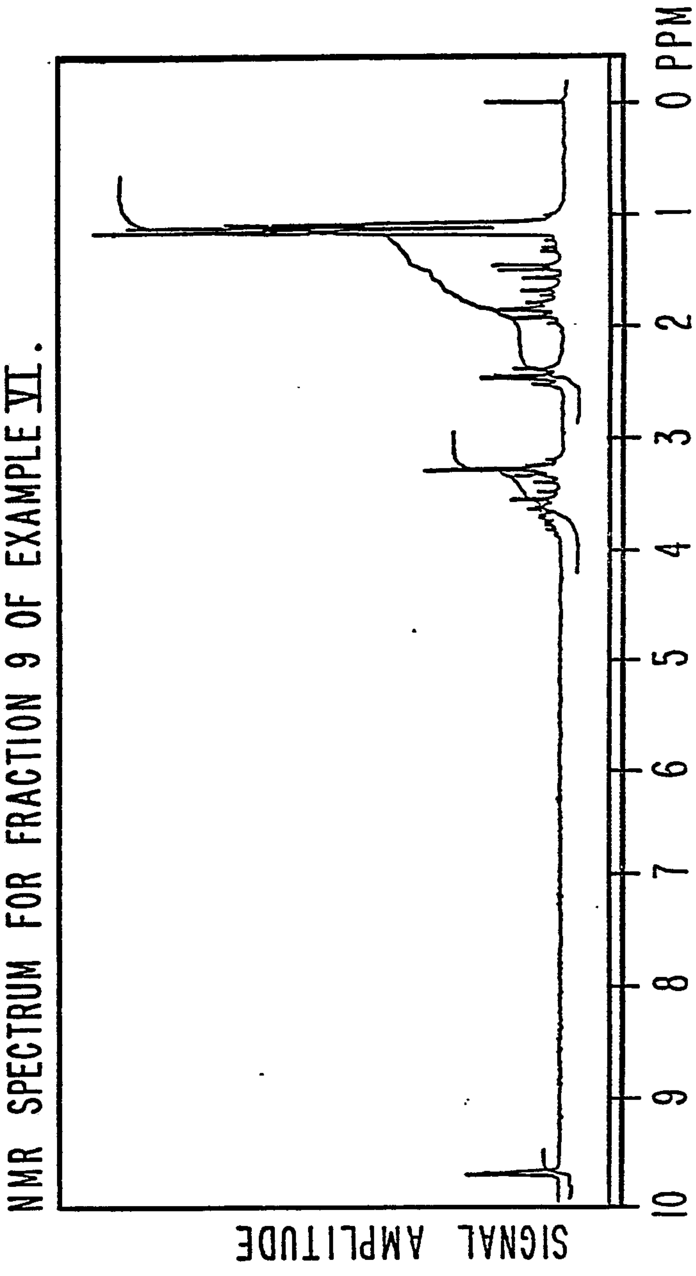

FIG.17

GLC PROFILE FOR EXAMPLE VII

CRUDE

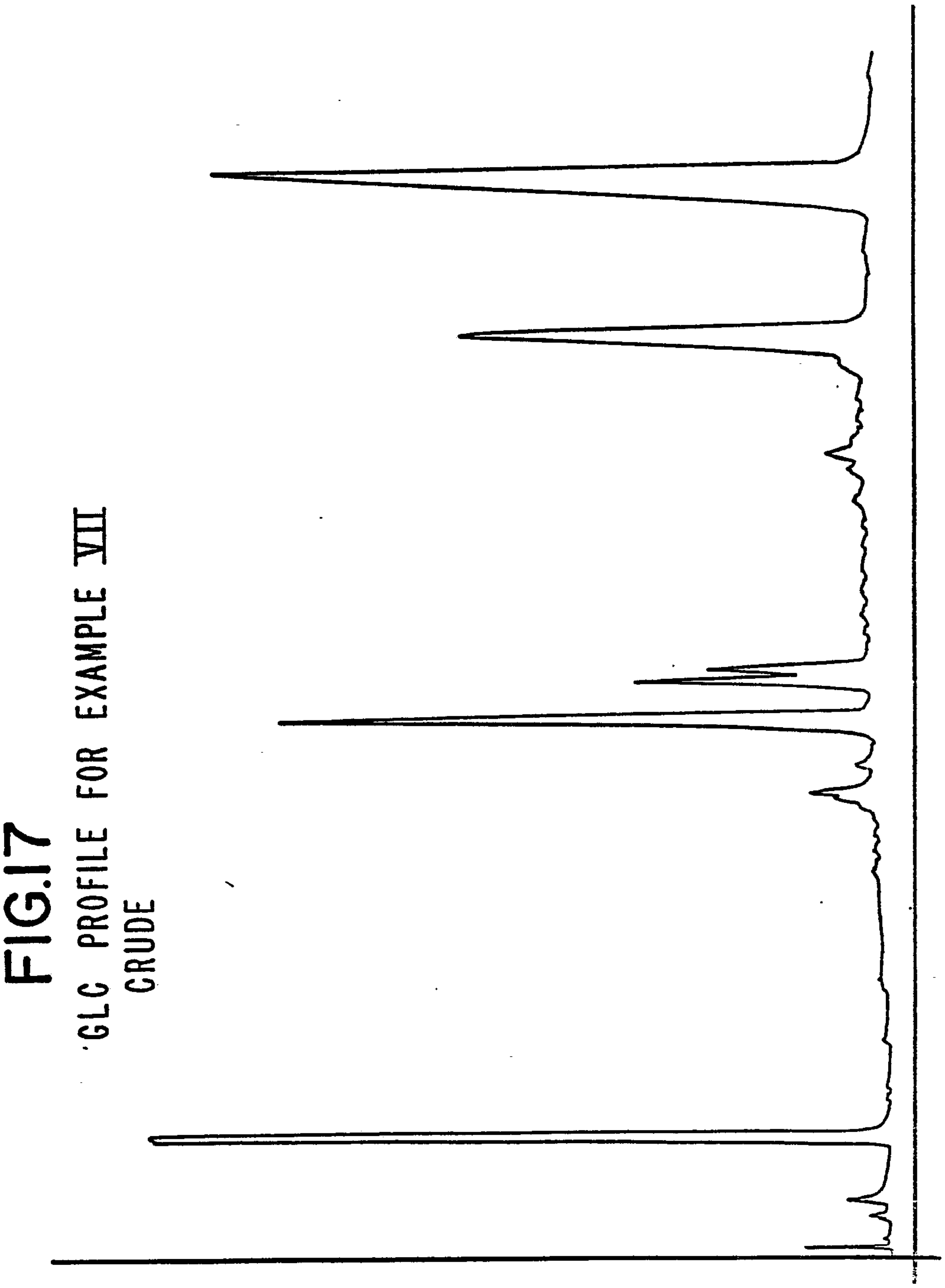

FIG.19

NMR SPECTRUM FOR FRACTION II OF EXAMPLE VII, PEAK 180 OF FIG.18

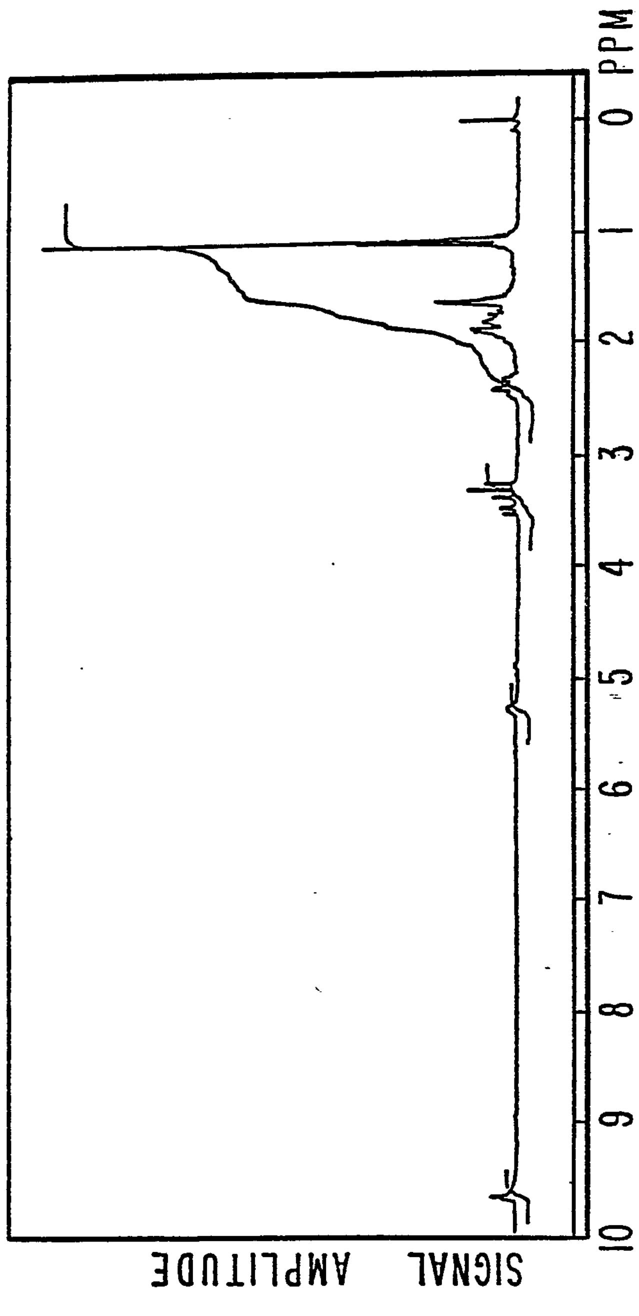

FIG.20

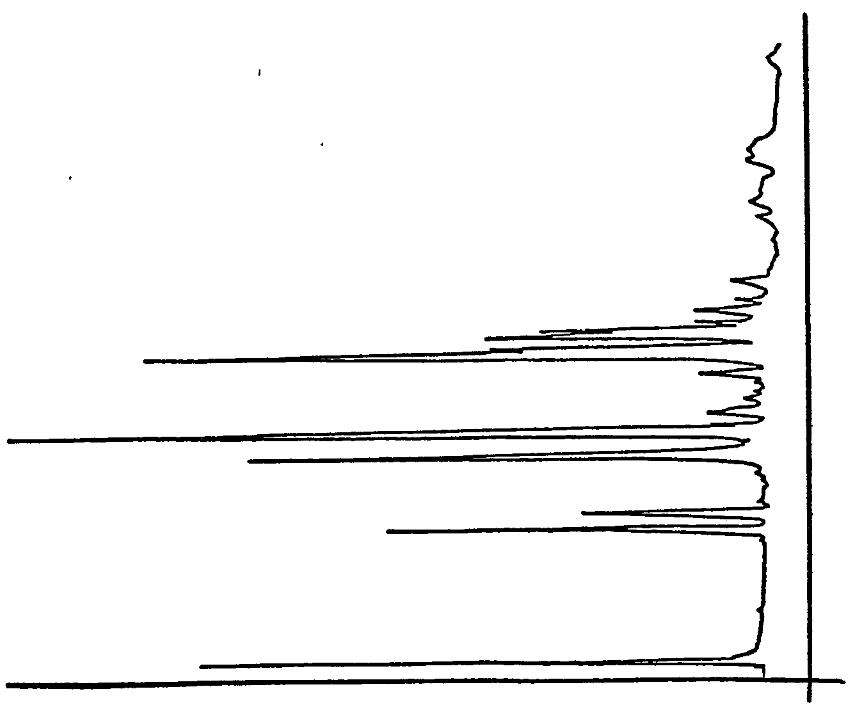

GLC PROFILE FOR EXAMPLE VIII.
CRUDE

FIG.21

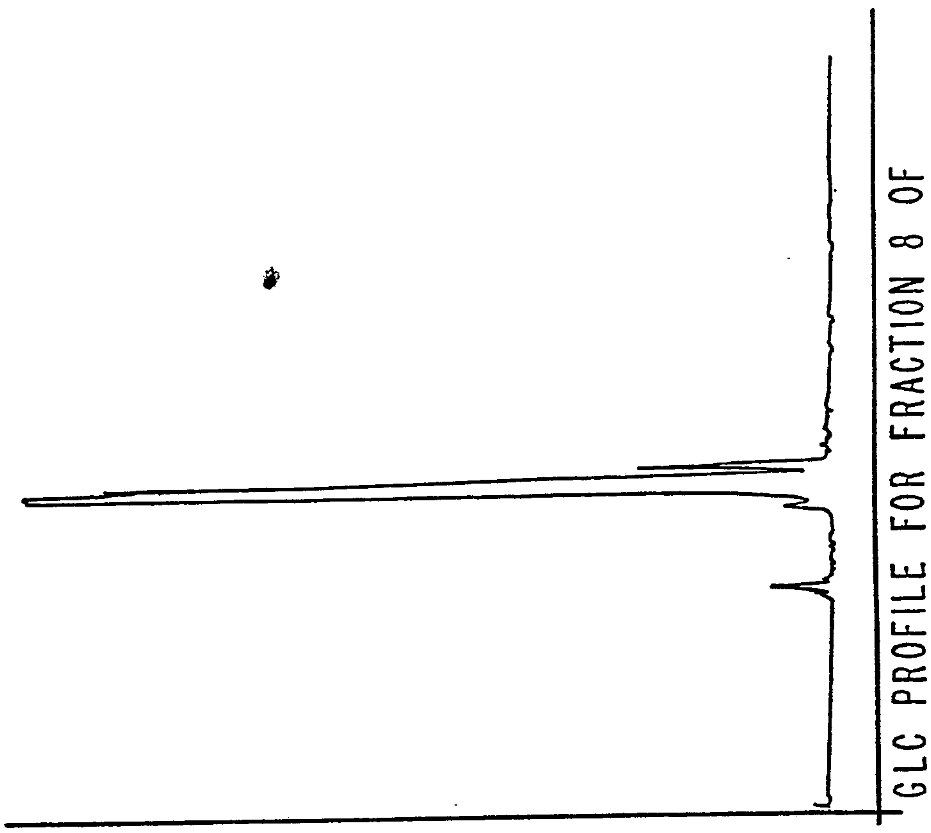

GLC PROFILE FOR FRACTION 8 OF
EXAMPLE VIII.

FIG.22

NMR SPECTRUM FOR EXAMPLE VIII.

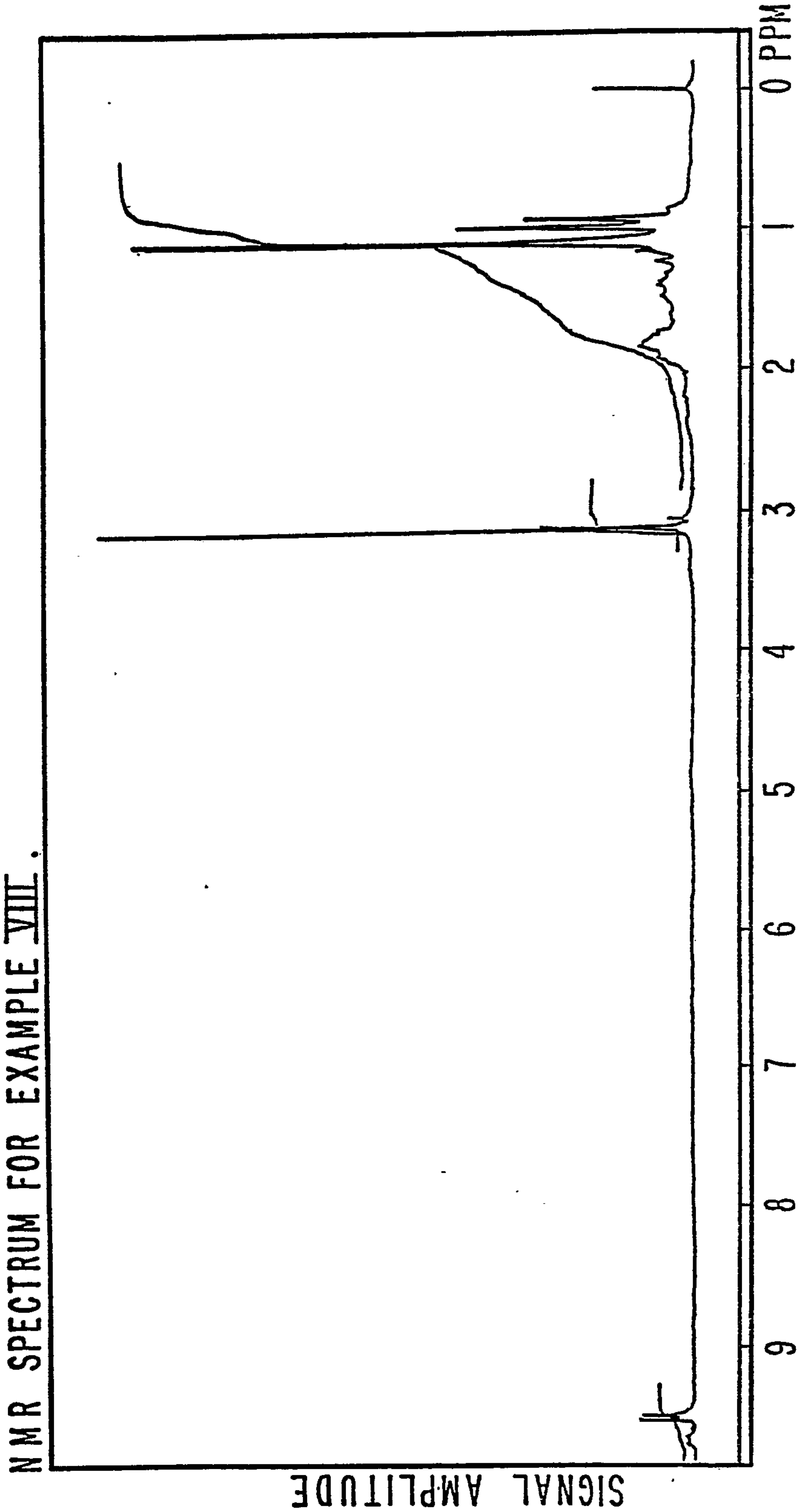

FIG.24

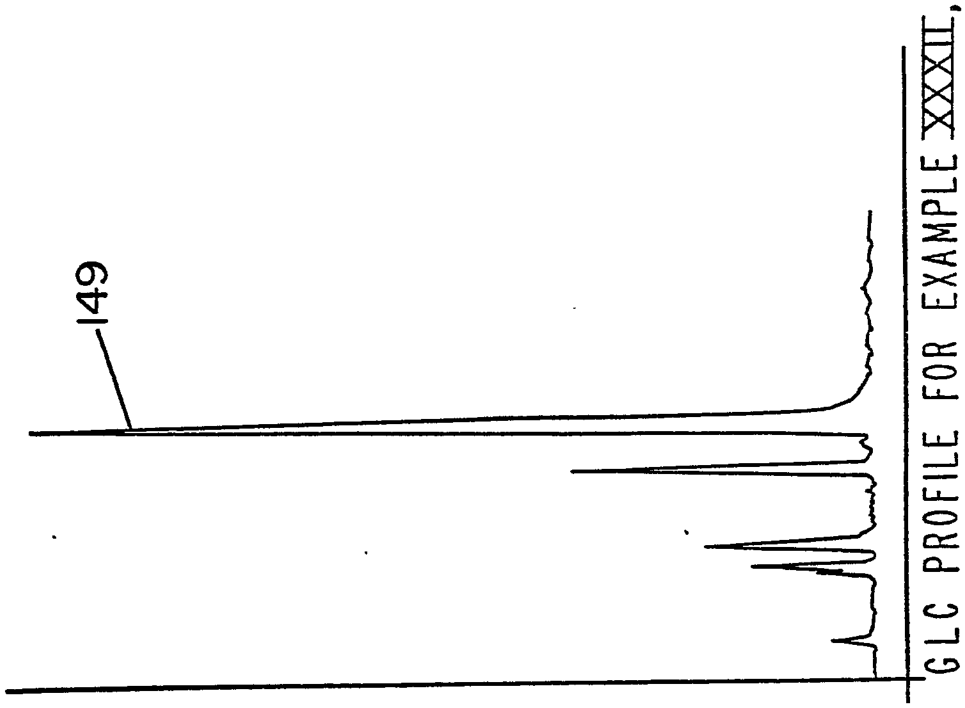


GLC PROFILE FOR EXAMPLE XXXII, FRACTION 5.

FIG.23

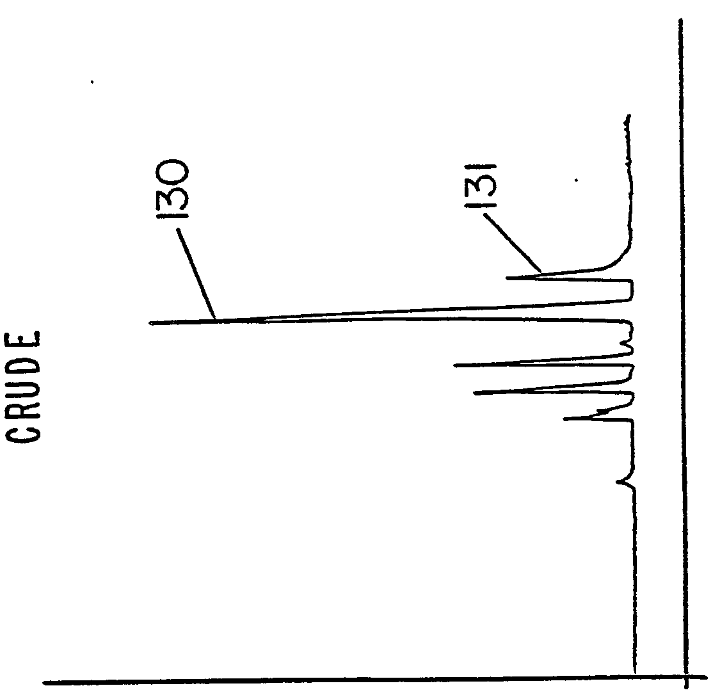


GLC PROFILE FOR EXAMPLE XXXII CRUDE

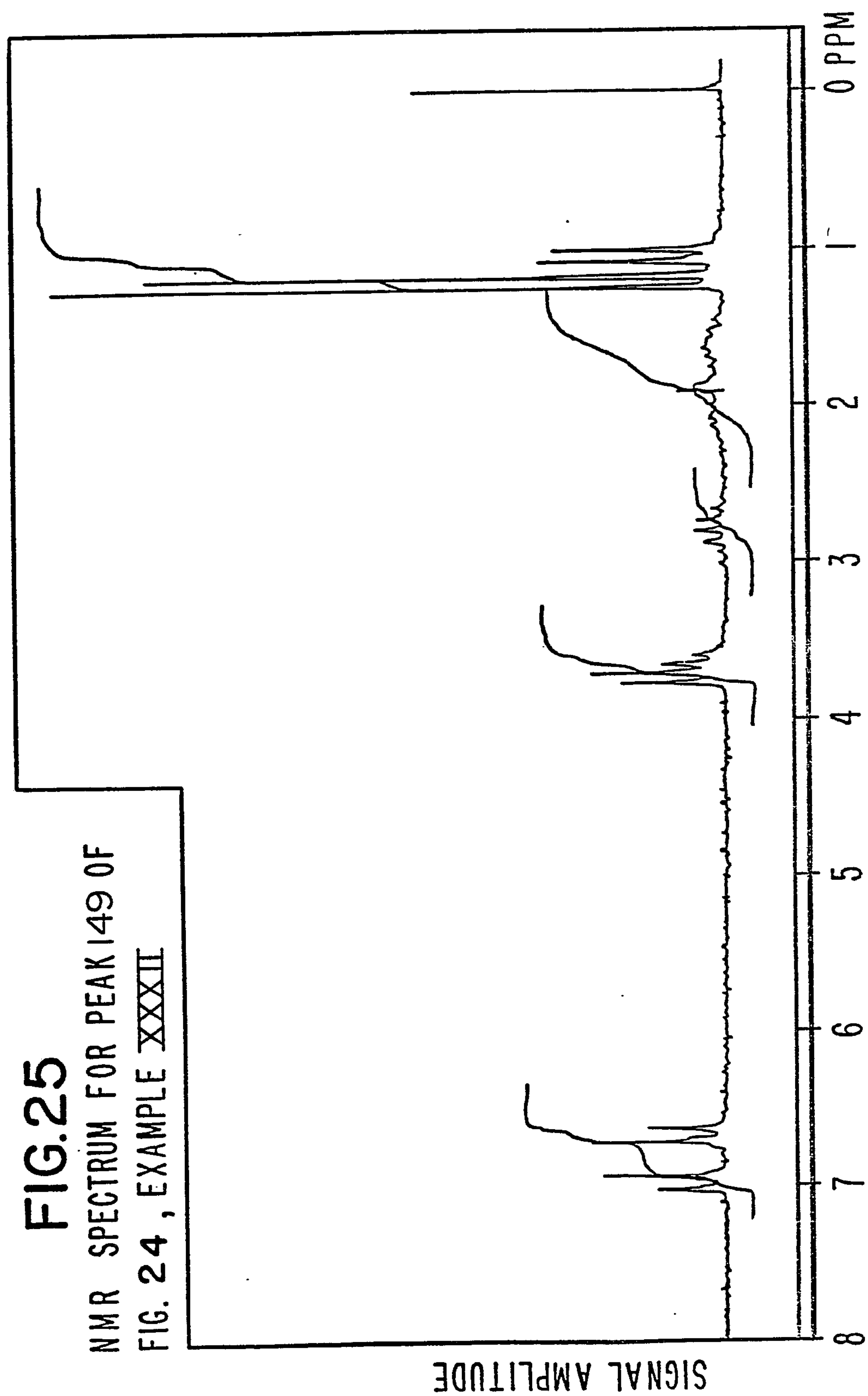


FIG.25

NMR SPECTRUM FOR PEAK 149 OF FIG. 24, EXAMPLE XXXII

FIG.26

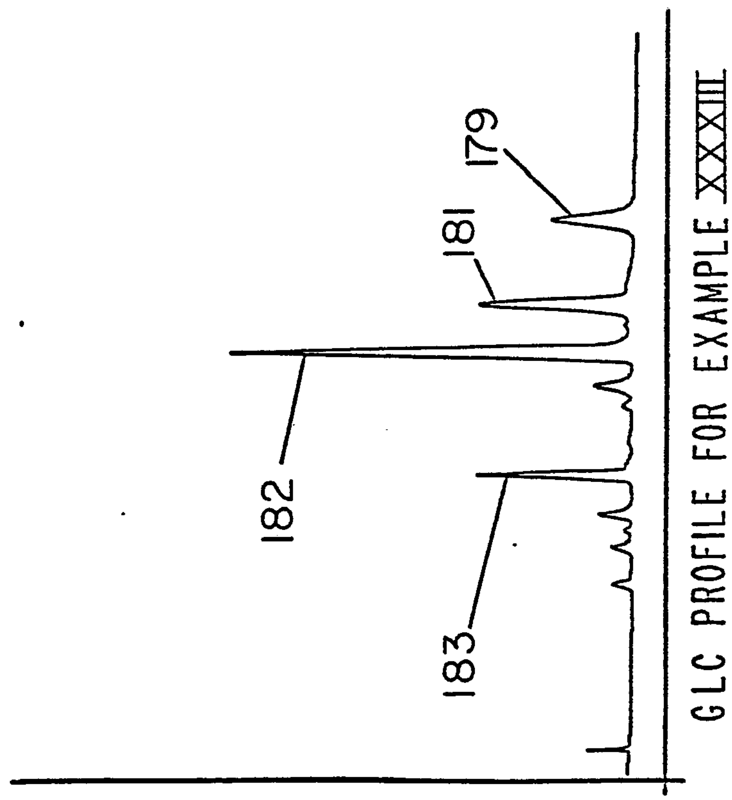
182
183
181
179
GLC PROFILE FOR EXAMPLE XXXII

FIG.27

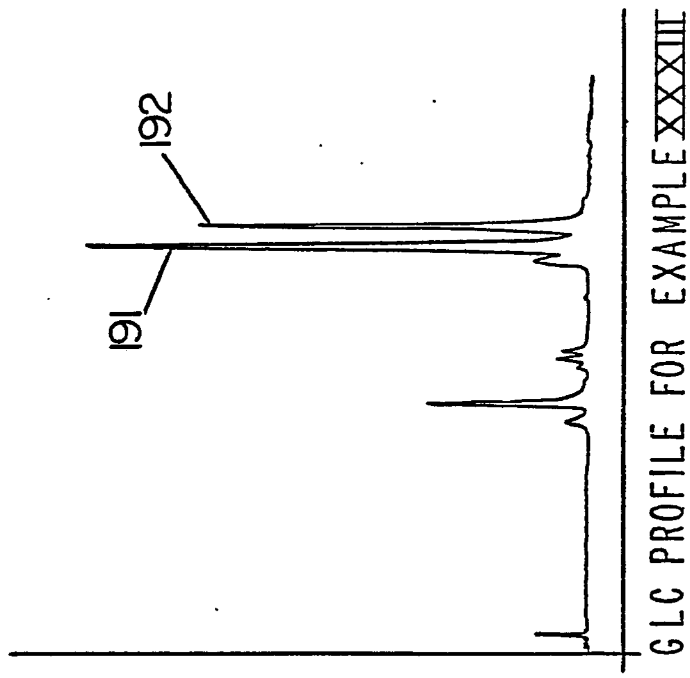
191
192
GLC PROFILE FOR EXAMPLE XXXIII

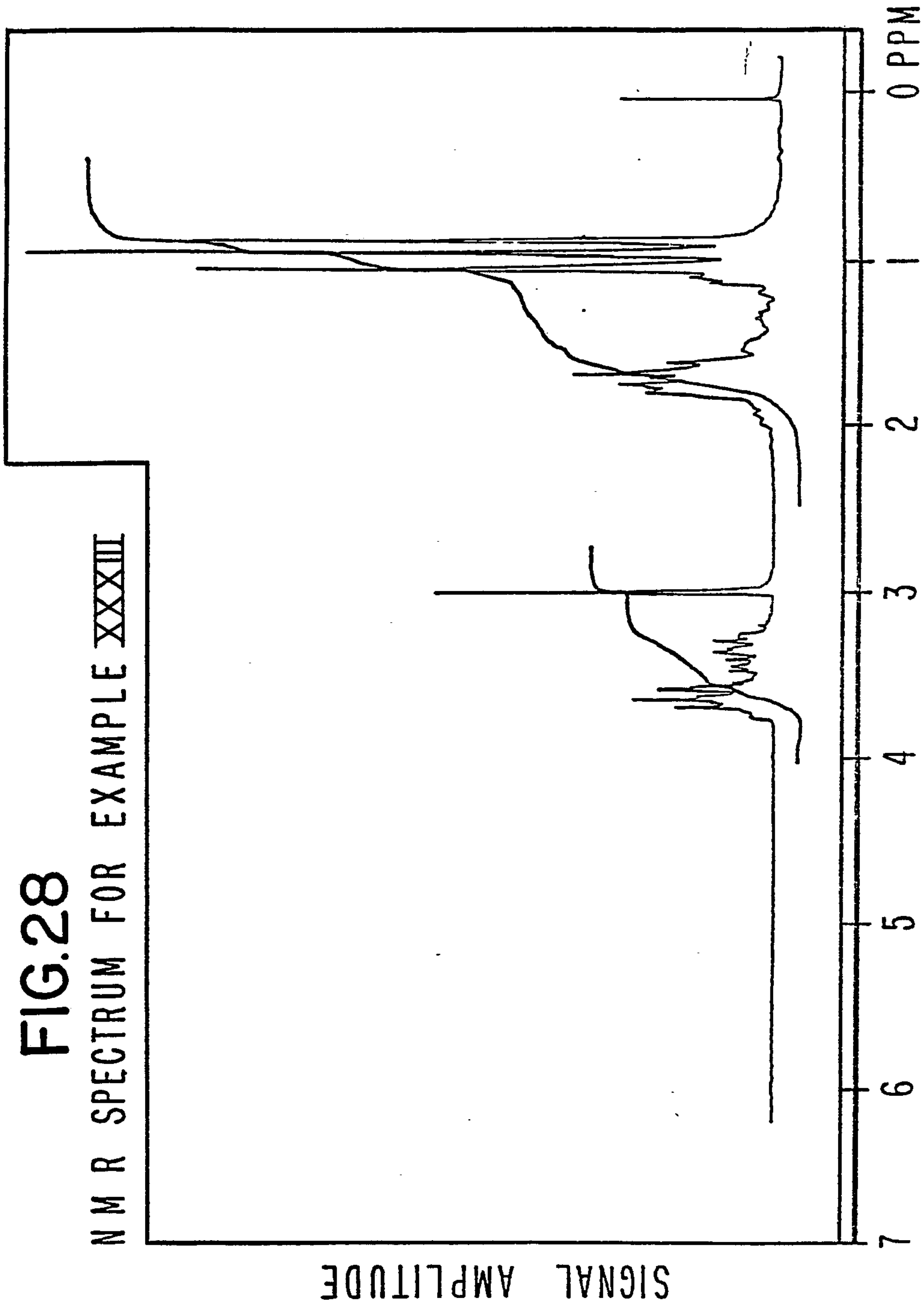


FIG.28
NMR SPECTRUM FOR EXAMPLE XXXIII

FIG.29

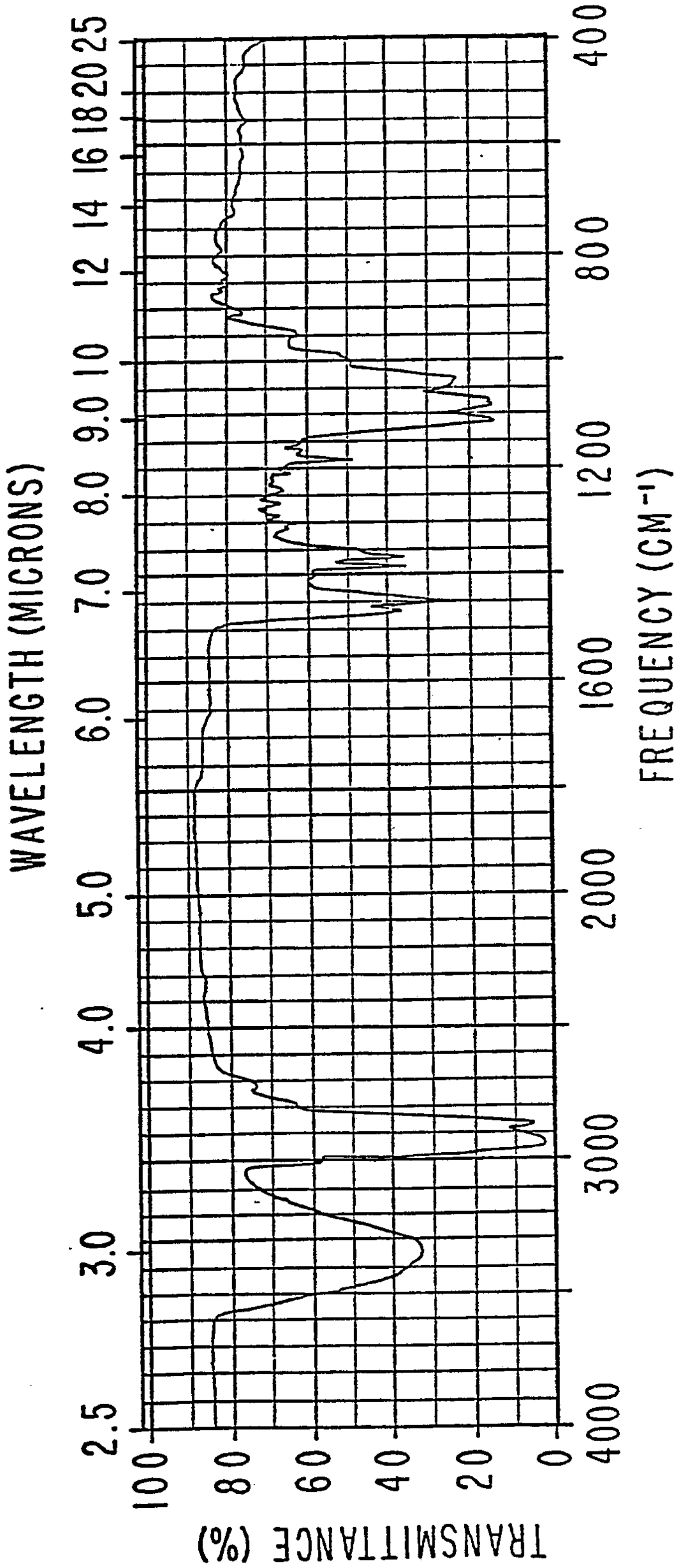


IR SPECTRUM FOR EXAMPLE XXXIII

FIG.30

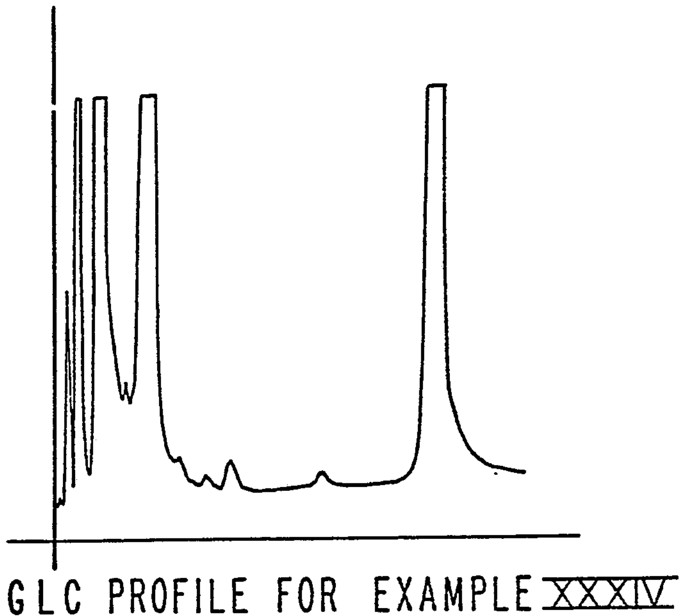

GLC PROFILE FOR EXAMPLE XXXIV
CRUDE

FIG.32

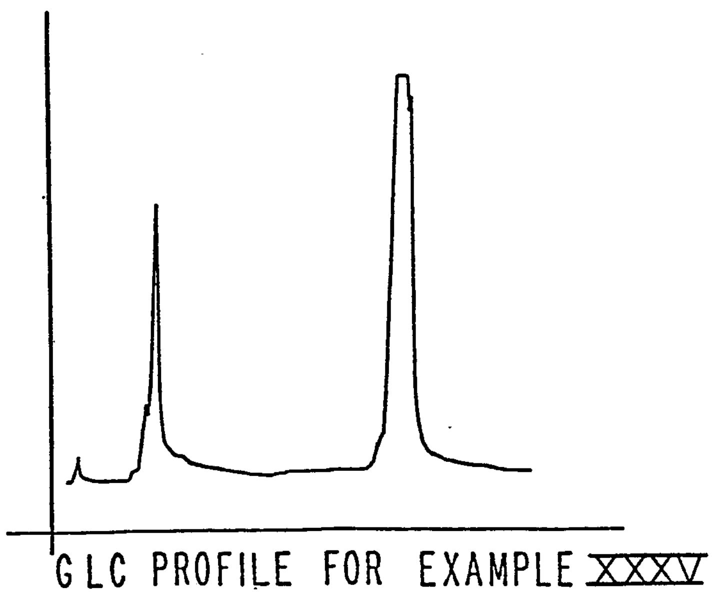

GLC PROFILE FOR EXAMPLE XXXV
CRUDE

FIG.3I

NMR SPECTRUM FOR FRACTION 4 OF EXAMPLE XXXIV

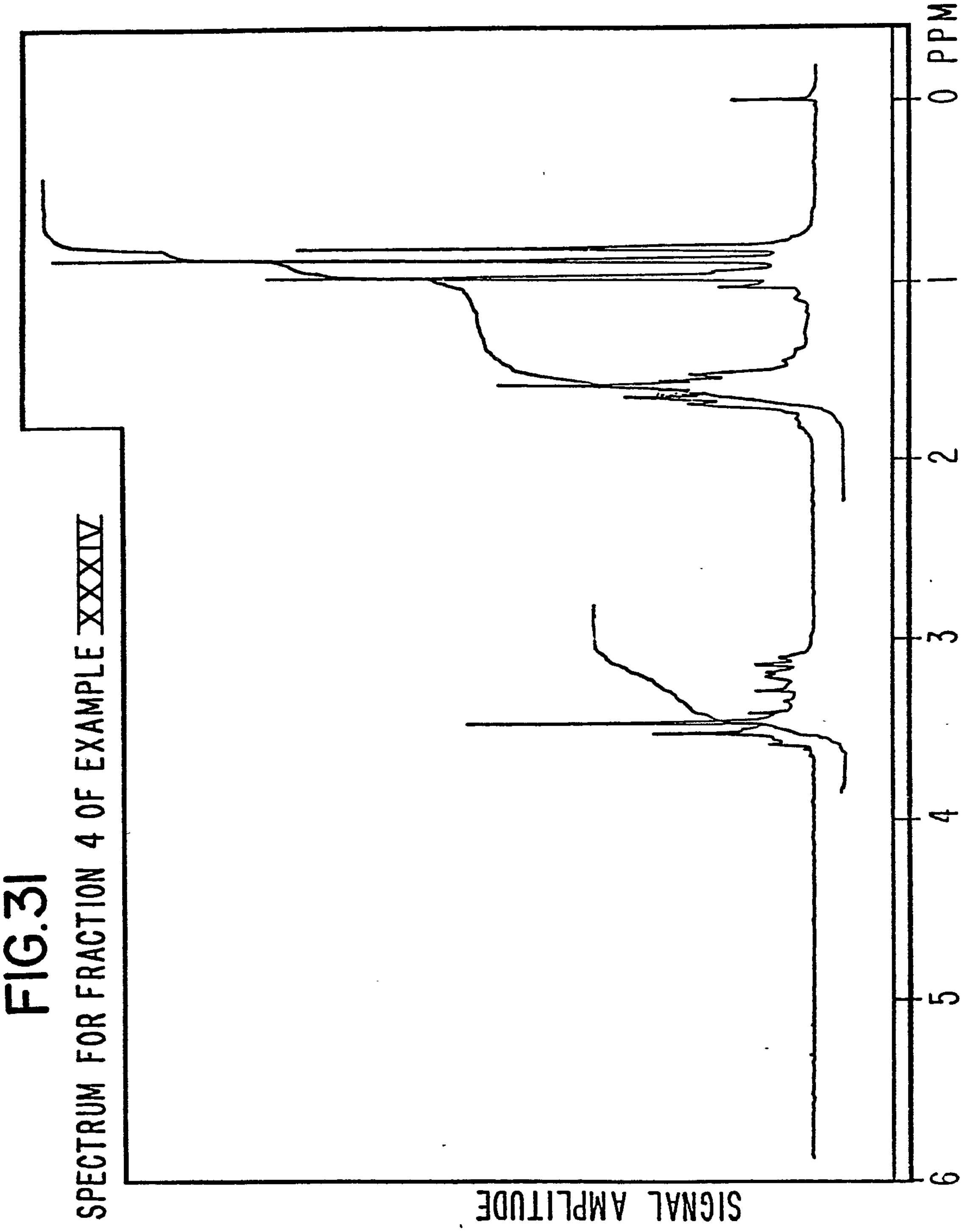

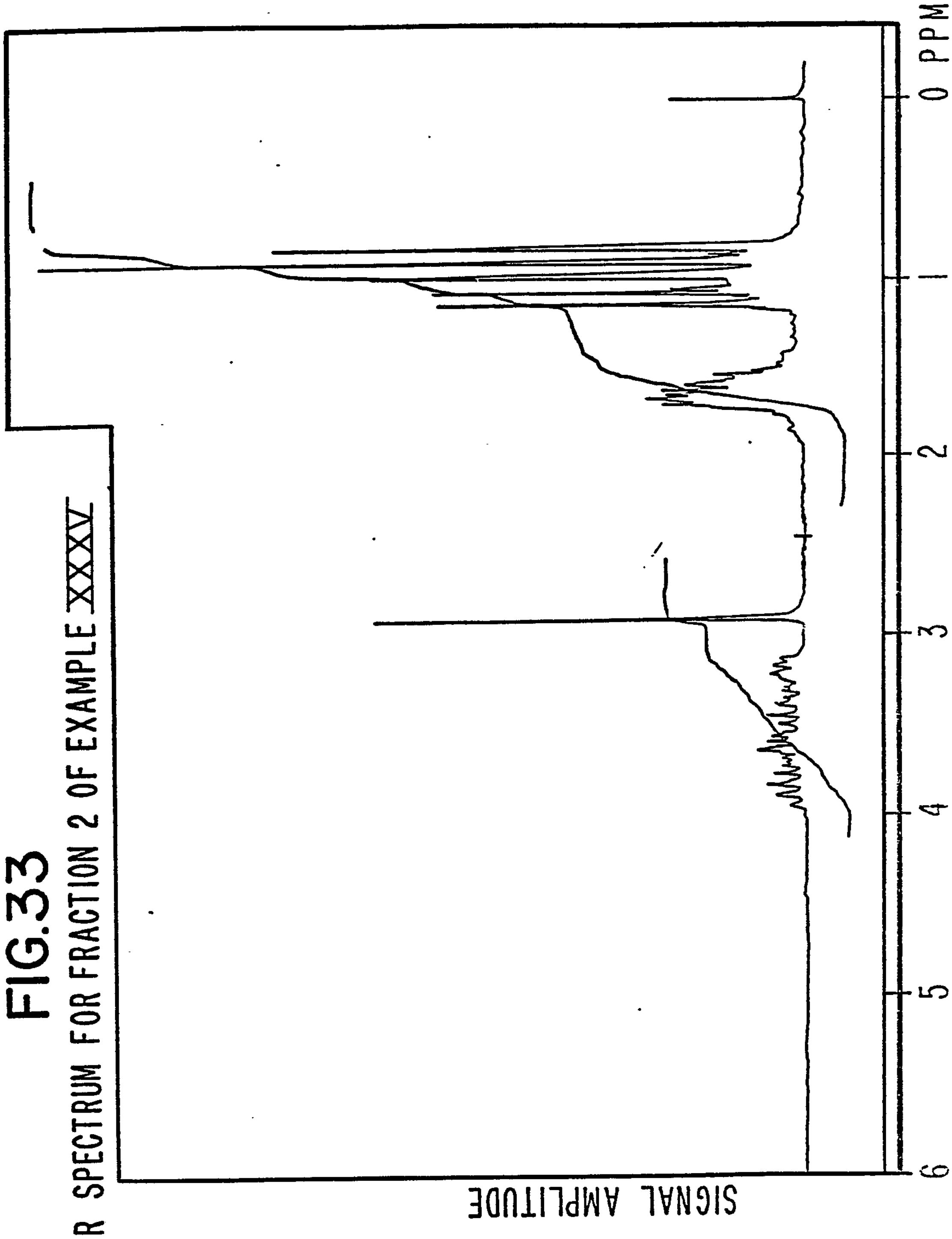


FIG.33

NMR SPECTRUM FOR FRACTION 2 OF EXAMPLE XXXV

FIG.34

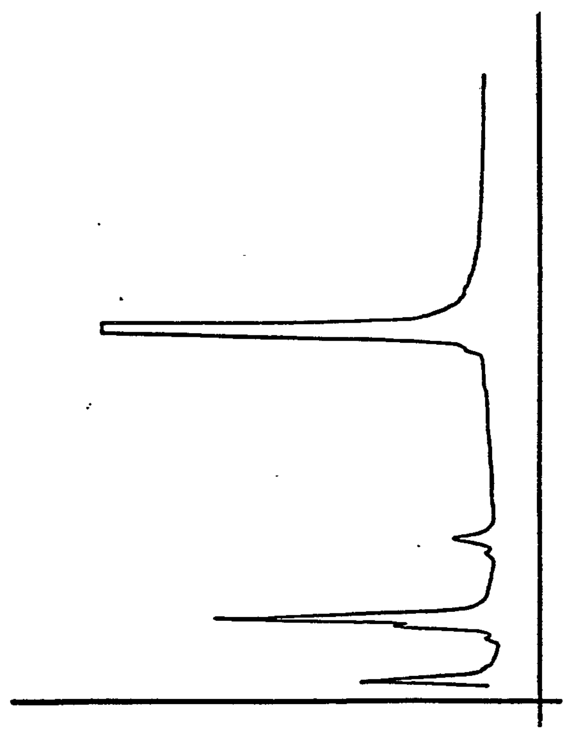

GLC PROFILE FOR EXAMPLE XXXVI
CRUDE

FIG.36

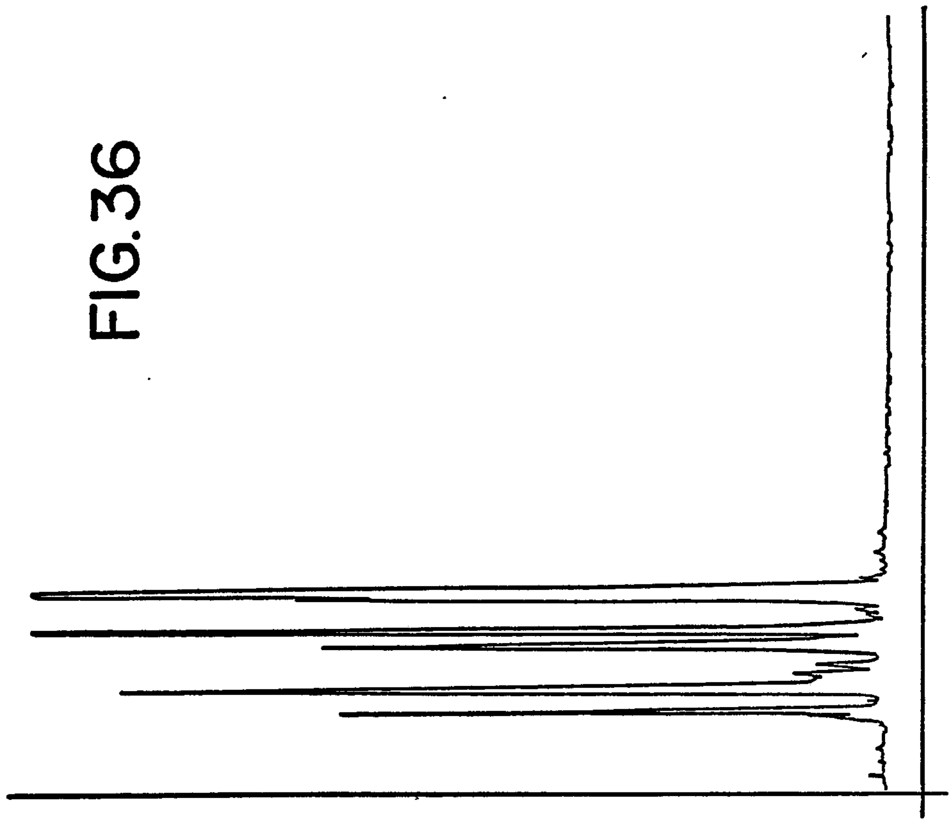

GLC PROFILE FOR FRACTION 4 OF
EXAMPLE XXXVII, CRUDE

FIG.35

NMR SPECTRUM FOR FRACTION 4 OF EXAMPLE XXXXVI

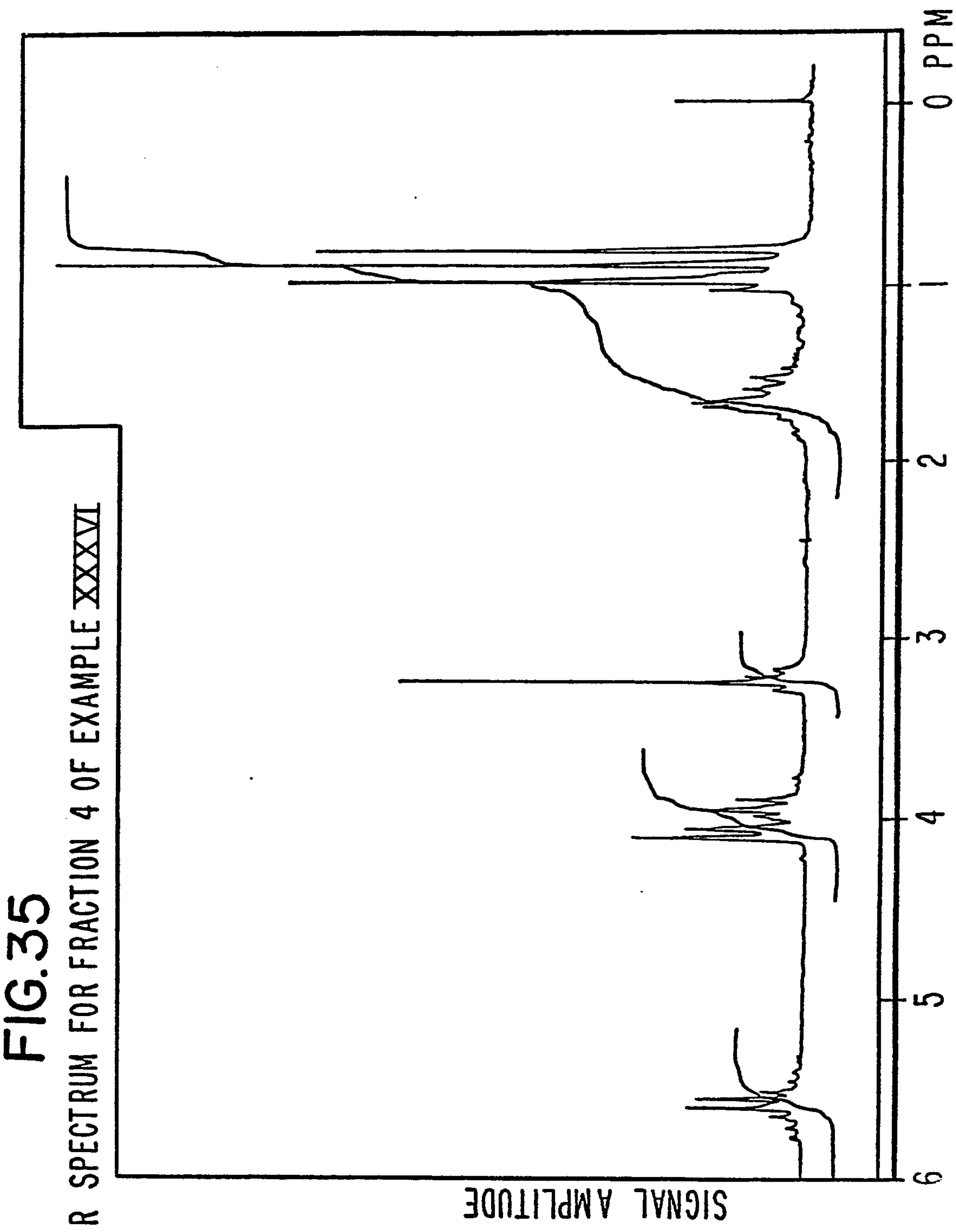

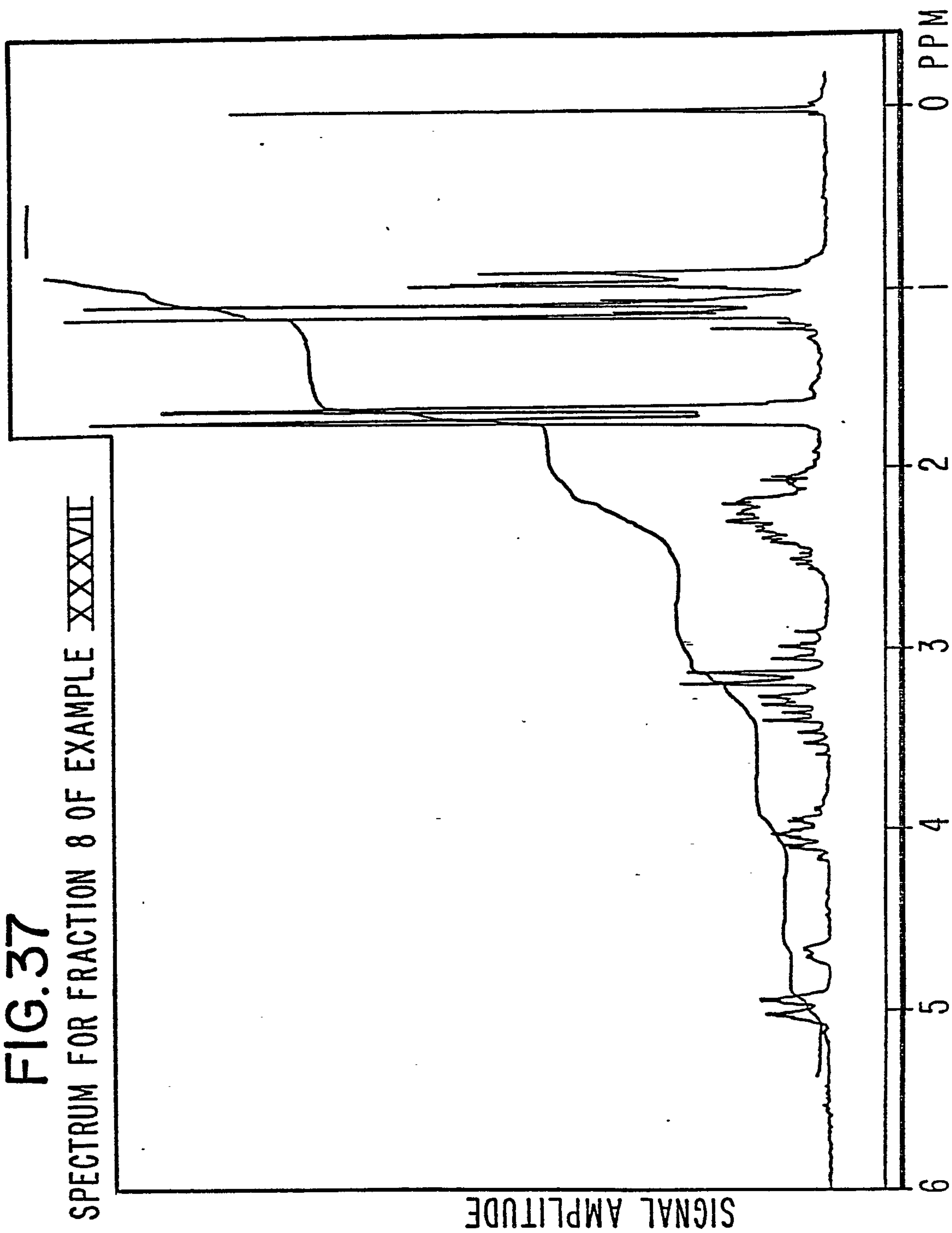


FIG.37

NMR SPECTRUM FOR FRACTION 8 OF EXAMPLE XXXVII

FIG. 38

GLC PROFILE FOR EXAMPLE XXXVIII
CRUDE

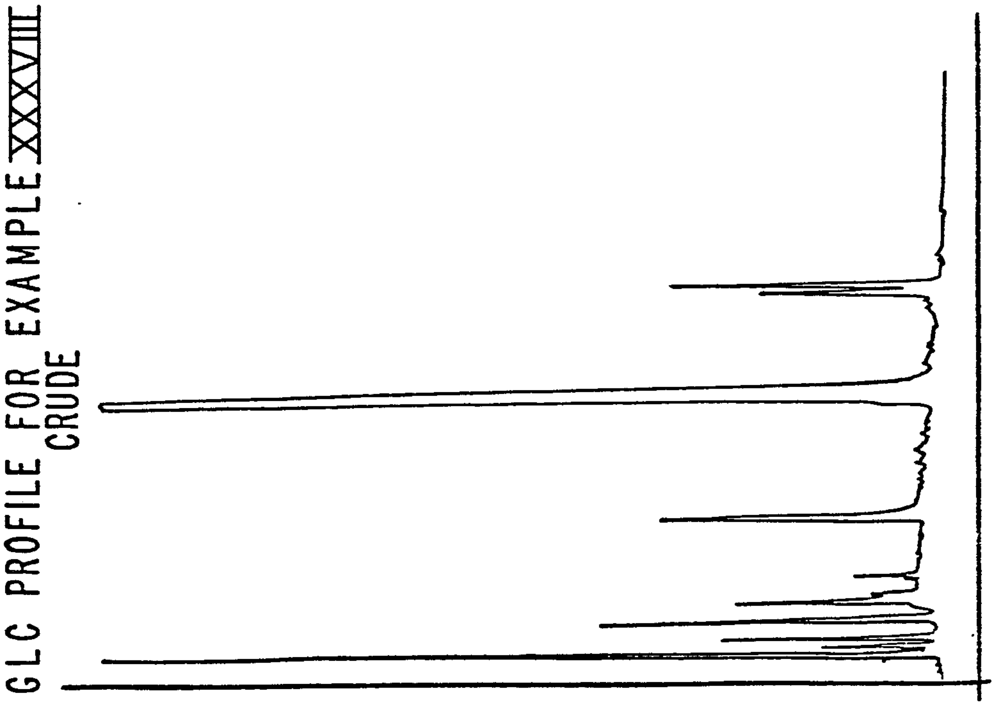

FIG. 40 A

GLC PROFILE FOR EXAMPLE XXXIX
CRUDE

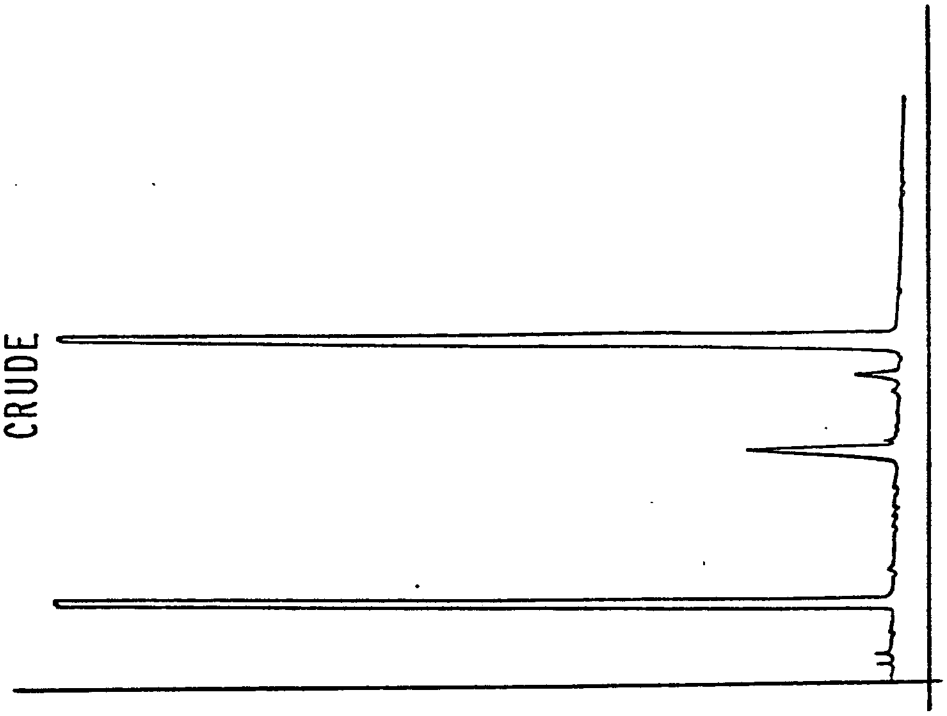

FIG.39

NMR SPECTRUM FOR FRACTION 4 OF EXAMPLE XXXVIII

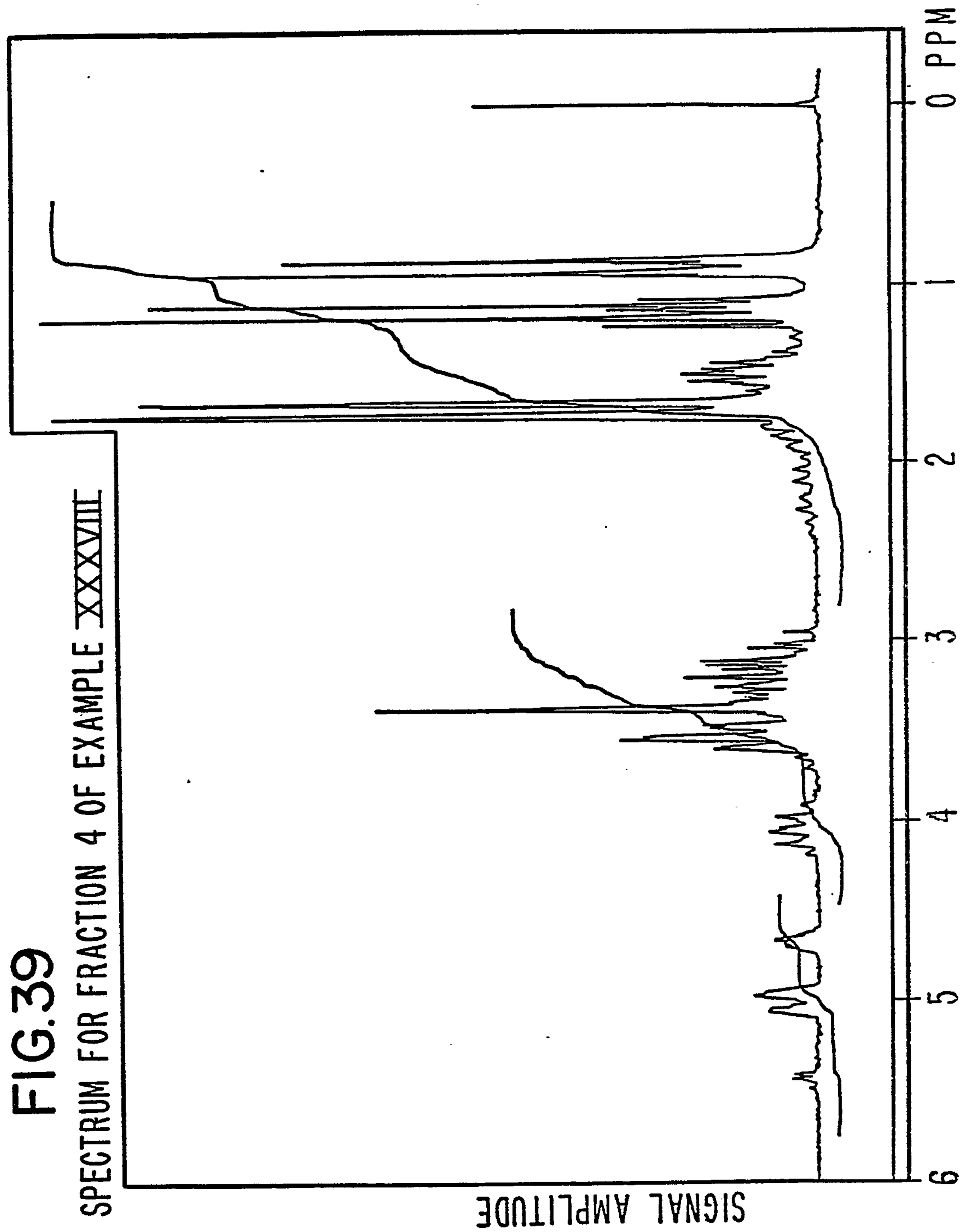

FIG. 40B

NMR SPECTRUM FOR EXAMPLE XXXIX

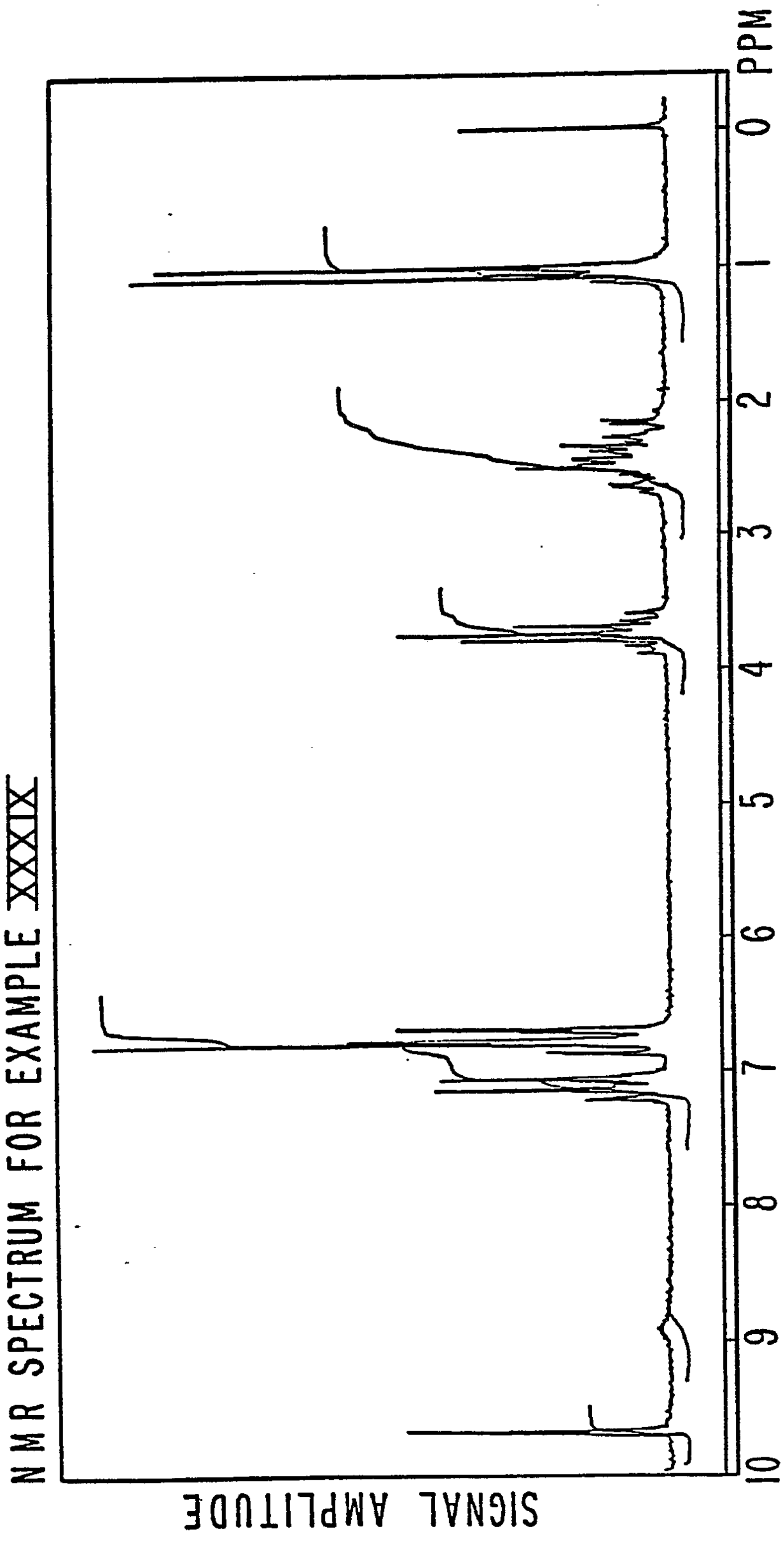

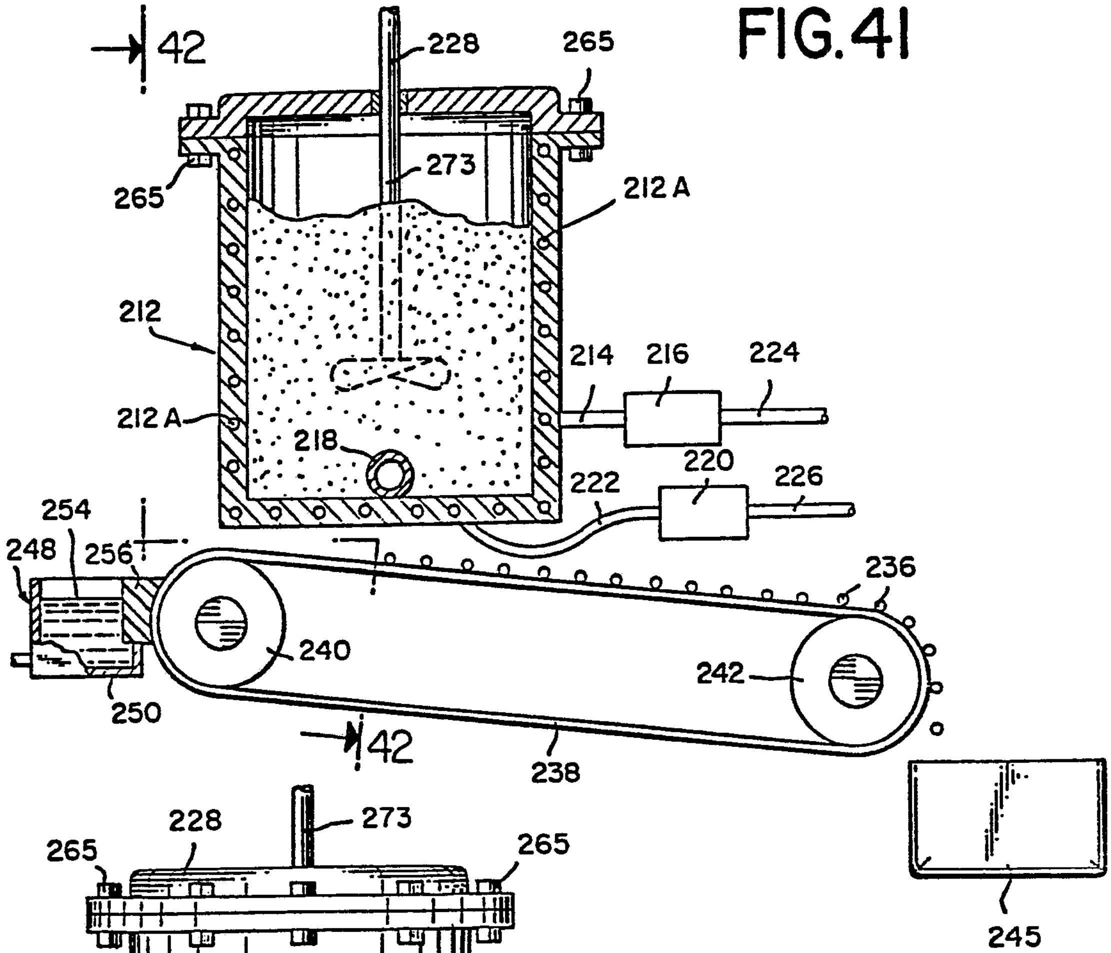
42
228
265
273
265
212 A
212
214
216
224
212 A
218
220
222
226
254
248
256
236
240
242
250
42
238
245

FIG. 41

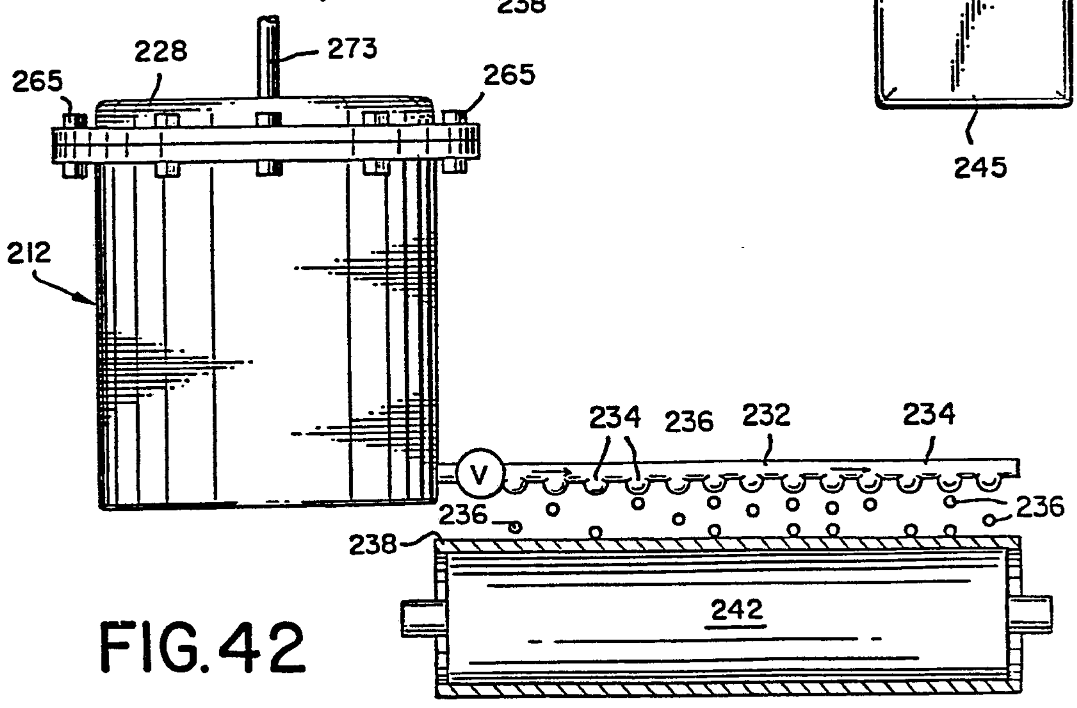
228
273
265
265
212
234
236
232
234
V
236
236
238
242

FIG. 42